# EUROPEAN PATENT APPLICATION

(11) **EP 4 516 899 A2**
(43) Date of publication of application: **05.03.2025**
(21) Application number: 24220619.1
(22) Date of filing: 20.01.2023
(51) Int. Cl.: C12N 9/22

(54) **NUCLEASE HAVING IMPROVED SALT TOLERANCE AND/OR TEMPERATURE PERFORMANCE**

(30) Priority: 20.01.2022 EP 22152536
(62) Divisional of application: 23701877.5
(71) Applicant: c-LEcta GmbH, 04103 Leipzig (DE)
(72) Inventor: FELLER, Claudia, 04155 Leipzig (DE); VOGEL, Andreas, 04105 Leipzig (DE); BARTSCH, Sebastian, 04317 Leipzig (DE)
(74) Representative: Kutzenberger Wolff & Partner

(57) **Abstract**

The invention relates to nucleases with improved properties such as enzymatic activity at high temperature, at low temperature, and/or high salt concentration. The invention also relates to methods for hydrolyzing polynucleotide substrates using the nucleases at high temperatures, at low temperature, or at high salt concentration. The invention further relates to uses of the nucleases for hydrolyzing polynucleotides in the manufacture of biopharmaceuticals, pharmaceutical compositions, vaccines, or viral vectors. Furthermore, the invention relates to kits comprising the nucleases.

## Description

### Field of the invention

The invention relates to nucleases with improved properties such as enzymatic activity at high temperature, at low temperature, and/or high salt concentration. The invention also relates to methods for hydrolyzing polynucleotide substrates using the nucleases at high temperatures, at low temperature, or at high salt concentration. The invention further relates to uses of the nucleases for hydrolyzing polynucleotides in the manufacture of biopharmaceuticals, pharmaceutical compositions, vaccines, or viral vectors. Furthermore, the invention relates to kits comprising the nucleases.

### Background art

Nucleases are hydrolytic enzymes that cleave nucleic acids (DNA or RNA) and are of widespread economic importance. Exonucleases are distinguished from endonucleases. Exonucleases cleave mononucleotides from the 5'- or the 3'terminus of the nucleic acid, while cleavage by endonucleases occurs within the nucleic acid and oligonucleotides are produced, respectively. Furthermore, specific nucleases (such as restriction enzymes) are distinguished from non-specific nucleases (such as RNase A). Specific nucleases specifically recognize and cleave nucleic acids at defined sequences or structures, while non-specific nucleases cleave nucleic acids randomly. Restriction enzymes have become indispensable tools in molecular biology and serve to specifically split different DNA molecules into fragments, which again may be selectively joined with other fragments using ligases to form new DNA molecule constructs. Non-specific nucleases are mainly used for the decomposition of nucleic acids in various processes. When a nuclease cleaves only DNA, it is referred to as "DNase", and when a nuclease cleaves only RNA, it is referred to as "RNase".

The removal of DNA and RNA from a sample by non-specific hydrolysis under catalysis of non-specific nucleases, in particular of endonucleases, is an important processing step in the manufacturing of several biotechnology products of economic interest, e.g. antibodies or enzymes, polysaccharides, lipids, low-molecular substances such as antibiotics, metabolic end products, intermediate products or chemicals, and the like. The necessity to remove the nucleic acids becomes particularly significant if production of the biotechnology products occurs intracellularly in production cells and nucleic acids are released during the production process, or if a proportion of the production cells is lysed during production. In consequence, considerable amounts of nucleic acids are released into the preparations (fermentation broth) which may contaminate the desired biotechnology product or make further purification thereof more difficult. A similar problem arises, for example, in the production of proteins (biotechnology product) using cell-free in-vitro translation. The nucleic acids increase the viscosity of the preparations to such an extent that subsequent steps for separation or purification of the biotechnology product such as filtration or chromatography operations are not possible or made more difficult. The use of endonucleases allows to break down the nucleic acids to small fragments thereby reducing the viscosity of the preparations, and the resulting cleaved nucleic acid and other unwanted components of the preparations can be separated from the biotechnology product using simple methods such as ultrafiltration.

The endonuclease from the species *Serratia marcescens* (SEQ ID NO: 1), also called *"Serratia marcescens nuclease"* or *"Serratia marcescens endonuclease",* classifies as EC 3.1.30.2 according to the Enzyme Class number of the International Union of Biochemistry and Molecular Biology. This endonuclease catalyzes the non-specific hydrolysis of phosphodiester bonds of both DNA and RNA, double- or single-stranded to produce smaller 5'-phosphorylated oligonucleotides. *Serratia marcescens* endonuclease is commercially available under the brand names Benzonase^{®} or DENARASE^{®}. This endonuclease is "engineered" for ease of manufacture, but its amino acid sequences is still identical to the wild-type *Serratia marcescens* endonuclease. This endonuclease exhibits a high activity and stability with a temperature optimum at 37°C, a pH optimum at pH 8.0-9.0, a requirement for Mg²⁺ ions at an optimal concentration of 1-2 mM, and optimal performance at monovalent cation concentrations (Na+, K+, etc.) of 0-20 mM. By nature, this endonuclease exhibits a lower performance or even no performance at reaction conditions outside of these optimum ranges.

Accordingly, the industrial use of the enzyme is limited under reaction conditions that are outside of optimum ranges, but which nevertheless are relevant for industrial manufacturing processes, especially high temperature, low temperature, or high salt concentration. For example, enzymatic cleavage of nucleic acids in certain industrial manufacturing processes of biotechnology products requires hydrolytic activity of nucleases under reaction conditions other than the optimum ranges of the wild-type enzyme. Such conditions can be comparatively harsh, e.g. in the presence of salts like NaCl or KCl at salt concentrations of as low as 0.05 M or as high as 1.00 M, or at temperatures as low as 4°C or as high as 60°C.

As the conventional nucleases that are known from the prior art do not satisfy these reaction requirements that are relevant for various industrial applications, there is a need for nucleases which are advantageous over the known nucleases. In particular, there is a demand for nucleases with an increased tolerance to high temperature, low temperature, or high salt concentration.

### Objects of the invention

It is an object of the invention to provide improved nucleases; preferably endonucleases; more preferably non-specific endonucleases, that have advantages compared to the nucleases of the prior art, particularly compared to *Serratia marcescens* nuclease (SEQ ID NO: 1). The improved nucleases should exhibit satisfactory enzymatic performance under conditions of high temperature, low temperature, or high salt concentration. The nucleases should be suitable for use in industrial hydrolysis of polynucleotides (RNA and/or DNA) to mononucleotides (e.g. due to exonuclease activity) or to oligonucleotides (e.g. due to non-specific endonuclease activity).

This object has been achieved by the subject-matter of the patent claims.

### Summary of the invention

In summary, the invention relates to improved modified nucleases, especially endonuclease variants, that are characterized by a broader industrial applicability spectrum. In particular, the invention relates to variants of the endonuclease of the organism *Serratia marcescens* (SEQ IDO NO:1) with substitutions of amino acids at sequence positions within defined subsequences of the amino acid sequence of the wild-type enzyme.

It has been surprisingly found that improved modified nucleases, especially endonuclease variants, can be provided that are characterized by a high activity at concentration of salts within the range of from 0.05M and 1.00M.

Further it has been surprisingly found that improved modified nucleases, especially endonuclease variants, can be provided that are characterized by a high activity at a temperature within the range of from 40°C to 60°C. Still further, it has been surprisingly found that improved modified nucleases, especially endonuclease variants, can be provided that at a temperature within the range of from 4°C to 25°C are characterized by a high activity.

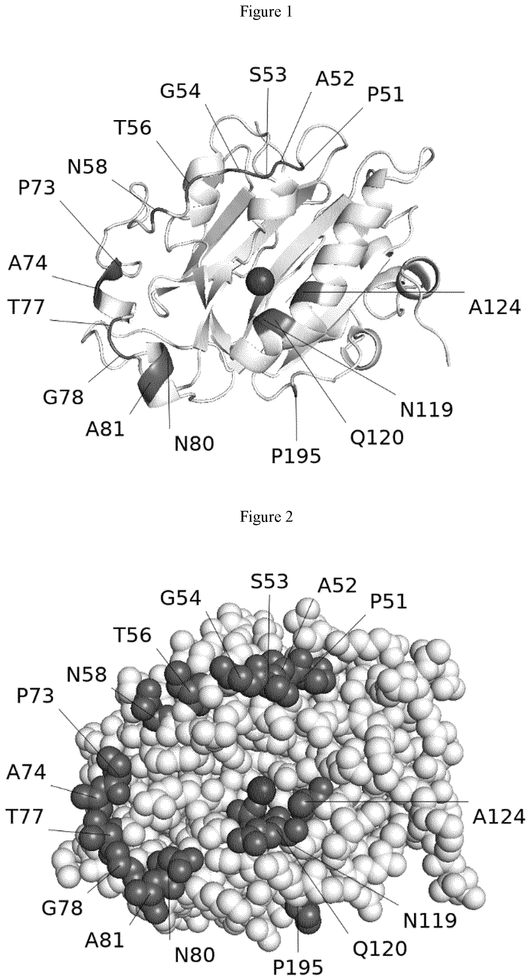

Figures 1 to 4 show a three-dimensional representation of the final model calculated by methods shown in example 8.

A first aspect of the invention relates to a nuclease comprising an amino acid sequence with at least 65% identity to SEQ ID NO:1, wherein the amino acid sequence is substituted compared to SEQ ID NO:1 in at least one amino acid position selected from the group consisting of P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239.

A particularly preferred embodiment of the invention relates to a nuclease; preferably an endonuclease; more preferably a non-specific endonuclease, comprising an amino acid sequence with at least 75%; identity to SEQ ID NO:1; and comprising
(i) a substitution in position P51, wherein P has been substituted by an amino acid selected from the group consisting of L, G, A, V, and I;
(ii) a substitution in position T77, wherein T has been substituted by an amino acid selected from the group consisting of R, H, and K; and
(iii) at least one substitution in a position selected from the group consisting of
   (a) A124, A52, G54, P73, A74, G78, A81, and P195; or
   (b) T56, S53, N58, N80, N119, and Q120;
wherein in each case (a) or (b) the amino acid of SEQ ID NO: 1 has been substituted by an amino acid selected from the group consisting of R, H, and K.

Another particularly preferred embodiment of the invention relates to a nuclease; preferably an endonuclease; more preferably a non-specific endonuclease, comprising an amino acid sequence with at least 65% identity to SEQ ID NO: 1, wherein the amino acid sequence is substituted compared to SEQ ID NO:1 in at least one amino acid position selected from the group consisting of E239, T56, K84, D191, T77, and N58, wherein in each case the native amino acid of the selected position is substituted by an amino acid selected from the group consisting of A, G, V, L, I, and P; preferably selected from the group consisting of A, G, V, L, and P; more preferably selected from the group consisting of A, G, L, and P; still more preferably selected from the group consisting of A, G, and P; yet more preferably selected from the group consisting of A, and G; and most preferably A.

Another further particularly preferred embodiment of the invention relates to nuclease; preferably an endonuclease, with at least 70% identity to SEQ ID NO: 1, wherein the amino acid sequence is substituted compared to SEQ ID NO: 1 in at least two amino acid position selected from the group consisting of P51, T56, K84, and N58, wherein in each case the native amino acid of the selected position is substituted by an amino acid selected from the group consisting of L, P, A, G, V, and I; preferably selected from the group consisting of L, P, I, G, and V; more preferably selected from the group consisting of L, P, I, and G; still more preferably selected from the group consisting of L, P, and G; yet more preferably selected from the group consisting of L, and P.

### Definitions

For the purpose of the specification, nucleases (also known as nucleodepolymerases or polynucleotidases) are hydrolytic enzymes capable of cleaving the phosphodiester bonds between nucleotides of nucleic acids (DNA or RNA). Exonucleases digest nucleic acids from the from their 5'- or the 3'-terminus forming mononucleotides, and endonucleases act on regions in the middle of nucleic acids forming oligonucleotides. Furthermore, specific nucleases are distinguished from non-specific nucleases: specific nucleases recognize and cleave nucleic acids at defined sequences or structures, and non-specific nucleases cleave nucleic acids randomly. Certain nucleases are capable of cleaving cleave only DNA, other nucleases are capable of cleaving cleave only RNA, and other nucleases are capable of cleaving DNA and RNA. The nuclease according to the invention is preferably an endonuclease; more preferably a non-specific endonuclease. In preferred embodiments, the nucleases are deoxyribonucleases; preferably deoxyriboendonucleases. In other preferred embodiments, the nucleases are ribonucleases; preferably riboendonucleases.

It is within the meaning of the invention, that whenever an embodiment of any of the aspects of the invention refer to a nuclease, any such embodiment is meant to also refer to an endonuclease providing the same embodiments and/or to a polypeptide providing the same embodiments. In this respect the invention also relates to an endonuclease, and/or to a polypeptide comprising an amino acid sequence with at least 65% identity to SEQ ID NO:1, wherein the amino acid sequence is substituted compared to SEQ ID NO:1 in at least one amino acid position selected from the group consisting of P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239. Accordingly, all embodiment of any aspect referring to the nuclease of the invention shall also refer to an endonuclease and/or a polypeptide correspondingly.

The nuclease according to the invention comprises an amino acid sequence with a defined identity to the amino acid sequence of SEQ ID NO:1. This means that any nuclease according to the invention may comprise said amino acid sequence as a subsequence of its overall amino acid sequence, or may essentially consist of said amino acid sequence. When the nuclease according to the invention comprises said amino acid sequence as a subsequence of its overall amino acid sequence, said overall amino acid sequence may be extended, i.e. may comprise additional amino acid residues, at the N-terminus and/or at the C-terminus of said subsequence. Such extension may be advantageous, for example, when the nuclease is to be immobilized on a solid support, e.g. for purification purposes.

For the purpose of the specification, the percent identity is calculated as: Sequence Identity [%] = number of Matches/ L x 100, wherein L is the number of aligned positions, i.e. identities and noni-dentities (including gaps, if any). Identity is preferably calculated using BLASTP (see, for example, Altschul SF et al. (1997) "Gapped BLAST and PSI-BLAST: a new generation of protein database search programs", Nucleic Acids Res. 25:3389-3402; or Altschul SF (2005) "Protein database searches using compositionally adjusted substitution matrices." FEBS J. 272:5101-5109); preferably with the following algorithm parameters: Matrix: BLOSUM62; Gap Costs: Existence: 11 Extension: 1, Expect threshold: 0.05, and Word size: 6. BlastP can be accessed online at the NCBI Homepage (https:// blast.ncbi.nlm. nih.gov/Blast.cgi?PROGRAM=blastp&PAGE_TYPE=BlastSearch&LINK_LOC=blasthome). Other program settings can be adjusted as desired, for example using the following settings:
- Field "Enter Query Sequence": Query subrange: none;
- Field "Choose Search Set": Database: non-redundant protein sequences (nr); optional parameters: none
- Field "Program Selection": Algorithm: blastp (protein-protein BLAST);
- Algorithm parameters: Field "General parameters": Max target sequences: 5000; Short queries: Automatically adjust parameters for short input sequences; Expect threshold: 0.05; Word size: 6; Max matches in a query range: 0;
- Algorithm parameters: Field "Scoring parameters": Matrix: BLOSUM62; Gap Costs: Existence: 11 Extension: 1; Compositional adjustments: Conditional compositional score matrix adjustment;
- Algorithm parameters: Field "Filters and Masking": Filter: none; Mask: none.

In addition to the default parameters for calculation of percent identity, when aligning a reference sequence, referred to as "query sequence", with another sequence, referred to as "subject sequence", in particular wherein the query sequence is any of the sequences provided by the present invention; preferably SEQ ID NO:1, and wherein the subject sequence is any other sequence, for example a sequence disclosed in a database, the subject sequence must align over a sequence stretch covering at least 70% of the overall length of the query sequence ("sequence coverage" of at least 70%); the alignment of both sequences must cover at least 70% of the query sequence; for the purpose of specification, the sequence coverage is calculated as: the number of aligned positions, i.e. covering identities and non-identities (including gaps, if any) divided by the overall length of the query sequence and multiplied by 100. Alignments with a lower sequence coverage of the query sequence by the subject sequence are excluded for the determination of sequence identity for the purposes of this application. However, the subject sequence may be longer than the length of the alignment.

During expression of naturally or recombinantly expressed nucleases in certain host organisms, nuclease genes may be linked with a so-called signal peptide ("SP") gene sequence. The SP gene sequence is co-expressed together with the nuclease to form a single precursor protein comprising the SP and the nuclease protein part. The SP element of the SP-nuclease fusion protein usually serves as a secretion signal and is cleaved from the nuclease during the secretion process. For the purpose of specification, gene sequences or peptide sequences of a signal peptide of nucleases are disregarded in calculation of the identity of a nuclease according to the invention. Tools for identifying SP gene or protein sequences are known to the person skilled in the art and are described for example under https://www.uniprot.org/help/signal, Section 2, in which predictive tools Phobius, Predotar, SignalP and TargetP are given as examples.

For the purpose of the specification, a certain target amino acid sequence being substituted at a certain "amino acid position number compared to SEQ ID NO:1" means that such target amino acid sequence is substituted at such target amino acid position number of the target amino acid sequence that aligns with the "amino acid position number of SEQ ID NO:1" in an alignment which is prepared in accordance with the requirements of sequence identity hereof, irrespective of whether the amino acid position number of SEQ ID NO:1 and the target amino acid position number are identical or different.

For the purpose of the specification, a certain target amino acid sequence being substituted at a certain "amino acid position number compared to SEQ ID NO: 1" furthermore means that in any certain amino acid position number of SEQ ID NO: 1 the amino acid of SEQ ID NO: 1 in such position is substituted by any differing amino acid selected from the group of natural proteinogenic amino acids, i.e. A, R, N, D, C, Q, E, G, H, I, L, K, M, F, T, W, Y, V, P or S.

For the purpose of specification, the at least amino acid position of the nuclease being substituted means that the at least one amino acid position is substituted by (i) either a positively charged amino acid selected from R, K, and H, or (ii) a negatively charged amino acid selected from E and D, or (iii) an aliphatic nonpolar amino acid selected from G, A, V, L, I, and P, or (iv) an aromatic amino acid selected from W, F, and Y, or (v) an aliphatic polar non-charged amino acid selected from N, Q, S, T, C, and M.

For the purpose of specification, the at least amino acid position of the nuclease being substituted may be (i) a positively charged amino acid which is substituted by another positively charged amino acid, or (ii) a negatively charged amino acid which is substituted by another negatively charged amino acid, or (iii) an aliphatic nonpolar amino acid which is substituted by another aliphatic nonpolar amino acid, or (iv) which is an aromatic amino acid which is substituted by another aromatic amino acid, or (v) an aliphatic polar non-charged amino acid which is substituted by another aliphatic polar non-charged amino acid.

For the purpose of specification, the at least amino acid position of the nuclease being substituted may be (i) a positively charged amino acid and is substituted by an aliphatic nonpolar amino acid, or an aromatic amino acid, or an aliphatic polar non-charged, or a negatively charged amino acid; or (ii) a negatively charged amino acid and is substituted by an aliphatic nonpolar amino acid, or an aromatic amino acid, or an aliphatic polar non-charged, or a positively charged amino acid; or (iii) an aliphatic nonpolar amino acid and is substituted by an aromatic amino acid, or an aliphatic polar non-charged, or a positively charged amino acid, or a negatively charged amino acid; or (iv) an aromatic amino acid and is substituted by an aliphatic nonpolar amino acid, or an aliphatic polar non-charged, or a positively charged amino acid, or a negatively charged amino acid; or (v) an aliphatic polar non-charged amino acid and is substituted by an aliphatic nonpolar amino acid, or an aromatic amino acid, or a positively charged amino acid, or a negatively charged amino acid.

For the purpose of the specification, the status of charge of any amino acids being substituted in any position compared to SEQ ID NO: 1, in particular the charge of the positively charged amino acids R, K, and H and the charge of the negatively charged amino acids E and D is determined at a pH of 7.

For the purpose of the specification, specific substitution positions disclosed herein are described in accordance with common practice in the field of amino acid substitutions, wherein the format [non-substituted amino acid - sequence position] is chosen and the sequence position is directly flanked with the amino acids letter (one letter code) of the not yet mutated amino acid on the left side, wherein no specific amino acid is selected as a substituent for such amino acid position. For clarification only, substitution "P51" describes the substitution of the amino acid proline (P) in sequence position 51 by any substituent.

For the purpose of the specification, specific substitutions disclosed herein are described in accordance with common practice in the field of amino acid substitutions, wherein the format [non-substituted amino acid - sequence position - substituted amino acid] is chosen and the sequence position is directly flanked with the amino acids letter (one letter code) on the left or right side. For clarification only, substitution "P51R" describes the substitution of the amino acid proline (P) in sequence position 51 by the amino acid arginine (R), i.e. P is replaced by R.

### Structural characteristics of the nucleases according to the invention

In preferred embodiments of the nuclease according to the invention, the nuclease is present in a liquid formulation.

In preferred embodiments of the nuclease according to the invention, the nuclease is present in a liquid formulation containing (i) buffer, (ii) glycerol, sucrose, or other stabilizing agents and/or (iii) benzoate or other preservative agents.

In preferred embodiments of the nuclease according to the invention, the nuclease is a solid or present in a solid formulation; preferably a powder.

In preferred embodiments of the nuclease according to the invention, the nuclease is present as a powder or contained in such a powder obtained by lyophilization, spray drying or other techniques for drying known in the state of the art.

In preferred embodiments of the nuclease according to the invention, the nuclease is contained in a crude cell lysate, or a crude cell supernatant, or a partially purified enzyme from any of the foregoing, or a purified enzyme from any of the foregoing.

In preferred embodiments of the nuclease according to the invention, the nuclease is present immobilized on a carrier.

In preferred embodiments of the nuclease according to the invention, the amino acid sequence of the nuclease has an identity to SEQ ID NO: 1 of at least 66%; preferably at least 69%; more preferably at least 72%; still more preferably at least 75%; yet more preferably at least 78%; even more preferably at least 80%; even more preferably at least 81%; most preferably at least 84%, and in particular at least 87%. In preferred embodiments of the nuclease according to the invention, the amino acid sequence has an identity to SEQ ID NO: 1 of at least 88%; preferably at least 89%; more preferably at least 90%; still more preferably at least 91%; yet more preferably at least 92%; even more preferably at least 93%; most preferably at least 94%, and in particular at least 95%.

In preferred embodiments of the nuclease according to the invention, the identity of the amino acid sequence of the nuclease with the sequence of SEQ ID NO: 1 is at least 65%, or at least 66%, or at least 67%, or at least 68%, or at least 69%, or at least 70%, or at least 71%, or at least 72%, or at least 73%, or at least 74%, or at least 75%, or at least 76%, or at least 77%, or at least 78%, or at least 79%, or at least 80%, or at least 81%, or at least 82%, or at least 83%, or at least 84%, or at least 85%; preferably at least 86%, or at least 87%, or at least 88%, or at least 89%, or at least 90%; more preferably at least 91%, or at least 92%, or at least 93%, or at least 94%, or at least 95%; still more preferably at least 96%, or at least 97%, or at least 98%, or at least 99%; yet more preferably at least 99.1%, or at least 99.2%, or at least 99.3%, or at least 99.4, or at least 99.5%; even more preferably at least 99.6%.

In preferred embodiments of the nuclease according to the invention, the identity of the amino acid sequence of the nuclease with the sequence of SEQ ID NO:1 is at least 89%, or at least 90%, or at least 91%, or at least 92%, or at least 93%, or at least 94%, or at least 95%; preferably at least 96%, or at least 97%, or at least 98%, or at least 99%; more preferably at least 99.1%, or at least 99.2%, or at least 99.3%, or at least 99.4, or at least 99.5%; still more preferably at least 99.6%.

In preferred embodiments of the nuclease according to the invention, the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, or 195.

In preferred embodiments of the nuclease according to the invention, the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, or 147.

In preferred embodiments of the nuclease according to the invention, the amino acid sequence is substituted compared to SEQ ID NO:1 in position P51, wherein the substitution is selected from the group consisting of P51A, P51R, P51N, P51D, P51C, P51Q, P51E, P51G, P51H, P51I, P51L, P51K, P51M, P51F, P51T, P51W, P51Y, P51V, and P51S; preferably P51D, P51E, P51R, P51K, P51H, P51G, P51A, P51V, P51L, and P51I; more preferably P51L, P51R, P51G, P51D, P51A, P51V, P51I; even more preferably P51D, P51R, P51G, and P51L; and most preferably P51R, P51G, and P51L.

In preferred embodiments of the nuclease according to the invention, the amino acid sequence is substituted compared to SEQ ID NO:1 in position A52, wherein the substitution is selected from the group consisting of A52R, A52N, A52D, A52C, A52Q, A52E, A52G, A52H, A52I, A52L, A52K, A52M, A52F, A52P, A52T, A52W, A52Y, A52V, and A52S; preferably A52R, A52K, A52H; more preferably A52R.

In preferred embodiments of the nuclease according to the invention, the amino acid sequence is substituted compared to SEQ ID NO:1 in position S53, wherein the substitution is selected from the group consisting of S53A, S53R, S53N, S53D, S53C, S53Q, S53E, S53G, S53H, S53I, S53L, S53K, S53M, S53F, S53P, S53T, S53W, S53Y, and S53V; preferably S53R, S53K, S53H, S53G, S53A, S53V, S53L, S53I, S53P, S53W, S53F, S53Y, and S53D; more preferably S53R, S53K, S53H, S53G, S53A, S53V, S53L, S53I and S53P; even more preferably S53K, and S53A; most preferably S53K.

In preferred embodiments of the nuclease according to the invention, the amino acid sequence is substituted compared to SEQ ID NO: 1 in position G54, wherein the substitution is selected from the group consisting of G54A, G54R, G54N, G54D, G54C, G54Q, G54E, G54H, G54I, G54L, G54K, G54M, G54F, G54P, G54T, G54W, G54Y, G54V, and G54S; preferably G54D, G54E, G54R, G54K, G54H, G54A, G54V, G54L, G54I, and G54P; preferably G54K, G54H, and G54R; more preferably G54R.

In preferred embodiments of the nuclease according to the invention, the amino acid sequence is substituted compared to SEQ ID NO: 1 in position K55, wherein the substitution is selected from the group consisting of K55A, K55R, K55N, K55D, K55C, K55Q, K55E, K55G, K55H, K55I, K55L, K55M, K55F, K55P, K55T, K55W, K55Y, K55V, and K55S; preferably K55G, K55A, K55V, K55L, K55I, K55P, K55R, K55H, K55N, K55Q, K55S, K55T, K55C, and K55M; more preferably K55H and K55R; most preferably K55R.

In preferred embodiments of the nuclease according to the invention, the amino acid sequence is substituted compared to SEQ ID NO: 1 in position T56, wherein the substitution is selected from the group consisting of T56A, T56R, T56N, T56D, T56C, T56Q, T56E, T56G, T56H, T56I, T56L, T56K, T56M, T56F, T56P, T56W, T56Y, T56V, and T56S; preferably T56P, T56D, T56R, T56K, T56S, T56G, T56A, T56V, T56I, T56F, T56L, and T56M; and even more preferably T56P, T56D, T56R, T56K, T56S, and T56G; most preferably T56R, T56K, T56S, and T56G.

In preferred embodiments of the nuclease according to the invention, the amino acid sequence is substituted compared to SEQ ID NO: 1 in position N58, wherein the substitution is selected from the group consisting of N58A, N58R, N58D, N58C, N58Q, N58E, N58G, N58H, N58I, N58L, N58K, N58M, N58F, N58P, N58T, N58W, N58Y, N58V, and N58S; preferably N58R, N58K, N58H, N58G, N58A, N58V, N58L, N58I, N58P, N58W, N58F, N58Y, and N58D; more preferably N58R, N58K, N58H, N58G, N58A, N58V, N58L, N58I, and N58P; more preferably N58K, N58R, and N58V; most preferably N58K, and N58R.

In preferred embodiments of the nuclease according to the invention, the amino acid sequence is substituted compared to SEQ ID NO: 1 in position A72, wherein the substitution is selected from the group consisting of A72R, A72N, A72D, A72C, A72Q, A72E, A72G, A72H, A72I, A72L, A72K, A72M, A72F, A72P, A72T, A72W, A72Y, A72V, and A72S; preferably A72D, A72E, A72R, A72K, A72H, A72G, A72V, A72L, A72I, and A72P; more preferably A72K, A72H and A72R; most preferably A72K.

In preferred embodiments of the nuclease according to the invention, the amino acid sequence is substituted compared to SEQ ID NO:1 in position P73, wherein the substitution is selected from the group consisting of P73A, P73R, P73N, P73D, P73C, P73Q, P73E, P73G, P73H, P73I, P73L, P73K, P73M, P73F, P73T, P73W, P73Y, P73V, and P73S; preferably P73D, P73E, P73R, P73K, P73H, P73G, P73V, P73L, P73I, and P73A; more preferably P73K, P73H and P73R; most preferably P73K, and P73R.

In preferred embodiments of the nuclease according to the invention, the amino acid sequence is substituted compared to SEQ ID NO: 1 in position A74, wherein the substitution is selected from the group consisting of A74R, A74N, A74D, A74C, A74Q, A74E, A74G, A74H, A74I, A74L, A74K, A74M, A74F, A74P, A74T, A74W, A74Y, A74V, and A74S; preferably A74D, A74E, A74R, A74K, A74H, A74G, A74V, A74L, A74I, and A74P; more preferably A74K, A74H, and A74R; most preferably A74K, and A74R.

In preferred embodiments of the nuclease according to the invention, the amino acid sequence is substituted compared to SEQ ID NO: 1 in position T77, wherein the substitution is selected from the group consisting of T77A, T77R, T77N, T77D, T77C, T77Q, T77E, T77G, T77H, T77I, T77L, T77K, T77M, T77F, T77P, T77W, T77Y, T77V, and T77S; preferably T77R, T77K, T77H, T77G, T77A, T77V, T77L, T77I, T77P, T77W, T77F, T77Y, and T77D; more preferably T77K, T77R, T77H, T77F, and T77L, and most preferably T77K, T77R, T77F, and T77L.

In preferred embodiments of the nuclease according to the invention, the amino acid sequence is substituted compared to SEQ ID NO: 1 in position G78, wherein the substitution is selected from the group consisting of G78A, G78R, G78N, G78D, G78C, G78Q, G78E, G78H, G78I, G78L, G78K, G78M, G78F, G78P, G78T, G78W, G78Y, G78V, and G78S; preferably G78R, G78K, and G78H; more preferably G78R.

In preferred embodiments of the nuclease according to the invention, the amino acid sequence is substituted compared to SEQ ID NO: 1 in position N80, wherein the substitution is selected from the group consisting of N80A, N80R, N80D, N80C, N80Q, N80E, N80G, N80H, N80I, N80L, N80K, N80M, N80F, N80P, N80T, N80W, N80Y, N80V, and N80S; preferably N80R, N80K, N80H, N80G, N80A, N80V, N80L, N80I, N80P, N80W, N80F, N80Y, and N80D; preferably N80K, N80R, and N80H; more preferably N80H and N80R; most preferably N80H.

In preferred embodiments of the nuclease according to the invention, the amino acid sequence is substituted compared to SEQ ID NO: 1 in position A81, wherein the substitution is selected from the group consisting of A81R, A81N, A81D, A81C, A81Q, A81E, A81G, A81H, A81I, A81L, A81K, A81M, A81F, A81P, A81T, A81W, A81Y, A81V, and A81S; preferably A81K, A81H, and A81R; more preferably A81K.

In preferred embodiments of the nuclease according to the invention, the amino acid sequence is substituted compared to SEQ ID NO: 1 in position A82, wherein the substitution is selected from the group consisting of A82R, A82N, A82D, A82C, A82Q, A82E, A82G, A82H, A82I, A82L, A82K, A82M, A82F, A82P, A82T, A82W, A82Y, A82V, and A82S; preferably A82K, A82H, and A82R; more preferably A82R.

In preferred embodiments of the nuclease according to the invention, the amino acid sequence is substituted compared to SEQ ID NO: 1 in position K84, wherein the substitution is selected from the group consisting of K84A, K84R, K84N, K84D, K84C, K84Q, K84E, K84G, K84H, K84I, K84L, K84M, K84F, K84P, K84T, K84W, K84Y, K84V, and K84S; preferably K84G, K84A, K84V, K84L, K84I, K84P, K84R, K84H, K84N, K84Q, K84S, K84T, K84C, and K84M; preferably K84G, K84A, K84V, K84L, K84I, and K84P; more preferably K84G.

In preferred embodiments of the nuclease according to the invention, the amino acid sequence is substituted compared to SEQ ID NO:1 in position D86, wherein the substitution is selected from the group consisting of D86A, D86R, D86N, D86C, D86Q, D86E, D86G, D86H, D86I, D86L, D86K, D86M, D86F, D86P, D86T, D86W, D86Y, D86V, and D86S; D86G, D86A, D86V, D86L, D86I, D86P, D86C, D86M, D86S, D86T, and D86Q; preferably D86C, D86M, D86S, D86T, and D86Q; more preferably D86S.

In preferred embodiments of the nuclease according to the invention, the amino acid sequence is substituted compared to SEQ ID NO: 1 in position A94, wherein the substitution is selected from the group consisting of A94R, A94N, A94D, A94C, A94Q, A94E, A94G, A94H, A94I, A94L, A94K, A94M, A94F, A94P, A94T, A94W, A94Y, A94V, and A94S; preferably A94G, A94V, A94L, A94I, and A94P; more preferably A94G.

In preferred embodiments of the nuclease according to the invention, the amino acid sequence is substituted compared to SEQ ID NO: 1 in position S116, wherein the substitution is selected from the group consisting of S116A, S116R, S116N, S116D, S116C, S116Q, S116E, S116G, S116H, S116I, S116L, S116K, S116M, S116F, S116P, S116T, S116W, S116Y, and S116V; preferably S116R, S116K, S116H, S116G, S116A, S116V, S116L, S1161, S116P, S116W, S116F, S116Y, and S116D; preferably S116K, S116H, and S116R; more preferably S116K.

In preferred embodiments of the nuclease according to the invention, the amino acid sequence is substituted compared to SEQ ID NO: 1 in position D117, wherein the substitution is selected from the group consisting of D117G, D117A, D117V, D117L, D1171, D117P, D117W, D117F, D117Y, D117C, D117M, D117S, D117Q, D117T, D117N, D117E, D117H, D117R and D117K; preferably D117G, D117A, D117V, D117L, D117I, D117P, D117W, D117F, D117Y, D117C, D117M, D117S, D117Q, D117T, D117N, D117H, D117R and D117K; more preferably D117K, D117A, D117S, D117N, D117R, D117H, and D117Y; even more preferably D117A, D117N, D117R, D117K, and D117S; yet more preferably D117A, D117N, D117K, and D117S; and most preferably D117A, D117N, and D117S.

In preferred embodiments of the nuclease according to the invention, the amino acid sequence is substituted compared to SEQ ID NO:1 in position N119, wherein the substitution is selected from the group consisting of N119A, N119R, N119D, N119C, N119Q, N119E, N119G, N119H, N119I, N119L, N119K, N119M, N119F, N119P, N119T, N119W, N119Y, N119V, and N119S; preferably N119R, N119K, and N119H; more preferably N119R.

In preferred embodiments of the nuclease according to the invention, the amino acid sequence is substituted compared to SEQ ID NO:1 in position Q120, wherein the substitution is selected from the group consisting of Q120A, Q120R, Q120N, Q120D, Q120C, Q120E, Q120G, Q120H, Q120I, Q120L, Q120K, Q120M, Q120F, Q120P, Q120T, Q120W, Q120Y, Q120V, and Q120S; preferably Q120R, Q120K, Q120H, Q120G, Q120A, Q120V, Q120L, Q120I, Q120P, Q120W, Q120F, Q120Y, and Q120D; more preferably Q120K, Q120H, and Q120R; most preferably Q120R.

In preferred embodiments of the nuclease according to the invention, the amino acid sequence is substituted compared to SEQ ID NO:1 in position A124, wherein the substitution is selected from the group consisting of A 124R, A124N, A124D, A124C, A124Q, A124E, A124G, A124H, A124I, A124L, A124K, A124M, A124F, A124P, A124T, A124W, A124Y, A124V, and A124S; preferably A124D, A124E, A124R, A124K, A124H, A124G, A124V, A124L, A124I, and A124P; preferably A124K, A124H and A124R; more preferably A124K, and A124R.

In preferred embodiments of the nuclease according to the invention, the amino acid sequence is substituted compared to SEQ ID NO:1 in position D128, wherein the substitution is selected from the group consisting of D128A, D128R, D128N, D128C, D128Q, D128E, D128G, D128H, D128I, D128L, D128K, D128M, D128F, D128P, D128T, D128W, D128Y, D128V, and D128S; preferably D128G, D128A, D128V, D128L, D128I, D128P, D128W, D128F, D128Y, D128C, D128M, D128N, D128Q, D128S, D128T, D128H, D128Rand D128K; most preferably D128G, D128Y, D128F, D128M, D128N, D128S, D128R and D128K.

In preferred embodiments of the nuclease according to the invention, the amino acid sequence is substituted compared to SEQ ID NO:1 in position Q129, wherein the substitution is selected from the group consisting of Q129A, Q129R, Q129N, Q129D, Q129C, Q129E, Q129G, Q129H, Q129I, Q129L, Q129K, Q129M, Q129F, Q129P, Q129T, Q129W, Q129Y, Q129V, and Q129S; preferably Q129R, Q129K, Q129H, Q129G, Q129A, Q129V, Q129L, Q129I, Q129P, Q129W, Q129F, Q129Y, and Q129D; preferably Q129K, Q129H, and Q129R; more preferably Q129K, Q129R; still more preferably Q129R.

In preferred embodiments of the nuclease according to the invention, the amino acid sequence is substituted compared to SEQ ID NO:1 in position K132, wherein the substitution is selected from the group consisting of K132A, K132R, K132N, K132D, K132C, K132Q, K132E, K132G, K132H, K132I, K132L, K132M, K132F, K132P, K132T, K132W, K132Y, K132V, and K132S; preferably K132R, and K132H; most preferably K132R.

In preferred embodiments of the nuclease according to the invention, the amino acid sequence is substituted compared to SEQ ID NO: 1 in position D135, wherein the substitution is selected from the group consisting of D135A, D135R, D135N, D135C, D135Q, D135E, D135G, D135H, D135I, D135L, D135K, D135M, D135F, D135P, D135T, D135W, D135Y, D135V, and D135S; preferably D135G, D135A, D135V, D135L, D135I, D135P, D135W, D135F, D135Y, D135C, D135M, D135N, D135Q, D135S, D135T, D135R, D135H, and D135K; more preferably D135G, D135A, D135V, D135L, D135I, D135P, D135C, D135M, D135N, D135Q, D135S, D135T, D135R, D135H, and D135K; most preferably D135A, D135M, D135G, D135N, D135R and D135K.

In preferred embodiments of the nuclease according to the invention, the amino acid sequence is substituted compared to SEQ ID NO:1 in position E151, wherein the substitution is selected from the group consisting of E151A, E151R, E151N, E151D, E151C, E151Q, E151G, E151H, E151I, E151L, E151K, E151M, E151F, E151P, E151T, E151W, E151Y, E151V, and E151S; E151G, E151A, E151V, E151L, E151I, E151P, E151W, E151F, E151Y, E151C, E151M, E151N, E151Q, E151S, E151T, E151H and E151K; preferably E151A, E151G, E151V, E151L, E151I, and E151P; more preferably E151L.

In preferred embodiments of the nuclease according to the invention, the amino acid sequence is substituted compared to SEQ ID NO: 1 in position K162, wherein the substitution is selected from the group consisting of K162A, K162R, K162N, K162D, K162C, K162Q, K162E, K162G, K162H, K162I, K162L, K162M, K162F, K162P, K162T, K162W, K162Y, K162V, and K162S; preferably K162N, K162Q, K162S, K162T, K162C and K162M; most preferably K162N.

In preferred embodiments of the nuclease according to the invention, the amino acid sequence is substituted compared to SEQ ID NO: 1 in position D191, wherein the substitution is selected from the group consisting of D191G, D191A, D191V, D191L, D191I, D191P, D191W, D191F, D191Y, D191C, D191M, D191S, D191Q, D191T, D191N, D191E, D191H, D191R and D191K; preferably D191G, D191A, D191V, D191L, D191I, D191P, D191C, D191M, D191S, D191Q, and D191T; more preferably D191G, D191A, D191V, D191L, D191I, and D191P; most preferably D191P.

In preferred embodiments of the nuclease according to the invention, the amino acid sequence is substituted compared to SEQ ID NO:1 in position P195, wherein the substitution is selected from the group consisting of P195A, P195R, P195N, P195D, P195C, P195Q, P195E, P195G, P195H, P195I, P195L, P195K, P195M, P195F, P195T, P195W, P195Y, P195V, and P195S; preferably P195K, P195H and P195R; most preferably P195R.

In preferred embodiments of the nuclease according to the invention, the amino acid sequence is substituted compared to SEQ ID NO: 1 in position K196, wherein the substitution is selected from the group consisting of K196A, K196R, K196N, K196D, K196C, K196Q, K196E, K196G, K196H, K196I, K196L, K196M, K196F, K196P, K196T, K196W, K196Y, K196V, and K196S; preferably K196G, K196A, K196V, K196L, K196I, K196P, K196R, K196H, K196N, K196Q, K196S, K196T, K196C, and K196M; more preferably K196R, and K196H; most preferably K196R.

In preferred embodiments of the nuclease according to the invention, the amino acid sequence is substituted compared to SEQ ID NO: 1 in position G197, wherein the substitution is selected from the group consisting of G197A, G197R, G197N, G197D, G197C, G197Q, G197E, G197H, G197I, G197L, G197K, G197M, G197F, G197P, G197T, G197W, G197Y, G197V, and G197S; preferably G197D, G197E, G197R, G197K, G197H, G197A, G197V, G197L, G197I, and G197P; preferably G197K, G197H, and G197R; more preferably G197K.

In preferred embodiments of the nuclease according to the invention, the amino acid sequence is substituted compared to SEQ ID NO: 1 in position D199, wherein the substitution is selected from the group consisting of D199G, D199A, D199V, D199L, D199I, D199P, D199W, D199F, D199Y, D199C, D199M, D199S, D199Q, D199T, D199N, D199E, D199H, D199R and D199K; preferably D199G, D199A, D199V, D199L, D199I, D199P, D199W, D199F, D199Y, D199C, D199M, D199N, D199S, D199Q, D199T, and D199K; preferably D199N, D199S, D199Q, D199T, D199C, D199M, D199H, and D199K; more preferably D199N, D199S, and D199K.

In preferred embodiments of the nuclease according to the invention, the amino acid sequence is substituted compared to SEQ ID NO: 1 in position R204, wherein the substitution is selected from the group consisting of R204A, R204N, R204D, R204C, R204Q, R204E, R204G, R204H, R204I, R204L, R204K, R204M, R204F, R204P, R204T, R204W, R204Y, R204V, and R204S; preferably R204G, R204A, R204V, R204L, R204I, R204P, R204K, R204H, R204N, R204Q, R204S, R204T, R204C, and R204M; more preferably R204Q, R204N, R204S, R204T, R204C and R204M; most preferably R204Q.

In preferred embodiments of the nuclease according to the invention, the amino acid sequence is substituted compared to SEQ ID NO: 1 in position I218, wherein the substitution is selected from the group consisting of I218R, I218N, I218D, I218C, I218Q, I218E, I218G, I218H, I218A, I218L, I218K, I218M, I218F, I218P, I218T, I218W, I218Y, I218V, and I218S; preferably I218F, I218W and I218Y; more preferably I218F.

In preferred embodiments of the nuclease according to the invention, the amino acid sequence is substituted compared to SEQ ID NO: 1 in position A228, wherein the substitution is selected from the group consisting of A228R, A228N, A228D, A228C, A228Q, A228E, A228G, A228H, A228I, A228L, A228K, A228M, A228F, A228P, A228T, A228W, A228Y, A228V, and A228S; preferably A228K, A228H and A228R; more preferably A228R.

In preferred embodiments of the nuclease according to the invention, the amino acid sequence is substituted compared to SEQ ID NO: 1 in position E239, wherein the substitution is selected from the group consisting of E239A, E239R, E239N, E239D, E239C, E239Q, E239G, E239H, E239I, E239L, E239K, E239M, E239F, E239P, E239T, E239W, E239Y, E239V, and E239S; preferably E239G, E239A, E239V, E239L, E239I, E239P, E239W, E239F, E239Y, E239C, E239M, E239N, E239Q, E239S, E239T, E239H and E239K; preferably E239A, E239G, E239V, E239L, E239I, and E239P; more preferably E239A.

In preferred embodiments of the nuclease according to the invention
- a substitution in position P51, P73, or P195 means that the P is substituted by an amino acid selected from the group consisting of A, R, N, D, C, Q, E, G, H, I, L, K, M, F, T, W, Y, V, or S; and/or
- a substitution in position S53, or S116 means that the S is substituted by an amino acid selected from the group consisting of A, R, N, D, C, Q, E, G, H, I, L, K, M, F, P, T, W, Y, or V; and/or
- a substitution in position K55, K84, K132, K162, or K196, means that the K is substituted by an amino acid selected from the group consisting of A, R, N, D, C, Q, E, G, H, I, L, M, F, P, T, W, Y, V, or S; and/or
- a substitution in position T56, or T77 means that the T is substituted by an amino acid selected from the group consisting of A, R, N, D, C, Q, E, G, H, I, L, K, M, F, P, W, Y, V, or S; and/or
- a substitution in position N58, N80, or N119 means that the N is substituted by an amino acid selected from the group consisting of A, R, D, C, Q, E, G, H, I, L, K, M, F, P, T, W, Y, V, or S; and/or
- a substitution in position A52, A72, A74, A81, A82, A94, A124, or A228 means that the A is substituted by an amino acid selected from the group consisting of R, N, D, C, Q, E, G, H, I, L, K, M, F, P, T, W, Y, V, or S; and/or
- a substitution in position G54, G78, or G197 means that the G is substituted by an amino acid selected from the group consisting of A, R, N, D, C, Q, E, H, I, L, K, M, F, P, T, W, Y, V, or S; and/or
- a substitution in position D86, D117, D128, D135, D191, or D199 means that the D is substituted by an amino acid selected from the group consisting of A, R, N, C, Q, E, G, H, I, L, K, M, F, P, T, W, Y, V, or S; and/or
- a substitution in position Q120, or Q129 means that the Q is substituted by an amino acid selected from the group consisting of A, R, N, D, C, E, G, H, I, L, K, M, F, P, T, W, Y, V, or S; and/or
- a substitution in position R204 means that the R is substituted by an amino acid selected from the group consisting of A, N, D, C, Q, E, G, H, I, L, K, M, F, P, T, W, Y, V, or S; and/or
- a substitution in position I218 means that the I is substituted by an amino acid selected from the group consisting of A, R, N, D, C, Q, E, G, H, L, K, M, F, P, T, W, Y, V, or S.
- a substitution in position E151 or E239 means that the E is substituted by an amino acid selected from the group consisting of A, R, N, D, C, Q, G, H, I, L, K, M, F, P, T, W, Y, V, or S.

In preferred embodiments of the nuclease according to the invention, the at least one amino acid position is substituted by
- a positively charged amino acid selected from R, K, and H; or
- a negatively charged amino acid selected from E and D;
- an aliphatic nonpolar amino acid selected from G, A, V, L, I, and P; or
- an aromatic amino acid selected from W, F, and Y; or
- an aliphatic polar non-charged amino acid selected from N, Q, S, T, C, and M.

In preferred embodiments of the nuclease according to the invention, the at least one amino acid position is
- a positively charged amino acid and substituted by another positively charged amino acid; or
- a negatively charged amino acid and substituted by another negatively charged amino acid; or
- an aliphatic nonpolar amino acid and substituted by another aliphatic nonpolar amino acid; or
- an aromatic amino acid and substituted by another aromatic amino acid; or
- an aliphatic polar non-charged amino acid and substituted by another aliphatic polar non-charged amino acid.

In preferred embodiments of the nuclease according to the invention, the at least one amino acid position is
- a positively charged amino acid and is substituted by an aliphatic nonpolar amino acid, or an aromatic amino acid, or an aliphatic polar non-charged, or a negatively charged amino acid; or
- a negatively charged amino acid and is substituted by an aliphatic nonpolar amino acid, or an aromatic amino acid, or an aliphatic polar non-charged, or a positively charged amino acid; or
- an aliphatic nonpolar amino acid and is substituted by an aromatic amino acid, or an aliphatic polar non-charged, or a positively charged amino acid, or a negatively charged amino acid; or
- an aromatic amino acid and is substituted by an aliphatic nonpolar amino acid, or an aliphatic polar non-charged, or a positively charged amino acid, or a negatively charged amino acid; or
- an aliphatic polar non-charged amino acid and is substituted by an aliphatic nonpolar amino acid, or an aromatic amino acid, or a positively charged amino acid, or a negatively charged amino acid.

In preferred embodiments of the nuclease according to the invention, the amino acid sequence is substituted compared to SEQ ID NO: 1 in one, two, three, four, five, six, seven, eight, nine, ten, eleven, twelve, thirteen, fourteen, fifteen, sixteen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, thirty-seven, or thirty-eight amino acid positions.

In preferred embodiments of the nuclease according to the invention, the amino acid sequence is substituted compared to SEQ ID NO:1 in 1 to 86 amino acid positions; preferably in 1 to 84 amino acid positions; more preferably in 1 to 50 amino acid positions; still more preferably in 1 to 39 amino acid positions; still more preferably in 1 to 27 amino acid positions; yet more preferably in 1 to 11 amino acid positions, and even more preferably 1 to 6 amino acid positions.

A preferred embodiment of the invention relates to a nuclease; preferably an endonuclease; more preferably a non-specific endonuclease; still more preferably the nuclease according to any of the invention as described above, comprising an amino acid sequence with at least 65% identity to SEQ ID NO:1, wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239;
wherein a substitution in position P51, A72, P73, A74, A124, or G197 means that the P, A or G is substituted by
   - a positively charged amino acid; or
   - a negatively charged amino acid; or
   - a different aliphatic nonpolar amino acid; and
wherein a substitution in position in S53, T56, N58, T77, N80, or S116 means that the S, T or N is substituted by
   - a positively charged amino acid; or
   - an aliphatic nonpolar amino acid; or
   - an aromatic amino acid; or
   - an aliphatic polar non-charged amino acid; or
   - a negatively charged amino acid; and
wherein a substitution in position in K55, K84, R204, or K196 means that the K or R is substituted by
   - an aliphatic nonpolar amino acid; or
   - a different positively charged amino acid; or
   - an aliphatic polar non-charged amino acid; and
wherein a substitution in position D86, or D191 means that the D is substituted by
   - an aliphatic nonpolar amino acid; or
   - a polar non-charged amino acid; and
wherein a substitution in position D128, D135, D117, E239 or D199 means that the D or E is substituted by
   - an aliphatic nonpolar amino acid; or
   - an aromatic amino acid; or
   - an aliphatic polar non-charged amino acid; or
   - a positively charged amino acid.

Another preferred embodiment of the invention relates to a nuclease; preferably an endonuclease; more preferably a non-specific endonuclease; still more preferably the nuclease according to the invention as described above comprising an amino acid sequence with at least 65% identity to SEQ ID NO:1, wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239;
wherein a substitution in position P51, A72, P73, A74, A124, or G197 means that the P, A or G is substituted by
   - a positively charged amino acid selected from R, K, and H; or
   - a negatively charged amino acid selected from D, and E; or
   - a different aliphatic nonpolar amino acid selected from G, A, V, L, I, and P; and
wherein a substitution in position in S53, T56, N58, T77, N80, or S116 means that the S, T or N is substituted by
   - a positively charged amino acid selected from R, K, and H; or
   - an aliphatic nonpolar amino acid selected from G, A, V, L, I, and P; or
   - an aromatic amino acid selected from W, F, and Y; or
   - an aliphatic polar non-charged amino acid selected from N, Q, S, T, C, and M; preferably S, and M; or
   - D; and
wherein a substitution in position in K55, K84, R204, or K196 means that the K or R is substituted by
   - an aliphatic nonpolar amino acid selected from of G, A, V, L, I, and P; or
   - a different positively charged amino acid selected from R, K, and H; or
   - an aliphatic polar non-charged amino acid selected from N, Q, S, T, C, and M; and
wherein a substitution in position D86, or D191 means that the D is substituted by
   - an aliphatic nonpolar amino acid selected from G, A, V, L, I, and P; or
   - a polar non-charged amino acid selected from N, Q, S, T, C, and M; preferably Q, S, T, C, and M; and
wherein a substitution in position D128, D135, D117, E239 or D199 means that the D or E is substituted by
   - an aliphatic nonpolar amino acid selected from G, A, V, L, I, and P; or
   - an aromatic amino acid selected from W, F, and Y; or
   - an aliphatic polar non-charged amino acid selected from N, Q, S, T, C, and M; or
   - a positively charged amino acid selected from R, K, and H; preferably K, and H.

Another preferred embodiment of the invention relates to a nuclease; preferably an endonuclease; more preferably a non-specific endonuclease; still more preferably the nuclease according to the invention as described above, comprising an amino acid sequence with at least 67% identity to SEQ ID NO:1; wherein the amino acid sequence is substituted compared to SEQ ID NO: 1 in at least one amino acid position selected from the group consisting of P51, T56, N58, P73, T77, A124, D128, D135, G197, R204, and E239.

Another preferred embodiment of the invention relates to a nuclease; preferably an endonuclease; more preferably a non-specific endonuclease; still more preferably the nuclease according to the invention as described above, comprising an amino acid sequence with at least 89% identity to SEQ ID NO:1;
wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239;
wherein a substitution in position P51, A72, P73, A74, A124, or G197 means that the P, A or G is substituted by
   - a positively charged amino acid; or
   - a negatively charged amino acid; or
   - a different aliphatic nonpolar amino acid; and
wherein a substitution in position in S53, T56, N58, T77, N80, or S116 means that the S, T or N is substituted by
   - a positively charged amino acid; or
   - an aliphatic nonpolar amino acid; or
   - an aromatic amino acid; or
   - an aliphatic polar non-charged amino acid; or
   - a negatively charged amino acid; and
wherein a substitution in position in K55, K84, R204, or K196 means that the K or R is substituted by
   - an aliphatic nonpolar amino acid; or
   - a different positively charged amino acid; or
   - an aliphatic polar non-charged amino acid; and
wherein a substitution in position D86, or D191 means that the D is substituted by
   - an aliphatic nonpolar amino acid; or
   - a polar non-charged amino acid; and
wherein a substitution in position D128, D135, D117, E239, or D199 means that the D or E is substituted by
   - an aliphatic nonpolar amino acid; or
   - an aromatic amino acid; or
   - an aliphatic polar non-charged amino acid; or
   - a positively charged amino acid.

Another preferred embodiment of the invention relates to a nuclease; preferably an endonuclease; more preferably a non-specific endonuclease; still more preferably the nuclease according to the invention as described above, comprising an amino acid sequence with at least 89% identity to SEQ ID NO:1;
wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239;
wherein a substitution in position P51, A72, P73, A74, A124, or G197 means that the P, A or G is substituted by
   - a positively charged amino acid selected from R, K, and H; or
   - a negatively charged amino acid selected from D, and E; or
   - a different aliphatic nonpolar amino acid selected from G, A, V, L, I, and P; and
wherein a substitution in position in S53, T56, N58, T77, N80, or S116 means that the S, T or N is substituted by
   - a positively charged amino acid selected from R, K, and H; or
   - an aliphatic nonpolar amino acid selected from G, A, V, L, I, and P; or
   - an aromatic amino acid selected from W, F, and Y; or
   - an aliphatic polar non-charged amino acid selected from N, Q, S, T, C, and M; preferably S, and M; more preferably M; or
   - D; and
wherein a substitution in position in K55, K84, R204, or K196 means that the K or R is substituted by
   - an aliphatic nonpolar amino acid selected from of G, A, V, L, I, and P; or
   - a different positively charged amino acid selected from R, K, and H; or
   - an aliphatic polar non-charged amino acid selected from N, Q, S, T, C, and M; and
wherein a substitution in position D86, or D191 means that the D is substituted by
   - an aliphatic nonpolar amino acid selected from G, A, V, L, I, and P; or
   - a polar non-charged amino acid selected from N, Q, S, T, C, and M; preferably Q, S, T, C, and M; and
wherein a substitution in position D128, D135, D117, E239, or D199 means that the D or E is substituted by
   - an aliphatic nonpolar amino acid selected from G, A, V, L, I, and P; or
   - an aromatic amino acid selected from W, F, and Y; or
   - an aliphatic polar non-charged amino acid selected from N, Q, S, T, C, and M; preferably Q, S, T, C, and M; or
   - a positively charged amino acid selected from R, K, and H. preferably K, and H.

Another preferred embodiment of the invention relates to a nuclease; preferably an endonuclease; more preferably a non-specific endonuclease; still more preferably the nuclease according to the invention as described above, comprising an amino acid sequence with at least 67%; preferably with at least 89% identity to SEQ ID NO:1;
wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239;
wherein a substitution in position P51, A72, P73, A74, A124, or G197 means that the P, A or G is substituted by a positively charged amino acid selected from R, K, and H;
wherein P51 is to be substituted by an aliphatic nonpolar amino acid selected from G, A, V, L, I;
wherein P51 is to be substituted by a negatively charged amino acid selected from D, and E;
wherein a substitution in position S53, T56, N58, T77, N80, or S116 means that the S, T or N is substituted by a positively charged amino acid selected from R, K, and H;
wherein a substitution in position S53, T56, N58, or T77 means that the S, T or N is substituted by an aliphatic nonpolar amino acid selected from G, A, V, L, I, and P;
wherein T77 is to be substituted by an aromatic amino acid selected from W, F, and Y;
wherein T56 is to be substituted by D;
wherein K55 is to be substituted by R, and H;
wherein K84 is to be substituted by G, A, V, L, I, and P;
wherein R204 is to be substituted by N, Q, S, T, C, and M;
wherein a substitution in position D117, D128, D135, D191, or E239 means that the D or E is substituted by an aliphatic nonpolar amino acid selected from G, A, V, L, I, and P;
wherein a substitution in position D86 means that the D is substituted by a polar non-charged amino acid selected from Q, S, T, C, and M;
wherein a substitution in position D117, D128, D135, or D199 means that the D or E is substituted by
   - an aliphatic polar non-charged amino acid selected from N, Q, S, T, C, and M; or
   - a positively charged amino acid selected from K, and H; and
wherein D128 is to be substituted by an aromatic amino acid selected from W, F, and Y.

Another preferred embodiment of the invention relates to a nuclease; preferably an endonuclease; more preferably a non-specific endonuclease; still more preferably the nuclease according to the invention as described above, comprising an amino acid sequence with at least 67%; preferably with at least 89% identity to SEQ ID NO: 1;
wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239;
wherein a substitution in position S53, D1 17, or E239 means that the S, D or E is substituted by A;
wherein a substitution in position P51, or T56 means that the P or T is substituted by D;
wherein a substitution in position T77, or D128 means that the T or D is substituted by F;
wherein a substitution in position P51, K84, D128, or D135 means that the P, K, or D is substituted by G;
wherein N80 is to be substituted by H;
wherein a substitution in position S53, T56, N58, A72, P73, A74, T77, S116, D117, A124, D128, D135, G197, or D199 means that the A, D, G, N, P, S, or T is substituted by K;
wherein a substitution in position P51, or T77 means that the P or T is substituted by L;
wherein D128 is to be substituted by M;
wherein a substitution in position D117, D128, D135, or D199 means that the D is substituted by N;
wherein a substitution in position T56, or D191 means that the D or T is substituted by P;
wherein R204 is to be substituted by Q;
wherein a substitution in position P51, K55, T56, N58, P73, A74, T77, or A124 means that the A, K, N, P, or T is substituted by R;
wherein a substitution in position D86, D117, D128, or D199 means that the D is substituted by S;
wherein N58 is to be substituted by V; and
wherein D128 is to be substituted by Y.

In preferred embodiments, the nuclease according to the invention comprises an amino acid sequence with at least 67%; preferably with at least 89% identity to SEQ ID NO:1;
wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least four-teen, at least fifteen, at least sixteen, at least seventeen, at least eighteen, at least nineteen, at least twenty, at least twenty-one, or at least twenty-two amino acid position selected from the group consisting of P51L, P51G, P51R, P51D, S53A, S53K, K55R, T56D, T56P, T56R, T56K, T56S, T56M, D56G, D56A, D56V, D56I, D56L, D56F, N58V, N58R, N58K, A72K, P73R, P73K, A74R, A74K, T77L, T77F, T77R, T77K, N80H, K84G, D86S, S116K, D117H, D117I, D117R, D117K, D117A, D117N, D117S, A124R, A124K, D128K, D128N, D128M, D128S, D128Y, D128G, D128F, D135A, D135M, D135K, D135N, D135G, D191P, G197K, D199K, D199N, D199S, R204Q, and E239A; preferably P51L, P51G, P51R, P51D, S53A, S53K, K55R, T56D, T56P, T56R, T56K, N58V, N58R, N58K, A72K, P73R, P73K, A74R, A74K, T77L, T77F, T77R, T77K, N80H, K84G, D86S, S116K, D117K, D117A, D117N, D117S, A124R, A124K, D128K, D128N, D128M, D128S, D128Y, D128G, D128F, D135K, D135N, D135G, D191P, G197K, D199K, D199N, D199S, R204Q, and E239A.

In another preferred embodiment, the nuclease according to the invention comprises an amino acid sequence with at least 67%; preferably with at least 80%; more preferably with at least 89% identity, and most preferably with at least 95% identity to SEQ ID NO:1;
wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least four-teen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, at least thirty-one, at least thirty-two, at least thirty-three, at least thirty-four, at least thirty-five, at least thirty-six, at least thirty-seven, or at least thirty-eight amino acid position selected from the group consisting of P51L, P51R, P51G, P51D, P51A, P51V, P51I, A52R, S53K, S53A, G54R, K55R, T56P, T56D, T56R, T56K, T56S, T56G, T56A, T56V, T56I, T56F, T56L, T56M, N58V, N58R, N58K, A72K, P73K, P73R, A74R, A74K, T77L, T77K, T77F, T77R, T77H, G78R, N80H, N80R, A81K, A82R, K84G, D86S, A94G, S116K, D117K, D117A, D117S, D117N, D117R, D117H, D117Y, N119R, Q120R, A124K, A124R, A124H, D128M, D128Y, D128K, D128S, D128N, D128F, D128G, D128R, Q129R, K132R, D135N, D135K, D135G, D135R, D135A, D135M, E151L, K162N, D191P, P195R, K196R, G197K, D199N, D199K, D199S, R204Q, I218F, A228R, and E239A.

In another preferred embodiment, the nuclease according to the invention comprises an amino acid sequence with at least 67%; preferably with at least 80%; more preferably with at least 89% identity, and most preferably with at least 95% identity to SEQ ID NO: 1;
wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least four-teen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, at least thirty-one, at least thirty-two, or at least thirty-three amino acid position selected from the group consisting of P51L, P51R, P51G, P51D, P51A, P51V, P51I, S53K, S53A, K55R, T56P, T56D, T56R, T56K, T56S, T56G, T56A, T56V, T56I, T56F, T56L, T56M, N58V, N58R, N58K, A72K, P73K, P73R, A74K, T77L, T77K, T77F, T77R, T77H, N80H, A81K, A82R, K84G, D86S, A94G, S116K, D117K, D117A, D117S, D117N, D117R, D117H, D117Y, Q120R, A124K, A124R, A124H, D128M, D128Y, D128K, D128S, D128N, D128F, D128G, D128R, Q129R, K132R, D135N, D135K, D135G, D135R, D135A, D135M, E151L, K162N, D191P, K196R, G197K, D199N, D199K, D199S, R204Q, I218F, A228R, and E239A.

Another preferred embodiment of the invention relates to a nuclease; preferably an endonuclease; more preferably a non-specific endonuclease; still more preferably the nuclease according to the invention as described above, comprising an amino acid sequence with at least 67%; preferably at least 89% identity to SEQ ID NO: 1;
wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239;
wherein an aliphatic nonpolar amino acid selected from G, A, V, L, I, and P substitutes P51, S53, T56, N58, T77, K84, D117, D128, D135, D191, or E239;
wherein a positively charged amino acid selected from R, K, and H substitutes P51, S53, K55, T56, N58, A72, P73, A74, T77, N80, S116, A124, or G197;
wherein a positively charged amino acid selected from R, K and H; preferably from K and H substitutes D117, D128, D135, or D199;
wherein an aromatic amino acid selected from W, F, and Y substitutes T77, or D128;
wherein D substitutes P51, or T56;
wherein an aliphatic polar non-charged amino acid selected from N, Q, S, T, C, and M substitutes D117, D128, D135, D199, or R204; and
wherein an aliphatic polar non-charged amino acid selected from Q, S, T, C, and M substitutes D86.

In preferred embodiments, the nuclease according to the invention comprises an amino acid sequence with at least 67%; preferably at least 89% identity to SEQ ID NO:1;
wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239;
wherein the amino acid A substitutes S53, D117, or E239;
wherein the amino acid D substitutes P51, or T56;
wherein the amino acid F substitutes T77, or D128;
wherein the amino acid G substitutes P51, K84, D128, or D135;
wherein the amino acid H substitutes N80;
wherein the amino acid K substitutes S53, T56, N58, A72, P73, A74, T77, S116, D117, A124, D128, D135, G197, or D199;
wherein the amino acid L substitutes P51, or T77;
wherein the amino acid M substitutes D128;
wherein the amino acid N substitutes D117, D128, D135, or D199;
wherein the amino acid P substitutes T56, or D191;
wherein the amino acid Q substitutes R204;
wherein the amino acid R substitutes P51, K55, T56, N58, P73, A74, T77, or A124;
wherein the amino acid S substitutes D86, D117, D128, or D199;
wherein the amino acid V substitutes N58; and
wherein the amino acid Y substitutes D128.

Another preferred embodiment of the invention relates to a nuclease; preferably an endonuclease; more preferably a non-specific endonuclease, comprising an amino acid sequence with at least 67%; preferably with at least 89% identity to SEQ ID NO:1;
wherein the amino acid sequence is substituted compared to SEQ ID NO:1 in at least a first amino acid position and a second amino acid position;
wherein the first amino acid position is selected from the group consisting of P51, T56, T77, and A124; and
wherein the second amino acid position is selected from the group consisting of P51, S53, K55, T56, N58, A72, P73, A74, T77, N80, D86, D117, Q120, A124, D128, Q129, D135, D191, K196, G197, D199, R204, and E239.

Another preferred embodiment of the invention relates to a nuclease; preferably an endonuclease; more preferably a non-specific endonuclease, comprising an amino acid sequence with at least 89% identity to SEQ ID NO: 1;
wherein the amino acid sequence is substituted compared to SEQ ID NO: 1 in at least a first amino acid position and a second amino acid position;
wherein the first amino acid position is selected from the group consisting of P51, T56, T77, and A124; and
wherein the second amino acid position is selected from the group consisting of P51, S53, K55, T56, N58, A72, P73, A74, T77, N80, D86, D117, A124, D128, D135, D191, K196, G197, D199, R204, and E239; and
wherein when the amino acid sequence is substituted in amino acid position P51 the substitution is P51R, P51K, P51H, P51D, P51E, P51G, P51A, P51V, P51L, P51I; preferably P51L, P51G, P51R, P51D; and
wherein when the amino acid sequence is substituted in amino acid position T56 the substitution is T56R, T56K, T56H, T56G, T56A, T56V, T56L, T56I, T56P, T56S, T56M, and T56D; preferably T56K, T56R, T56P, and T56D, and more preferably T56K, T56R, and T56D; and
wherein when the amino acid sequence is substituted in amino acid position T77 the substitution is T77R, T77K, T77H, T77G, T77A, T77V, T77L, T77I, T77P, T77W, T77F, T77Y, and T77D; preferably T77K, T77R, T77F, and T77L; and
wherein when the amino acid sequence is substituted in amino acid position A124 the substitution is A124D, A124E, A124R, A124K, A124H, A124G, A124V, A124L, A124I, and A124P; preferably A124K, A124H and A124R, and more preferably A124K, and A124R.

In preferred embodiments of the nuclease according to the invention, the first amino acid position and the second amino acid position are both selected from the group consisting of P51, T56, T77, and A124.

In preferred embodiments of the nuclease according to the invention,
a substitution in position P51, A72, P73, A74, A124, or G197 means that the P, A or G is substituted by
   - a positively charged amino acid selected from R, K, and H; or
   - a different aliphatic nonpolar amino acid selected from G, A, V, L, I, and P; or
   - D; and
a substitution in position in S53, T56, N58, T77, N80, Q120, or Q129 means that the S, T, Q, or N is substituted by
   - a positively charged amino acid selected from R, K, and H; or
   - an aliphatic nonpolar amino acid selected from G, A, V, L, I, and P; or
   - an aromatic amino acid selected from W, F, and Y; or
   - an aliphatic polar non-charged amino acid selected from N, Q, S, T, C, and M; preferably S, and M; more preferably M; or
   - D; and
a substitution in position in K55, R204, or K196 means that the K or R is substituted by
   - an aliphatic nonpolar amino acid selected from of G, A, V, L, I, and P; or
   - a different positively charged amino acid selected from R, K, and H; or
   - an aliphatic polar non-charged amino acid selected from N, Q, S, T, C, and M; and
a substitution in position D86, or D191 means that the D is substituted by
   - an aliphatic nonpolar amino acid selected from G, A, V, L, I, and P; or
   - a polar non-charged amino acid selected from N, Q, S, T, C, and M; preferably Q, S, T, C, and M; and
a substitution in position D128, D135, D117, E239, or D199 means that the D or E is substituted by
   - an aliphatic nonpolar amino acid selected from G, A, V, L, I, and P; or
   - an aromatic amino acid selected from W, F, and Y; or
   - an aliphatic polar non-charged amino acid selected from N, Q, S, T, C, and M; preferably Q, S, T, C, and M; or
   - a positively charged amino acid selected from R, K, and H; preferably K, and H.

Another preferred embodiment of invention relates to the following:
[1]: A nuclease; preferably an endonuclease, comprising an amino acid sequence with at least 67%; preferably at least 89% identity to SEQ ID NO: 1; wherein the amino acid sequence is substituted compared to SEQ ID NO:1 in at least a first amino acid position and a second amino acid position; wherein the first amino acid position is selected from the group consisting of P51, T56, T77, and A124; and wherein the second amino acid position is selected from the group consisting of P51, S53, K55, T56, N58, A72, P73, A74, T77, N80, D86, D117, Q120, A124, D128, Q129, D135, D191, K196, G197, D199, R204, and E239.
[2]: The nuclease of [1], wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51, S53, K55, T56, N58, A72, P73, A74, T77, N80, D86, D117, Q120, A124, D128, Q129, K132, D135, D191, K196, G197, D199, R204, and E239; preferably P51, T56, N58, T77, Q120, A124, D128, Q129, D135, K196, and G197.
[3]: The nuclease of [1] or [2], wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, D86S, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A; preferably P51R, P51G, P51L, T56S, T56M, T56R, T56K, T56G, T56A, T56V, T56L, T56I, T56F, T56P, T56D, N58K, N58R, N58V, T77K, T77R, T77F, T77L, Q120R, A124K, A124R, D128G, D128Y, D128F, D128M, D128N, D128S, D128R, D128K, Q129R, D135A, D135M, D135G, D135N, D135R, D135K, K196R, and G197K.
[4]: The nuclease of any of [1]-[3], wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, or 102.
[5]: The nuclease of any of [1]-[4], which is characterized by either (A) an increased thermal stability in comparison to the wildtype nuclease of SEQ ID NO:1; and/or (B) an increased relative salt activity in comparison to the relative salt activity of the wildtype nuclease of SEQ ID NO:1; and/or (C) an increased S_{high} activity in comparison to the S_{high} activity of the wildtype nuclease of SEQ ID NO:1; and/or (D) an increased relative cold activity in comparison to the relative cold activity of the wildtype nuclease of SEQ ID NO:1; and/or (E) an increased T_{low} activity in comparison to the T_{low} activity of the wildtype nuclease of SEQ ID NO:1.
[6]: The nuclease of any of [1]-[5], which has an increased thermal stability than SEQ ID NO:1, wherein said thermal stability is characterized by a higher residual activity after incubation for 15 minutes at a temperature T_{stability}.
[7]: The nuclease of any of [1]-[6], wherein the nuclease is capable of hydrolyzing of phosphodiesters of polynucleotide substrate into cleaved polynucleotides or oligonucleotides.
[8]: The nuclease of any of [1]-[7], wherein the polynucleotide substrates are selected from the group consisting of single strand RNA, single strand DNA, double strand DNA, double strand RNA and hybrid strand DNA/RNA; preferably double strand DNA.
[9]: A method of hydrolyzing polynucleotide substrates; preferably being contained in a sample, comprising the steps of (a) providing a nuclease according to any of [1]-[8], (b) providing a composition containing a polynucleotide substrate, (c) contacting the polynucleotide substrate in the composition provided in step (b) with the nuclease provided in step (a) and allowing the nuclease to hydrolyze the polynucleotide substrate thereby obtaining a composition containing cleaved polynucleotide.
[10]: The method according to [9], wherein step (c) is carried out at a temperature, (i) between 4°C and 90°C; preferably between 10°C and 80°C; more preferably between 20°C and 65°C; still more preferably between 25°C and 65°; yet more preferably between 30°C and 50°C; even more preferably between 34°C and 42°C; most preferably at 37°C; or (ii) between 20°C and 90°C; preferably between 23°C and 80°C; more preferably between 25°C and 65°C; still more preferably between 30°C and 65°C; yet more preferably between 37°C and 65°C; even more preferably 50°C and 65°C; most preferably at 51°C; or (iii) between 4°C and 65°C; preferably between 4°C and 38°C; more preferably between 4°C and 25°C; still more preferably between 4°C and 20°C; yet more preferably between 4°C and 15°C; even more preferably between 4°C and 10°C; most preferably between 4°C and 6°C, and utmost preferably at 5°C.
[11]: The method according to [9] or [10], wherein step (c) is carried out in the presence of salts, wherein the salt is NaCl or KCl; preferably NaCl.
[12]: The method according to [9] or [11], wherein step (c) is carried out at a concentration of salt from 0.00M to 1.00M, or from 0.01M to 1.00M; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.
[13]: The method according to [9] or [12], wherein step (c) is carried out in the absence of salts.
[14]: The method according to [9] or [13] comprising the additional step of (d) inactivating the nuclease after step (c) thereby obtaining a composition containing cleaved polynucleotides and an inactivated nuclease.
[15]: The method According to [9] or [14], comprising the additional step of (e) separating the nuclease from the composition containing cleaved polynucleotide obtained in step (c) and optionally inactivated nuclease obtained in step (d).

Another preferred embodiment of the invention relates to a nuclease; preferably an endonuclease; more preferably a non-specific endonuclease, comprising an amino acid sequence with at least 75%; identity to SEQ ID NO: 1; and comprising
(i) a substitution in position P51, wherein P has been substituted by an amino acid selected from the group consisting of L, G, A, V, and I;
(ii) a substitution in position T77, wherein T has been substituted by an amino acid selected from the group consisting of R, H, and K; and
(iii) at least one substitution in a position selected from the group consisting of
   (a) A124, A52, G54, P73, A74, G78, A81, and P195; or
   (b) T56, S53, N58, N80, N119, and Q120;
   wherein in each case (a) or (b) the amino acid of SEQ ID NO: 1 has been substituted by an amino acid selected from the group consisting of R, H, and K.

In preferred embodiments of the nuclease according to the invention the nuclease; preferably the endonuclease, comprises
(i) a substitution in position P51, wherein P has been substituted by an amino acid selected from the group consisting of L, G, A, V, and I; preferably L;
(ii) a substitution in position T77, wherein T has been substituted by an amino acid selected from the group consisting of R, H, and K; preferably R; and
(iii) a substitution in position A124, wherein A has been substituted by an amino acid selected from the group consisting of R, H, and K; preferably K.

In preferred embodiments of the nuclease according to the invention the nuclease; preferably the endonuclease, comprises
(i) a substitution in position P51, wherein P has been substituted by an amino acid selected from the group consisting of L, G, A, V, and I; preferably L;
(ii) a substitution in position T77, wherein T has been substituted by an amino acid selected from the group consisting of R, H, and K; preferably R; and
(iii) a substitution in position A81, wherein A has been substituted by an amino acid selected from the group consisting of R, H, and K; preferably K.

In preferred embodiments of the nuclease according to the invention the nuclease; preferably the endonuclease, comprises an amino acid sequence, which is substituted compared to SEQ ID NO:1 in at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, or at least thirty-one, at least thirty-two, at least thirty-three, at least thirty-four, at least thirty-five, at least thirty-six, at least thirty-seven, or at least thirty-eight positions selected from the group consisting of P51, T77, A52, S53, G54, K55, T56, N58, A72, P73, A74, G78, N80, A81, A82, K84, D86, A94, S116, D117, N119, Q120, A124, D128, Q129, K132, D135, E151, K162, D191, P195, K196, G197, D199, R204, I218, A228, and E239.

Preferably, the nuclease; preferably the endonuclease, according to the invention comprises an amino acid sequence, which is substituted compared to SEQ ID NO:1 in at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, or at least thirty-one, at least thirty-two, at least thirty-three, at least thirty-four, at least thirty-five, at least thirty-six, at least thirty-seven, or at least thirty-eight positions selected from the group consisting of P51, T77, S53, K55, T56, N58, A72, P73, A74, G78, N80, A81, A82, K84, D86, A94, S116, D117, Q120, A124, D128, Q129, K132, D135, E151, K162, D191, K196, G197, D199, R204, I218, A228, and E239.

In preferred embodiments of the nuclease according to the invention the nuclease; preferably the endonuclease, comprises an amino acid sequence, which is substituted compared to SEQ ID NO:1 in one, two, three, four, five, six, seven, eight, nine, ten, eleven, twelve, thirteen, fourteen, fifteen, sixteen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, or thirty-six additional positions selected from the group consisting of A52, S53, G54, K55, T56, N58, A72, P73, A74, G78, N80, A81, A82, K84, D86, A94, S116, D117, N119, Q120, A124, D128, Q129, K132, D135, E151, K162, D191, P195, K196, G197, D199, R204, I218, A228, and E239; preferably selected from the group consisting of S53, K55, T56, N58, A72, P73, A74, G78, N80, A81, A82, K84, D86, A94, S116, D117, Q120, A124, D128, Q129, K132, D135, E151, K162, D191, K196, G197, D199, R204, I218, A228, and E239.

In preferred embodiments of the nuclease according to the invention the amino acid sequence of the nuclease; preferably of the endonuclease, comprises
(iii) a third substitution in a position selected from the group consisting of (a) A124, A52, G54, P73, A74, G78, A81, and P195; or (b) T56, S53, N58, N80, N119, and Q120; wherein in each case (a) or (b) the amino acid of SEQ ID NO:1 has been substituted by an amino acid selected from the group consisting of R, H, and K; and additionally
(iv) a fourth substitution in another position selected from the group consisting of (a) A124, A52, G54, P73, A74, G78, A81, and P195; or (b) T56, S53, N58, N80, N119, and Q120; wherein in each case (a) or (b) the amino acid of SEQ ID NO:1 has been substituted by an amino acid selected from the group consisting of G, P, A, V, L, I, M, C, F, Y, W, H, K, R, Q, N, E, D, S, and T.

In preferred embodiments of the nuclease according to the invention the amino acid sequence of the nuclease; preferably of the endonuclease, comprises
(iv) a fourth substitution, wherein the fourth substitution is selected from the group consisting of
- A124R, A124N, A124D, A124C, A124Q, A124E, A124G, A124H, A124I, A124L, A124K, A124M, A124F, A124P, A124T, A124W, A124Y, A124V, and A124S; preferably A124D, A124E, A124R, A124K, A124H, A124G, A124V, A124L, A1241, and A124P; preferably A124K, A124H and A124R; more preferably A124K, and A124R;
- A52R, A52N, A52D, A52C, A52Q, A52E, A52G, A52H, A52I, A52L, A52K, A52M, A52F, A52P, A52T, A52W, A52Y, A52V, and A52S; preferably A52R, A52K, A52H; more preferably A52R;
- G54A, G54R, G54N, G54D, G54C, G54Q, G54E, G54H, G54I, G54L, G54K, G54M, G54F, G54P, G54T, G54W, G54Y, G54V, and G54S; preferably G54D, G54E, G54R, G54K, G54H, G54A, G54V, G54L, G54I, and G54P; preferably G54K, G54H, and G54R; more preferably G54R;
- P73A, P73R, P73N, P73D, P73C, P73Q, P73E, P73G, P73H, P73I, P73L, P73K, P73M, P73F, P73T, P73W, P73Y, P73V, and P73S; preferably P73D, P73E, P73R, P73K, P73H, P73G, P73V, P73L, P73I, and P73A; more preferably P73K, P73H and P73R; most preferably P73K, and P73R;
- A74R, A74N, A74D, A74C, A74Q, A74E, A74G, A74H, A74I, A74L, A74K, A74M, A74F, A74P, A74T, A74W, A74Y, A74V, and A74S; preferably A74D, A74E, A74R, A74K, A74H, A74G, A74V, A74L, A74I, and A74P; more preferably A74K, A74H, and A74R; most preferably A74K, and A74R;
- G78A, G78R, G78N, G78D, G78C, G78Q, G78E, G78H, G78I, G78L, G78K, G78M, G78F, G78P, G78T, G78W, G78Y, G78V, and G78S; preferably G78R, G78K, and G78H; more preferably G78R;
- A81R, A81N, A81D, A81C, A81Q, A81E, A81G, A81H, A81I, A81L, A81K, A81M, A81F, A81P, A81T, A81W, A81Y, A81V, and A81S; preferably A81K, A81H, and A81R; more preferably A81K;
- P195A, P195R, P195N, P195D, P195C, P195Q, P195E, P195G, P195H, P195I, P195L, P195K, P195M, P195F, P195T, P195W, P195Y, P195V, and P195S; preferably P195K, P195H and P195R; most preferably P195R;
- T56A, T56R, T56N, T56D, T56C, T56Q, T56E, T56G, T56H, T56I, T56L, T56K, T56M, T56F, T56P, T56W, T56Y, T56V, and T56S; preferably T56P, T56D, T56R, T56K, T56S, T56G, T56A, T56V, T56I, T56F, T56L, and T56M; and even more preferably T56P, T56D, T56R, T56K, T56S, and T56G; most preferably T56R, T56K, T56S, and T56G;
- S53A, S53R, S53N, S53D, S53C, S53Q, S53E, S53G, S53H, S53I, S53L, S53K, S53M, S53F, S53P, S53T, S53W, S53Y, and S53V; preferably S53R, S53K, S53H, S53G, S53A, S53V, S53L, S53I, S53P, S53W, S53F, S53Y, and S53D; more preferably S53R, S53K, S53H, S53G, S53A, S53V, S53L, S53I and S53P; even more preferably S53R, S53K, S53H and S53A; yet more preferably S53K, and S53A; most preferably S53K;
- N58A, N58R, N58D, N58C, N58Q, N58E, N58G, N58H, N58I, N58L, N58K, N58M, N58F, N58P, N58T, N58W, N58Y, N58V, and N58S; preferably N58R, N58K, N58H, N58G, N58A, N58V, N58L, N58I, N58P, N58W, N58F, N58Y, and N58D; more preferably N58R, N58K, N58H, N58G, N58A, N58V, N58L, N58I, and N58P; more preferably N58K, N58R, N58H, and N58V; most preferably N58K, and N58R;
- N80A, N80R, N80D, N80C, N80Q, N80E, N80G, N80H, N80I, N80L, N80K, N80M, N80F, N80P, N80T, N80W, N80Y, N80V, and N80S; preferably N80R, N80K, N80H, N80G, N80A, N80V, N80L, N80I, N80P, N80W, N80F, N80Y, and N80D; preferably N80K, N80R, and N80H; more preferably N80H and N80R; most preferably N80H;
- N119A, N119R, N119D, N119C, N119Q, N119E, N119G, N119H, N119I, N119L, N119K, N119M, N119F, N119P, N119T, N119W, N119Y, N119V, and N119S; preferably N119R, N119K, and N119H; more preferably N119R; and
- Q120A, Q120R, Q120N, Q120D, Q120C, Q120E, Q120G, Q120H, Q120I, Q120L, Q120K, Q120M, Q120F, Q120P, Q120T, Q120W, Q120Y, Q120V, and Q120S; preferably Q120R, Q120K, Q120H, Q120G, Q120A, Q120V, Q120L, Q120I, Q120P, Q120W, Q120F, Q120Y, and Q120D; more preferably Q120K, Q120H, and Q120R; most preferably Q 120R.

In preferred embodiments of the nuclease according to the invention the nuclease; preferably the endonuclease, comprises an amino acid sequence, which is substituted compared to SEQ ID NO:1 in at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, at least thirty-one, at least thirty-two, at least thirty-three, at least thirty-four, at least thirty-five, at least thirty-six, at least thirty-seven, or at least thirty-eight positions selected from the group consisting of P51L, P51G, P51A, P51V, P51I, A52R, A52K, A52H, S53R, S53K, S53H, G54R, G54K, G54H, K55R, T56R, T56K, T56H, N58R, N58K, N58H, A72K, P73K, P73R, P73H, A74R, A74K, A74H, T77K, T77R, T77H, G78R, G78K, G78H, N80R, N80K, N80H, A81R, A81K, A81H, A82R, K84G, D86S, A94G, S116K, D117K, D117A, D117S, D117N, D117R, D117H, D117Y, N119R, N119K, N119H, Q120R, Q120K, Q120H, A124K, A124R, A124H, D128M, D128Y, D128K, D128S, D128N, D128F, D128G, D128R, Q129R, K132R, D135N, D135K, D135G, D135R, D135A, D135M, E151L, K162N, D191P, P195R, P195K, P195H, K196R, G197K, D199N, D199K, D199S, R204Q, I218F, A228R, and E239A.

Preferably, the nuclease; preferably the endonuclease, comprises an amino acid sequence, which is substituted compared to SEQ ID NO:1 in at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, at least thirty-one, at least thirty-two, at least thirty-three, at least thirty-four, at least thirty-five, at least thirty-six, at least thirty-seven, or at least thirty-eight positions selected from the group consisting of P51L, P51G, P51A, P51V, P51I, A52R, S53K, G54R, K55R, T56R, T56K, N58R, N58K, A72K, P73K, P73R, A74R, A74K, T77K, T77R, T77H, G78R, N80R, A81K, A82R, K84G, D86S, A94G, S116K, D117K, D117A, D117S, D117N, D117R, D117H, D117Y, N119R, Q120R, A124K, A124R, A124H, D128M, D128Y, D128K, D128S, D128N, D128F, D128G, D128R, Q129R, K132R, D135N, D135K, D135G, D135R, D135A, D135M, E151L, K162N, D191P, P195R, K196R, G197K, D199N, D199K, D199S, R204Q, I218F, A228R, and E239A.

Preferably, the nuclease; preferably the endonuclease, comprises an amino acid sequence corresponding to SEQ ID NO: 2, 3, 4, 6, 7, 8, 11, 12, 14, 15, 16, 17, 18, 19, 20, 22, 23, 24, 27, 28, 29, 30, 31, 32, 33, 34, 35, 36, 37, 38, 39, 40, 41, 42, 43, 44, 45, 46, 47, 49, 50, 51, 52, 55, 56, 57, 58, 59, 60, 61, 62, 63, 64, 65, 66, 67, 68, 69, 70, 71, 72, 73, 74, 75, 78, 79, 80, 81, 82, 83, 84, 85, 86, 87, 88, 89, 90, 91, 92, 93, 94, 95, 96, 97, 98, 107, 113, 114, 115, 116, 117, 118, 119, 120, 121, 122, 123, 124, 125, 126, 127, 128, 129, 131, 132, 133, 134, 135, 136, 137, 138, 139, 140, 141, 142, 143,145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, and 195; preferably 55, 57, 58, 59, 61, 64, 66, 69, 86, 128, 131, 132, 133, 134, 137, 138, 139, 140, 143, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, and 195.

Another preferred embodiment of the invention relates to a nuclease; preferably an endonuclease; more preferably a non-specific endonuclease, comprising an amino acid sequence with at least 65%; identity to SEQ ID NO:1; wherein the amino acid sequence is substituted compared to SEQ ID NO:1 in at least one amino acid position selected from the group consisting of E239, T56, K84, D191, T77, and N58, wherein in each case the native amino acid of the selected position is substituted by an amino acid selected from the group consisting of A, G, V, L, I, and P; preferably selected from the group consisting of A, G, V, L, and P; more preferably selected from the group consisting of A, G, L, and P; still more preferably selected from the group consisting of A, G, and P; yet more preferably selected from the group consisting of A, and G; and most preferably A.

In a preferred embodiment, the nuclease; preferably the endonuclease, comprises an amino acid sequence, which is substituted compared to SEQ ID NO:1
- in position E239; preferably wherein the substitution is selected from the group consisting of E239A, E239G, E239V, E239L, E239I, and E239P; preferably E239A;
- in position T56; preferably wherein the substitution is selected from the group consisting of T56A, T56G, T56V, T56L, T56I, and T56P; preferably T56P and T56G; more preferably T56P;
- in position K84; preferably wherein the substitution is selected from the group consisting of K84A, K84G, K84V, K84L, K84I, and K84P; preferably K84G;
- in position D191; preferably wherein the substitution is selected from the group consisting of D191A, D191G, D191V, D191L, D191I, and D191P; preferably D191P;
- in position T77; preferably wherein the substitution is selected from the group consisting of T77A, T77G, T77V, T77L, T77I, and T77P; preferably T77L;
in position N58; preferably wherein the substitution is selected from the group consisting of N58A, N58G, N58V, N58L, N58I, and N58P; preferably N58V.

In a preferred embodiment, the nuclease; preferably the endonuclease, comprises an amino acid sequence, which is substituted compared to SEQ ID NO:1 in one, two, three, four, five, six, seven, eight, nine, ten, eleven, twelve, thirteen, fourteen, fifteen, sixteen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, or thirty-seven additional positions selected from the group consisting of P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239; preferably selected from the group consisting of P51, K55, T56, N58, A74, T77, N80, A81, K84, D86, A94, S116, D117, A124, D128, K132, D135, E151, K162, D191, G197, R204, I218, A228, and E239; more preferably selected from the group consisting ofP51, K55, T56, N58, A74, T77, N80, K84, D86, A94, S116, D117, A124, D128, K132, D135, E151, K162, D191, R204, I218, A228, and E239.

In a preferred embodiment, the nuclease; preferably the endonuclease, comprises an amino acid sequence, which is substituted compared to SEQ ID NO:1, wherein the amino acid sequence comprises
(i) a substitution in a position selected from the group consisting of E239, T56, K84, D191, T77, and N58; wherein in each case the native amino acid of SEQ ID NO:1 has been substituted by an amino acid selected from the group consisting of A, G, V, L, I, and P; and additionally
(ii) a second substitution in another position selected from the group consisting of E239, T56, K84, D191, T77, and N58; wherein in each case the native amino acid of SEQ ID NO:1 has been substituted by an amino acid selected from the group consisting of G, P, A, V, L, I, M, C, F, Y, W, H, K, R, Q, N, E, D, S, and T.

In a preferred embodiment, the nuclease; preferably the endonuclease, wherein the (ii) second substitution is selected from the group consisting of
- E239A, E239R, E239N, E239D, E239C, E239Q, E239G, E239H, E239I, E239L, E239K, E239M, E239F, E239P, E239T, E239W, E239Y, E239V, and E239S; preferably E239G, E239A, E239V, E239L, E239I, E239P, E239W, E239F, E239Y, E239C, E239M, E239N, E239Q, E239S, E239T, E239H and E239K; preferably E239A, E239G, E239V, E239L, E239I, and E239P; more preferably E239A;
- T56A, T56R, T56N, T56D, T56C, T56Q, T56E, T56G, T56H, T56I, T56L, T56K, T56M, T56F, T56P, T56W, T56Y, T56V, and T56S; preferably T56P, T56D, T56R, T56K, T56S, T56G, T56A, T56V, T56I, T56F, T56L, and T56M; and even more preferably T56P, T56D, T56R, T56K, T56S, and T56G; most preferably T56R, T56K, T56S, and T56G;
- K84A, K84R, K84N, K84D, K84C, K84Q, K84E, K84G, K84H, K84I, K84L, K84M, K84F, K84P, K84T, K84W, K84Y, K84V, and K84S; preferably K84G, K84A, K84V, K84L, K84I, K84P, K84R, K84H, K84N, K84Q, K84S, K84T, K84C, and K84M; preferably K84G, K84A, K84V, K84L, K84I, and K84P; more preferably K84G;
- D191G, D191A, D191V, D191L, D191I, D191P, D191W, D191F, D191Y, D191C, D191M, D191S, D191Q, D191T, D191N, D191E, D191H, D191R and D191K; preferably D191G, D191A, D191V, D191L, D191I, D191P, D191C, D191M, D191S, D191Q, and D191T; more preferably D191G, D191A, D191V, D191L, D191I, and D191P; most preferably D191P;
- T77A, T77R, T77N, T77D, T77C, T77Q, T77E, T77G, T77H, T77I, T77L, T77K, T77M, T77F, T77P, T77W, T77Y, T77V, and T77S; preferably T77R, T77K, T77H, T77G, T77A, T77V, T77L, T77I, T77P, T77W, T77F, T77Y, and T77D; more preferably T77K, T77R, T77H, T77F, and T77L, and most preferably T77K, T77R, T77F, and T77L; and
- N58A, N58R, N58D, N58C, N58Q, N58E, N58G, N58H, N58I, N58L, N58K, N58M, N58F, N58P, N58T, N58W, N58Y, N58V, and N58S; preferably N58R, N58K, N58H, N58G, N58A, N58V, N58L, N58I, N58P, N58W, N58F, N58Y, and N58D; more preferably N58R, N58K, N58H, N58G, N58A, N58V, N58L, N58I, and N58P; more preferably N58K, N58R, and N58V; most preferably N58K, and N58R.

In a preferred embodiment, the nuclease; preferably the endonuclease, comprises an amino acid sequence wherein the amino acid sequence is substituted compared to SEQ ID NO:1
(i) in position P51; preferably wherein the substitution is selected from the group consisting of P51A, P51R, P51N, P51D, P51C, P51Q, P51E, P51G, P51H, P51I, P51L, P51K, P51M, P51F, P51T, P51W, P51Y, P51V, and P51S; preferably P51D, P51E, P51R, P51K, P51H, P51G, P51A, P51V, P51L, and P51I; more preferably P51L, P51R, P51G, P51D, P51A, P51V, P51I; even more preferably P51D, P51R, P51G, and P51L; and most preferably P51R, P51G, and P51L;
(ii) in position P51; preferably wherein the substitution is selected from the group consisting of P51A, P51R, P51N, P51D, P51C, P51Q, P51E, P51G, P51H, P51I, P51L, P51K, P51M, P51F, P51T, P51W, P51Y, P51V, and P51S; preferably P51D, P51E, P51R, P51K, P51H, P51G, P51A, P51V, P51L, and P51I; more preferably P51L, P51R, P51G, P51D, P51A, P51V, P51I; even more preferably P51D, P51R, P51G, and P51L; and most preferably P51R, P51G, and P51L;
(iii) in position A52; preferably wherein the substitution is selected from the group consisting of A52R, A52N, A52D, A52C, A52Q, A52E, A52G, A52H, A52I, A52L, A52K, A52M, A52F, A52P, A52T, A52W, A52Y, A52V, and A52S; preferably A52R, A52K, A52H; more preferably A52R;
(iv) in position S53; preferably wherein the substitution is selected from the group consisting of S53A, S53R, S53N, S53D, S53C, S53Q, S53E, S53G, S53H, S53I, S53L, S53K, S53M, S53F, S53P, S53T, S53W, S53Y, and S53V; preferably S53R, S53K, S53H, S53G, S53A, S53V, S53L, S53I, S53P, S53W, S53F, S53Y, and S53D; more preferably S53R, S53K, S53H, S53G, S53A, S53V, S53L, S53I and S53P; even more preferably S53K, and S53A; most preferably S53K;
(v) in position G54; preferably wherein the substitution is selected from the group consisting of G54A, G54R, G54N, G54D, G54C, G54Q, G54E, G54H, G54I, G54L, G54K, G54M, G54F, G54P, G54T, G54W, G54Y, G54V, and G54S; preferably G54D, G54E, G54R, G54K, G54H, G54A, G54V, G54L, G54I, and G54P; preferably G54K, G54H, and G54R; more preferably G54R;
(vi) in position K55; preferably wherein the substitution is selected from the group consisting of K55A, K55R, K55N, K55D, K55C, K55Q, K55E, K55G, K55H, K55I, K55L, K55M, K55F, K55P, K55T, K55W, K55Y, K55V, and K55S; preferably K55G, K55A, K55V, K55L, K55I, K55P, K55R, K55H, K55N, K55Q, K55S, K55T, K55C, and K55M; more preferably K55H and K55R; most preferably K55R;
(vii) in position A72; preferably wherein the substitution is selected from the group consisting of A72R, A72N, A72D, A72C, A72Q, A72E, A72G, A72H, A72I, A72L, A72K, A72M, A72F, A72P, A72T, A72W, A72Y, A72V, and A72S; preferably A72D, A72E, A72R, A72K, A72H, A72G, A72V, A72L, A72I, and A72P; more preferably A72K, A72H and A72R; most preferably A72K;
(viii) in position P73; preferably wherein the substitution is selected from the group consisting of P73A, P73R, P73N, P73D, P73C, P73Q, P73E, P73G, P73H, P73I, P73L, P73K, P73M, P73F, P73T, P73W, P73Y, P73V, and P73S; preferably P73D, P73E, P73R, P73K, P73H, P73G, P73V, P73L, P73I, and P73A; more preferably P73K, P73H and P73R; most preferably P73K, and P73R;
(ix) in position A74; preferably wherein the substitution is selected from the group consisting of A74R, A74N, A74D, A74C, A74Q, A74E, A74G, A74H, A74I, A74L, A74K, A74M, A74F, A74P, A74T, A74W, A74Y, A74V, and A74S; preferably A74D, A74E, A74R, A74K, A74H, A74G, A74V, A74L, A74I, and A74P; more preferably A74K, A74H, and A74R; most preferably A74K, and A74R;
(x) in position G78; preferably wherein the substitution is selected from the group consisting of G78A, G78R, G78N, G78D, G78C, G78Q, G78E, G78H, G78I, G78L, G78K, G78M, G78F, G78P, G78T, G78W, G78Y, G78V, and G78S; preferably G78R, G78K, and G78H; more preferably G78R;
(xi) in position N80; preferably wherein the substitution is selected from the group consisting of N80A, N80R, N80D, N80C, N80Q, N80E, N80G, N80H, N80I, N80L, N80K, N80M, N80F, N80P, N80T, N80W, N80Y, N80V, and N80S; preferably N80R, N80K, N80H, N80G, N80A, N80V, N80L, N80I, N80P, N80W, N80F, N80Y, and N80D; preferably N80K, N80R, and N80H; more preferably N80H and N80R; most preferably N80H;
(xii) in position A81; preferably wherein the substitution is selected from the group consisting of A81R, A81N, A81D, A81C, A81Q, A81E, A81G, A81H, A81I, A81L, A81K, A81M, A81F, A81P, A81T, A81W, A81Y, A81V, and A81S; preferably A81K, A81H, and A81R; more preferably A81K;
(xiii) in position A82; preferably wherein the substitution is selected from the group consisting of A82R, A82N, A82D, A82C, A82Q, A82E, A82G, A82H, A82I, A82L, A82K, A82M, A82F, A82P, A82T, A82W, A82Y, A82V, and A82S; preferably A82K, A82H, and A82R; more preferably A82R;
(xiv) in position D86; preferably wherein the substitution is selected from the group consisting of D86A, D86R, D86N, D86C, D86Q, D86E, D86G, D86H, D86I, D86L, D86K, D86M, D86F, D86P, D86T, D86W, D86Y, D86V, and D86S; D86G, D86A, D86V, D86L, D86I, D86P, D86C, D86M, D86S, D86T, and D86Q; preferably D86C, D86M, D86S, D86T, and D86Q; more preferably D86S;
(xv) in position A94; preferably wherein the substitution is selected from the group consisting of A94R, A94N, A94D, A94C, A94Q, A94E, A94G, A94H, A94I, A94L, A94K, A94M, A94F, A94P, A94T, A94W, A94Y, A94V, and A94S; preferably A94G, A94V, A94L, A94I, and A94P; more preferably A94G;
(xvi) in position S116; preferably wherein the substitution is selected from the group consisting of S116A, S116R, S116N, S116D, S116C, S116Q, S116E, S116G, S116H, S116I, S116L, S116K, S116M, S116F, S116P, S116T, S116W, S116Y, and S116V; preferably S116R, S116K, S116H, S116G, S116A, S116V, S116L, S116I, S116P, S116W, S116F, S116Y, and S116D; preferably S116K, S116H, and S116R; more preferably S116K;
(xvii) in position D117; preferably wherein the substitution is selected from the group consisting of D117G, D117A, D117V, D117L, D117I, D117P, D117W, D117F, D117Y, D117C, D117M, D117S, D117Q, D117T, D117N, D117E, D117H, D117R and D117K; preferably D117G, D117A, D117V, D117L, D117I, D117P, D117W, D117F, D117Y, D117C, D117M, D117S, D117Q, D117T, D117N, D117H, D117R and D117K; more preferably D117K, D117A, D117S, D117N, D117R, D117H, and D 117Y; even more preferably D117A, D117N, D117R, D117K, and D117S; yet more preferably D117A, D117N, D117K, and D117S; and most preferably D117A, D117N, and D117S;
(xviii) in position N119; preferably wherein the substitution is selected from the group consisting of N119A, N119R, N119D, N119C, N119Q, N119E, N119G, N119H, N119I, N119L, N119K, N119M, N119F, N119P, N119T, N119W, N119Y, N119V, and N119S; preferably N119R, N119K, and N119H; more preferably N119R;
(xix) in position Q120; preferably wherein the substitution is selected from the group consisting of Q120A, Q120R, Q120N, Q120D, Q120C, Q120E, Q120G, Q120H, Q120I, Q120L, Q120K, Q120M, Q120F, Q120P, Q120T, Q120W, Q120Y, Q120V, and Q120S; preferably Q120R, Q120K, Q120H, Q120G, Q120A, Q120V, Q120L, Q120I, Q120P, Q120W, Q120F, Q120Y, and Q120D; more preferably Q120K, Q120H, and Q120R; most preferably Q 120R;
(xx) in position A124; preferably wherein the substitution is selected from the group consisting of A124R, A124N, A124D, A124C, A124Q, A124E, A124G, A124H, A1241, A124L, A124K, A124M, A124F, A124P, A124T, A124W, A124Y, A124V, and A124S; preferably A124D, A124E, A124R, A124K, A124H, A124G, A124V, A124L, A124I, and A124P; preferably A124K, A124H and A124R; more preferably A124K, and A124R;
(xxi) in position D128; preferably wherein the substitution is selected from the group consisting of D128A, D128R, D128N, D128C, D128Q, D128E, D128G, D128H, D128I, D128L, D128K, D128M, D128F, D128P, D128T, D128W, D128Y, D128V, and D128S; preferably D128G, D128A, D128V, D128L, D128I, D128P, D128W, D128F, D128Y, D128C, D128M, D128N, D128Q, D128S, D128T, D128H, D128R and D128K; most preferably D128G, D128Y, D128F, D128M, D128N, D128S, D128R and D128K;
(xxii) in position Q129; preferably wherein the substitution is selected from the group consisting of Q129A, Q129R, Q129N, Q129D, Q129C, Q129E, Q129G, Q129H, Q129I, Q129L, Q129K, Q129M, Q129F, Q129P, Q129T, Q129W, Q129Y, Q129V, and Q129S; preferably Q129R, Q129K, Q129H, Q129G, Q129A, Q129V, Q129L, Q129I, Q129P, Q129W, Q129F, Q129Y, and Q129D; preferably Q129K, Q129H, and Q129R; more preferably Q129K, Q129R; still more preferably Q 129R;
(xxiii) in position K132; preferably wherein the substitution is selected from the group consisting of K132A, K132R, K132N, K132D, K132C, K132Q, K132E, K132G, K132H, K132I, K132L, K132M, K132F, K132P, K132T, K132W, K132Y, K132V, and K132S; preferably K132R, and K132H; most preferably K132R;
(xxiv) in position D135; preferably wherein the substitution is selected from the group consisting of D135A, D135R, D135N, D135C, D135Q, D135E, D135G, D135H, D135I, D135L, D135K, D135M, D135F, D135P, D135T, D135W, D135Y, D135V, and D135S; preferably D135G, D135A, D135V, D135L, D135I, D135P, D135W, D135F, D135Y, D135C, D135M, D135N, D135Q, D135S, D135T, D135R, D135H, and D135K; more preferably D135G, D135A, D135V, D135L, D135I, D135P, D135C, D135M, D135N, D135Q, D135S, D135T, D135R, D135H, and D135K; most preferably D135A, D135M, D135G, D135N, D135R and D135K;
(xxv) in position E151; preferably wherein the substitution is selected from the group consisting of E151A, E151R, E151N, E151D, E151C, E151Q, E151G, E151H, E151I, E151L, E151K, E151M, E151F, E151P, E151T, E151W, E151Y, E151V, and E151S; E151G, E151A, E151V, E151L, E151I, E151P, E151W, E151F, E151Y, E151C, E151M, E151N, E151Q, E151S, E151T, E151H and E151K; preferably E151A, E151G, E151V, E151L, E151I, and E151P; more preferably E151L;
(xxvi) in position K162; preferably wherein the substitution is selected from the group consisting of K162A, K162R, K162N, K162D, K162C, K162Q, K162E, K162G, K162H, K162I, K162L, K162M, K162F, K162P, K162T, K162W, K162Y, K162V, and K162S; preferably K162N, K162Q, K162S, K162T, K162C and K162M; most preferably K162N;
(xxvii) in position P195; preferably wherein the substitution is selected from the group consisting of P195A, P195R, P195N, P195D, P195C, P195Q, P195E, P195G, P195H, P195I, P195L, P195K, P195M, P195F, P195T, P195W, P195Y, P195V, and P195S; preferably P195K, P195H and P195R; most preferably P195R;
(xxviii) in position K196; preferably wherein the substitution is selected from the group consisting of K196A, K196R, K196N, K196D, K196C, K196Q, K196E, K196G, K196H, K196I, K196L, K196M, K196F, K196P, K196T, K196W, K196Y, K196V, and K196S; preferably K196G, K196A, K196V, K196L, K196I, K196P, K196R, K196H, K196N, K196Q, K196S, K196T, K196C, and K196M; more preferably K196R, and K196H; most preferably K196R;
(xxix) in position G197; preferably wherein the substitution is selected from the group consisting of G197A, G197R, G197N, G197D, G197C, G197Q, G197E, G197H, G197I, G197L, G197K, G197M, G197F, G197P, G197T, G197W, G197Y, G197V, and G197S; preferably G197D, G197E, G197R, G197K, G197H, G197A, G197V, G197L, G197I, and G197P; preferably G197K, G197H, and G197R; more preferably G197K;
(xxx) in position D199; preferably wherein the substitution is selected from the group consisting of D199G, D199A, D199V, D199L, D199I, D199P, D199W, D199F, D199Y, D199C, D199M, D199S, D199Q, D199T, D199N, D199E, D199H, D199R and D199K; preferably D199G, D199A, D199V, D199L, D199I, D199P, D199W, D199F, D199Y, D199C, D199M, D199N, D199S, D199Q, D199T, and D199K; preferably D199N, D199S, D199Q, D199T, D199C, D199M, D199H, and D199K; more preferably D199N, D199S, and D199K;
(xxxi) in position R204; preferably wherein the substitution is selected from the group consisting of R204A, R204N, R204D, R204C, R204Q, R204E, R204G, R204H, R204I, R204L, R204K, R204M, R204F, R204P, R204T, R204W, R204Y, R204V, and R204S; preferably R204G, R204A, R204V, R204L, R204I, R204P, R204K, R204H, R204N, R204Q, R204S, R204T, R204C, and R204M; more preferably R204Q, R204N, R204S, R204T, R204C and R204M; most preferably R204Q;
(xxxii) in position I218; preferably wherein the substitution is selected from the group consisting of 1218R, I218N, I218D, I218C, I218Q, I218E, I218G, I218H, I218A, I218L, I218K, I218M, I218F, I218P, I218T, I218W, I218Y, I218V, and I218S; preferably I218F, I218W and I218Y; more preferably I218F;
(xxxiii) in position A228; preferably wherein the substitution is selected from the group consisting of A228R, A228N, A228D, A228C, A228Q, A228E, A228G, A228H, A228I, A228L, A228K, A228M, A228F, A228P, A228T, A228W, A228Y, A228V, and A228S; preferably A228K, A228H and A228R; more preferably A228R.

In a preferred embodiment, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five substitution selected from the group consisting of the substitutions P51L, P51G, P51D, K55R, T56A, T56V, T56L, T56I, T56P, T56D, T56S, T56G, T56R, N58A, N58G, N58V, N58L, N58I, N58P, N58R, A74R, T77K, T77F, T77A, T77G, T77V, T77I, T77P, T77L, T77R, N80H, A81K, K84G, K84A, K84V; K84L, K84I, K84P, D86S, A94G, S116K, D117N, A124K, D128R, D128M, D128Y, D128F, K132R, D135A, D135G, D135N, D135M, D135R, E151L, K162N, D191A, D191G, D191V, D191L, D191I, D191P, G197K, R204Q, I218F, A228R, E239G, E239V, E239L, E239I, E239P, and E239A; preferably P51L, P51G, P51D, K55R, T56P, T56D, T56S, T56G, T56R, N58V, N58R, A74R, T77K, T77F, T77L, T77R, N80H, A81K, K84G, D86S, A94G, S116K, D117N, A124K, D128R, D128M, D128Y, D128F, K132R, D135A, D135G, D135N, D135M, D135R, E151L, K162N, D191P, G197K, R204Q, I218F, A228R, and E239A; more preferably P51L, P51G, P51D, K55R, T56P, T56G, N58V, A74R, T77L, N80H, A81K, K84G, D86S, A94G, S116K, D117N, A124K, D128R, D128M, D128Y, D128F, K132R, D135A, D135G, D135N, D135M, D135R, E151L, K162N, D191P, G197K, R204Q, I218F, A228R, and E239A.

In a preferred embodiment, the nuclease; preferably the endonuclease, comprises an amino acid sequence with at least 65%; identity to SEQ ID NO:1; and comprising
[1]: (i) a substitution in position T56, wherein the position is substituted by an amino acid selected from the group consisting of A, G, V, L, I, and P; preferably P; and (ii) a substitution in position K84, wherein the position is substituted by an amino acid selected from the group consisting of A, G, V, L, I, and P; preferably G;
[2]: (i) a substitution in position T56, wherein the position is substituted by an amino acid selected from the group consisting of A, G, V, L, I, and P; preferably P; (ii) a substitution in position K84, wherein the position is substituted by an amino acid selected from the group consisting of A, G, V, L, I, and P; preferably G; and (iii) a substitution in position P51, wherein the position is substituted by an amino acid selected from the group consisting of A, G, V, L, and I; preferably L;
[3]: (i) a substitution in position P51, wherein the position is substituted by an amino acid selected from the group consisting of A, G, V, L, and I; preferably L; and (ii) a substitution in position T56, wherein the position is substituted by an amino acid selected from the group consisting of A, G, V, L, I, and P; preferably P;
[4]: (i) a substitution in position P51, wherein the position is substituted by an amino acid selected from the group consisting of A, G, V, L, and I; preferably L; (ii) a substitution in position T56, wherein the position is substituted by an amino acid selected from the group consisting of A, G, V, L, I, and P; preferably P; and (iii) a substitution in position D135, wherein the position is substituted by an amino acid selected from the group consisting of N, T, Q, S, C, M, A, G, V, L, I, and P; preferably N, and A;
[5]: (i) a substitution in position E239, wherein the position is substituted by an amino acid selected from the group consisting of A, G, V, L, I, and P; preferably A; (ii) a substitution in position P51, wherein the position is substituted by an amino acid selected from the group consisting of A, G, V, L, and I; preferably L; (iii) a substitution in position T56, wherein the position is substituted by an amino acid selected from the group consisting of D, E, C, M, N, Q, S, A, G, V, L, I, and P; preferably P, G, D, and S; more preferably D.

In a preferred embodiment, the nuclease; preferably the endonuclease, comprises an amino acid sequence, wherein the amino acid sequence has an identity to SEQ ID NO:1 of at least 76%; yet more preferably at least 78%; even more preferably at least 80%; most preferably at least 84%, and in particular at least 87%.

Preferably, the amino acid sequence of the nuclease; preferably the endonuclease, according to the invention has an identity to SEQ ID NO:1 of at least 88%; preferably at least 89%; more preferably at least 90%; still more preferably at least 91%; yet more preferably at least 92%; even more preferably at least 93%; most preferably at least 94%, and in particular at least 95%.

In a preferred embodiment, the nuclease; preferably the endonuclease, comprises an amino acid sequence, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 4, 5, 8, 9, 10, 13, 19, 21, 22, 23, 24, 25, 26, 27, 28, 32, 34, 36, 37, 41, 44, 46, 48, 53, 54, 68, 72, 73, 74, 75, 76, 77, 81, 99, 100, 101, 102, 106, 107, 108, 109, 110, 111, 122, 123, 124, 128, 129, 130, 131, or 137.

In preferred embodiments of the nuclease; preferably the endonuclease, the nuclease is characterized by (A) an increased thermal stability in comparison to the wildtype nuclease of SEQ ID NO:1.

Another preferred embodiment of the invention relates to a nuclease; preferably an endonuclease, with at least 70% identity to SEQ ID NO:1, wherein the amino acid sequence is substituted compared to SEQ ID NO:1 in at least two amino acid position selected from the group consisting of P51, T56, K84, and N58, wherein in each case the native amino acid of the selected position is substituted by an amino acid selected from the group consisting of L, P, A, G, V, and I; preferably selected from the group consisting of L, P, I, G, and V; more preferably selected from the group consisting of L, P, I, and G; still more preferably selected from the group consisting of L, P, and G; yet more preferably selected from the group consisting of L, and P.

In a preferred embodiment, the amino acid sequence is substituted compared to SEQ ID NO:1 in position P51; preferably wherein the substitution is selected from the group consisting of P51G, P51A, P51V, P51I, and P51L; preferably P51G, and P51L; more preferably P51L.

In a preferred embodiment, the amino acid sequence is substituted compared to SEQ ID NO:1 in position T56; preferably wherein the substitution is selected from the group consisting of T56G, T56A, T56V, T56L, T56I, and T56P; preferably T56G, and T56P.

In a preferred embodiment, the amino acid sequence is substituted compared to SEQ ID NO:1 in position K84; preferably wherein the substitution is selected from the group consisting of K84G, K84A, K84V, K84L, K84I, and K84P; preferably K84G.

In a preferred embodiment, the amino acid sequence is substituted compared to SEQ ID NO:1 in position N58; preferably wherein the substitution is selected from the group consisting of N58G, N58A, N58V, N58L, N58I, and N58P; preferably N58V.

In a preferred embodiment, the amino acid sequence is substituted compared to SEQ ID NO:1 in one, two, three, four, five, six, seven, eight, nine, ten, eleven, twelve, thirteen, fourteen, fifteen, sixteen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, or thirty-seven additional positions selected from the group consisting of P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239; preferably P51, K55, T56, N58, P73, A74, T77, G78, A81, K84, D86, S116, D117, Q120, A124, D128, Q129, D135, K196, G197, D199, and E239; more preferably P51, T56, N58, T77, A81, K84, D86, S116, D117, Q120, A124, D128, Q129, D135, K196, and E239; still more preferably P51, T56, N58, T77, K84, D86, S116, and D135.

In a preferred embodiment, the nuclease; preferably the endonuclease, comprises
[1]: (i) two substitutions in a position selected from the group consisting of P51, T56, K84, and N58; wherein in each case the native amino acid of SEQ ID NO:1 has been substituted by an amino acid selected from the group consisting of L, P, A, G, V, and I; and additionally
[2] : (ii) a third substitution in another position selected from the group consisting of P51, T56, K84, and N58; wherein in each case the native amino acid of SEQ ID NO:1 has been substituted by an amino acid selected from the group consisting of G, P, A, V, L, I, M, C, F, Y, W, H, K, R, Q, N, E, D, S, and T.

In a preferred embodiment, the (ii) third substitution is selected from the group consisting of
- P51L, P51D, P51E, P51R, P51K, P51H, P51G, P51A, P51V, P51I, P51F, P51W, P51Y, P51N, P51T, P51S, P51Q, P51C, and P51M; preferably P51G, P51A, P51V, P51I, and P51L; more preferably P51G, and P51L; still more preferably P51L;
- T56P, T56G, T56D, T56E, T56R, T56K, T56H, T56A, T56V, T56L, T56I, T56F, T56W, T56Y, T56N, T56S, T56Q, T56C, and T56M; preferably T56R, T56K, T56H, T56G, T56A, T56V, T56L, T56I, T56P, and T56D; more preferably T56G, T56A, T56V, T56L, T56I, T56P, and T56D; still more preferably T56G, T56P, and T56D;
- K84G, K84D, K84E, K84R, K84H, K84A, K84V, K84L, K84I, K84P, K84F, K84W, K84Y, K84N, K84T, K84S, K84Q, K84C, and K84M; preferably K84G, K84A, K84V, K84L, K84I, and K84P; more preferably K84G; and
- N58V, N58D, N58E, N58R, N58K, N58H, N58G, N58A, N58L, N58I, N58P, N58F, N58W, N58Y, N58T, N58S, N58Q, N58C, and N58M; N58R, N58K, N58H, N58G, N58A, N58V, N58L, N58I, and N58P; more preferably N58R, N58K, and N58V; still more preferably N58R, and N58V.

In a preferred embodiment, the amino acid sequence is substituted compared to SEQ ID NO: 1 in position A52; preferably wherein the substitution is selected from the group consisting of A52R, A52N, A52D, A52C, A52Q, A52E, A52G, A52H, A52I, A52L, A52K, A52M, A52F, A52P, A52T, A52W, A52Y, A52V, and A52S; preferably A52R, A52K, A52H; more preferably A52R.

In a preferred embodiment, the amino acid sequence is substituted compared to SEQ ID NO: 1 in position S53; preferably wherein the substitution is selected from the group consisting of S53A, S53R, S53N, S53D, S53C, S53Q, S53E, S53G, S53H, S53I, S53L, S53K, S53M, S53F, S53P, S53T, S53W, S53Y, and S53V; preferably S53R, S53K, S53H, S53G, S53A, S53V, S53L, S53I, S53P, S53W, S53F, S53Y, and S53D; more preferably S53R, S53K, S53H, S53G, S53A, S53V, S53L, S53I and S53P; even more preferably S53K, and S53A; most preferably S53K.

In a preferred embodiment, the amino acid sequence is substituted compared to SEQ ID NO: 1 in position G54; preferably wherein the substitution is selected from the group consisting of G54A, G54R, G54N, G54D, G54C, G54Q, G54E, G54H, G54I, G54L, G54K, G54M, G54F, G54P, G54T, G54W, G54Y, G54V, and G54S; preferably G54D, G54E, G54R, G54K, G54H, G54A, G54V, G54L, G54I, and G54P; preferably G54K, G54H, and G54R; more preferably G54R.

In a preferred embodiment, the amino acid sequence is substituted compared to SEQ ID NO: 1 in position K55; preferably wherein the substitution is selected from the group consisting of K55A, K55R, K55N, K55D, K55C, K55Q, K55E, K55G, K55H, K55I, K55L, K55M, K55F, K55P, K55T, K55W, K55Y, K55V, and K55S; preferably K55G, K55A, K55V, K55L, K55I, K55P, K55R, K55H, K55N, K55Q, K55S, K55T, K55C, and K55M; more preferably K55H and K55R; most preferably K55R.

In a preferred embodiment, the amino acid sequence is substituted compared to SEQ ID NO: 1 in position A72; preferably wherein the substitution is selected from the group consisting of A72R, A72N, A72D, A72C, A72Q, A72E, A72G, A72H, A72I, A72L, A72K, A72M, A72F, A72P, A72T, A72W, A72Y, A72V, and A72S; preferably A72D, A72E, A72R, A72K, A72H, A72G, A72V, A72L, A72I, and A72P; more preferably A72K, A72H and A72R; most preferably A72K.

In a preferred embodiment, the amino acid sequence is substituted compared to SEQ ID NO: 1 in position P73; preferably wherein the substitution is selected from the group consisting of P73A, P73R, P73N, P73D, P73C, P73Q, P73E, P73G, P73H, P73I, P73L, P73K, P73M, P73F, P73T, P73W, P73Y, P73V, and P73S; preferably P73D, P73E, P73R, P73K, P73H, P73G, P73V, P73L, P73I, and P73A; more preferably P73K, P73H and P73R; most preferably P73K, and P73R.

In a preferred embodiment, the amino acid sequence is substituted compared to SEQ ID NO: 1 in position A74; preferably wherein the substitution is selected from the group consisting of A74R, A74N, A74D, A74C, A74Q, A74E, A74G, A74H, A74I, A74L, A74K, A74M, A74F, A74P, A74T, A74W, A74Y, A74V, and A74S; preferably A74D, A74E, A74R, A74K, A74H, A74G, A74V, A74L, A74I, and A74P; more preferably A74K, A74H, and A74R; most preferably A74K, and A74R.

In a preferred embodiment, the amino acid sequence is substituted compared to SEQ ID NO: 1 in position T77; preferably wherein the substitution is selected from the group consisting of T77R, T77N, T77D, T77C, T77Q, T77E, T77G, T77H, T77I, T77L, T77K, T77M, T77F, T77P, T77A T77W, T77Y, T77V, and T77S; preferably T77R, T77K, T77H, T77G, T77A, T77V, T77L, T77I, T77P, T77W, T77F, T77Y, and T77D; preferably T77L, T77F, T77K, T77R, and T77H; more preferably T77K, T77R, and T77H; still more preferably T77R.

In a preferred embodiment, the amino acid sequence is substituted compared to SEQ ID NO: 1 in position G78; preferably wherein the substitution is selected from the group consisting of G78A, G78R, G78N, G78D, G78C, G78Q, G78E, G78H, G78I, G78L, G78K, G78M, G78F, G78P, G78T, G78W, G78Y, G78V, and G78S; preferably G78R, G78K, and G78H; more preferably G78R.

In a preferred embodiment, the amino acid sequence is substituted compared to SEQ ID NO: 1 in position N80; preferably wherein the substitution is selected from the group consisting of N80A, N80R, N80D, N80C, N80Q, N80E, N80G, N80H, N80I, N80L, N80K, N80M, N80F, N80P, N80T, N80W, N80Y, N80V, and N80S; preferably N80R, N80K, N80H, N80G, N80A, N80V, N80L, N80I, N80P, N80W, N80F, N80Y, and N80D; preferably N80K, N80R, and N80H; more preferably N80H and N80R; most preferably N80H.

In a preferred embodiment, the amino acid sequence is substituted compared to SEQ ID NO: 1 in position A81; preferably wherein the substitution is selected from the group consisting of A81R, A81N, A81D, A81C, A81Q, A81E, A81G, A81H, A81I, A81L, A81K, A81M, A81F, A81P, A81T, A81W, A81Y, A81V, and A81S; preferably A81K, A81H, and A81R; more preferably A81K.

In a preferred embodiment, the amino acid sequence is substituted compared to SEQ ID NO: 1 in position A82; preferably wherein the substitution is selected from the group consisting of A82R, A82N, A82D, A82C, A82Q, A82E, A82G, A82H, A82I, A82L, A82K, A82M, A82F, A82P, A82T, A82W, A82Y, A82V, and A82S; preferably A82K, A82H, and A82R; more preferably A82R.

In a preferred embodiment, the amino acid sequence is substituted compared to SEQ ID NO: 1 in position D86; preferably wherein the substitution is selected from the group consisting of D86A, D86R, D86N, D86C, D86Q, D86E, D86G, D86H, D86I, D86L, D86K, D86M, D86F, D86P, D86T, D86W, D86Y, D86V, and D86S; D86G, D86A, D86V, D86L, D86I, D86P, D86C, D86M, D86S, D86T, and D86Q; preferably D86C, D86M, D86S, D86T, and D86Q; more preferably D86S.

In a preferred embodiment, the amino acid sequence is substituted compared to SEQ ID NO: 1 in position A94; preferably wherein the substitution is selected from the group consisting of A94R, A94N, A94D, A94C, A94Q, A94E, A94G, A94H, A94I, A94L, A94K, A94M, A94F, A94P, A94T, A94W, A94Y, A94V, and A94S; preferably A94G, A94V, A94L, A94I, and A94P; more preferably A94G.

In a preferred embodiment, the amino acid sequence is substituted compared to SEQ ID NO:1 in position S116; preferably wherein the substitution is selected from the group consisting of S116A, S116R, S116N, S116D, S116C, S116Q, S116E, S116G, S116H, S116I, S116L, S116K, S116M, S116F, S116P, S116T, S116W, S116Y, and S116V; preferably S116R, S116K, S116H, S116G, S116A, S116V, S116L, S116I, S116P, S116W, S116F, S116Y, and S116D; preferably S116K, S116H, and S116R; more preferably S116K.

In a preferred embodiment, the amino acid sequence is substituted compared to SEQ ID NO:1 in position D117; preferably wherein the substitution is selected from the group consisting of D117G, D117A, D117V, D117L, D117I, D117P, D117W, D117F, D117Y, D117C, D117M, D117S, D117Q, D117T, D117N, D117E, D117H, D117R and D117K; preferably D117G, D117A, D117V, D117L, D117I, D117P, D117W, D117F, D117Y, D117C, D117M, D117S, D117Q, D117T, D117N, D117H, D117R and D117K; more preferably D117K, D117R, D117H, D117W, D117F and D117Y; still more preferably D117R, D117K, D 117H, and D 117Y; yet more preferably D117R, D117K, and D 117H; even more preferably D117K.

In a preferred embodiment, the amino acid sequence is substituted compared to SEQ ID NO:1 in position N119; preferably wherein the substitution is selected from the group consisting of N119A, N119R, N119D, N119C, N119Q, N119E, N119G, N119H, N119I, N119L, N119K, N119M, N119F, N119P, N119T, N119W, N119Y, N119V, and N119S; preferably N119R, N119K, and N119H; more preferably N119R.

In a preferred embodiment, the amino acid sequence is substituted compared to SEQ ID NO:1 in position Q120; preferably wherein the substitution is selected from the group consisting of Q120A, Q120R, Q120N, Q120D, Q120C, Q120E, Q120G, Q120H, Q120I, Q120L, Q120K, Q120M, Q120F, Q120P, Q120T, Q120W, Q120Y, Q120V, and Q120S; preferably Q120R, Q120K, Q120H, Q120G, Q120A, Q120V, Q120L, Q120I, Q120P, Q120W, Q120F, Q120Y, and Q120D; more preferably Q120K, Q120H, and Q120R; most preferably Q 120R.

In a preferred embodiment, the amino acid sequence is substituted compared to SEQ ID NO:1 in position A124; preferably wherein the substitution is selected from the group consisting of A124R, A124N, A124D, A124C, A124Q, A124E, A124G, A124H, A1241, A124L, A124K, A124M, A124F, A124P, A124T, A124W, A124Y, A124V, and A124S; preferably A124D, A124E, A124R, A124K, A124H, A124G, A124V, A124L, A1241, and A124P; preferably A124K, A124H and A124R; more preferably A124K.

In a preferred embodiment, the amino acid sequence is substituted compared to SEQ ID NO:1 in position D128; preferably wherein the substitution is selected from the group consisting of D128A, D128R, D128N, D128C, D128Q, D128E, D128G, D128H, D128I, D128L, D128K, D128M, D128F, D128P, D128T, D128W, D128Y, D128V, and D128S; preferably D128G, D128A, D128V, D128L, D128I, D128P, D128W, D128F, D128Y, D128C, D128M, D128N, D128Q, D128S, D128T, D128H, D128R and D128K; most preferably D128G, D128Y, D128F, D128M, D128N, D128S, D128R and D128K.

In a preferred embodiment, the amino acid sequence is substituted compared to SEQ ID NO: 1 in position Q129; preferably wherein the substitution is selected from the group consisting of Q129A, Q129R, Q129N, Q129D, Q129C, Q129E, Q129G, Q129H, Q129I, Q129L, Q129K, Q129M, Q129F, Q129P, Q129T, Q129W, Q129Y, Q129V, and Q129S; preferably Q129R, Q129K, Q129H, Q129G, Q129A, Q129V, Q129L, Q129I, Q129P, Q129W, Q129F, Q129Y, and Q129D; preferably Q129K, Q129H, and Q129R; more preferably Q129K, Q129R; still more preferably Q129R.

In a preferred embodiment, the amino acid sequence is substituted compared to SEQ ID NO: 1 in position K132; preferably wherein the substitution is selected from the group consisting of K132A, K132R, K132N, K132D, K132C, K132Q, K132E, K132G, K132H, K132I, K132L, K132M, K132F, K132P, K132T, K132W, K132Y, K132V, and K132S; preferably K132R, and K132H; most preferably K132R.

In a preferred embodiment, the amino acid sequence is substituted compared to SEQ ID NO: 1 in position D135; preferably wherein the substitution is selected from the group consisting of D135A, D135R, D135N, D135C, D135Q, D135E, D135G, D135H, D135I, D135L, D135K, D135M, D135F, D135P, D135T, D135W, D135Y, D135V, and D135S; preferably D135G, D135A, D135V, D135L, D135I, D135P, D135C, D135M, D135N, D135Q, D135S, D135T, D135R, D135H, and D135K; more preferably D135G, D135A, D135V, D135L, D135I, D135P, D135C, D135M, D135N, D135Q, D135S, and D135T; still more preferably D135A, and D135N.

In a preferred embodiment, the amino acid sequence is substituted compared to SEQ ID NO: 1 in position E151; preferably wherein the substitution is selected from the group consisting of E151A, E151R, E151N, E151D, E151C, E151Q, E151G, E151H, E151I, E151L, E151K, E151M, E151F, E151P, E151T, E151W, E151Y, E151V, and E151S; E151G, E151A, E151V, E151L, E151I, E151P, E151W, E151F, E151Y, E151C, E151M, E151N, E151Q, E151S, E151T, E151H and E151K; preferably E151A, E151G, E151V, E151L, E151I, and E151P; more preferably E151L.

In a preferred embodiment, the amino acid sequence is substituted compared to SEQ ID NO: 1 in position K162; preferably wherein the substitution is selected from the group consisting of K162A, K162R, K162N, K162D, K162C, K162Q, K162E, K162G, K162H, K162I, K162L, K162M, K162F, K162P, K162T, K162W, K162Y, K162V, and K162S; preferably K162N, K162Q, K162S, K162T, K162C and K162M; most preferably K162N.

In a preferred embodiment, the amino acid sequence is substituted compared to SEQ ID NO: 1 in position D191, wherein the substitution is selected from the group consisting of D191G, D191A, D191V, D191L, D191I, D191P, D191W, D191F, D191Y, D191C, D191M, D191S, D191Q, D191T, D191N, D191E, D191H, D191R and D191K; preferably D191G, D191A, D191V, D191L, D191I, D191P, D191C, D191M, D191S, D191Q, and D191T; more preferably D191G, D191A, D191V, D191L, D191I, and D191P; most preferably D191P.

In a preferred embodiment, the amino acid sequence is substituted compared to SEQ ID NO: 1 in position P195; preferably wherein the substitution is selected from the group consisting of P195A, P195R, P195N, P195D, P195C, P195Q, P195E, P195G, P195H, P195I, P195L, P195K, P195M, P195F, P195T, P195W, P195Y, P195V, and P195S; preferably P195K, P195H and P195R; most preferably P195R.

In a preferred embodiment, the amino acid sequence is substituted compared to SEQ ID NO: 1 in position K196; preferably wherein the substitution is selected from the group consisting of K196A, K196R, K196N, K196D, K196C, K196Q, K196E, K196G, K196H, K196I, K196L, K196M, K196F, K196P, K196T, K196W, K196Y, K196V, and K196S; preferably K196G, K196A, K196V, K196L, K196I, K196P, K196R, K196H, K196N, K196Q, K196S, K196T, K196C, and K196M; more preferably K196R, and K196H; most preferably K196R.

In a preferred embodiment, the amino acid sequence is substituted compared to SEQ ID NO: 1 in position G197; preferably wherein the substitution is selected from the group consisting of G197A, G197R, G197N, G197D, G197C, G197Q, G197E, G197H, G197I, G197L, G197K, G197M, G197F, G197P, G197T, G197W, G197Y, G197V, and G197S; preferably G197D, G197E, G197R, G197K, G197H, G197A, G197V, G197L, G197I, and G197P; preferably G197K, G197H, and G197R; more preferably G197K.

In a preferred embodiment, the amino acid sequence is substituted compared to SEQ ID NO: 1 in position D199; preferably wherein the substitution is selected from the group consisting of D199G, D199A, D199V, D199L, D199I, D199P, D199W, D199F, D199Y, D199C, D199M, D199S, D199Q, D199T, D199N, D199E, D199H, D199R and D199K; preferably D199G, D199A, D199V, D199L, D199I, D199P, D199W, D199F, D199Y, D199C, D199M, D199N, D199S, D199Q, D199T, and D199K; preferably D199N, D199S, D199Q, D199T, D199C, D199M, D199H, and D199K; more preferably D199N, D199S, and D199K.

In a preferred embodiment, the amino acid sequence is substituted compared to SEQ ID NO: 1 in position R204; preferably wherein the substitution is selected from the group consisting of R204A, R204N, R204D, R204C, R204Q, R204E, R204G, R204H, R204I, R204L, R204K, R204M, R204F, R204P, R204T, R204W, R204Y, R204V, and R204S; preferably R204G, R204A, R204V, R204L, R204I, R204P, R204K, R204H, R204N, R204Q, R204S, R204T, R204C, and R204M; more preferably R204Q, R204N, R204S, R204T, R204C and R204M; most preferably R204Q.

In a preferred embodiment, the amino acid sequence is substituted compared to SEQ ID NO: 1 in position I218; preferably wherein the substitution is selected from the group consisting of I218R, I218N, I218D, I218C, I218Q, I218E, I218G, I218H, I218A, I218L, I218K, I218M, I218F, I218P, I218T, I218W, I218Y, I218V, and I218S; preferably I218F, I218W and I218Y; more preferably I218F.

In a preferred embodiment, the amino acid sequence is substituted compared to SEQ ID NO: 1 in position A228; preferably wherein the substitution is selected from the group consisting of A228R, A228N, A228D, A228C, A228Q, A228E, A228G, A228H, A228I, A228L, A228K, A228M, A228F, A228P, A228T, A228W, A228Y, A228V, and A228S; preferably A228K, A228H and A228R; more preferably A228R.

In a preferred embodiment, the amino acid sequence is substituted compared to SEQ ID NO: 1 in positionE239, wherein the substitution is selected from the group consisting of E239A, E239R, E239N, E239D, E239C, E239Q, E239G, E239H, E239I, E239L, E239K, E239M, E239F, E239P, E239T, E239W, E239Y, E239V, and E239S; preferably E239G, E239A, E239V, E239L, E239I, E239P, E239W, E239F, E239Y, E239C, E239M, E239N, E239Q, E239S, E239T, E239H and E239K; preferably E239A, E239G, E239V, E239L, E239I, and E239P; more preferably E239A.

In a preferred embodiment, the amino acid sequence is substituted compared to SEQ ID NO: 1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions selected from the group consisting of the positions P51, K55, T56, N58, P73, A74, T77, G78, A81, K84, D86, S116, D117, Q120, A124, D128, Q129, D135, K196, G197, D199, and E239; preferably P51, T56, N58, T77, A81, K84, D86, S116, D117, Q120, A124, D128, Q129, D135, K196, and E239; more preferably P51, T56, N58, T77, K84, D86, S116, and D135, and exhibits an increased Tlow activity in comparison to the Tlow activity of SEQ ID NO: 1, wherein,
(i) when the amino acid substitution comprises a substitution at amino acid position P51, then the amino acid substitution at position P51 is selected from the group consisting of the substitutions D, E, R, K, H, G, A, V, L, and I; preferably G, A, V, I, and L; more preferably G, and L; still more preferably L;
(ii)when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
(iii)when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably R, K, H, G, A, V, L, I, P, and D; more preferably G, A, V, L, I, P, and D; still more preferably G, P, and D;
(iv) when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably R, K, and V; still more preferably R, and V;
(v) when the amino acid substitution comprises a substitution at amino acid position P73, then the amino acid substitution at position P73 is selected from the group consisting of the substitutions D, E, R, K, H, G, V, L, I, and A; preferably K, H and R; more preferably K, and R;
(vi) when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
(vii)when the amino acid substitution comprises a substitution at amino acid position T77, then the amino acid substitution at position T77 is selected from the group consisting of the substitutions R, K, H, G, A, V, L, I, P, W, F, Y, and D; preferably L, F, K, R, and H; more preferably K, R, and H; still more preferably R;
(viii) when the amino acid substitution comprises a substitution at amino acid position G78, then the amino acid substitution at position G78 is selected from the group consisting of the substitutions R, K, H, A, V, L, I, P, W, F, Y, N, Q, S, T, C, M and D; preferably K, R, and H; more preferably R;
(ix) when the amino acid substitution comprises a substitution at amino acid position A81, then the amino acid substitution at position A81 is selected from the group consisting of the substitutions R, K, and H; preferably K;
(x) when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
(xi) when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
(xii) when the amino acid substitution comprises a substitution at amino acid position S116, then the amino acid substitution at position S 116 is selected from the group consisting of the substitutions R, K, H, G, A, V, L, I, P, W, F, Y, and D; preferably K, H, and R; more preferably K;
(xiii) when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably G, V, L, I, P, W, F, Y, C, M, S, N, Q, H, R, and K; more preferably Y, W, F, K, R, and H; still more preferably Y, K, R, and H; yet preferably K, R, and H; even more preferably K;
(xiv) when the amino acid substitution comprises a substitution at amino acid position Q120, then the amino acid substitution at position Q120 is selected from the group consisting of the substitutions R, K, H, G, A, V, L, I, P, W, F, Y, and D; preferably K, H, and R; more preferably R;
(xv) when the amino acid substitution comprises a substitution at amino acid position A124, then the amino acid substitution at position A124 is selected from the group consisting of the substitutions D, E, R, K, H, G, V, L, I, and P; preferably K, H and R; more preferably K;
(xvi) when the amino acid substitution comprises a substitution at amino acid position D128, then the amino acid substitution at position D 128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, R, and K; more preferably G, Y, F, M, N, S, R, and K;
(xvii) when the amino acid substitution comprises a substitution at amino acid position Q129, then the amino acid substitution at position Q 129 is selected from the group consisting of the substitutions R, K, H, G, A, V, L, I, P, W, F, Y, and D; preferably K, H, and R; more preferably R;
(xviii) when the amino acid substitution comprises a substitution at amino acid position D 135, then the amino acid substitution at position D 135 is selected from the group consisting of the substitutions G, A, V, L, I, P, W, F, Y, C, M, N, Q, S, T, H, R, and K; preferably R, K, G, A, V, L, I, P, C, M, N, Q, S, and T; more preferably G, A, V, L, I, P, C, M, N, Q, S, and T; still more preferably A, and N;
(xix) when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
(xx) when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K;
(xxi) when the amino acid substitution comprises a substitution at amino acid position D 199, then the amino acid substitution at position D 199 is selected from the group consisting of the substitutions G, A, V, L, I, P, W, F, Y, C, M, Q, T, N, S, H, R, and K; preferably G, A, V, L, I, P, W, F, Y, C, M, Q, T, N, S, H, and K; more preferably S, Q, T, N, C, M, H, and K; still more preferably S, N, and K; or
(xxii) when the amino acid substitution comprises a substitution at amino acid position E239, then the amino acid substitution at position E239 is selected from the group consisting of the substitutions G, A, V, L, I, P, W, F, Y, C, M, Q, T, N, S, H, R, K, and D; preferably G, A, V, L, I, and P; more preferably A.

In a preferred embodiment, the amino acid sequence is substituted compared to SEQ ID NO: 1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two substitutions selected from the group consisting of the substitutions P51L, P51D, P51G, P51R, K55R, T56M, T56L, T56S, T56A, T56V, T56I, T56G, T56D, T56P, T56R, T56K, T56F, N58V, N58K, N58R, P73K, P73R, A74K, A74R, T77K, T77L, T77R, T77F, G78R, K84G, D86S, S116K, D117K, D117H, D117Y, D117R, Q120R, A124K, D128Y, D128M, D128N, D128K, D128F, D128R, D128S, D128G, Q129R, D135A, D135K, D135R, D135N, D135M, D135G, K196R, G197K, D199K, D199S, D199N, and E239A; preferably P51L, T56A, T56V, T56I, T56M, T56L, T56F, T56D, T56P, T56G, T56K, T56S, N58V, N58R, T77K, T77R, K84G, D86S, S116K, D117H, D117R, D117Y, Q120R, A124K, D128G, D128N, D128S, Q129R, D135A, D135M, D135N, K196R, and E239A; more preferably P51L, T56D, T56P, T56G, N58V, N58R, T77R, K84G, D86S, S116K, D135A, and D135N.

In a preferred embodiment, the nuclease; preferably the endonuclease, comprising an amino acid sequence with at least 70%; identity to SEQ ID NO:1; and comprising
(i) a substitution in position P51, wherein the position is substituted by an amino acid selected from the group consisting of A, G, V, L, and I; preferably L; and
(ii) a substitution in position T56, wherein the position is substituted by an amino acid selected from the group consisting of A, G, V, L, I, and P; preferably G, A, L, I, and P; more preferably G, and P; still more preferably P.

In a preferred embodiment, the nuclease; preferably the endonuclease, comprising an amino acid sequence with at least 70%; identity to SEQ ID NO:1; and comprising
(i) a substitution in position T56, wherein the position is substituted by an amino acid selected from the group consisting of A, G, V, L, I, and P; preferably G, A, L, I, and P; more preferably G, and P; still more preferably P; and
(ii) a substitution in position K84, wherein the position is substituted by an amino acid selected from the group consisting of A, G, V, L, I, and P; preferably G.

In a preferred embodiment, the nuclease; preferably the endonuclease, comprising an amino acid sequence with at least 70%; identity to SEQ ID NO:1; and comprising
(i) a substitution in position T56, wherein the position is substituted by an amino acid selected from the group consisting of A, G, V, L, I, and P; preferably G, A, L, I, and P; more preferably G, and P; still more preferably P;
(ii) a substitution in position K84, wherein the position is substituted by an amino acid selected from the group consisting of A, G, V, L, I, and P; preferably G; and
(iii) a substitution in position P51, wherein the position is substituted by an amino acid selected from the group consisting of A, G, V, L, and I; preferably L.

In a preferred embodiment, the nuclease; preferably the endonuclease, comprising an amino acid sequence with at least 70%; identity to SEQ ID NO: 1; and comprising
(i) a substitution in position P51, wherein the position is substituted by an amino acid selected from the group consisting of A, G, V, L, and I; preferably L;
(ii) a substitution in position T56, wherein the position is substituted by an amino acid selected from the group consisting of A, G, V, L, I, and P; preferably G, A, L, I, and P; more preferably G, and P; still more preferably P; and
(iii) a substitution in position D135, wherein the position is substituted by an amino acid selected from the group consisting of N, T, Q, S, C, M, A, G, V, L, I, and P; preferably N, and A.

In a preferred embodiment, the nuclease; preferably the endonuclease, comprising an amino acid sequence with at least 70%; identity to SEQ ID NO: 1; and comprising
(i) a substitution in position P51, wherein the position is substituted by an amino acid selected from the group consisting of A, G, V, L, and I; preferably L;
(ii) a substitution in position T56, wherein the position is substituted by an amino acid selected from the group consisting of A, G, V, L, I, and P; preferably G, A, L, I, and P; more preferably G, and P; still more preferably G; and
(iii) a substitution in position A81, wherein the position is substituted by an amino acid selected from the group consisting of R, K, and H; preferably R, and K; more preferably K.

In a preferred embodiment, the nuclease; preferably the endonuclease, comprising an amino acid sequence with at least 70%; identity to SEQ ID NO: 1; and comprising
(i) a substitution in position P51, wherein the position is substituted by an amino acid selected from the group consisting of A, G, V, L, and I; preferably L;
(ii) a substitution in position T56, wherein the position is substituted by an amino acid selected from the group consisting of D, E, C, M, N, Q, S, A, G, V, L, I, and P; preferably P, G, D, and S; more preferably D;

In a preferred embodiment, the nuclease; preferably the endonuclease, comprising an amino acid sequence with at least 70%; identity to SEQ ID NO: 1; and comprising
(i) a substitution in position P51, wherein the position is substituted by an amino acid selected from the group consisting of A, G, V, L, and I; preferably L;
(ii) a substitution in position T56, wherein the position is substituted by an amino acid selected from the group consisting of D, E, C, M, N, Q, S, A, G, V, L, I, and P; preferably P, G, D, and S; more preferably D;
(iii) a substitution in position E239, wherein the position is substituted by an amino acid selected from the group consisting of A, G, V, L, I, and P; preferably A;

In a preferred embodiment, the nuclease; preferably the endonuclease, wherein the amino acid sequence has an identity to SEQ ID NO:1 of at least 76%; yet more preferably at least 78%; even more preferably at least 80%; most preferably at least 84%, and in particular at least 87%.

In a preferred embodiment, the nuclease; preferably the endonuclease, wherein the amino acid sequence has an identity to SEQ ID NO:1 of at least 88%; preferably at least 89%; more preferably at least 90%; still more preferably at least 91%; yet more preferably at least 92%; even more preferably at least 93%; most preferably at least 94%, and in particular at least 95%.

In a preferred embodiment, the nuclease; preferably the endonuclease, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 8, 9, 10, 11, 12, 13, 14, 15, 17, 18, 19, 20, 21, 22, 23, 24, 26, 27, 28, 29, 36, 37, 38, 39, 40, 41, 42, 43, 44, 45, 46, 47, 49, 50, 51, 52, 57, 60, 61, 62, 63, 64, 65, 66, 73, 74, 75, 76, 77, 78, 79, 80, 81, 82, 83, 84, 85, 86, 87, 88, 89, 90, 91, 92, 93, 94, 95, 96, 97, 98, 99, 102, 112, 116, 119, 120, 121, 122, 123, 124, 125, 126, 127, 128, 129, 130, 131, 132, 133, 134, 135, 136, 137, 138, 139, 140, 141, 142, 143, or 144.

In a preferred embodiment, the nuclease; preferably the endonuclease, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 8, 9, 10, 11, 12, 13, 14, 15, 17, 18, 19, 20, 21, 22, 23, 24, 26, 27, 28, 29, 36, 37, 38, 39, 40, 41, 42, 43, 44, 45, 46, 47, 49, 50, 51, 52, 57, 60, 61, 62, 63, 64, 65, 66, 73, 74, 75, 76, 77, 112, 116, or 119.

In a preferred embodiment, the nuclease; preferably the endonuclease, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to
(i) SEQ ID NO: 64, 65, 66, 73, 74, 75, 76, 81, 83, 88, 89, 90, 94, 120, 122, 123, 128, 131, and 135; or
(ii) SEQ ID NO: 64, 65, 66, 73, 74, 75, 76.

In a preferred embodiment, the nuclease; preferably the endonuclease, which is characterized by,
(A) an increased Tlow activity in comparison to the Tlow activity of SEQ ID NO:1; and/or
(B) an increased relative cold activity compared to the relative cold activity of SEQ ID NO:1.

In a preferred embodiment, the nuclease; preferably the endonuclease, which has a relative cold activity that compared to SEQ ID NO:1 is increased,
(i) at least 1.05-fold; preferably at least 1.5-fold; more preferably at least 2.0-fold, and still more preferably at least 3.0-fold.; or
(ii) is increased from 1.05-fold to 5-fold; preferably from 1.5-fold to 5-fold; more preferably from 2-fold to 5-fold, and still more preferably about 3-fold.

In a preferred embodiment, the nuclease; preferably the endonuclease, which has a Tlow activity that compared to SEQ ID NO:1 is increased,
(i) at least 1.01-fold; preferably at least 1.1-fold; more preferably at least 1.3-fold; still more preferably at least 1.5-fold, and yet more preferably at least 1.55-fold; or
(ii) is increased from 1.01-fold to 2-fold; preferably from 1.1-fold to 2-fold; more preferably from 1.3-fold to 1.6-fold; still more preferably from 1.5-fold to 1.6-fold, and yet more preferably about 1.55-fold.

In a preferred embodiment, the nuclease; preferably the endonuclease, wherein the cold activity and the relative cold activity are determined at a Tlow of 5°C and a Thigh of 30°C.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51, S53, K55, T56, N58, A72, P73, A74, T77, N80, K84, D86, S116, D117, Q120, A124, D128, Q129, K132, D135, D191, K196, G197, D199, R204, and E239; preferably P51, T56, N58, T77, K84, Q120, A124, D128, Q129, D135, K196, and G197.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51, S53, K55, T56, N58, A72, P73, A74, T77, N80, K84, D86, S116, D117, Q120, A124, D128, Q129, K132, D135, D191, K196, G197, D199, R204, and E239; preferably P51, T56, N58, T77, K84, Q120, A124, D128, Q129, D135, K196, and G197, and wherein,
when the amino acid substitution comprises a substitution at amino acid position P51, then the amino acid substitution at position P51 is selected from the group consisting of the substitutions D, E, R, K, H, G, A, V, L, and I; preferably D, R, G, and L;
when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, and A;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions S, M, R, K, H, G, A, V, L, I, P, W, F, Y, and D; preferably S, M, R, K, G, A, V, L, I, F, P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and V;
when the amino acid substitution comprises a substitution at amino acid position A72, then the amino acid substitution at position A72 is selected from the group consisting of the substitutions D, E, R, K, H, G, V, L, I, and P; preferably K, H and R; more preferably K;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L;
when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K;
when the amino acid substitution comprises a substitution at amino acid position D 117, then the amino acid substitution at position D 1 17 is selected from the group consisting of the substitutions G, A, V, L, I, P, W, F, Y, C, M, S, N, T, Q, H, R, and K; preferably A, G, V, L, I, P, C, M, N, T, Q, K, H, R, and S; more preferably A, N, Y, K, H, R, and S;
when the amino acid substitution comprises a substitution at amino acid position Q120, then the amino acid substitution at position Q 120 is selected from the group consisting of the substitutions R, K, H, G, A, V, L, I, P, W, F, Y, and D; preferably K, H, and R; more preferably R;
when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R;
when the amino acid substitution comprises a substitution at amino acid position D128, then the amino acid substitution at position D 128 is selected from the group consisting of the substitutions G, A, V, L, I, P, W, F, Y, C, M, N, Q, S, T, H, R, and K; preferably G, Y, F, M, N, S, R, and K;
when the amino acid substitution comprises a substitution at amino acid position Q129, then the amino acid substitution at position Q 129 is selected from the group consisting of the substitutions R, K, H, G, A, V, L, I, P, W, F, Y, and D; preferably K, H, and R; more preferably K, and R;
when the amino acid substitution comprises a substitution at amino acid position K132, then the amino acid substitution at position K132 is selected from the group consisting of the substitutions G, A, V, L, I, P, R, H, N, Q, S, T, C, and M; preferably H, and R; more preferably R;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably A, M, G, N, R, and K;
when the amino acid substitution comprises a substitution at amino acid position D191, then the amino acid substitution at position D191 is selected from the group consisting of the substitutions G, A, V, L, I, P, C, M, S, Q, and T; preferably G, A, V, L, I, and P; more preferably P;
when the amino acid substitution comprises a substitution at amino acid position K196, then the amino acid substitution at position K196 is selected from the group consisting of the substitutions G, A, V, L, I, P, R, H, N, Q, S, T, C, and M; preferably H, and R; more preferably R;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K;
when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably S, Q, T, N, C, M, H, R, and K; more preferably N, S, and K;
when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q; or
when the amino acid substitution comprises a substitution at amino acid position E239, then the amino acid substitution at position E239 is selected from the group consisting of the substitutions G, A, V, L, I, P, W, F, Y, C, M, N, Q, S, T, H, R, and K; preferably A, G, V, L, I, and P; more preferably A.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A; preferably P51R, P51G, P51L, T56S, T56M, T56R, T56K, T56G, T56A, T56V, T56L, T56I, T56F, T56P, T56D, N58K, N58R, N58V, T77K, T77R, T77F, T77L, K84G, Q120R, A124K, A124R, D128G, D128Y, D128F, D128M, D128N, D128S, D128R, D128K, Q129R, D135A, D135M, D135G, D135N, D135R, D135K, K196R, and G197K.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five, or at least twenty-six, or at least twenty-seven, or at least twenty-eighth amino acid positions selected from the group consisting of P51, S53, K55, T56, N58, A72, P73, A74, T77, G78, N80, A81, A82, K84, D86, S116, D117, Q120, A124, D128, Q129, K132, D135, D191, K196, G197, D199, R204, and E239, and wherein the amino acid sequence in addition comprises at least one further substitution, wherein the at least one further substitution position is selected from the group consisting ofP51, S53, K55, T56, N58, A72, P73, A74, T77, G78, N80, A81, A82, K84, D86, S116, D117, Q120, A124, D128, Q129, K132, D135, D191, K196, G197, D199, R204, and E239.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five, or at least twenty-six, or at least twenty-seven, or at least twenty-eighth amino acid positions selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A, and wherein the amino acid sequence in addition comprises at least one further substitution, wherein the at least one further substitution position is selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A.

### Functional characteristics of the nucleases according to the invention

In preferred embodiments of the nuclease according to the invention, the nuclease is capable of hydrolyzing of phosphodiesters of polynucleotide substrate into cleaved polynucleotides or oligonucleotides.

In preferred embodiments of the nuclease according to the invention, the nuclease catalyzes the hydrolysis of phosphodiesters of polynucleotide substrate into cleaved polynucleotides or oligonucleotides.

In preferred embodiments of the nuclease according to the invention, the polynucleotide substrates are selected from the group consisting of single strand RNA, single strand DNA, double strand DNA, double strand RNA and hybrid strand DNA/RNA; preferably double strand DNA.

In preferred embodiments of the nuclease according to the invention, the cleaved polynucleotide substrate is an oligonucleotide; preferably, wherein the oligonucleotide is at least 200 nucleotides long; more preferably at least 150 nucleotides long; still more preferably at least 100 nucleotides long; yet more preferably at least 50 nucleotides long; even more preferably at least 25 nucleotides long; most preferably at least 13 nucleotides long, utmost preferably at least 5 nucleotides long, and in particular 2 nucleotides long; optionally, wherein the cleaved polynucleotide is a mononucleotide.

In preferred embodiments, the nuclease according to the invention is characterized by either
(A) an increased thermal stability in comparison to the wildtype nuclease of SEQ ID NO:1; and/or
(B) an increased relative salt activity in comparison to the relative salt activity of the wildtype nuclease of SEQ ID NO: 1; and/or
(C) an increased S_{high} activity in comparison to the S_{high} activity of the wildtype nuclease of SEQ ID NO: 1; and/or
(D) an increased relative cold activity in comparison to the relative cold activity of the wildtype nuclease of SEQ ID NO: 1; and/or
(E) an increased T_{low} activity in comparison to the T_{low} activity of the wildtype nuclease of SEQ ID NO: 1.

For the purpose of the specification, the enzymatic activity of a nuclease according to the invention is detected by a nuclease activity assay as described in Example 2. The Nuclease Activity Assay bases upon the release of short, acid soluble cleaved polynucleotides or oligonucleotides from DNA polynucleotide substrate which leads to an increase in absorbance at 260 nm. The term A260 describes the absorbance of a sample in the nuclease activity assay and corresponds to the difference between the absorbance at 260nm of such enzyme sample and the absorbance at 260nm of a control sample without nuclease.

In preferred embodiments, the nuclease according to the invention has an increased thermal stability than SEQ ID NO: 1, wherein said thermal stability is characterized by a higher residual activity after incubation for 15 minutes at a temperature T_{stability}.

In preferred embodiments of the nuclease according to the invention, the residual activity of an individual nuclease is detected by
(i) separately incubating two vessels with a specific amount of nuclease in a buffer containing 50 mM Tris-HCl, pH 8.0, 5 mM MgCl2, 0.1 mg/ml BSA for 15 min at either 23°C or T_{stability}, respectively, and
(ii) subsequently cooling down the two vessels by incubation for 15 min on ice, centrifugation of vessels for removal of denatured protein and isolating the supernatant containing the nuclease, and
(iii) measuring the residual activity of either nuclease from step (ii) by transferring amounts of either nuclease in a specific ratio into new separate vessels and mixing with DNA sodium salt from salmon testes to a final concentration of 0.8 mg/ml, and incubating each new vessel for 1 h at 30°C, then adding 100 µl of 4 % perchloric acid to terminate the reaction, incubating the two obtained composition on ice for 45 min, and subsequently centrifuging the obtained composition at 3,800xg at 4°C for 30 minutes, and measuring the absorbance of each obtained supernatant at 260 nm which absorbance correlates to the enzymatic activity of the nuclease of the corresponding vessel, and
(iv) calculating the residual activity as the ratio of absorbances measured for the sample at T_{stability} and at 23°C expressed as percentage (A260 [T_{stability}]: A260 [T_{23°C}])
wherein for any nuclease variant the specific ratio of the amounts of nucleases being transferred into the two vessels in step (ii) is defined by the amounts of nuclease of SEQ ID NO:1 after incubation at either 23°C or T_{stability} that are required to obtain a detectable and linear signal in measuring the residual activity in step (iii).

In preferred embodiments of the nuclease according to the invention, the residual activity is determined at a temperature T_{stability} from 40°C to 60°C; preferably from 45°C to 55°C; more preferably from 49°C to 53°C, and still more preferably 51°C.

In preferred embodiments of the nuclease according to the invention, the thermal stability of the nuclease is at least 16%; preferably at least 23%; more preferably at least 29%, and still more preferably at least 59% increased in comparison to SEQ ID NO:1, when the residual activity is determined at a temperature T_{stability} of 51°C.

In preferred embodiments of the nuclease according to the invention, the thermal stability of the nuclease is at least 16%; preferably at least 23%; more preferably at least 29%, and still more preferably at least 59% increased in comparison to SEQ ID NO: 1.

In preferred embodiments, the nuclease according to the invention has a thermal stability that compared to SEQ ID NO:1 is increased at least 1.051-fold, at least 1.5-fold, or at least 2-fold; more preferably at least 3-fold, at least 4-fold, or at least 5-fold, and still more preferably at least 6-fold, at least 6.5-fold, or at least 7-fold.

In preferred embodiments, the nuclease according to the invention has a thermal stability that compared to SEQ ID NO:1 is increased from 1.051-fold to 100-fold; preferably from 1.5-fold to 50-fold; more preferably from 2-fold to 40-fold; still more preferably from 3-fold, to 30-fold; yet more preferably from 4-fold to 23-fold; even more preferably from 5-fold to 21-fold, and most preferably from 6-fold to 15-fold.

In preferred embodiments, the nuclease according to the invention has
(i) a higher enzymatic activity at a salt concentration S_{high} (S_{high} activity) than SEQ ID NO:1; and/or
(ii) a higher relative salt activity in comparison to SEQ ID NO:1, wherein said relative salt activity is the ratio of enzymatic activity at the salt concentration S_{high} in comparison to the salt concentration S_{low}.

In preferred embodiments of the nuclease according to the invention, the S_{high} activity, the S_{low} activity, and the relative salt activity are determined by
(i) separately detecting the enzymatic activity of the nuclease at salt concentrations S_{low} and S_{high} by transferring amounts of such nuclease in a specific ratio into two separate vessels, mixing with buffer to a final concentration of 50 mM Tris-HCl, pH 8.0, 5.0 mM MgCl2, 0.1 mg/ml BSA, and 0.8 mg/ml DNA sodium salt from salmon testes, and a salt concentration of S_{low} or S_{high}, respectively, incubating at 30°C for 1 h, then adding 100 µl of 4 % perchloric acid to terminate the reaction, incubating the two obtained compositions on ice for 45 min, and subsequently centrifuging the obtained compositions at 3,800xg at 4°C for 30 minutes, and measuring the absorbance of each obtained supernatant at 260 nm, wherein the detected absorbances correlate to the enzymatic activity of the nuclease at salt concentrations S_{low} (S_{low} activity) and S_{high} (S_{high} activity), respectively, and
(ii) calculating the relative salt activity as the ratio of absorbances measured for the sample incubated at S_{high} (S_{high} activity) and the sample at S_{low} (S_{low} activity) ex-pressed as percentage (A260 [S_{high}]: A260 [S_{low}]),
wherein for any nuclease variant the specific ratio of the amounts of nucleases being transferred into the two vessels in step (i) is defined by the amounts of nuclease of SEQ ID NO:1 being required to obtain a detectable and linear signal in measuring the S_{low} activity or the S_{high} activity in step (ii), respectively.

In preferred embodiments of the nuclease according to the invention, the salt is NaCl or KCl; preferably NaCl.

In preferred embodiments of the nuclease according to the invention, in determining of the S_{high} activity, the S_{low} activity, and the relative salt activity the concentration of
- S_{low} is within the range of from 0.00M to 0.15M; preferably at 0.00M; and
- S_{high} is from 0.01M to LOOM; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.

In preferred embodiments, the nuclease according to the invention has a relative salt activity that compared to SEQ ID NO:1 is increased at least 1.05-fold; preferably at least 7-fold; more preferably at least 9-fold; still more preferably at least 13-fold; yet more preferably at least 60-fold, and even more preferably at least 110-fold.

In preferred embodiments, the nuclease according to the invention has a relative salt activity that compared to SEQ ID NO:1 is increased from 1.05-fold to 300-fold preferably from 7-fold to 250-fold; more preferably from 9-fold to 200-fold; still more preferably from 13-fold to 150-fold, and yet more preferably about 115-fold.

In preferred embodiments of the nuclease according to the invention, the relative salt activity is at least 3%; preferably at least 25%; more preferably at least 50%; still more preferably at least 100%; yet more preferably at least 130%; even more preferably at least 200%, and most preferably at least 800% increased in comparison to SEQ ID NO:1.

In preferred embodiments, the nuclease according to the invention has a S_{high} activity that compared to the S_{high} activity of SEQ ID NO:1 is increased at least 1.01-fold; preferably at least 1.5-fold; more preferably at least 2-fold; still more preferably at least 5-fold, yet more prefer-ably at least 12-fold, and even more preferably at least 19-fold.

In preferred embodiments, the nuclease according to the invention has a S_{high} activity that compared to the SEQ ID NO:1 is increased from 1.01-fold to 200-fold; preferably from 1.5-fold to 150-fold; more preferably from 2-fold to 100-fold; still more preferably from 5-fold to 75-fold; yet more preferably from 12-fold to 50-fold, and even more preferably about 20-fold.

In preferred embodiments of the nuclease according to the invention, the salt activity and the relative salt activity are determined at a S_{low} of 0.00M and S_{high} of 500 mM.

In preferred embodiments, the nuclease according to the invention has
(i) a higher enzymatic activity at a temperature T_{low} below 30°C (T_{low} activity) than SEQ ID NO:1; and/or
(ii) a higher relative cold activity than SEQ ID NO:1, wherein said relative cold activity is the ratio of the enzymatic activity at a temperature T_{low} below 30°C to the enzymatic activity at a temperature T_{high} of 30°C or above (T_{low} activity: T_{high} activity).

In preferred embodiments of the nuclease according to the invention, the T_{low} activity, the T_{high} activity, and the relative cold activity are determined by
(i) separately detecting the enzymatic activity of the nuclease at temperatures T_{low} and T_{high} by transferring amounts of such nuclease in a specific ratio into two separate vessels, mixing with buffer to a final concentration of 50 mM Tris-HCl, pH 8.0, 5.0 mM MgCl2, 0.1 mg/ml BSA, and 0.8 mg/ml DNA sodium salt from salmon testes, and incubating at temperatures T_{low} and at T_{high}, respectively for 1.0 h, then adding 100 µl of 4 % perchloric acid to terminate the reaction, incubating the two obtained composition on ice for 45 min, and subsequently centrifuging the obtained composition at 3,800xg at 4°C for 30 minutes, and measuring the absorbance of each obtained supernatant at 260 nm, wherein the detected absorbances correlate to the enzymatic activity of the nuclease at T_{low} (T_{low} activity) and at T_{high} (T_{high} activity), respectively, and
(ii) calculating the relative cold activity as the ratio of absorbances measured for the sample incubated at T_{low} (T_{low} activity) and the sample at T_{high} (T_{high} activity) ex-pressed as percentage (A260 [T_{low}]: A260 [T_{high}])
wherein for any nuclease variant the specific ratio of the amounts of nucleases being transferred into the two vessels in step (i) is defined by the amounts of nuclease of SEQ ID NO:1 being required to obtain a detectable and linear signal in measuring the T_{low} activity or a T_{high} activity in step (ii), respectively.

In preferred embodiments of the nuclease according to the invention,
- T_{low} is within the range of from 4°C to 25°C; preferably from 4.0°C to 15°C; more preferably from 4.0°C to 10°C; still more preferably from 4.0°C to 6.0°C; yet more preferably 5.0°C; and
- T_{high} is within the range of from 30°C to 60°C; preferably from 30°C to 50°C; more preferably from more preferably from 30°C to 40°C; still more preferably 30°C.

In preferred embodiments of the nuclease according to the invention, T_{low} is from 4.0°C to 15°C and T_{high} is from 25°C to 60°C; preferably T_{low} is from 4.0°C to 10°C and T_{high} is from 25°C to 50°C; more preferably T_{low} is from 4.0°C to 6.0°C and T_{high} is from 25°C to 40°C; still more preferably T_{low} is 5.0°C and T_{high} is 30°C.

In preferred embodiments, the nuclease according to the invention has a relative cold activity that compared to SEQ ID NO:1 is increased at least 1.05-fold; preferably at least 1.5-fold; more prefer-ably at least 2.0-fold, and still more preferably at least 3.0-fold.

In preferred embodiments, the nuclease according to the invention has a relative cold activity that compared to SEQ ID NO:1 is increased from 1.05-fold to 5-fold; preferably from 1.5-fold to 5-fold; more preferably from 2-fold to 5-fold, and still more preferably about 3-fold.

In preferred embodiments, the nuclease according to the invention has a T_{low} activity that compared to SEQ ID NO:1 is increased at least 1.01-fold; preferably at least 1.1-fold; more preferably at least 1.3-fold; still more preferably at least 1.5-fold, and yet more preferably at least 1.55-fold.

In preferred embodiments, the nuclease according to the invention has a T_{low} activity that compared to SEQ ID NO:1 is increased from 1.01-fold to 2-fold; preferably from 1.1-fold to 2-fold; more preferably from 1.3-fold to 1.6-fold; still more preferably from 1.5-fold to 1.6-fold, and yet more preferably about 1.55-fold.

In preferred embodiments of the nuclease according to the invention, the cold activity and the relative cold activity are determined at a T_{low} of 5°C and a T_{high} of 30°C.

### Combinations of structural and functional characteristics of the nucleases according to the invention

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, or at least twenty-three amino acid positions selected from the group consisting of the positions P51, K55, T56, N58, A74, T77, N80, K84, D86, A94, S116, D117, A124, D128, K132, D135, E151, K162, D191, R204, I218, A228, and E239, and exhibits an increased thermal stability in comparison to SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, or at least twenty-three positions selected from the group consisting of the positions P51, K55, T56, N58, A74, T77, N80, K84, D86, A94, S116, D117, A124, D128, K132, D135, E151, K162, D191, R204, I218, A228, and E239, and exhibits an increased thermal stability of at least 1,051- fold, at least 1,5-fold, or at least 2-fold; more preferably at least 3-fold, at least 4-fold, or at least 5-fold, and still more preferably at least 6-fold, at least 6,5-fold, or at least 7-fold in comparison to the thermal stability of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, or at least twenty-three positions selected from the group consisting of the positions P51, K55, T56, N58, T77, A74, N80, K84, D86, A94, S116, D117, A124, D128, K132, D135, E151, K162, D191, R204, I218, A228, and E239, and exhibits an increased thermal stability in comparison to SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably D, E, G, A, V, L, and I; more preferably G, L, and D;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably G, A, V, L, I, P, and D; still more preferably P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably G, A, V, L, I, and P; still more preferably V;
when the amino acid substitution comprises a substitution at amino acid position A74, then the amino acid substitution at position A74 is selected from the group consisting of the substitutions G, I, V, L, P, W, F, Y, C, M, N, Q, S, T, D, E, H, R, and K; preferably R, Q, and H; more preferably R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L;
when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position A94, then the amino acid substitution at position A94 is selected from the group consisting of the substitutions G, I, V, L, P, W, F, Y, C, M, N, Q, S, T, D, E, H, R, and K; preferably G, I, V, L, and P; more preferably G;
when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K;
when the amino acid substitution comprises a substitution at amino acid position D117, then the amino acid substitution at position D117 is selected from the group consisting of the substitutions R, K, H, G, A, V, L, I, P, W, F, Y, C, M, S, Q, T, and N; preferably C, M, S, Q, T, and N; more preferably C, M, S, Q, and N; still more preferably N;
when the amino acid substitution comprises a substitution at amino acid position A124, then the amino acid substitution at position A124 is selected from the group consisting of the substitutions D, E, R, K, H, G, V, L, I, and P; preferably K, Hand R; more preferably K;
when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably W, F, Y, C, M, N, Q, S, T; still more preferably M, F, and Y;
when the amino acid substitution comprises a substitution at amino acid position K132, then the amino acid substitution at position K132 is selected from the group consisting of the substitutions R, H, G, A, V, L, I, P, W, F, Y, C, M, S, Q, T, and N; preferably R, and H; more preferably R;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R and K; preferably G, A, V, L, I, P, W, F, Y, C, M, N, Q, S, T, H, and K; more preferably G, A, V, L, I, P, C, M, N, Q, S, T, H, and K; still more preferably G, A, V, L, I, P, C, M, Q, S, T and N; yet more preferably G, and N;
when the amino acid substitution comprises a substitution at amino acid position E151, then the amino acid substitution at position E151 is selected from the group consisting of the substitutions D, R, K, H, G, A, V, L, I, P, W, F, Y, C, M, S, Q, T, and N; preferably G, A, V, L, I, and P; more preferably L;
when the amino acid substitution comprises a substitution at amino acid position K162, then the amino acid substitution at position K162 is selected from the group consisting of the substitutions R, H, G, A, V, L, I, P, W, F, Y, C, M, S, Q, T, and N; preferably C. M, S, Q, T, and N; more preferably N;
when the amino acid substitution comprises a substitution at amino acid position D191, then the amino acid substitution at position D191 is selected from the group consisting of the substitutions G, A, V, L, I, P, C, M, S, Q, and T; preferably G, A, V, L, I, and P; more preferably P;
when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q;
when the amino acid substitution comprises a substitution at amino acid position I218, then the amino acid substitution at position I218 is selected from the group consisting of the substitutions G, A, V, L, P, W, F, Y, C, M, N, Q, S, T, D, E, H, R, and K; preferably W, Y, and F; more preferably F;
when the amino acid substitution comprises a substitution at amino acid position A228, then the amino acid substitution at position A228 is selected from the group consisting of the substitutions G, I, V, L, P, W, F, Y, C, M, N, Q, S, T, D, E, H, R, and K; preferably H, R, and K; more preferably R; or
when the amino acid substitution comprises a substitution at amino acid position E239, then the amino acid substitution at position E239 is selected from the group consisting of the substitutions G, A, V, L, I, P, W, F, Y, C, M, N, Q, S, T, H, R, and K; preferably A, G, V, L, I, and P; more preferably A.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, or at least twenty-three positions such that it comprises at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, or at least twenty-three substitution selected from the group consisting of the substitutions P51L, P51G, P51D, K55R, T56P, T56D, N58V, A74R, T77K, T77F, T77L, T77R, N80H, K84G, D86S, A94G, S116K, D117N, A124K, D128M, D128Y, D128F, K132R, D135G, D135N, E151L, K162N, D191P, R204Q, I218F, A228R, and E239A, exhibits an in-creased thermal stability in comparison to SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen , at least eighteen, at least nineteen, at least twenty, at least twenty-one, at least twenty-two, at least twenty-three, at least twenty-four, at least twenty-five, at least twenty-six, or at least twenty-seven positions, wherein the substitution is selected from the group consisting of the positions P51, A52, S53, G54, K55, T56, N58, A72, P73, A74, T77, G78, N80, A81, A82, D86, S116, D117, N119, Q120, A124, D128, D135, P195, K196, G197, and D199; preferably P51, S53, K55, T56, N58, A72, P73, A74, T77, A81, A82, D86, S116, D117, A124, D128, D135, K196, G197, and D199, and wherein the nuclease exhibits an increased relative salt activity compared to the relative salt activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions, wherein the substitution is selected from the group consisting of the positions P51, A52, S53, G54, K55, T56, N58, A72, P73, A74, T77, G78, N80, A81, A82, D86, S116, D117, N119, Q120, A124, D128, D135, P195, K196, G197, and D199; preferably P51, S53, K55, T56, N58, A72, P73, A74, T77, A81, A82, D86, S116, D117, A124, D128, D135, K196, G197, and D199, and wherein the nuclease exhibits an increased relative salt activity of at least 1.05-fold; preferably at least 7-fold; more preferably at least 9-fold; still more preferably at least 13-fold; yet more preferably at least 60-fold, and even more preferably at least 110-fold compared to the relative salt activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions selected from the group consisting of the positions P51, A52, S53, G54, K55, T56, N58, A72, P73, A74, T77, G78, N80, A81, A82, D86, S116, D117,N119, Q120, A124, D128, D135, P195, K196, G197, and D199; preferably P51, S53, K55, T56, N58, A72, P73, A74, T77, A81, A82, D86, S116, D117, A124, D128, D135, K196, G197, and D199, and exhibits an increased relative salt activity compared to the relative salt activity of SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably R, G, and L;
when the amino acid substitution comprises a substitution at amino acid position A52, then the amino acid substitution at position A52 is selected from the group consisting of the substitutions G, V, L, I, P, W, F, Y, C, M, S, N, T, Q, H, R, K, D and E; preferably K, H and R; more preferably R;
when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, and A;
when the amino acid substitution comprises a substitution at amino acid position G54, then the amino acid substitution at position G54 is selected from the group consisting of the substitutions A, V, L, I, P, W, F, Y, C, M, S, N, T, Q, H, R, K, D and E; preferably K, Hand R; more preferably R;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, C, M, S, N, T, Q and D; more preferably K, R, S and G;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R;
when the amino acid substitution comprises a substitution at amino acid position A72, then the amino acid substitution at position A72 is selected from the group consisting of the substitutions D, E, R, K, H, G, V, L, I, and P; preferably K, H and R; more preferably K;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, H, W, F, and Y; more preferably K, R, and F;
when the amino acid substitution comprises a substitution at amino acid position G78, then the amino acid substitution at position G78 is selected from the group consisting of the substitutions A, V, L, I, P, W, F, Y, C, M, S, N, T, Q, H, R, K, D and E; preferably K, H and R; more preferably R;
when the amino acid substitution comprises a substitution at amino acid position N80, then the amino acid substitution at position N80 is selected from the group consisting of the substitutions G, A, V, L, I, P, W, F, Y, C, M, S, T, Q, H, R, K, D and E; preferably R, K, and H; more preferably R;
when the amino acid substitution comprises a substitution at amino acid position A81, then the amino acid substitution at position A81 is selected from the group consisting of the substitutions R, K, and H; preferably K;
when the amino acid substitution comprises a substitution at amino acid position A82, then the amino acid substitution at position A82 is selected from the group consisting of the substitutions R, K, and H; preferably R;
when the amino acid substitution comprises a substitution at amino acid position D86, then the amino acid substitution at position D86 is selected from the group consisting of the substitutions N, Q, S, T, C, and M; preferably S;
when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K;
when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably A, G, V, L, I, P, C, M, N, T, Q, K, H, R, and S; more preferably A, K, R, N, and S;
when the amino acid substitution comprises a substitution at amino acid position N119, then the amino acid substitution at position N119 is selected from the group consisting of the substitutions G, A, V, L, I, P, W, F, Y, C, M, S, T, Q, H, R, K, D and E; preferably R, K, and H; more preferably R;
when the amino acid substitution comprises a substitution at amino acid position Q120, then the amino acid substitution at position Q120 is selected from the group consisting of the substitutions G, A, V, L, I, P, W, F, Y, C, M, N, T, S, H, R, K, D and E; preferably R, K, and H; more preferably R;
when the amino acid substitution comprises a substitution at amino acid position A124, then the amino acid substitution at position A124 is selected from the group consisting of the substitutions D, E, R, K, H, G, V, L, I, and P; preferably K, Hand R; more preferably K, and R;
when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, R, and K; more preferably G, Y, F, M, N, S, R, and K;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably G, A, V, L, I, P, C, M, N, Q, S, T, H, and K; still more preferably G, A, N, and K;
when the amino acid substitution comprises a substitution at amino acid position P195, then the amino acid substitution at position P195 is selected from the group consisting of the substitutions A, V, L, I, G, W, F, Y, C, M, S, N, T, Q, H, R, K, D and E; preferably K, H and R; more preferably R;
when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or
when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably G, A, V, L, I, P, W, F, Y, C, M, Q, T, N, S, H, and K; more preferably S, Q, T, N, C, M, H, and K; still more preferably S, N, and K.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions such that it comprises at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven substitutions selected from the group consisting of the substitutions P51R, P51G, P51L, A52R, S53K, S53A, G54R, K55R, T56G, T56S, T56R, T56K, N58R, N58K, A72K, P73K, P73R, A74R, A74K, T77F, T77K, T77R, G78R, N80R, A81K, A82R, D86S, S116K, D117A, D117S, D117K, D117R, D117N, N119R, Q120R, A124K, A124R, D128K, D128R, D128Y, D128S, D128M, D128N, D128F, D128G, D135A, D135G, D135N, D135K, P195R, K196R, G197K, D199K, D199N, and D199S; preferably P51R, P51G, P51L, S53K, S53A, K55R, T56G, T56S, T56R, T56K, N58R, N58K, A72K, P73K, P73R, A74R, A74K, T77F, T77K, T77R, A81K, A82R, D86S, S116K, D117A, D117S, D117K, D117R, D117N, A124K, A124R, D128K, D128R, D128Y, D128S, D128M, D128N, D128F, D128G, D135A, D135G, D135N, D135K, K196R, G197K, D199K, D199N, and D199S, and exhibits an increased relative salt activity compared to the relative salt activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions selected from the group consisting of the positions P51, A52, S53, G54, K55, T56, N58, A72, P73, A74, T77, G78, N80, A81, A82, D86, S116, D117, N119, Q120, A124, D128, D135, P195, K196, G197, and D199; preferably P51, A52, S53, G54, T56, N58, A72, P73, A74, T77, G78, N80, A81, A82, D117, N119, Q120, A124, D128, D135, P195, K196, and G197 ; more preferably P51, S53, T56, N58, A72, A74, T77, A81, A82, D117, A124, D128, D135, K196, and G197, and exhibits an increased S_{high} activity compared to the S_{high} activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions selected from the group consisting of the positions P51, A52, S53, G54, K55, T56, N58, A72, P73, A74, T77, G78, N80, A81, A82, D86, S116, D117, N119, Q120, A124, D128, D135, P195, K196, G197, and D199; preferably P51, A52, S53, G54, T56, N58, A72, P73, A74, T77, G78, N80, A81, A82, D117, N119, Q120, A124, D128, D135, P195, K196, and G197; more preferably P51, S53, T56, N58, A72, A74, T77, A81, A82, D117, A124, D128, D135, K196, and G197, and exhibits an increased S_{high} activity of at least 1.01-fold; preferably at least 1.5-fold; more preferably at least 2-fold; still more preferably at least 5-fold, yet more prefer-ably at least 12-fold, and even more preferably at least 19-fold compared to the S_{high} activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions selected from the group consisting of the positions P51, A52, S53, G54, K55, T56, N58, A72, P73, A74, T77, G78, N80, A81, A82, D86, S116, D117, N119, Q120, A124, D128, D135, P195, K196, G197, and D199; preferably P51, A52, S53, G54, T56, N58, A72, P73, A74, T77, G78, N80, A81, A82, D117, N119, Q120, A124, D128, D135, P195, K196, and G197 ; more preferably P51, S53, T56, N58, A72, A74, T77, A81, A82, D117, A124, D128, D135, K196, and G197, and exhibits an increased S_{high} activity compared to the S_{high} activity of SEQ ID NO:1 wherein,
when the amino acid substitution comprises a substitution at amino acid position P51, then the amino acid substitution at position P51 is selected from the group consisting of the substitutions D, E, R, K, H, G, A, V, L, and I; preferably R, G, and L;
when the amino acid substitution comprises a substitution at amino acid position A52, then the amino acid substitution at position A52 is selected from the group consisting of the substitutions G, V, L, I, P, W, F, Y, C, M, S, N, T, Q, H, R, K, D and E; preferably K, H and R; more preferably R;
when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, R, and H; still more preferably K;
when the amino acid substitution comprises a substitution at amino acid position G54, then the amino acid substitution at position G54 is selected from the group consisting of the substitutions A, V, L, I, P, W, F, Y, C, M, S, N, T, Q, H, R, K, D and E; preferably K, H and R; more preferably R;
when the amino acid substitution comprises a substitution at amino acid position K55, then the amino acid substitution at position K55 is selected from the group consisting of the substitutions D, E, R, H, G, V, L, I, P, C, M, S, N, T, Q, W, F, and Y; preferably R, and H; more preferably R;
when the amino acid substitution comprises a substitution at amino acid position T56, then the amino acid substitution at position T56 is selected from the group consisting of the substitutions R, K, H, G, A, V, L, I, P, W, F, Y, C, M, S, N, T, Q and D; preferably R, K, H, G, A, V, L, I, P, C, M, S, N, T, Q and D; more preferably K, R, G and S;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R;
when the amino acid substitution comprises a substitution at amino acid position A72, then the amino acid substitution at position A72 is selected from the group consisting of the substitutions D, E, R, K, H, G, V, L, I, and P; preferably K, H and R; more preferably K;
when the amino acid substitution comprises a substitution at amino acid position P73, then the amino acid substitution at position P73 is selected from the group consisting of the substitutions G, V, L, I, A, W, F, Y, C, M, S, N, T, Q, H, R, K, D and E; preferably R, K, and H; more preferably R, and K;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably W, F, Y, K, R, and H; more preferably K, R, and H; still more preferably K, and R;
when the amino acid substitution comprises a substitution at amino acid position G78, then the amino acid substitution at position G78 is selected from the group consisting of the substitutions A, V, L, I, P, W, F, Y, C, M, S, N, T, Q, H, R, K, D and E; preferably K, H and R; more preferably R;
when the amino acid substitution comprises a substitution at amino acid position N80, then the amino acid substitution at position N80 is selected from the group consisting of the substitutions G, A, V, L, I, P, W, F, Y, C, M, S, T, Q, H, R, K, D and E; preferably R, K, and H; more preferably R;
when the amino acid substitution comprises a substitution at amino acid position A81, then the amino acid substitution at position A81 is selected from the group consisting of the substitutions G, V, L, I, P, W, F, Y, C, M, S, N, T, Q, H, R, K, D and E; preferably R, K, and H; more preferably K;
when the amino acid substitution comprises a substitution at amino acid position A82, then the amino acid substitution at position A82 is selected from the group consisting of the substitutions G, V, L, I, P, W, F, Y, C, M, S, N, T, Q, H, R, K, D and E; preferably R, K, and H; more preferably R;
when the amino acid substitution comprises a substitution at amino acid position D86, then the amino acid substitution at position D86 is selected from the group consisting of the substitutions G, A, V, L, I, P, W, F, Y, C, M, S, N, T, Q, H, R, K, and E; preferably C, M, S, N, T, and Q; more preferably S;
when the amino acid substitution comprises a substitution at amino acid position S116, then the amino acid substitution at position S116 is selected from the group consisting of the substitutions G, A, V, L, I, P, W, F, Y, C, M, N, T, Q, H, R, K, D and E; preferably R, K, and H; more preferably K;
when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably A, G, V, L, I, P, C, M, N, T, Q, R, K, H, and S; more preferably A, G, V, L, I, P, C, M, N, T, Q, and S; still more preferably A, N, and S;
when the amino acid substitution comprises a substitution at amino acid position N119, then the amino acid substitution at position N119 is selected from the group consisting of the substitutions G, A, V, L, I, P, W, F, Y, C, M, S, T, Q, H, R, K, D and E; preferably R, K, and H; more preferably R;
when the amino acid substitution comprises a substitution at amino acid position Q120, then the amino acid substitution at position Q120 is selected from the group consisting of the substitutions G, A, V, L, I, P, W, F, Y, C, M, N, T, S, H, R, K, D and E; preferably R, K, and H; more preferably R;
when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R;
when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, Y, F, M, N, S, R, and K;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably G, A, V, L, I, P, C, M, N, Q, S, T, H, and K; still more preferably A, G, N, and K;
when the amino acid substitution comprises a substitution at amino acid position P195, then the amino acid substitution at position P195 is selected from the group consisting of the substitutions A, V, L, I, G, W, F, Y, C, M, S, N, T, Q, H, R, K, D and E; preferably K, H and R; more preferably R;
when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or
when the amino acid substitution comprises a substitution at amino acid position D199, then the amino acid substitution at position D199 is selected from the group consisting of the substitutions G, A, V, L, I, P, W, F, Y, C, M, S, N, T, Q, H, R, K, and E; preferably C, M, S, N, T, Q, K, H, and R; more preferably K, S, and N.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven positions such that it comprises at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, or at least twenty-seven substitutions selected from the group consisting of the substitutions P51L, P51R, P51G, A52R, S53A, S53K, K55R, G54R, T56G, T56S, T56R, T56K, N58R, N58K, A72K, P73K, P73R, A74K, A74R, T77R, T77K, T77F, A78R, N80R, A81K, A82R, D86S, S116K, D117A, D117S, D117N, D117R, N119R, Q120R, A124K, A124R, D128S, D128N, D128K, D128R, D128G, D128M, D128Y, D128F, D135A, D135K, D135G, D135N, P195R, K196R, G197K, D199K, D199S, and D199N; preferably P51R, P51G, A52R, S53K, G54R, T56G, T56S, T56R, T56K, N58R, N58K, A72K, P73R, A74K, A74R, T77R, T77K, G78R, N80R, A81K, A82R, D117A, D117S, D1 17N, N119R, Q120R, A124K, A124R, D128S, D128N, D128K, D128R, D128G, D128M, D128Y, D128F, D135A, D135K, D135G, D135N, P195R, K196R, and G197K; more preferably P51R, P51G, S53K, T56G, T56S, T56R, T56K, N58R, N58K, A72K, A74K, A74R, T77R, T77K, A81K, A82R, D117A, D117S, D117N, A124K, A124R, D128S, D128N, D128K, D128R, D128G, D128M, D128Y, D128F, D135A, D135K, D135G, D135N, K196R, and G197K, and exhibits an increased S_{high} activity compared to the S_{high} activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions select-ed from the group consisting of the positions P51, K55, T56, N58, P73, A74, T77, G78, K84, D86, S116, D117, D128, D135, G197, and D199, and exhibits an increased relative cold activity compared to the relative cold activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen position selected from the group consisting of the positions P51, K55, T56, N58, P73, A74, T77, G78, K84, D86, S116, D117, D128, D135, G197, and D199, and exhibits an increased relative cold activity of at least 1.05-fold; preferably at least 1.5-fold; more preferably at least 2.0-fold, and still more preferably at least 3.0-fold compared to the relative cold activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions select-ed from the group consisting of the positions P51, K55, T56, N58, P73, A74, T77, G78, K84, D86, S116, D117, D128, D135, G197, and D199, and exhibits an increased relative cold activity compared to the relative cold activity of SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably D, R, G, and L;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably K, R, P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and V;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L;
when the amino acid substitution comprises a substitution at amino acid position G78, then the amino acid substitution at position G78 is selected from the group consisting of the substitutions R, K, H, A, V, L, I, P, W, F, Y, N, Q, S, T, C, M and D; preferably K, R, and H; more preferably R;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K;
when the amino acid substitution comprises a substitution at amino acid position D 117, then the amino acid substitution at position D 1 17 is selected from the group consisting of the substitutions G, A, V, L, I, P, W, F, Y, C, M, S, N, T, Q, H, R and K; preferably G, A, V, L, I, P, W, F, Y, C, M, S, N, T, Q, H, and K; more preferably A, G, V, L, I, P, C, M, N, T, Q, K, H, and S; even more preferably H, and K; most preferably K;
when the amino acid substitution comprises a substitution at amino acid position D128, then the amino acid substitution at position D 128 is selected from the group consisting of the substitutions G, A, V, L, I, P, W, F, Y, C, M, N, Q, S, T, H, R and K; preferably G, Y, F, M, N, S, R, and K;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R and K; preferably G, A, V, L, I, P, C, M, N, Q, S, T, H, R, and K; more preferably G, A, N, R, and K; still more preferably G, A, N, and K;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or
when the amino acid substitution comprises a substitution at amino acid position D199, then the amino acid substitution at position D199 is selected from the group consisting of the substitutions G, A, V, L, I, P, W, F, Y, C, M, Q, T, N, S, H, and K; preferably S, Q, T, N, C, M, H, and K; more preferably N, S, and K.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions such that it comprises at least one, at least two, at least three, at least four, at least five, at least six, at least seven, at least eight, at least nine, at least ten, at least eleven, at least twelve, at least thirteen, at least fourteen, at least fifteen, or at least sixteen substitutions selected from the group consisting of the substitutions P51L, P51D, P51G, P51R, K55R, T56D, T56R, T56P, T56K, N58V, N58K, N58R, P73R, P73K, A74K, A74R, T77K, T77L, T77R, T77F, G78R, K84G, D86S, S116K, D117K, D128Y, D128M, D128N, D128K, D128R, D128F, D128S, D128G, D135A, D135K, D135R, D135N, D135G, G197K, D199K, D199N, and D199S; preferably P51L, P51D, P51G, P51R, K55R, T56D, T56R, T56P, T56K, N58V, N58K, N58R, P73R, P73K, A74K, A74R, T77K, T77L, T77R, T77F, G78R, K84G, D86S, S116K, D117K, D128Y, D128M, D128N, D128K, D128R, D128F, D128S, D128G, D135A, D135K, D135N, D135G, G197K, D199K, D199N, and D199S and exhibits an increased relative cold activity compared to the relative cold activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions selected from the group consisting of the positions P51, K55, T56, N58, P73, A74, T77, G78, K84, D86, S116, D117, D128, D135, G197, and D199; preferably P51, T56, N58, K84, D86, S116, and D135, and exhibits an increased T_{low} activity in comparison to the T_{low} activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions selected from the group consisting of the positions P51, K55, T56, N58, P73, A74, T77, G78, K84, D86, S116, D117, D128, D135, G197, and D199; preferably P51, T56, N58, K84, D86, S116, and D135, and exhibits an increased T_{low} activity of at least 1.01-fold ; preferably at least 1.1-fold; more preferably at least 1.3-fold; still more preferably at least 1.5-fold, and yet more preferably at least 1.55-fold in comparison to the T_{low} activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions selected from the group consisting of the positions P51, K55, T56, N58, P73, A74, T77, G78, K84, D86, S116, D117, D128, D135, G197, and D199; preferably P51, T56, N58, K84, D86, S116, and D135, and exhibits an increased T_{low} activity in comparison to the T_{low} activity of SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably G, A, V, I, and L; more preferably L;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably G, A, V, L, I, P, and D; still more preferably G, P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably G, A, L, I, P, and V; still more preferably V;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L; when the amino acid substitution comprises a substitution at amino acid position G78, then the amino acid substitution at position G78 is selected from the group consisting of the substitutions R, K, H, A, V, L, I, P, W, F, Y, N, Q, S, T, C, M and D; preferably K, R, and H; more preferably R;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K;
when the amino acid substitution comprises a substitution at amino acid position D 117, then the amino acid substitution at position D 1 17 is selected from the group consisting of the substitutions G, A, V, L, I, P, W, F, Y, C, M, S, N, T, Q, H, R, and K; preferably G, V, L, I, P, W, F, Y, C, M, S, N, Q, H, R, and K; more preferably K, R, and H; still more preferably K;
when the amino acid substitution comprises a substitution at amino acid position D 128, then the amino acid substitution at position D 128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, R, and K; more preferably G, Y, F, M, N, S, R, and K;
when the amino acid substitution comprises a substitution at amino acid position D135, then the amino acid substitution at position D 135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably G, A, V, L, I, P, C, M, N, Q, S, T, H, and K; still more preferably G, A, V, L, I, and P; yet more preferably A;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or
when the amino acid substitution comprises a substitution at amino acid position D 199, then the amino acid substitution at position D 199 is selected from the group consisting of the substitutions G, A, V, L, I, P, W, F, Y, C, M, Q, T, N, S, H, R, and K; preferably G, A, V, L, I, P, W, F, Y, C, M, Q, T, N, S, H, and K; more preferably S, Q, T, N, C, M, H, and K; still more preferably S, N, and K.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, or at least sixteen positions such that it comprises at least one, at least two, at least three, at least four, at least five, at least six, at least seven, at least eight, at least nine, at least ten, at least eleven, at least twelve, at least thirteen, at least fourteen, at least fifteen, or at least sixteen substitutions selected from the group consisting of the substitutions P51L, P51D, P51G, P51R, K55R, T56G, T56D, T56P, T56R, T56K, N58V, N58K, N58R, P73K, P73R, A74K, A74R, T77K, T77L, T77R, T77F, G78R, K84G, D86S, S116K, D117K, D128Y, D128M, D128N, D128K, D128F, D128R, D128S, D128G, D135A, D135K, D135R, D135N, D135G, G197K, D199K, D199S, and D199N; preferably P51L, T56D, T56P, T56G, N58V, K84G, D86S, S116K, and D135A, and exhibits an increased T_{low} activity in comparison to the T_{low} activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five amino acid positions selected from the group consisting of the positions P51, K55, T56, N58, A74, T77, N80, A81, K84, D86, A94, S116, D117, A124, D128, K132, D135, E151, K162, D191, G197, R204, I218, A228, and E239, and exhibits an increased thermal stability in comparison to SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five positions selected from the group consisting of the positions P51, K55, T56, N58, A74, T77, N80, A81, K84, D86, A94, S116, D117, A124, D128, K132, D135, E151, K162, D191, G197, R204, I218, A228, and E239, and exhibits an increased thermal stability of at least 1.051-fold, at least 1.5-fold, or at least 2-fold; more preferably at least 3-fold, at least 4-fold, or at least 5-fold, and still more prefer-ably at least 6-fold, at least 6.5-fold, or at least 7-fold in comparison to the thermal stability of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five positions selected from the group consisting of the positions P51, K55, T56, N58, A74, T77, N80, A81, K84, D86, A94, S116, D117, A124, D128, K132, D135, E151, K162, D191, G197, R204, I218, A228, and E239, and exhibits an increased thermal stability in comparison to SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably D, E, G, A, V, L, and I; more preferably G, L, and D;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, N, Q, S, C, M, and D; preferably R, K, H, G, A, V, L, I, P, N, Q, S, C, M, and D; more preferably R, G, S, P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably R, and V;
when the amino acid substitution comprises a substitution at amino acid position A74, then the amino acid substitution at position A74 is selected from the group consisting of the substitutions G, I, V, L, P, W, F, Y, C, M, N, Q, S, T, D, E, H, R, and K; preferably R, Q, and H; more preferably R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L;
when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H;
when the amino acid substitution comprises a substitution at amino acid position A81, then the amino acid substitution at position A81 is selected from the group consisting of the substitutions G, V, L, I, P, W, F, Y, C, M, S, N, T, Q, H, R, K, D and E; preferably R, K, and H; more preferably K;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position A94, then the amino acid substitution at position A94 is selected from the group consisting of the substitutions G, I, V, L, P, W, F, Y, C, M, N, Q, S, T, D, E, H, R, and K; preferably G, I, V, L, and P; more preferably G;
when the amino acid substitution comprises a substitution at amino acid position S 116, then the amino acid substitution at position S116 is selected from the group consisting of the substitutions R, K, H, G, A, V, L, I, P, W, F, Y, and D; preferably K, H, and R; more preferably K;
when the amino acid substitution comprises a substitution at amino acid position D117, then the amino acid substitution at position D117 is selected from the group consisting of the substitutions R, K, H, G, A, V, L, I, P, W, F, Y, C, M, S, Q, T, and N; preferably C, M, S, Q, T, and N; more preferably C, M, S, Q, and N; still more preferably N;
when the amino acid substitution comprises a substitution at amino acid position A124, then the amino acid substitution at position A124 is selected from the group consisting of the substitutions D, E, R, K, H, G, V, L, I, and P; preferably K, H and R; more preferably K;
when the amino acid substitution comprises a substitution at amino acid position D128, then the amino acid substitution at position D 128 is selected from the group consisting of the substitutions G, A, V, L, I, P, W, F, Y, C, M, N, Q, S, T, H, R, and K; preferably W, F, Y, C, M, N, Q, S, T, and R; more preferably R, M, F, and Y;
when the amino acid substitution comprises a substitution at amino acid position K132, then the amino acid substitution at position K132 is selected from the group consisting of the substitutions R, H, G, A, V, L, I, P, W, F, Y, C, M, S, Q, T, and N; preferably R, and H; more preferably R;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably G, A, M, R, and N;
when the amino acid substitution comprises a substitution at amino acid position E151, then the amino acid substitution at position E151 is selected from the group consisting of the substitutions D, R, K, H, G, A, V, L, I, P, W, F, Y, C, M, S, Q, T, and N; preferably G, A, V, L, I, and P; more preferably L;
when the amino acid substitution comprises a substitution at amino acid position K162, then the amino acid substitution at position K162 is selected from the group consisting of the substitutions R, H, G, A, V, L, I, P, W, F, Y, C, M, S, Q, T, and N; preferably C. M, S, Q, T, and N; more preferably N;
when the amino acid substitution comprises a substitution at amino acid position D191, then the amino acid substitution at position D 191 is selected from the group consisting of the substitutions G, A, V, L, I, P, C, M, S, Q, and T; preferably G, A, V, L, I, and P; more preferably P;
when the amino acid substitution comprises a substitution at amino acid position G197, then the amino acid substitution at position G197 is selected from the group consisting of the substitutions D, E, R, K, H, G, V, L, I, N, T, S, Q, C, M, Y, F, Wand P; preferably R, K, and H; more preferably K;
when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q;
when the amino acid substitution comprises a substitution at amino acid position I218, then the amino acid substitution at position I218 is selected from the group consisting of the substitutions G, A, V, L, P, W, F, Y, C, M, N, Q, S, T, D, E, H, R, and K; preferably W, Y, and F; more preferably F;
when the amino acid substitution comprises a substitution at amino acid position A228, then the amino acid substitution at position A228 is selected from the group consisting of the substitutions G, I, V, L, P, W, F, Y, C, M, N, Q, S, T, D, E, H, R, and K; preferably H, R, and K; more preferably R; or
when the amino acid substitution comprises a substitution at amino acid position E239, then the amino acid substitution at position E239 is selected from the group consisting of the substitutions G, A, V, L, I, P, W, F, Y, C, M, N, Q, S, T, H, R, and K; preferably A, G, V, L, I, and P; more preferably A.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five substitution selected from the group consisting of the substitutions P51L, P51G, P51D, K55R, T56P, T56D, T56S, T56G, T56R, N58V, N58R, A74R, T77K, T77F, T77L, T77R, N80H, A81K, K84G, D86S, A94G, S116K, D117N, A124K, D128R, D128M, D128Y, D128F, K132R, D135A, D135G, D135N, D135M, D135R, E151L, K162N, D191P, G197K, R204Q, I218F, A228R, and E239A, exhibits an increased thermal stability in comparison to SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions, wherein the substitution is selected from the group consisting of the positions P51, A52, S53, G54, K55, T56, N58, A72, P73, A74, T77, G78, N80, A81, A82, K84, D86, S116, D117, N119, Q120, A124, D128, Q129, D135, P195, K196, G197, D199, and E239; preferably P51, S53, K55, T56, N58, A72, P73, A74, T77, A81, A82, K84, D86, S116, D117, Q120, A124, D128, Q129, D135, K196, G197, D199, and E239, and wherein the nuclease exhibits an increased relative salt activity compared to the relative salt activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions, wherein the substitution is selected from the group consisting of the positions P51, A52, S53, G54, K55, T56, N58, A72, P73, A74, T77, G78, N80, A81, A82, K84, D86, S116, D117, N119, Q120, A124, D128, Q129, D135, P195, K196, G197, D199, and E239; preferably P51, S53, K55, T56, N58, A72, P73, A74, T77, A81, A82, K84, D86, S116, D117, Q120, A124, D128, Q129, D135, K196, G197, D199, and E239, and wherein the nuclease exhibits an in-creased relative salt activity of at least 1.05-fold; preferably at least 7-fold; more preferably at least 9-fold; still more preferably at least 13-fold; yet more preferably at least 60-fold, and even more preferably at least 110-fold compared to the relative salt activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions selected from the group consisting of the positions P51, A52, S53, G54, K55, T56, N58, A72, P73, A74, T77, G78, N80, A81, A82, K84, D86, S116, D117, N119, Q120, A124, D128, Q129, D135, P195, K196, G197, D199, and E239; preferably P51, S53, K55, T56, N58, A72, P73, A74, T77, A81, A82, K84, D86, S116, D117, Q120, A124, D128, Q129, D135, K196, G197, D199, and E239, and exhibits an increased relative salt activity compared to the relative salt activity of SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably G, A, V, L, I, and R; more preferably R, G, and L;
when the amino acid substitution comprises a substitution at amino acid position A52, then the amino acid substitution at position A52 is selected from the group consisting of the substitutions G, V, L, I, P, W, F, Y, C, M, S, N, T, Q, H, R, K, D and E; preferably R, K, and H; more preferably R;
when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, and A;
when the amino acid substitution comprises a substitution at amino acid position G54, then the amino acid substitution at position G54 is selected from the group consisting of the substitutions A, V, L, I, P, W, F, Y, C, M, S, N, T, Q, H, R, K, D and E; preferably R, K, and H; more preferably R;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably R, K, G, A, V, L, I, P, F, S, M, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R;
when the amino acid substitution comprises a substitution at amino acid position A72, then the amino acid substitution at position A72 is selected from the group consisting of the substitutions D, E, R, K, H, G, V, L, I, and P; preferably K, H and R; more preferably K;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G,A, V, L, I, P, W, F, Y, and D; preferably K, R, H, W, F, and Y; more preferably H, K, R, and F;
when the amino acid substitution comprises a substitution at amino acid position G78, then the amino acid substitution at position G78 is selected from the group consisting of the substitutions A, V, L, I, P, W, F, Y, C, M, S, N, T, Q, H, R, K, D and E; preferably R, K, and H; more preferably R;
when the amino acid substitution comprises a substitution at amino acid position N80, then the amino acid substitution at position N80 is selected from the group consisting of the substitutions R, K, H, G, A, V, L, I, P, W, F, Y, C, M, T, Q, S, and D; preferably K, H, and R; more preferably R;
when the amino acid substitution comprises a substitution at amino acid position A81, then the amino acid substitution at position A81 is selected from the group consisting of the substitutions G, V, L, I, P, W, F, Y, C, M, S, N, T, Q, H, R, K, D and E; preferably R, K, and H; more preferably K;
when the amino acid substitution comprises a substitution at amino acid position A82, then the amino acid substitution at position A82 is selected from the group consisting of the substitutions G, V, L, I, P, W, F, Y, C, M, S, N, T, Q, H, R, K, D and E; preferably R, K, and H; more preferably R;
when the amino acid substitution comprises a substitution at amino acid position K84, then the amino acid substitution at position K84 is selected from the group consisting of the substitutions G, A, V, L, I, P, W, F, Y, C, M, S, N, T, Q, H, R, D and E; preferably G, A, V, L, I, P; more preferably G;
when the amino acid substitution comprises a substitution at amino acid position D86, then the amino acid substitution at position D86 is selected from the group consisting of the substitutions G, A, V, L, I, P, W, F, Y, C, M, S, N, T, Q, H, R, K, and E; preferably C, M, S, N, T, and Q; more preferably S;when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K;
when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably N, Y, A, K, H, R, and S;
when the amino acid substitution comprises a substitution at amino acid position N119, then the amino acid substitution at position N119 is selected from the group consisting of the substitutions R, K, H, G, A, V, L, I, P, W, F, Y, C, M, T, Q, S, and D; preferably K, H, and R; more preferably R;
when the amino acid substitution comprises a substitution at amino acid position Q120, then the amino acid substitution at position Q120 is selected from the group consisting of the substitutions R, K, H, G, A, V, L, I, P, W, F, Y, and D; preferably K, H, and R; more preferably R;
when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R;
when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, Y, F, M, N, S, R, and K;
when the amino acid substitution comprises a substitution at amino acid position Q129, then the amino acid substitution at position Q 129 is selected from the group consisting of the substitutions R, K, H, G, A, V, L, I, P, W, F, Y, and D; preferably K, H, and R; more preferably R;
when the amino acid substitution comprises a substitution at amino acid position D135, then the amino acid substitution at position D 135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably G, A, N, R, and K;
when the amino acid substitution comprises a substitution at amino acid position P195, then the amino acid substitution at position P195 is selected from the group consisting of the substitutions G, V, L, I, A, W, F, Y, C, M, S, N, T, Q, H, R, K, D and E; preferably R, K, and H; more preferably R;
when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K;
when the amino acid substitution comprises a substitution at amino acid position D 199, then the amino acid substitution at position D 199 is selected from the group consisting of the substitutions G, A, V, L, I, P, W, F, Y, C, M, Q, T, N, S, H, R, and K; preferably S, Q, T, N, C, M, H, R, and K; more preferably N, S, and K; or
when the amino acid substitution comprises a substitution at amino acid position E239, then the amino acid substitution at position E239 is selected from the group consisting of the substitutions G, A, V, L, I, P, W, F, Y, C, M, Q, T, N, S, H, R, K, and D; preferably G, A, V, L, I, and P; more preferably A.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty substitutions selected from the group consisting of the substitutions P51R, P51G, P51L, P51A, P51V, P51I, A52R, S53K, S53A, G54R, K55R, T56R, T56K, T56M, T56L, T56F, T56I, T56V, T56A, T56G, T56P, T56S, T56D, N58R, N58K, A72K, P73K, P73R, A74R, A74K, T77F, T77H, T77K, T77R, G78R, N80R, A81K, A82R, K84G, D86S, S116K, D117A, D117S, D117K, D117N, D117Y, D117H, D117R, N119R, Q120R, A124K, A124R, A124H, D128R, D128K, D128Y, D128S, D128M, D128N, D128F, D128G, Q129R, D135A, D135R, D135G, D135N, D135K, P195R, K196R, G197K, D199N, D199K, D199S, and E239A; preferably P51R, P51G, P51L, S53K, S53A, K55R, T56R, T56K, T56M, T56L, T56F, T56I, T56V, T56A, T56G, T56P, T56S, T56D, N58R, N58K, A72K, P73K, P73R, A74R, A74K, T77F, T77K, T77R, A81K, A82R, K84G, D86S, S116K, D117A, D117S, D117K, D117N, D117Y, D117H, D117R, Q120R, A124K, A124R, D128R, D128K, D128Y, D128S, D128M, D128N, D128F, D128G, Q129R, D135A, D135R, D135G, D135N, D135K, K196R, G197K, D199N, D199K, D199S, and E239A, and exhibits an increased relative salt activity compared to the relative salt activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions selected from the group consisting of the positions P51, A52, S53, G54, K55, T56, N58, A72, P73, A74, T77, G78, N80, A81, A82, K84, D86, S116, D117, N119, Q120, A124, D128, Q129, D135, P195, K196, G197, D199, and E239; preferably P51, A52, S53, G54, T56, N58, A72, P73, A74, T77, G78, N80, A81, A82, K84, D117, N119, Q120, A124, D128, Q129, D135, P195, K196, G197, and E239; more preferably P51, S53, T56, N58, A72, A74, T77, A81, A82, K84, D117, Q120, A124, D128, Q129, D135, K196, G197, and E239, and exhibits an increased S_{high} activity compared to the S_{high} activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions selected from the group consisting of the positions P51, A52, S53, G54, K55, T56, N58, A72, P73, A74, T77, G78, N80, A81, A82, K84, D86, S116, D117, N119, Q120, A124, D128, Q129, D135, P195, K196, G197, D199, and E239; preferably P51, A52, S53, G54, T56, N58, A72, P73, A74, T77, G78, N80, A81, A82, K84, D117, N119, Q120, A124, D128, Q129, D135, P195, K196, G197, and E239; more preferably P51, S53, T56, N58, A72, A74, T77, A81, A82, K84, D117, Q120, A124, D128, Q129, D135, K196, G197, and E239, and exhibits an increased S_{high} activity of at least 1.01-fold; preferably at least 1.5-fold; more preferably at least 2-fold; still more preferably at least 5-fold; yet more preferably at least 12-fold, and even more preferably at least 19-fold compared to the S_{high} activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions selected from the group consisting of the positions P51, A52, S53, G54, K55, T56, N58, A72, P73, A74, T77, G78, N80, A81, A82, K84, D86, S116, D117, N119, Q120, A124, D128, Q129, D135, P195, K196, G197, D199, and E239; preferably P51, A52, S53, G54, T56, N58, A72, P73, A74, T77, G78, N80, A81, A82, K84, D117, N119, Q120, A124, D128, Q129, D135, P195, K196, G197, and E239; more preferably P51, S53, T56, N58, A72, A74, T77, A81, A82, K84, D117, Q120, A124, D128, Q129, D135, K196, G197, and E239, and exhibits an increased S_{high} activity compared to the S_{high} activity of SEQ ID NO:1 wherein,
when the amino acid substitution comprises a substitution at amino acid position P51, then the amino acid substitution at position P51 is selected from the group consisting of the substitutions D, E, R, K, H, G, A, V, L, and I; preferably G, A, V, L, I, and R; more preferably R, G, and L;
when the amino acid substitution comprises a substitution at amino acid position A52, then the amino acid substitution at position A52 is selected from the group consisting of the substitutions G, V, L, I, P, W, F, Y, C, M, S, N, T, Q, H, R, K, D and E; preferably R, K, and H; more preferably R;
when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, R, and H; still more preferably K;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position G54, then the amino acid substitution at position G54 is selected from the group consisting of the substitutions A, V, L, I, P, W, F, Y, C, M, S, N, T, Q, H, R, K, D and E; preferably R, K, and H; more preferably R;
when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably G, A, V, L, I, P, F, S, M, D, K, and R;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R;
when the amino acid substitution comprises a substitution at amino acid position A72, then the amino acid substitution at position A72 is selected from the group consisting of the substitutions D, E, R, K, H, G, V, L, I, and P; preferably K, H and R; more preferably K;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably W, F, Y, K, R, and H; more preferably K, H, and R; still more preferably K, and R;
when the amino acid substitution comprises a substitution at amino acid position G78, then the amino acid substitution at position G78 is selected from the group consisting of the substitutions A, V, L, I, P, W, F, Y, C, M, S, N, T, Q, H, R, K, D and E; preferably R, K, and H; more preferably R;
when the amino acid substitution comprises a substitution at amino acid position N80, then the amino acid substitution at position N80 is selected from the group consisting of the substitutions R, K, H, G, A, V, L, I, P, W, F, Y, C, M, T, Q, S, and D; preferably K, H, and R; more preferably R;
when the amino acid substitution comprises a substitution at amino acid position A81, then the amino acid substitution at position A81 is selected from the group consisting of the substitutions G, V, L, I, P, W, F, Y, C, M, S, N, T, Q, H, R, K, D and E; preferably R, K, and H; more preferably K;
when the amino acid substitution comprises a substitution at amino acid position A82, then the amino acid substitution at position A82 is selected from the group consisting of the substitutions G, V, L, I, P, W, F, Y, C, M, S, N, T, Q, H, R, K, D and E; preferably R, K, and H; more preferably R;
when the amino acid substitution comprises a substitution at amino acid position K84, then the amino acid substitution at position K84 is selected from the group consisting of the substitutions G, A, V, L, I, P, W, F, Y, C, M, S, N, T, Q, H, R, D and E; preferably G, A, V, L, I, P; more preferably G;
when the amino acid substitution comprises a substitution at amino acid position D86, then the amino acid substitution at position D86 is selected from the group consisting of the substitutions G, A, V, L, I, P, W, F, Y, C, M, S, N, T, Q, H, R, K, and E; preferably C, M, S, N, T, and Q; more preferably S;
when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K;
when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably Y, N, H, R, A, and S;
when the amino acid substitution comprises a substitution at amino acid position N119, then the amino acid substitution at position N119 is selected from the group consisting of the substitutions R, K, H, G, A, V, L, I, P, W, F, Y, C, M, T, Q, S, and D; preferably K, H, and R; more preferably R;
when the amino acid substitution comprises a substitution at amino acid position Q120, then the amino acid substitution at position Q120 is selected from the group consisting of the substitutions R, K, H, G, A, V, L, I, P, W, F, Y, and D; preferably K, H, and R; more preferably R;
when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R;
when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, Y, F, M, N, S, R, and K;
when the amino acid substitution comprises a substitution at amino acid position Q129, then the amino acid substitution at position Q129 is selected from the group consisting of the substitutions R, K, H, G, A, V, L, I, P, W, F, Y, and D; preferably K, H, and R; more preferably R;
when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably A, G, N, R, and K;
when the amino acid substitution comprises a substitution at amino acid position P195, then the amino acid substitution at position P195 is selected from the group consisting of the substitutions G, V, L, I, A, W, F, Y, C, M, S, N, T, Q, H, R, K, D and E; preferably R, K, and H; more preferably R;
when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K;
when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably S, Q, T, N, C, M, H, R, and K; more preferably N, S, and K; or
when the amino acid substitution comprises a substitution at amino acid position E239, then the amino acid substitution at position E239 is selected from the group consisting of the substitutions G, A, V, L, I, P, W, F, Y, C, M, Q, T, N, S, H, R, K, and D; preferably G, A, V, L, I, and P; more preferably A.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, or at least thirty substitutions selected from the group consisting of the substitutions P51L, P51R, P51G, P51A, P51V, P51I, A52R, S53A, S53K, G54R, K55R, T56R, T56K, T56M, T56L, T56F, T56I, T56V, T56A, T56G, T56P, T56S, T56D, N58R, N58K, A72K, P73K, P73R, A74K, A74R, T77R, T77K, T77H, T77F, G78R, N80R, A81K, A82R, K84G, D86S, S116K, D117A, D117S, D117N, D117Y, D117H, D117R, N119R, Q120R, A124K, A124R, A124H, D128R, D128S, D128N, D128K, D128G, D128M, D128Y, D128F, Q129R, D135A, D135K, D135G, D135N, D135R, P195R, K196R, G197K, D199K, D199S, D199N, and E239A; preferably P51L, P51R, P51G, P51A, P51V, P51I, A52R, S53K, G54R, T56R, T56K, T56M, T56L, T56F, T56I, T56V, T56A, T56G, T56P, T56S, T56D, N58R, N58K, A72K, P73R, A74K, A74R, T77R, T77K, T77H, G78R, N80R, A81K, A82R, K84G, D117A, D117S, D117N, D117Y, D117H, D117R, N119R, Q120R, A124K, A124R, A124H, D128R, D128S, D128N, D128K, D128G, D128M, D128Y, D128F, Q129R, D135A, D135K, D135G, D135N, D135R, P195R, K196R, G197K, and E239A; more preferably P51L, P51R, P51G, S53K, T56R, T56K, T56M, T56L, T56F, T56I, T56V, T56A, T56G, T56P, T56S, T56D, N58R, N58K, A72K, A74K, A74R, T77R, T77K, A81K, A82R, K84G, D117A, D117S, D117N, D117Y, D117H, D117R, Q120R, A124K, A124R, D128R, D128S, D128N, D128K, D128G, D128M, D128Y, D128F, Q129R, D135A, D135K, D135G, D135N, D135R, K196R, G197K, and E239A, and exhibits an increased S_{high} activity compared to the S_{high} activity of SEQ ID NO: 1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, or at least twenty-one positions selected from the group consisting of the positions P51, K55, T56, N58, P73, A74, T77, G78, A81, K84, D86, S116, D117, Q120, A124, D128, Q129, D135, G197, D199, and E239; preferably P51, K55, T56, N58, P73, A74, T77, G78, K84, D86, S116, D117, A124, D128, D135, G197, and D199, and exhibits an increased relative cold activity compared to the relative cold activity of SEQ ID NO: 1.

I In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, or at least twenty-one position selected from the group consisting of the positions P51, K55, T56, N58, P73, A74, T77, G78, A81, K84, D86, S116, D117, Q120, A124, D128, Q129, D135, G197, D199, and E239; preferably P51, K55, T56, N58, P73, A74, T77, G78, K84, D86, S116, D117, A124, D128, D135, G197, and D199, and exhibits an increased relative cold activity of at least 1.05-fold; preferably at least 1.5-fold; more preferably at least 2.0-fold, and still more preferably at least 3.0-fold compared to the relative cold activity of SEQ ID NO: 1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, or at least twenty-one positions selected from the group consisting of the positions P51, K55, T56, N58, P73, A74, T77, G78, A81, K84, D86, S116, D117, Q120, A124, D128, Q129, D135, G197, D199, and E239; preferably P51, K55, T56, N58, P73, A74, T77, G78, K84, D86, S116, D117, A124, D128, D135, G197, and D199, and exhibits an increased relative cold activity compared to the relative cold activity of SEQ ID NO: 1, wherein,
when the amino acid substitution comprises a substitution at amino acid position P51, then the amino acid substitution at position P51 is selected from the group consisting of the substitutions D, E, R, K, H, G, A, V, L, and I; preferably D, R, G, and L;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, N, Q, S, C, M, and D; preferably R, K, H, G, A, V, L, I, P, and D; more preferably K, R, P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and V;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L;
when the amino acid substitution comprises a substitution at amino acid position G78, then the amino acid substitution at position G78 is selected from the group consisting of the substitutions R, K, H, A, V, L, I, P, W, F, Y, N, Q, S, T, C, M and D; preferably K, R, and H; more preferably R;
when the amino acid substitution comprises a substitution at amino acid position A81, then the amino acid substitution at position A81 is selected from the group consisting of the substitutions R, K, and H; preferably K;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K;
when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably A, G, V, L, I, P, , R, K, H, W, F, and Y; more preferably Y, H, R, and K; still more preferably K, H, and R; yet more preferably K;
when the amino acid substitution comprises a substitution at amino acid position Q120, then the amino acid substitution at position Q 120 is selected from the group consisting of the substitutions R, K, H, G, A, V, L, I, P, W, F, Y, and D; preferably K, H, and R; more preferably R;
when the amino acid substitution comprises a substitution at amino acid position A124, then the amino acid substitution at position A124 is selected from the group consisting of the substitutions D, E, R, K, H, G, V, L, I, and P; preferably K, H and R; more preferably K;
when the amino acid substitution comprises a substitution at amino acid position D128, then the amino acid substitution at position D 128 is selected from the group consisting of the substitutions G, A, V, L, I, P, W, F, Y, C, M, N, Q, S, T, H, R and K; preferably G, Y, F, M, N, S, R and K;
when the amino acid substitution comprises a substitution at amino acid position Q129, then the amino acid substitution at position Q 129 is selected from the group consisting of the substitutions R, K, H, G, A, V, L, I, P, W, F, Y, and D; preferably K, H, and R; more preferably R;
when the amino acid substitution comprises a substitution at amino acid position D135, then the amino acid substitution at position D 135 is selected from the group consisting of the substitutions G, A, V, L, I, P, W, F, Y, C, M, N, Q, S, T, H, R and K; preferably G, A, V, L, I, P, C, M, N, Q, S, T, H, R and K; more preferably M, G, A, N, R and K;
when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or
when the amino acid substitution comprises a substitution at amino acid position D 199, then the amino acid substitution at position D 199 is selected from the group consisting of the substitutions G, A, V, L, I, P, W, F, Y, C, M, Q, T, N, S, H, R, and K; preferably S, Q, T, N, C, M, H, R, and K; more preferably N, S, and K
when the amino acid substitution comprises a substitution at amino acid position E239, then the amino acid substitution at position E239 is selected from the group consisting of the substitutions G, A, V, L, I, P, W, F, Y, C, M, Q, T, N, S, H, R, K, and D; preferably G, A, V, L, I, and P; more preferably A.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, or at least twenty-one positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, or at least twenty-one substitutions selected from the group consisting of the substitutions P51L, P51D, P51G, P51R, K55R, T56D, T56R, T56P, T56K, T56S, T56G, T56A, T56M, T56F, T56V, T56L, T56I, N58V, N58K, N58R, P73R, P73K, A74K, A74R, T77K, T77L, T77R, T77F, G78R, A81K, K84G, D86S, S116K, D117K, D117H, D117Y, Q120R, A124K, D128Y, D128M, D128N, D128K, D128F, D128S, D128G, D128R, Q129R, D135A, D135K, D135R, D135N, D135G, D135M, G197K, D199N, D199K, D199S, E239A; preferably P51L, P51D, P51G, P51R, K55R, T56D, T56R, T56P, T56K, N58V, N58K, N58R, P73R, P73K, A74K, A74R, T77K, T77L, T77R, T77F, G78R, K84G, D86S, S116K, D117K, A124K, D128Y, D128M, D128N, D128K, D128F, D128S, D128G, D128R, D135A, D135K, D135R, D135N, D135G, G197K, D199N, D199K, and D199S, and exhibits an increased relative cold activity compared to the relative cold activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions selected from the group consisting of the positions P51, K55, T56, N58, P73, A74, T77, G78, A81, K84, D86, S116, D117, Q120, A124, D128, Q129, D135, K196, G197, D199, and E239; preferably P51, T56, N58, T77, A81, K84, D86, S116, D117, Q120, A124, D128, Q129, D135, K196, and E239; more preferably P51, T56, N58, T77, K84, D86, S116, and D135, and exhibits an increased T_{low} activity in comparison to the T_{low} activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions selected from the group consisting of the positions P51, K55, T56, N58, P73, A74, T77, G78, A81, K84, D86, S116, D117, Q120, A124, D128, Q129, D135, K196, G197, D199, and E239; preferably P51, T56, N58, T77, A81, K84, D86, S116, D117, Q120, A124, D128, Q129, D135, K196, and E239; more preferably P51, T56, N58, T77, K84, D86, S116, and D135, and exhibits an increased T_{low} activity of at least 1.01-fold; preferably at least 1.1-fold; more preferably at least 1.3-fold, still more prefer-ably at least 1.5-fold, and yet more preferably at least 1.55-fold in comparison to the T_{low} activity of SEQ ID NO:1.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions selected from the group consisting of the positions P51, K55, T56, N58, P73, A74, T77, G78, A81, K84, D86, S116, D117, Q120, A124, D128, Q129, D135, K196, G197, D199, and E239; preferably P51, T56, N58, T77, A81, K84, D86, S116, D117, Q120, A124, D128, Q129, D135, K196, and E239; more preferably P51, T56, N58, T77, K84, D86, S116, and D135, and exhibits an increased T_{low} activity in comparison to the T_{low} activity of SEQ ID NO:1, wherein,
when the amino acid substitution comprises a substitution at amino acid position P51, then the amino acid substitution at position P51 is selected from the group consisting of the substitutions D, E, R, K, H, G, A, V, L, and I; preferably G, A, V, I, and L; more preferably G, and L; still more preferably L;
when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably R, K, H, G, A, V, L, I, P, and D; more preferably G, A, V, L, I, P, and D; still more preferably G, P, and D;
when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably R, K, and V; still more preferably R, and V;
when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
when the amino acid substitution comprises a substitution at amino acid position T77, then the amino acid substitution at position T77 is selected from the group consisting of the substitutions R, K, H, G, A, V, L, I, P, W, F, Y, and D; preferably L, F, K, R, and H; more preferably K, R, and H; still more preferably R;
when the amino acid substitution comprises a substitution at amino acid position G78, then the amino acid substitution at position G78 is selected from the group consisting of the substitutions R, K, H, A, V, L, I, P, W, F, Y, N, Q, S, T, C, M and D; preferably K, R, and H; more preferably R;
when the amino acid substitution comprises a substitution at amino acid position A81, then the amino acid substitution at position A81 is selected from the group consisting of the substitutions R, K, and H; preferably K;
when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K;
when the amino acid substitution comprises a substitution at amino acid position D 117, then the amino acid substitution at position D 1 17 is selected from the group consisting of the substitutions G, A, V, L, I, P, W, F, Y, C, M, S, N, T, Q, H, R, and K; preferably G, V, L, I, P, W, F, Y, C, M, S, N, Q, H, R, and K; more preferably Y, W, F, K, R, and H; still more preferably Y, K, R, and H; yet preferably K, R, and H; even more preferably K;
when the amino acid substitution comprises a substitution at amino acid position Q120, then the amino acid substitution at position Q 120 is selected from the group consisting of the substitutions R, K, H, G, A, V, L, I, P, W, F, Y, and D; preferably K, H, and R; more preferably R;
when the amino acid substitution comprises a substitution at amino acid position A124, then the amino acid substitution at position A 124 is selected from the group consisting of the substitutions D, E, R, K, H, G, V, L, I, and P; preferably K, Hand R; more preferably K;
when the amino acid substitution comprises a substitution at amino acid position D 128, then the amino acid substitution at position D 128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, R, and K; more preferably G, Y, F, M, N, S, R, and K;when the amino acid substitution comprises a substitution at amino acid position Q129, then the amino acid substitution at position Q 129 is selected from the group consisting of the substitutions R, K, H, G, A, V, L, I, P, W, F, Y, and D; preferably K, H, and R; more preferably R;
when the amino acid substitution comprises a substitution at amino acid position D135, then the amino acid substitution at position D 135 is selected from the group consisting of the substitutions G, A, V, L, I, P, W, F, Y, C, M, N, Q, S, T, H, R, and K; preferably R, K, G, A, V, L, I, P, C, M, N, Q, S, and T; more preferably G, A, V, L, I, P, C, M, N, Q, S, and T; still more preferably A, and N;
when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
when the amino acid substitution comprises a substitution at amino acid position G197, then the amino acid substitution at position G 197 is selected from the group consisting of the substitutions D, E, R, K, H, A, V, L, I, and P; preferably K, H, and R; more preferably K;
when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably G, A, V, L, I, P, W, F, Y, C, M, Q, T, N, S, H, and K; more preferably S, Q, T, N, C, M, H, and K; still more preferably S, N, and K; or
when the amino acid substitution comprises a substitution at amino acid position E239, then the amino acid substitution at position E239 is selected from the group consisting of the substitutions G, A, V, L, I, P, W, F, Y, C, M, Q, T, N, S, H, R, K, and D; preferably G, A, V, L, I, and P; more preferably A.

In preferred embodiments of the nuclease according to the invention, the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two substitutions selected from the group consisting of the substitutions P51L, P51D, P51G, P51R, K55R, T56M, T56L, T56S, T56A, T56V, T56I, T56G, T56D, T56P, T56R, T56K, T56F, N58V, N58K, N58R, P73K, P73R, A74K, A74R, T77K, T77L, T77R, T77F, G78R, K84G, D86S, S116K, D117K, D117H, D117Y, D117R, Q120R, A124K, D128Y, D128M, D128N, D128K, D128F, D128R, D128S, D128G, Q129R, D135A, D135K, D135R, D135N, D135M, D135G, K196R, G197K, D199K, D199S, D199N, and E239A; preferably P51L, T56A, T56V, T56I, T56M, T56L, T56F, T56D, T56P, T56G, T56K, T56S, N58V, N58R, T77K, T77R, K84G, D86S, S116K, D117H, D117R, D117Y, Q120R, A124K, D128G, D128N, D128S, Q129R, D135A, D135M, D135N, K196R, and E239A; more preferably P51L, T56D, T56P, T56G, N58V, N58R, T77R, K84G, D86S, S116K, D135A, and D135N, and exhibits an increased T_{low} activity in comparison to the T_{low} activity of SEQ ID NO:1.

In preferred embodiments of the nuclease according to the invention, the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, at least thirty-one, at least thirty-two, at least thirty-three, at least thirty-four, at least thirty-five, at least thirty-six, at least thirty-seven, at least thirty-eight, at least or at thirty-nine amino acid positions, wherein the amino acid sequence in addition comprises at least one further substitution, wherein the position of the at least one further substitution is selected from the group consisting of amino acid positions P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239.

In preferred embodiments, the nuclease according to the invention is characterized by either
(A) an increased thermal stability in comparison to the wildtype nuclease of SEQ ID NO:1; and/or
(B) an increased relative salt activity in comparison to the relative salt activity of the wildtype nuclease of SEQ ID NO:1; and/or
(C) an increased S_{high} activity in comparison to the S_{high} activity of the wildtype nuclease of SEQ ID NO:1; and/or
(D) an increased relative cold activity in comparison to the relative cold activity of the wildtype nuclease of SEQ ID NO:1; and/or
(E) an increased T_{low} activity in comparison to the T_{low} activity of the wildtype nuclease of SEQ ID NO:1;
and wherein the identity of the amino acid sequence of the nuclease with the sequence of SEQ ID NO:1 is at least 65%, or at least 70%, or at least 75%, or at least 80%; preferably at least 86%, or at least 87%, or at least 88%, or at least 89%, or at least 90%; more preferably at least 91%, or at least 92%, or at least 93%, or at least 94%, or at least 95%; still more preferably at least 96%, or at least 97%, or at least 98%, or at least 99%; yet more preferably at least 99.1%, or at least 99.2%, or at least 99.3%, or at least 99.4, or at least 99.5%; even more preferably at least 99.6%.

Preferably, the disclosures of any one of the embodiments or preferred embodiments of the nuclease; preferably the endonuclease, under this first aspect of the invention are also embodiments or preferred embodiments of any other embodiment under this first aspect of the invention. It will be appreciated that the disclosures of any of the embodiments or preferred embodiments of the nuclease; preferably the endonuclease, under this first aspect of the invention refer to each other and are also deemed embodiments or preferred embodiments of each other.

### Method of hydrolyzing polynucleotide substrates

Another aspect of the invention relates to a method of hydrolyzing polynucleotide substrates; preferably being contained in a sample, comprising the steps of
(a) providing a nuclease according to the invention as described above;
(b) providing a composition containing a polynucleotide substrate;
(c) contacting the polynucleotide substrate in the composition provided in step (b) with the nuclease provided in step (a) and allowing the nuclease to hydrolyze the polynucleotide substrate thereby obtaining a composition containing cleaved polynucleotide.

In preferred embodiments of the method according to the invention, the nuclease is hydrolyzing the polynucleotide substrate into cleaved polynucleotides or oligonucleotides.

In preferred embodiments of the method according to the invention, step (c) is carried out at a temperature,
between 4°C and 90°C; preferably between 10°C and 80°C; more preferably between 20°C and 65°C; still more preferably between 25°C and 65°; yet more preferably between 30°C and 50°C; even more preferably between 34°C and 42°C; most preferably at 37°C; or
between 20°C and 90°C; preferably between 23°C and 80°C; more preferably between 25°C and 65°C; still more preferably between 30°C and 65°C; yet more preferably be-tween 37°C and 65°C; even more preferably 50°C and 65°C; most preferably at 51°C; or
between 4°C and 65°C; preferably between 4°C and 38°C; more preferably between 4°C and 25°C; still more preferably between 4°C and 20°C; yet more preferably between 4°C and 15°C; even more preferably between 4°C and 10°C; most preferably between 4°C and 6°C, and utmost preferably at 5°C.

In preferred embodiments of the method according to the invention, step (c) is carried out at a temperature between 20°C and 42°C; preferably between 25°C and 42°C; more preferably between 28°C and 40°C, and still more preferably 37°C.

In preferred embodiments of the method according to the invention, step (c) is carried out at a temperature between 30°C and 65°C; preferably between 30°C and 55°C; more preferably between 40°C and 52°C, and still more preferably 51°C.

In preferred embodiments of the method according to the invention, step (c) is carried out at a temperature between 4°C and 25°C; preferably between 4°C and 15°C; more preferably between 4°C and 10°C; still more preferably between 4°C and 6°C, and yet more preferably at 5°C.

In preferred embodiments of the method according to the invention, step (c) is carried out in the presence of salts, wherein the salt is NaCl or KCl; preferably NaCl.

In preferred embodiments of the method according to the invention, step (c) is carried out at a concentration of salt from 0.00M to 1.00M, or from 0.01M to 1.00M; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.

In preferred embodiments of the method according to the invention, step (c) is carried out in the absence of salts.

In preferred embodiments, the method according to the invention comprises the additional step of
(d) inactivating the nuclease after step (c) thereby obtaining a composition containing cleaved polynucleotides and an inactivated nuclease.

In preferred embodiments of the method according to the invention, the inactivation of the nuclease in step (d) is carried out
(a) by heat inactivation; and/or
(b) by decreasing the pH value of the composition to lower than pH 5; and/or
(c) by increasing the pH value of the composition to higher than pH 11; and/or
(d) by adding chelating agents to the composition; and/or
(e) by adding oxidizing agents to the composition; and/or
(f) by adding protein denaturant agents; preferably detergents; and/or
(g) by adding enzymes hydrolyzing proteins; preferably a protease.

In preferred embodiments, the method according to the invention comprises the additional step of
(e) separating the nuclease from the composition containing cleaved polynucleotide obtained in step
(c) and optionally inactivated nuclease obtained in step (d).

In preferred embodiments of the method according to the invention, step (e) is carried out by use of a chromato-graphic method, and/or ultrafiltration, and/or tangential flow filtration.

Another aspect of the invention relates to the use of a nuclease according to the invention as described above for hydrolyzing polynucleotide substrates; preferably being contained in a composition.

In preferred embodiments of the use according to the invention, the polynucleotide substrates in the composition are derived from a cell; preferably a cell selected from
- a cell from the domain Eukaryota; preferably an immortalized cell line, or a stem cell, or a tissue cell, or a fungal cell; more preferably from the specie Pichia pastoris, or from a specie from the genus Aspergillus, or from a specie from the genus Saccharomyces; and/or
- a cell from the domain Bacteria; preferably a bacterial cell; more preferably from the specie Escherichia coli, or from a specie from the genus Bacillus; and/or
- a cell from the domain Archea.

In preferred embodiments of the use according to the invention, the polynucleotide substrate is re-leased into the composition from a cell by cell disruption, secretion, lysis, or cell burst.

In preferred embodiments of the use according to the invention, the composition is derived from lysed cells from a cell fermentation process.

In preferred embodiments of the use according to the invention, the composition is an intermediate product or product from a production process for biopharmaceuticals, or pharmaceutical compositions, medicaments, or vaccines, or viral vectors.

In preferred embodiments of the use according to the invention, the viral vector is selected from the group consisting of adeno associated virus, retrovirus, lentivirus, adenovirus, retrovirus, plant virus, bacteriophage, and hybrid vector virus.

In preferred embodiments of the use according to the invention, the composition is an intermediate product or product during preparation of patient tissue samples for use in vitro diagnostics.

In preferred embodiments of the use according to the invention, the biopharmaceuticals, or pharmaceutical compositions, medicaments, or vaccines, or viral vectors are used in gene therapy, and/or tissue therapy, and/or in vitro diagnostic.

In preferred embodiments of the use according to the invention, the nuclease according to the invention is used for the reduction of viscosity in cell lysates.

In preferred embodiments of the use according to the invention, the nuclease according to the invention is used for sample preparation in electrophoresis and/or chromatographic methods.

In preferred embodiments of the use according to the invention, the nuclease is used at a temperature
(a) between 4°C and 90°C; preferably between 10°C and 80°C; more preferably be-tween 20°C and 65°C; still more preferably between 25°C and 65°; yet more preferably between 30°C and 50°C; even more preferably between 34°C and 42°C; most preferably at 37°C; or
(b) between 20°C and 90°C; preferably between 23°C and 80°C; more preferably between 25°C and 65°C; still more preferably between 30°C and 65°C; yet more preferably between 37°C and 65°C; even more preferably 50°C and 65°C; most preferably at 51°C; or
(c) between 4°C and 65°C; preferably between 4°C and 38°C; more preferably be-tween 4°C and 25°C; still more preferably between 4°C and 20°C; yet more preferably between 4°C and 15°C; even more preferably between 4°C and 10°C; most preferably between 4°C and 6°C, and utmost preferably at 5°C.

In preferred embodiments of the use according to the invention, the nuclease is used at a concentration of salt from 0.00M to 1.00M, or from 0.01M to 1.00M; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.

In preferred embodiments of the use according to the invention, the nuclease is used in the absence of salts.

Another aspect of the invention relates to a kit for cleaving a polynucleotide in method according to the invention as described above, or for being used according to the invention as defined above, the kit comprising the nuclease according to the invention as described above, a buffer for hydrolyzing polynucleotide substrates and optionally an agent and/or device for inactivation and/or removal of the nuclease.

The Table 1-A below shows the origin name of the wild-type sequences of certain selected nucleases described in this application. The Tables 1-B, 1-C, 1-D, 1-E, 1-F, 1-G, and 1-H below shows a summary of exemplary variants of the nuclease according to the present invention. For each individual variant with SEQ ID NO: 2 to SEQ ID NO: 102 the substitutions in comparison to the reference sequence SEQ ID NO: 1 (wild-type enzyme from *Serratia marcescens*) are listed in Tables 1-B, 1-C, 1-D, 1-E, 1-F, 1-G, and 1-H.

**Table 1-A: Wildtype sequences of selected nucleases; preferably endonucleases**

| SEQ ID NO | wildtype organism |
|---|---|
| 1 | *S. marcescens* |
| 103 | *V.salmonicida* |
| 104 | *S.ficaria* |
| 105 | *S.odorifera* |

**Table 1-B: Variants with one mutation (substitution) in comparison to SEQ ID NO:1 (=wild-type, S. marcescens)**

| SEQ ID NO | 1st |
|---|---|
| 2 | P51L |
| 3 | P51R |
| 4 | P51G |
| 5 | P51D |
| 6 | S53K |
| 7 | S53A |
| 8 | K55R |
| 9 | T56P |
| 10 | T56D |
| 11 | T56R |
| 12 | T56K |
| 13 | N58V |
| 14 | N58R |
| 15 | N58K |
| 16 | A72K |
| 17 | P73K |
| 18 | P73R |
| 19 | A74R |
| 20 | A74K |
| 21 | T77L |
| 22 | T77K |
| 23 | T77F |
| 24 | T77R |
| 25 | N80H |
| 26 | K84G |
| 27 | D86S |
| 28 | S116K |
| 29 | D117K |
| 30 | D117A |
| 31 | D117S |
| 32 | D117N |
| 33 | D117R |
| 34 | A124K |
| 35 | A124R |
| 36 | D128M |
| 37 | D128Y |
| 38 | D128K |
| 39 | D128S |
| 40 | D128N |
| 41 | D128F |
| 42 | D128G |
| 43 | D128R |
| 44 | D 135N |
| 45 | D135K |
| 46 | D135G |
| 47 | D135R |
| 48 | D191P |
| 49 | G197K |
| 50 | D 199N |
| 51 | D199K |
| 52 | D199S |
| 53 | R204Q |
| 54 | E239A |
| 106 | I218F |
| 107 | K132R |
| 108 | E151L |
| 109 | A94G |
| 110 | K162N |
| 111 | A228R |
| 112 | G78R |
| 113 | A81K |
| 114 | Q 120R |
| 115 | A82R |
| 116 | D135A |
| 117 | K196R |
| 118 | T56S |
| 119 | T56G |
| 145 | A52R |
| 146 | G54R |
| 147 | P73R |
| 148 | A74R |
| 149 | G78R |
| 150 | N80R |
| 151 | N119R |
| 152 | P195R |

**Table 1-C: Variants with two mutations (substitutions) in comparison to SEQ ID NO: 1 (=wild-type, S. marcescens)**

| SEQ ID NO | 1st | 2nd |
|---|---|---|
| 67 | T77K | A124K |
| 74 | P51L | T56P |
| 76 | T56P | K84G |
| 77 | P51L | T56D |
| 79 | P51L | T56K |
| 80 | P51L | T56S |
| 81 | P51L | T56P |
| 83 | P51L | T56G |
| 88 | P51L | T56A |
| 89 | P51L | T56V |
| 90 | P51L | T56I |
| 92 | P51L | T56F |
| 94 | P51L | T56L |
| 96 | P51L | T56M |
| 153 | P51L | T77R |
| 154 | P51L | T77K |
| 155 | P51G | T77K |
| 156 | P51G | T77R |
| 157 | P51L | T77H |
| 158 | P51G | T77H |
| 159 | P51A | T77R |
| 160 | P51V | T77R |
| 161 | P51I | T77R |

**Table 1-D: Variants with three mutations (substitutions) in comparison to SEQ ID NO:1 (=wild-type, S. marcescens)**

| SEQ ID NO | 1st | 2nd | 3rd |
|---|---|---|---|
| 62 | T56R | T77K | A124K |
| 64 | P51L | T77R | A124K |
| 65 | P51L | N58R | A124K |
| 66 | P51L | N58R | T77R |
| 73 | P51L | T56P | D 135N |
| 75 | P51L | T56P | K84G |
| 78 | P51L | T56D | Q 120R |
| 82 | P51L | T56D | D128S |
| 84 | P51L | T56D | D128N |
| 85 | P51L | T56D | D117R |
| 86 | P51L | T56D | T77K |
| 87 | P51L | T56D | D128G |
| 91 | P51L | T56D | Q 129R |
| 93 | P51L | T56D | D117H |
| 95 | P51L | T56D | K196R |
| 97 | P51L | T56D | D117Y |
| 98 | P51L | T56D | D135A |
| 99 | P51L | T56D | D135M |
| 100 | P51L | T56D | R204Q |
| 101 | P51L | T56D | N80H |
| 102 | P51L | T56D | E239A |
| 120 | P51L | T56G | A81K |
| 121 | P51L | T56K | D135A |
| 122 | P51L | T56P | A124K |
| 123 | P51L | T56P | D135A |
| 144 | P51L | T56D | A81K |
| 162 | P51L | T77R | A124H |
| 163 | P51L | T77R | A124R |
| 164 | P51L | T77R | Q 120R |
| 165 | P51L | T77R | A81K |
| 166 | P51L | T77R | S53K |
| 167 | P51L | T77R | A52R |
| 168 | P51L | T77R | G54R |
| 169 | P51L | T77R | P73R |
| 170 | P51L | T77R | A74R |
| 171 | P51L | T77R | G78R |
| 172 | P51L | T77R | N80R |
| 173 | P51L | T77R | N119R |
| 174 | P51L | T77R | P195R |
| 175 | P51L | T77R | T56K |
| 176 | P51L | T77R | T56R |
| 177 | P51L | T77R | N58K |
| 178 | P51L | T77K | N58R |
| 179 | P51L | T77K | A124H |
| 180 | P51L | T77K | A124K |
| 181 | P51G | T77K | A124K |
| 182 | P51G | T77K | A124R |
| 183 | P51G | T77K | A124H |
| 184 | P51G | T77R | A124R |
| 185 | P51G | T77R | A124K |
| 186 | P51G | T77R | A124H |
| 187 | P51L | T77H | A124R |
| 188 | P51L | T77H | A124K |
| 189 | P51L | T77H | A124H |
| 190 | P51G | T77H | A124R |
| 191 | P51G | T77H | A124K |
| 192 | P51G | T77H | A124H |
| 193 | P51A | T77R | A124K |
| 194 | P51V | T77R | A124K |
| 195 | P51I | T77R | A124K |

**Table 1-E: Variants with four mutations (substitutions) in comparison to SEQ ID NO:1 (=wild-type, S. marcescens)**

| SEQ ID NO | 1st | 2nd | 3rd | 4th |
|---|---|---|---|---|
| 56 | N58R | T77R | A124K | G197K |
| 57 | P51L | N58R | T77K | A124K |
| 59 | P51L | T77R | A124K | G197K |
| 60 | P51L | N58R | A124K | G197K |
| 61 | P51L | T56R | T77R | A124K |
| 63 | P51L | T56R | N58R | A124K |
| 70 | P51L | A124K | D128R | D135R |
| 124 | P51L | T56S | A81K | E239A |
| 125 | P51L | T56D | Q120R | A124K |
| 126 | P51L | T56D | A81K | A124K |
| 127 | P51L | T56K | A81K | A124K |

**Table 1-F: Variants with five mutations (substitutions) in comparison to SEQ ID NO: 1 (=wild-type, S. marcescens)**

| SEQ ID NO | 1st | 2nd | 3rd | 4th | 5th |
|---|---|---|---|---|---|
| 55 | P51L | N58R | T77K | A124K | G197K |
| 58 | P51L | T56R | T77K | A124K | G197K |
| 68 | T56R | T77K | A124K | D135R | G197K |
| 71 | P51L | N58R | A124K | D128R | G197K |
| 72 | P51L | N58R | A124K | D128R | D135R |
| 128 | P51L | T56G | T77K | A81K | E239A |
| 129 | P51L | T56D | A81K | A124K | E239A |
| 130 | P51L | T56D | A81K | D135A | E239A |
| 131 | P51L | T56G | T77K | D135A | E239A |
| 132 | P51L | T56D | T77K | Q120R | D135A |
| 133 | P51L | T56K | T77K | A81K | D135A |
| 134 | P51L | T56S | T77K | A81K | D135A |
| 135 | P51L | T56G | Q 120R | A124K | D135A |
| 136 | P51L | T56D | A81K | D117H | A124K |

**Table 1-G: Variants with six mutations (substitutions) in comparison to SEQ ID NO: 1 (=wild-type, S. marcescens)**

| SEQ ID NO | 1st | 2nd | 3rd | 4th | 5th | 6th |
|---|---|---|---|---|---|---|
| 69 | P51L | T56R | N58R | T77K | A124K | D135R |
| 137 | P51L | T56S | T77K | A81K | D135A | E239A |
| 138 | P51L | T56D | T77K | A81K | D117H | D135A |
| 139 | P51L | T56D | T77K | Q120R | D128N | D135A |
| 140 | P51L | T56D | T77K | A81K | Q120R | D135A |
| 141 | P51L | T56D | A81K | Q120R | A124K | D135A |

**Table 1-H: Variants with seven mutations (substitutions) in comparison to SEQ ID NO:1 (=wild-type, S. marcescens)**

| SEQ ID NO | 1st | 2nd | 3rd | 4th | 5th | 6th | 7th |
|---|---|---|---|---|---|---|---|
| 142 | P51L | T56D | A81K | Q120R | A124K | D135A | E239A |
| 143 | P51L | T56D | T77K | A81K | Q120R | A124K | D135A |

Preferred embodiments of the invention are compiled as clauses 1 to 158 hereinafter:
Clause 1: A nuclease; preferably an endonuclease; more preferably a non-specific endonuclease, comprising an amino acid sequence with at least 65% identity to SEQ ID NO:1, wherein the amino acid sequence is substituted compared to SEQ ID NO:1 in at least one amino acid position selected from the group consisting of P51, S53, K55, T56, N58, A72, P73, A74, T77, G78, N80, A81, A82, K84, D86, S116, D117, Q120, A124, D128, Q129, K132, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239.
Clause 2: The nuclease according to clause 1, wherein - a substitution in position P51, or P73 means that the P is substituted by an amino acid selected from the group consisting of A, R, N, D, C, Q, E, G, H, I, L, K, M, F, T, W, Y, V, or S; and/or - a substitution in position S53, or S116 means that the S is substituted by an amino acid selected from the group consisting of A, R, N, D, C, Q, E, G, H, I, L, K, M, F, P, T, W, Y, or V; and/or - a substitution in position K55, K84, K132, or K196, means that the K is substituted by an amino acid selected from the group consisting of A, R, N, D, C, Q, E, G, H, I, L, M, F, P, T, W, Y, V, or S; and/or - a substitution in position T56, or T77 means that the T is substituted by an amino acid selected from the group consisting of A, R, N, D, C, Q, E, G, H, I, L, K, M, F, P, W, Y, V, or S; and/or - a substitution in position N58, or N80 means that the N is substituted by an amino acid selected from the group consisting of A, R, D, C, Q, E, G, H, I, L, K, M, F, P, T, W, Y, V, or S; and/or - a substitution in position A72, A74, A81, A82, or A124 means that the A is substituted by an amino acid selected from the group consisting of R, N, D, C, Q, E, G, H, I, L, K, M, F, P, T, W, Y, V, or S; and/or - a substitution in position G78, or G197 means that the G is substituted by an amino acid selected from the group consisting of A, R, N, D, C, Q, E, H, I, L, K, M, F, P, T, W, Y, V, or S; and/or - a substitution in position D86, D117, D128, D135, D191, or D199 means that the D is substituted by an amino acid selected from the group consisting of A, R, N, C, Q, E, G, H, I, L, K, M, F, P, T, W, Y, V, or S; and/or - a substitution in position Q120, or Q129 means that the Q is substituted by an amino acid selected from the group consisting of A, R, N, D, C, E, G, H, I, L, K, M, F, P, T, W, Y, V, or S; and/or - a substitution in position R204 means that the R is substituted by an amino acid selected from the group consisting of A, N, D, C, Q, E, G, H, I, L, K, M, F, P, T, W, Y, V, or S; and/or - a substitution in position E239 means that the E is substituted by an amino acid selected from the group consisting of A, R, N, D, C, Q, G, H, I, L, K, M, F, P, T, W, Y, V, or S.
Clause 3: The nuclease according to any of the preceding clauses, wherein the at least one amino acid position is substituted by - a positively charged amino acid selected from R, K, and H; or - a negatively charged amino acid selected from E and D; - an aliphatic nonpolar amino acid selected from G, A, V, L, I, and P; or - an aromatic amino acid selected from W, F, and Y; or - an aliphatic polar non-charged amino acid selected from N, Q, S, T, C, and M.
Clause 4: The nuclease according to any of the preceding clauses, wherein the at least one amino acid position is - a positively charged amino acid and substituted by another positively charged amino acid; or - a negatively charged amino acid and substituted by another negatively charged amino acid; or - an aliphatic nonpolar amino acid and substituted by another aliphatic nonpolar amino acid; or - an aromatic amino acid and substituted by another aromatic amino acid; or - an aliphatic polar non-charged amino acid and substituted by another aliphatic polar non-charged amino acid.
Clause 5: The nuclease according to any of the preceding clauses, wherein the at least one amino acid position is - a positively charged amino acid and is substituted by an aliphatic nonpolar amino acid, or an aromatic amino acid, or an aliphatic polar non-charged, or a negatively charged amino acid; or - a negatively charged amino acid and is substituted by an aliphatic nonpolar amino acid, or an aromatic amino acid, or an aliphatic polar non-charged, or a positively charged amino acid; or - an aliphatic nonpolar amino acid and is substituted by an aromatic amino acid, or an aliphatic polar non-charged, or a positively charged amino acid, or a negatively charged amino acid; or - an aromatic amino acid and is substituted by an aliphatic nonpolar amino acid, or an aliphatic polar non-charged, or a positively charged amino acid, or a negatively charged amino acid; or - an aliphatic polar non-charged amino acid and is substituted by an aliphatic nonpolar amino acid, or an aromatic amino acid, or a positively charged amino acid, or a negatively charged amino acid.
Clause 6: The nuclease according to any of the preceding clauses, wherein the amino acid sequence is substituted compared to SEQ ID NO: 1 in one, two, three, four, five, six, seven, eight, nine, ten, eleven, twelve, thirteen, fourteen, fifteen, sixteen, eighteen, nineteen, twenty, twenty-one, twenty-two, twenty-three, twenty-four, twenty-five, twenty-six, twenty-seven, twenty-eight, or twenty-nine amino acid positions.
Clause 7: The nuclease according to any of the preceding clauses, wherein the amino acid sequence is substituted compared to SEQ ID NO: 1 in 1 to 86 amino acid positions; preferably in 1 to 84 amino acid positions; more preferably in 1 to 50 amino acid positions; still more preferably in 1 to 27 amino acid positions; yet more preferably in 1 to 11 amino acid positions, and even more preferably 1 to 6 amino acid positions.
Clause 8: A nuclease; preferably an endonuclease; more preferably a non-specific endonuclease; still more preferably the nuclease according to any of the preceding clauses, comprising an amino acid sequence with at least 65% identity to SEQ ID NO:1, wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239, wherein a substitution in position P51, A72, P73, A74, A124, or G197 means that the P, A or G is substituted by - a positively charged amino acid; or - a negatively charged amino acid; or - a different aliphatic nonpolar amino acid; and wherein a substitution in position in S53, T56, N58, T77, N80, or S116 means that the S, T or N is substituted by - a positively charged amino acid; or - an aliphatic nonpolar amino acid; or - an aromatic amino acid; or - an aliphatic polar non-charged amino acid; or - a negatively charged amino acid; and wherein a substitution in position in K55, K84, R204, or K196 means that the K or R is substituted by - an aliphatic nonpolar amino acid; or - a different positively charged amino acid; or - an aliphatic polar non-charged amino acid; and wherein a substitution in position D86, or D191 means that the D is substituted by - an aliphatic nonpolar amino acid; or - a polar non-charged amino acid; and wherein a substitution in position D128, D135, D117, E239 or D199 means that the D or E is substituted by - an aliphatic nonpolar amino acid; or - an aromatic amino acid; or - an aliphatic polar non-charged amino acid; or - a positively charged amino acid.
Clause 9: A nuclease; preferably an endonuclease; more preferably a non-specific endonuclease; still more preferably the nuclease according to any of the preceding clauses, comprising an amino acid sequence with at least 65% identity to SEQ ID NO:1, wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239; wherein a substitution in position P51, A72, P73, A74, A124, or G197 means that the P, A or G is substituted by - a positively charged amino acid selected from R, K, and H; or - a negatively charged amino acid selected from D, and E; or - a different aliphatic nonpolar amino acid selected from G, A, V, L, I, and P; and wherein a substitution in position in S53, T56, N58, T77, N80, or S116 means that the S, T or N is substituted by - a positively charged amino acid selected from R, K, and H; or - an aliphatic nonpolar amino acid selected from G, A, V, L, I, and P; or - an aromatic amino acid selected from W, F, and Y; or - an aliphatic polar non-charged amino acid selected from N, Q, S, T, C, and M; preferably S, and M; or - D; and wherein a substitution in position in K55, K84, R204, or K196 means that the K or R is substituted by - an aliphatic nonpolar amino acid selected from of G, A, V, L, I, and P; or - a different positively charged amino acid selected from R, K, and H; or - an aliphatic polar non-charged amino acid selected from N, Q, S, T, C, and M; and wherein a substitution in position D86, or D191 means that the D is substituted by - an aliphatic nonpolar amino acid selected from G, A, V, L, I, and P; or - a polar non-charged amino acid selected from N, Q, S, T, C, and M; preferably Q, S, T, C, and M; and wherein a substitution in position D128, D135, D117, E239 or D199 means that the D or E is substituted by - an aliphatic nonpolar amino acid selected from G, A, V, L, I, and P; or - an aromatic amino acid selected from W, F, and Y; or - an aliphatic polar non-charged amino acid selected from N, Q, S, T, C, and M; or - a positively charged amino acid selected from R, K, and H; preferably K, and H.
Clause 10: A nuclease; preferably an endonuclease; more preferably a non-specific endonuclease; still more preferably the nuclease according to any of the preceding clauses, comprising an amino acid sequence with at least 67% identity to SEQ ID NO:1; wherein the amino acid sequence is substituted compared to SEQ ID NO:1 in at least one amino acid position selected from the group consisting of P51, T56, N58, P73, T77, A124, D128, D135, G197, R204, and E239.
Clause 11: A nuclease; preferably an endonuclease; more preferably a non-specific endonuclease; still more preferably the nuclease according to any of the preceding clauses, comprising an amino acid sequence with at least 89% identity to SEQ ID NO:1; wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239; wherein a substitution in position P51, A72, P73, A74, A124, or G197 means that the P, A or G is substituted by - a positively charged amino acid; or - a negatively charged amino acid; or - a different aliphatic nonpolar amino acid; and wherein a substitution in position in S53, T56, N58, T77, N80, or S116 means that the S, T or N is substituted by - a positively charged amino acid; or - an aliphatic nonpolar amino acid; or - an aromatic amino acid; or - an aliphatic polar non-charged amino acid; or - a negatively charged amino acid; and wherein a substitution in position in K55, K84, R204, or K196 means that the K or R is substituted by - an aliphatic nonpolar amino acid; or - a different positively charged amino acid; or - an aliphatic polar non-charged amino acid; and wherein a substitution in position D86, or D191 means that the D is substituted by - an aliphatic nonpolar amino acid; or - a polar non-charged amino acid; and wherein a substitution in position D128, D135, D117, E239, or D199 means that the D or E is substituted by - an aliphatic nonpolar amino acid; or - an aromatic amino acid; or - an aliphatic polar non-charged amino acid; or - a positively charged amino acid.
Clause 12: A nuclease; preferably an endonuclease; more preferably a non-specific endonuclease; still more preferably the nuclease according to any of the preceding clauses, comprising an amino acid sequence with at least 89% identity to SEQ ID NO:1; wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239; wherein a substitution in position P51, A72, P73, A74, A124, or G197 means that the P, A or G is substituted by - a positively charged amino acid selected from R, K, and H; or - a negatively charged amino acid selected from D, and E; or - a different aliphatic nonpolar amino acid selected from G, A, V, L, I, and P; and wherein a substitution in position in S53, T56, N58, T77, N80, or S116 means that the S, T or N is substituted by - a positively charged amino acid selected from R, K, and H; or - an aliphatic nonpolar amino acid selected from G, A, V, L, I, and P; or - an aromatic amino acid selected from W, F, and Y; or - an aliphatic polar non-charged amino acid selected from N, Q, S, T, C, and M; preferably S, and M; more preferably M; or - D; and wherein a substitution in position in K55, K84, R204, or K196 means that the K or R is substituted by - an aliphatic nonpolar amino acid selected from of G, A, V, L, I, and P; or - a different positively charged amino acid selected from R, K, and H; or - an aliphatic polar non-charged amino acid selected from N, Q, S, T, C, and M; and wherein a substitution in position D86, or D191 means that the D is substituted by - an aliphatic nonpolar amino acid selected from G, A, V, L, I, and P; or - a polar non-charged amino acid selected from N, Q, S, T, C, and M; preferably Q, S, T, C, and M; and wherein a substitution in position D128, D135, D117, E239, or D199 means that the D or E is substituted by - an aliphatic nonpolar amino acid selected from G, A, V, L, I, and P; or - an aromatic amino acid selected from W, F, and Y; or - an aliphatic polar non-charged amino acid selected from N, Q, S, T, C, and M; preferably Q, S, T, C, and M; or - a positively charged amino acid selected from R, K, and H. preferably K, and H. Clause 13: The nuclease according to any of the preceding clauses comprising an amino acid sequence with at least 67% preferably of at least 89% identity to SEQ ID NO: 1; wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239; wherein a substitution in position P51, A72, P73, A74, A124, or G197 means that the P, A or G is substituted by a positively charged amino acid selected from R, K, and H; wherein P51 is to be substituted by an aliphatic nonpolar amino acid selected from G, A, V, L, I; wherein P51 is to be substituted by a negatively charged amino acid selected from D, and E; wherein a substitution in position S53, T56, N58, T77, N80, or S116 means that the S, T or N is substituted by a positively charged amino acid selected from R, K, and H; wherein a substitution in position S53, T56, N58, or T77 means that the S, T or N is substituted by an aliphatic nonpolar amino acid selected from G, A, V, L, I, and P; wherein T77 is to be substituted by an aromatic amino acid selected from W, F, and Y; wherein T56 is to be substituted by D; wherein K55 is to be substituted by R, and H; wherein K84 is to be substituted by G, A, V, L, I, and P; wherein R204 is to be substituted by N, Q, S, T, C, and M; wherein a substitution in position D117, D128, D135, D191, or E239 means that the D or E is substituted by an aliphatic nonpolar amino acid selected from G, A, V, L, I, and P; wherein a substitution in position D86 means that the D is substituted by a polar non-charged amino acid selected from Q, S, T, C, and M; wherein a substitution in position D117, D128, D135, or D199 means that the D or E is substituted by - an aliphatic polar non-charged amino acid selected from N, Q, S, T, C, and M; or - a positively charged amino acid selected from K, and H; and wherein D128 is to be substituted by an aromatic amino acid selected from W, F, and Y.
Clause 14: The nuclease according to any of the preceding clauses comprising an amino acid sequence with at least 67%; preferably at least 89% identity to SEQ ID NO: 1; wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239; wherein a substitution in position S53, D117, or E239 means that the S, D or E is substituted by A; wherein a substitution in position P51, or T56 means that the P or T is substituted by D; wherein a substitution in position T77, or D128 means that the T or D is substituted by F; wherein a substitution in position P51, K84, D128, or D135 means that the P, K, or D is substituted by G; wherein N80 is to be substituted by H; wherein a substitution in position S53, T56, N58, A72, P73, A74, T77, S116, D117, A124, D128, D135, G197, or D199 means that the A, D, G, N, P, S, or T is substituted by K; wherein a substitution in position P51, or T77 means that the P or T is substituted by L; wherein D128 is to be substituted by M; wherein a substitution in position D117, D128, D135, or D199 means that the D is substituted by N; wherein a substitution in position T56, or D191 means that the D or T is substituted by P; wherein R204 is to be substituted by Q; wherein a substitution in position P51, K55, T56, N58, P73, A74, T77, or A124 means that the A, K, N, P, or T is substituted by R; wherein a substitution in position D86, D117, D128, or D199 means that the D is substituted by S; wherein N58 is to be substituted by V; and wherein D128 is to be substituted by Y.
Clause 15: The nuclease according to any of the preceding clauses comprising an amino acid sequence with at least 67%; preferably at least 89% identity to SEQ ID NO: 1; wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least four-teen, at least fifteen, at least sixteen, at least seventeen, at least eighteen, at least nineteen, at least twenty, at least twenty-one, or at least twenty-two amino acid position selected from the group consisting ofP51L, P51G, P51R, P51D, S53A, S53K, K55R, T56D, T56P, T56R, T56K, T56S, T56M, D56G, D56A, D56V, D56I, D56L, D56F, N58V, N58R, N58K, A72K, P73R, P73K, A74R, A74K, T77L, T77F, T77R, T77K, N80H, K84G, D86S, S 116K, D117H, D117I, D117R, D117K, D117A, D117N, D117S, A124R, A124K, D128K, D128N, D128M, D128S, D128Y, D128G, D128F, D135A, D135M, D135K, D135N, D135G, D191P, G197K, D199K, D199N, D199S, R204Q, and E239A; preferably P51L, P51G, P51R, P51D, S53A, S53K, K55R, T56D, T56P, T56R, T56K, N58V, N58R, N58K, A72K, P73R, P73K, A74R, A74K, T77L, T77F, T77R, T77K, N80H, K84G, D86S, S116K, D117K, D117A, D117N, D117S, A124R, A124K, D128K, D128N, D128M, D128S, D128Y, D128G, D128F, D135K, D135N, D135G, D191P, G197K, D199K, D199N, D199S, R204Q, and E239A.
Clause 16: The nuclease of any of the preceding clauses comprising an amino acid sequence with at least 67%; preferably at least 89% identity to SEQ ID NO:1; wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239; wherein an aliphatic nonpolar amino acid selected from G, A, V, L, I, and P substitutes P51, S53, T56, N58, T77, K84, D117, D128, D135, D191, or E239; wherein a positively charged amino acid selected from R, K, and H substitutes P51, S53, K55, T56, N58, A72, P73, A74, T77, N80, S116, A124, or G197; wherein a positively charged amino acid selected from R, K and H; preferably from K and H substitutes D117, D128, D135, or D199; wherein an aromatic amino acid selected from W, F, and Y substitutes T77, or D128; wherein D substitutes P51, or T56; wherein an aliphatic polar non-charged amino acid selected from N, Q, S, T, C, and M substitutes D117, D128, D135, D199, or R204; and wherein an aliphatic polar non-charged amino acid selected from Q, S, T, C, and M substitutes D86.
Clause 17: The nuclease of any of the preceding clauses comprising an amino acid sequence with at least 67%; preferably at least 89% identity to SEQ ID NO:1; wherein the amino acid sequence is substituted compared to SEQ ID NO:1 in at least one amino acid position selected from the group consisting of P51, S53, K55, T56, N58, A72, P73, A74, T77, N80, K84, D86, S116, D117, A124, D128, D135, D191, G197, D199, R204, and E239; wherein the amino acid A substitutes S53, D117, or E239; wherein the amino acid D substitutes P51, or T56; wherein the amino acid F substitutes T77, or D128; wherein the amino acid G substitutes P51, K84, D128, or D135; wherein the amino acid H substitutes N80; wherein the amino acid K substitutes S53, T56, N58, A72, P73, A74, T77, S116, D117, A124, D128, D135, G197, or D199; wherein the amino acid L substitutes P51, or T77; wherein the amino acid M substitutes D128; wherein the amino acid N substitutes D117, D128, D135, or D199; wherein the amino acid P substitutes T56, or D191; wherein the amino acid Q substitutes R204; wherein the amino acid R substitutes P51, K55, T56, N58, P73, A74, T77, or A124; wherein the amino acid S substitutes D86, D117, D128, or D199; wherein the amino acid V substitutes N58; and wherein the amino acid Y substitutes D128.
Clause 18: A nuclease; preferably an endonuclease; more preferably a non-specific endonuclease, comprising an amino acid sequence with at least 67% ; preferably at least 89% identity to SEQ ID NO:1; wherein the amino acid sequence is substituted compared to SEQ ID NO:1 in at least a first amino acid position and a second amino acid position; wherein the first amino acid position is selected from the group consisting of P51, T56, T77, and A124; and wherein the second amino acid position is selected from the group consisting of P51, S53, K55, T56, N58, A72, P73, A74, T77, N80, K84, D86, S116, D117, Q120, A124, D128, Q129, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239.
Clause 19: A nuclease; preferably an endonuclease; more preferably a non-specific endonuclease, comprising an amino acid sequence with at least 89% identity to SEQ ID NO:1; wherein the amino acid sequence is substituted compared to SEQ ID NO:1 in at least a first amino acid position and a second amino acid position; wherein the first amino acid position is selected from the group consisting of P51, T56, T77, and A124; and wherein the second amino acid position is selected from the group consisting of P51, S53, K55, T56, N58, A72, P73, A74, T77, N80, K84, D86, S116, D117, A124, D128, D135, D191, K196, G197, D199, R204, and E239; preferably wherein the amino acid sequence is substituted compared to SEQ ID NO:1 in at least one amino acid position selected from the group consisting of P51, S53, K55, T56, N58, A72, P73, A74, T77, G78, N80, A81, A82, D86, D117, Q120, A124, D128, Q129, K132, D135, D191, K196, G197, D199, R204, and E239; and wherein when the amino acid sequence is substituted in amino acid position P51 the substitution is P51R, P51K, P51H, P51D, P51E, P51G, P51A, P51V, P51L, P51I; preferably P51L, P51G, P51R, P51D; and wherein when the amino acid sequence is substituted in amino acid position T56 the substitution is T56R, T56K, T56H, T56G, T56A, T56V, T56L, T56I, T56P, T56S, T56M, and T56D; preferably T56K, T56R, T56P, and T56D, and more preferably T56K, T56R, and T56D; and wherein when the amino acid sequence is substituted in amino acid position T77 the substitution is T77R, T77K, T77H, T77G, T77A, T77V, T77L, T77I, T77P, T77W, T77F, T77Y, and T77D; preferably T77K, T77R, T77F, and T77L; and wherein when the amino acid sequence is substituted in amino acid position A124 the substitution is A124D, A124E, A124R, A124K, A124H, A124G, A124V, A124L, A124I, and A124P; preferably A124K, A124H and A124R, and more preferably A124K, and A124R.
Clause 20: The nuclease according to clause 18 or clause 19, wherein the first amino acid position and the second amino acid position are both selected from the group consisting of P51, T56, T77, and A124.
Clause 21: The nuclease according to any of clauses 18 to 20, wherein a substitution in position P51, A72, P73, A74, A124, or G197 means that the P, A or G is substituted by - a positively charged amino acid selected from R, K, and H; or - a different aliphatic nonpolar amino acid selected from G, A, V, L, I, and P; or - D; and wherein a substitution in position in S53, T56, N58, T77, N80, S116, Q120, or Q129 means that the S, T, Q, or N is substituted by - a positively charged amino acid selected from R, K, and H; or - an aliphatic nonpolar amino acid selected from G, A, V, L, I, and P; or - an aromatic amino acid selected from W, F, and Y; or - an aliphatic polar non-charged amino acid selected from N, Q, S, T, C, and M; preferably S, and M; more preferably M; or - D; and wherein a substitution in position in K55, K84, R204, or K196 means that the K or R is substituted by - an aliphatic nonpolar amino acid selected from of G, A, V, L, I, and P; or - a different positively charged amino acid selected from R, K, and H; or - an aliphatic polar non-charged amino acid selected from N, Q, S, T, C, and M; and wherein a substitution in position D86, or D191 means that the D is substituted by - an aliphatic nonpolar amino acid selected from G, A, V, L, I, and P; or - a polar non-charged amino acid selected from N, Q, S, T, C, and M; preferably Q, S, T, C, and M; and wherein a substitution in position D128, D135, D117, E239, or D199 means that the D or E is substituted by - an aliphatic nonpolar amino acid selected from G, A, V, L, I, and P; or - an aromatic amino acid selected from W, F, and Y; or - an aliphatic polar non-charged amino acid selected from N, Q, S, T, C, and M; preferably Q, S, T, C, and M; or - a positively charged amino acid selected from R, K, and H. preferably K, and H.
Clause 21A-1: A nuclease; preferably an endonuclease, comprising an amino acid sequence with at least 75%; identity to SEQ ID NO:1; and comprising (i) a substitution in position P51, wherein P has been substituted by an amino acid selected from the group consisting of L, G, A, V, and I; (ii) a substitution in position T77, wherein T has been substituted by an amino acid selected from the group consisting of R, H, and K; and (iii) at least one substitution in a position selected from the group consisting of (a) A124, A52, G54, P73, A74, G78, A81, and P195; or (b) T56, S53, N58, N80, N119, and Q120; wherein in each case (a) or (b) the amino acid of SEQ ID NO:1 has been substituted by an amino acid selected from the group consisting of R, H, and K.
Clause 21A-2: The nuclease; preferably the endonuclease, according to Clause 21A-1, wherein the nuclease; preferably the endonuclease, comprises (i) a substitution in position P51, wherein P has been substituted by an amino acid selected from the group consisting of L, G, A, V, and I; preferably L; (ii) a substitution in position T77, wherein T has been substituted by an amino acid selected from the group consisting of R, H, and K; preferably R; and (iii) a substitution in position A124, wherein A has been substituted by an amino acid selected from the group consisting of R, H, and K; preferably K.
Clause 21A-3: The nuclease; preferably the endonuclease, according to Clause 21A-1, wherein the nuclease; preferably the endonuclease, comprises (i) a substitution in position P51, wherein P has been substituted by an amino acid selected from the group consisting of L, G, A, V, and I; preferably L; (ii) a substitution in position T77, wherein T has been substituted by an amino acid selected from the group consisting of R, H, and K; preferably R; and (iii) a substitution in position A81, wherein A has been substituted by an amino acid selected from the group consisting of R, H, and K; preferably K.
Clause 21A-4: The nuclease; preferably the endonuclease, according to any of Clauses 21A-1 to 21A-3, wherein the amino acid sequence is substituted compared to SEQ ID NO:1 in at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five, at least twenty-six, at least twenty-seven, at least twenty-eight, at least twenty-nine, at least thirty, or at least thirty-one, at least thirty-two, at least thirty-three, at least thirty-four, at least thirty-five, at least thirty-six, at least thirty-seven, or at least thirty-eight positions selected from the group consisting of P51, T77, A52, S53, G54, K55, T56, N58, A72, P73, A74, G78, N80, A81, A82, K84, D86, A94, S116, D117, N119, Q120, A124, D128, Q129, K132, D135, E151, K162, D191, P195, K196, G197, D199, R204, I218, A228, and E239; preferably selected from the group consisting of P51, T77, S53, K55, T56, N58, A72, P73, A74, G78, N80, A81, A82, K84, D86, A94, S116, D117, Q120, A124, D128, Q129, K132, D135, E151, K162, D191, K196, G197, D199, R204, I218, A228, and E239.
Clause 21B-1: A nuclease; preferably an endonuclease, with at least 65% identity to SEQ ID NO:1, wherein the amino acid sequence is substituted compared to SEQ ID NO:1 in at least one amino acid position selected from the group consisting of E239, T56, K84, D191, T77, and N58, wherein in each case the native amino acid of the selected position is substituted by an amino acid selected from the group consisting of A, G, V, L, I, and P; preferably selected from the group consisting of A, G, V, L, and P; more preferably selected from the group consisting of A, G, L, and P; still more preferably selected from the group consisting of A, G, and P; yet more preferably selected from the group consisting of A, and G; and most preferably A.
Clause 21B-2: The nuclease; preferably the endonuclease, according to any of the preceding Clauses; preferably Clause 21B-1, wherein the amino acid sequence is substituted compared to SEQ ID NO:1 in position E239; preferably wherein the substitution is selected from the group consisting of E239A, E239G, E239V, E239L, E239I, and E239P; preferably E239A.
Clause 21B-3: The nuclease; preferably the endonuclease, according to any of the preceding Clauses; preferably Clause 21B-1 or Clause 21B-2, wherein the amino acid sequence is substituted compared to SEQ ID NO:1 in position T56; preferably wherein the substitution is selected from the group consisting of T56A, T56G, T56V, T56L, T56I, and T56P; preferably T56P and T56G; more preferably T56P.
Clause 21B-4: The nuclease; preferably the endonuclease, according to any of the preceding Clauses; preferably Clause 21B-1, Clause 21B-2 or Clause 21B-3, wherein the amino acid sequence is substituted compared to SEQ ID NO:1 in position K84; preferably wherein the substitution is selected from the group consisting of K84A, K84G, K84V, K84L, K84I, and K84P; preferably K84G.
Clause 21B-5: The nuclease; preferably the endonuclease, according to any of the preceding Clauses; preferably Clause 21B-1, Clause 21B-2, Clause 21B-3 or Clause 21B-4, wherein the amino acid sequence is substituted compared to SEQ ID NO: 1 in position D191; preferably wherein the substitution is selected from the group consisting of D191A, D191G, D191V, D191L, D191I, and D191P; preferably D191P.
Clause 21B-6: The nuclease; preferably the endonuclease, according to any of the preceding Clauses, wherein the amino acid sequence is substituted compared to SEQ ID NO: 1 in position T77; preferably wherein the substitution is selected from the group consisting of T77A, T77G, T77V, T77L, T77I, and T77P; preferably T77L.
Clause 21B-7: The nuclease; preferably the endonuclease, according to any of the preceding Clauses, wherein the amino acid sequence is substituted compared to SEQ ID NO: 1 in position N58; preferably wherein the substitution is selected from the group consisting ofN58A, N58G, N58V, N58L, N58I, and N58P; preferably N58V.
Clause 21B-8: The nuclease; preferably the endonuclease, according to any of the preceding Clauses, wherein the amino acid sequence is substituted compared to SEQ ID NO:1 in one, two, three, four, five, six, seven, eight, nine, ten, eleven, twelve, thirteen, fourteen, fifteen, sixteen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, or thirty-seven additional positions selected from the group consisting of P51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239; preferably selected from the group consisting of P51, K55, T56, N58, A74, T77, N80, A81, K84, D86, A94, S116, D117, A124, D128, K132, D135, E151, K162, D191, G197, R204, I218, A228, and E239; more preferably selected from the group consisting of P51, K55, T56, N58, A74, T77, N80, K84, D86, A94, S116, D117, A124, D128, K132, D135, E151, K162, D191, R204, I218, A228, and E239
Clause 21B-9: The nuclease; preferably the endonuclease, according to any of the preceding Clauses, which comprises (i) a substitution in a position selected from the group consisting of E239, T56, K84, D191, T77, and N58; wherein in each case the native amino acid of SEQ ID NO:1 has been substituted by an amino acid selected from the group consisting of A, G, V, L, I, and P; and additionally (ii) a second substitution in another position selected from the group consisting of E239, T56, K84, D191, T77, and N58; wherein in each case the native amino acid of SEQ ID NO:1 has been substituted by an amino acid selected from the group consisting of G, P, A, V, L, I, M, C, F, Y, W, H, K, R, Q, N, E, D, S, and T.
Clause 21B-10: The nuclease; preferably endonuclease, according to any of the preceding Clauses, wherein the (ii) second substitution is selected from the group consisting of (i) E239A, E239R, E239N, E239D, E239C, E239Q, E239G, E239H, E239I, E239L, E239K, E239M, E239F, E239P, E239T, E239W, E239Y, E239V, and E239S; preferably E239G, E239A, E239V, E239L, E239I, E239P, E239W, E239F, E239Y, E239C, E239M, E239N, E239Q, E239S, E239T, E239H and E239K; preferably E239A, E239G, E239V, E239L, E239I, and E239P; more preferably E239A; (ii) T56A, T56R, T56N, T56D, T56C, T56Q, T56E, T56G, T56H, T56I, T56L, T56K, T56M, T56F, T56P, T56W, T56Y, T56V, and T56S; preferably T56P, T56D, T56R, T56K, T56S, T56G, T56A, T56V, T56I, T56F, T56L, and T56M; and even more preferably T56P, T56D, T56R, T56K, T56S, and T56G; most preferably T56R, T56K, T56S, and T56G; (iii) K84A, K84R, K84N, K84D, K84C, K84Q, K84E, K84G, K84H, K84I, K84L, K84M, K84F, K84P, K84T, K84W, K84Y, K84V, and K84S; preferably K84G, K84A, K84V, K84L, K84I, K84P, K84R, K84H, K84N, K84Q, K84S, K84T, K84C, and K84M; preferably K84G, K84A, K84V, K84L, K84I, and K84P; more preferably K84G; (iv) D191G, D191A, D191V, D191L, D191I, D191P, D191W, D191F, D191Y, D191C, D191M, D191S, D191Q, D191T, D191N, D191E, D191H, D191R and D191K; preferably D191G, D191A, D191V, D191L, D191I, D191P, D191C, D191M, D191S, D191Q, and D191T; more preferably D191G, D191A, D191V, D191L, D191I, and D191P; most preferably D191P; (v) T77A, T77R, T77N, T77D, T77C, T77Q, T77E, T77G, T77H, T77I, T77L, T77K, T77M, T77F, T77P, T77W, T77Y, T77V, and T77S; preferably T77R, T77K, T77H, T77G, T77A, T77V, T77L, T77I, T77P, T77W, T77F, T77Y, and T77D; more preferably T77K, T77R, T77H, T77F, and T77L, and most preferably T77K, T77R, T77F, and T77L; and (vi) N58A, N58R, N58D, N58C, N58Q, N58E, N58G, N58H, N58I, N58L, N58K, N58M, N58F, N58P, N58T, N58W, N58Y, N58V, and N58S; preferably N58R, N58K, N58H, N58G, N58A, N58V, N58L, N58I, N58P, N58W, N58F, N58Y, and N58D; more preferably N58R, N58K, N58H, N58G, N58A, N58V, N58L, N58I, and N58P; more preferably N58K, N58R, and N58V; most preferably N58K, and N58R.
Clause 21B-11: The nuclease; preferably the endonuclease, according to any of the preceding Clauses, wherein the amino acid sequence is substituted compared to SEQ ID NO:1 in position P51; preferably wherein the substitution is selected from the group consisting of P51A, P51R, P51N, P51D, P51C, P51Q, P51E, P51G, P51H, P51I, P51L, P51K, P51M, P51F, P51T, P51W, P51Y, P51V, and P51S; preferably P51D, P51E, P51R, P51K, P51H, P51G, P51A, P51V, P51L, and P51I; more preferably P51L, P51R, P51G, P51D, P51A, P51V, P51I; even more preferably P51D, P51R, P51G, and P51L; and most preferably P51R, P51G, and P51L.
Clause 21B-12: The nuclease; preferably the endonuclease, according to any of the preceding Clauses, wherein the amino acid sequence is substituted compared to SEQ ID NO:1 in position A52; preferably wherein the substitution is selected from the group consisting of A52R, A52N, A52D, A52C, A52Q, A52E, A52G, A52H, A52I, A52L, A52K, A52M, A52F, A52P, A52T, A52W, A52Y, A52V, and A52S; preferably A52R, A52K, A52H; more preferably A52R.
Clause 21B-13: The nuclease; preferably the endonuclease, according to any of the preceding Clauses, wherein the amino acid sequence is substituted compared to SEQ ID NO:1 in position S53; preferably wherein the substitution is selected from the group consisting of S53A, S53R, S53N, S53D, S53C, S53Q, S53E, S53G, S53H, S53I, S53L, S53K, S53M, S53F, S53P, S53T, S53W, S53Y, and S53V; preferably S53R, S53K, S53H, S53G, S53A, S53V, S53L, S53I, S53P, S53W, S53F, S53Y, and S53D; more preferably S53R, S53K, S53H, S53G, S53A, S53V, S53L, S53I and S53P; even more preferably S53K, and S53A; most preferably S53K.
Clause 21B-14: The nuclease; preferably the endonuclease, according to any of the preceding Clauses, wherein the amino acid sequence is substituted compared to SEQ ID NO:1 in position G54; preferably wherein the substitution is selected from the group consisting of G54A, G54R, G54N, G54D, G54C, G54Q, G54E, G54H, G54I, G54L, G54K, G54M, G54F, G54P, G54T, G54W, G54Y, G54V, and G54S; preferably G54D, G54E, G54R, G54K, G54H, G54A, G54V, G54L, G54I, and G54P; preferably G54K, G54H, and G54R; more preferably G54R.
Clause 21B-15: The nuclease; preferably the endonuclease, according to any of the preceding Clauses, wherein the amino acid sequence is substituted compared to SEQ ID NO:1 in position K55; preferably wherein the substitution is selected from the group consisting of K55A, K55R, K55N, K55D, K55C, K55Q, K55E, K55G, K55H, K55I, K55L, K55M, K55F, K55P, K55T, K55W, K55Y, K55V, and K55S; preferably K55G, K55A, K55V, K55L, K55I, K55P, K55R, K55H, K55N, K55Q, K55S, K55T, K55C, and K55M; more preferably K55H and K55R; most preferably K55R.
Clause 21B-16: The nuclease; preferably the endonuclease, according to any of the preceding Clauses, wherein the amino acid sequence is substituted compared to SEQ ID NO: 1 in position A72; preferably wherein the substitution is selected from the group consisting of A72R, A72N, A72D, A72C, A72Q, A72E, A72G, A72H, A72I, A72L, A72K, A72M, A72F, A72P, A72T, A72W, A72Y, A72V, and A72S; preferably A72D, A72E, A72R, A72K, A72H, A72G, A72V, A72L, A72I, and A72P; more preferably A72K, A72H and A72R; most preferably A72K.
Clause 21B-17: The nuclease; preferably the endonuclease, according to any of the preceding Clauses, wherein the amino acid sequence is substituted compared to SEQ ID NO: 1 in position P73; preferably wherein the substitution is selected from the group consisting of P73A, P73R, P73N, P73D, P73C, P73Q, P73E, P73G, P73H, P73I, P73L, P73K, P73M, P73F, P73T, P73W, P73Y, P73V, and P73S; preferably P73D, P73E, P73R, P73K, P73H, P73G, P73V, P73L, P73I, and P73A; more preferably P73K, P73H and P73R; most preferably P73K, and P73R.
Clause 21B-18: The nuclease; preferably the endonuclease, according to any of the preceding Clauses, wherein the amino acid sequence is substituted compared to SEQ ID NO: 1 in position A74; preferably wherein the substitution is selected from the group consisting of A74R, A74N, A74D, A74C, A74Q, A74E, A74G, A74H, A74I, A74L, A74K, A74M, A74F, A74P, A74T, A74W, A74Y, A74V, and A74S; preferably A74D, A74E, A74R, A74K, A74H, A74G, A74V, A74L, A74I, and A74P; more preferably A74K, A74H, and A74R; most preferably A74K, and A74R.
Clause 21B-19: The nuclease; preferably the endonuclease, according to any of the preceding Clauses, wherein the amino acid sequence is substituted compared to SEQ ID NO: 1 in position G78; preferably wherein the substitution is selected from the group consisting of G78A, G78R, G78N, G78D, G78C, G78Q, G78E, G78H, G78I, G78L, G78K, G78M, G78F, G78P, G78T, G78W, G78Y, G78V, and G78S; preferably G78R, G78K, and G78H; more preferably G78R.
Clause 21B-20: The nuclease; preferably the endonuclease, according to any of the preceding Clauses, wherein the amino acid sequence is substituted compared to SEQ ID NO: 1 in position N80; preferably wherein the substitution is selected from the group consisting of N80A, N80R, N80D, N80C, N80Q, N80E, N80G, N80H, N80I, N80L, N80K, N80M, N80F, N80P, N80T, N80W, N80Y, N80V, and N80S; preferably N80R, N80K, N80H, N80G, N80A, N80V, N80L, N80I, N80P, N80W, N80F, N80Y, and N80D; preferably N80K, N80R, and N80H; more preferably N80H and N80R; most preferably N80H.
Clause 21B-21: The nuclease; preferably the endonuclease, according to any of the preceding Clauses, wherein the amino acid sequence is substituted compared to SEQ ID NO: 1 in position A81; preferably wherein the substitution is selected from the group consisting of A81R, A81N, A81D, A81C, A81Q, A81E, A81G, A81H, A81I, A81L, A81K, A81M, A81F, A81P, A81T, A81W, A81Y, A81V, and A81S; preferably A81K, A81H, and A81R; more preferably A81K.
Clause 21B-22: The nuclease; preferably the endonuclease, according to any of the preceding Clauses, wherein the amino acid sequence is substituted compared to SEQ ID NO:1 in position A82; preferably wherein the substitution is selected from the group consisting of A82R, A82N, A82D, A82C, A82Q, A82E, A82G, A82H, A82I, A82L, A82K, A82M, A82F, A82P, A82T, A82W, A82Y, A82V, and A82S; preferably A82K, A82H, and A82R; more preferably A82R.
Clause 21B-23: The nuclease; preferably the endonuclease, according to any of the preceding Clauses, wherein the amino acid sequence is substituted compared to SEQ ID NO:1 in position D86; preferably wherein the substitution is selected from the group consisting of D86A, D86R, D86N, D86C, D86Q, D86E, D86G, D86H, D86I, D86L, D86K, D86M, D86F, D86P, D86T, D86W, D86Y, D86V, and D86S; D86G, D86A, D86V, D86L, D86I, D86P, D86C, D86M, D86S, D86T, and D86Q; preferably D86C, D86M, D86S, D86T, and D86Q; more preferably D86S.
Clause 21B-24: The nuclease; preferably the endonuclease, according to any of the preceding Clauses, wherein the amino acid sequence is substituted compared to SEQ ID NO:1 in position A94; preferably wherein the substitution is selected from the group consisting of A94R, A94N, A94D, A94C, A94Q, A94E, A94G, A94H, A94I, A94L, A94K, A94M, A94F, A94P, A94T, A94W, A94Y, A94V, and A94S; preferably A94G, A94V, A94L, A94I, and A94P; more preferably A94G.
Clause 21B-25: The nuclease; preferably the endonuclease, according to any of the preceding Clauses, wherein the amino acid sequence is substituted compared to SEQ ID NO:1 in position S116; preferably wherein the substitution is selected from the group consisting of S116A, S116R, S116N, S116D, S116C, S116Q, S116E, S116G, S116H, S116I, S116L, S116K, S116M, S116F, S116P, S116T, S116W, S116Y, and S116V; preferably S116R, S116K, S116H, S116G, S116A, S116V, S116L, S116I, S116P, S116W, S116F, S116Y, and S116D; preferably S116K, S116H, and S116R; more preferably S116K.
Clause 21B-26: The nuclease; preferably the endonuclease, according to any of the preceding Clauses, wherein the amino acid sequence is substituted compared to SEQ ID NO:1 in position D117; preferably wherein the substitution is selected from the group consisting of D117G, D117A, D117V, D117L, D117I, D117P, D117W, D117F, D117Y, D117C, D117M, D117S, D117Q, D117T, D117N, D117E, D117H, D117R and D117K; preferably D117G, D117A, D117V, D117L, D117I, D117P, D117W, D117F, D117Y, D117C, D117M, D117S, D117Q, D117T, D117N, D117H, D117R and D117K; more preferably D117K, D1 17A, D117S, D117N, D117R, D117H, and D117Y; even more preferably D117A, D117N, D117R, D117K, and D117S; yet more preferably D1 17A, D117N, D117K, and D117S; and most preferably D117A, D117N, and D117S.
Clause 21B-27: The nuclease; preferably the endonuclease, according to any of the preceding Clauses, wherein the amino acid sequence is substituted compared to SEQ ID NO:1 in position N119; preferably wherein the substitution is selected from the group consisting of N119A, N119R, N119D, N119C, N119Q, N119E, N119G, N119H, N119I, N119L, N119K, N119M, N119F, N119P, N119T, N119W, N119Y, N119V, and N119S; preferably N119R, N119K, and N119H; more preferably N119R.
Clause 21B-28: The nuclease; preferably the endonuclease, according to any of the preceding Clauses, wherein the amino acid sequence is substituted compared to SEQ ID NO: 1 in position Q120; preferably wherein the substitution is selected from the group consisting of Q120A, Q120R, Q120N, Q120D, Q120C, Q120E, Q120G, Q120H, Q120I, Q120L, Q120K, Q120M, Q120F, Q120P, Q120T, Q120W, Q120Y, Q120V, and Q120S; preferably Q120R, Q120K, Q120H, Q120G, Q120A, Q120V, Q120L, Q120I, Q120P, Q120W, Q120F, Q120Y, and Q120D; more preferably Q120K, Q120H, and Q120R; most preferably Q120R.
Clause 21B-29: The nuclease; preferably the endonuclease, according to any of the preceding Clauses, wherein the amino acid sequence is substituted compared to SEQ ID NO:1 in position A124; preferably wherein the substitution is selected from the group consisting of A124R, A124N, A124D, A124C, A124Q, A124E, A124G, A124H, A1241, A124L, A124K, A124M, A124F, A124P, A124T, A124W, A124Y, A124V, and A124S; preferably A124D, A124E, A124R, A124K, A124H, A124G, A124V, A124L, A1241, and A124P; preferably A124K, A124H and A124R; more preferably A124K, and A124R.
Clause 21B-30: The nuclease; preferably the endonuclease, according to any of the preceding Clauses, wherein the amino acid sequence is substituted compared to SEQ ID NO: 1 in position D128; preferably wherein the substitution is selected from the group consisting of D128A, D128R, D128N, D128C, D128Q, D128E, D128G, D128H, D128I, D128L, D128K, D128M, D128F, D128P, D128T, D128W, D128Y, D128V, and D128S; preferably D128G, D128A, D128V, D128L, D128I, D128P, D128W, D128F, D128Y, D128C, D128M, D128N, D128Q, D128S, D128T, D128H, D128R and D128K; most preferably D128G, D128Y, D128F, D128M, D128N, D128S, D128R and D128K.
Clause 21B-31: The nuclease; preferably the endonuclease, according to any of the preceding Clauses, wherein the amino acid sequence is substituted compared to SEQ ID NO:1 in position Q129; preferably wherein the substitution is selected from the group consisting of Q129A, Q129R, Q129N, Q129D, Q129C, Q129E, Q129G, Q129H, Q129I, Q129L, Q129K, Q129M, Q129F, Q129P, Q129T, Q129W, Q129Y, Q129V, and Q129S; preferably Q129R, Q129K, Q129H, Q129G, Q129A, Q129V, Q129L, Q129I, Q129P, Q129W, Q129F, Q129Y, and Q129D; preferably Q129K, Q129H, and Q129R; more preferably Q129K, Q129R; still more preferably Q129R.
Clause 21B-32: The nuclease; preferably the endonuclease, according to any of the preceding Clauses, wherein the amino acid sequence is substituted compared to SEQ ID NO:1 in position K132; preferably wherein the substitution is selected from the group consisting of K132A, K132R, K132N, K132D, K132C, K132Q, K132E, K132G, K132H, K132I, K132L, K132M, K132F, K132P, K132T, K132W, K132Y, K132V, and K132S; preferably K132R, and K132H; most preferably K132R.
Clause 21B-33: The nuclease; preferably the endonuclease, according to any of the preceding Clauses, wherein the amino acid sequence is substituted compared to SEQ ID NO:1 in position D135; preferably wherein the substitution is selected from the group consisting of D135A, D135R, D135N, D135C, D135Q, D135E, D135G, D135H, D135I, D135L, D135K, D135M, D135F, D135P, D135T, D135W, D135Y, D135V, and D135S; preferably D135G, D135A, D135V, D135L, D135I, D135P, D135W, D135F, D135Y, D135C, D135M, D135N, D135Q, D135S, D135T, D135R, D135H, and D135K; more preferably D135G, D135A, D135V, D135L, D135I, D135P, D135C, D135M, D135N, D135Q, D135S, D135T, D135R, D135H, and D135K; most preferably D135A, D135M, D135G, D135N, D135R and D135K.
Clause 21B-34: The nuclease; preferably the endonuclease, according to any of the preceding Clauses, wherein the amino acid sequence is substituted compared to SEQ ID NO: 1 in position E151; preferably wherein the substitution is selected from the group consisting of E151A, E151R, E151N, E151D, E151C, E151Q, E151G, E151H, E151I, E151L, E151K, E151M, E151F, E151P, E151T, E151W, E151Y, E151V, and E151S; E151G, E151A, E151V, E151L, E151I, E151P, E151W, E151F, E151Y, E151C, E151M, E151N, E151Q, E151S, E151T, E151H and E151K; preferably E151A, E151G, E151V, E151L, E151I, and E151P; more preferably E151L.
Clause 21B-35: The nuclease; preferably the endonuclease, according to any of the preceding Clauses, wherein the amino acid sequence is substituted compared to SEQ ID NO:1 in position K162; preferably wherein the substitution is selected from the group consisting of K162A, K162R, K162N, K162D, K162C, K162Q, K162E, K162G, K162H, K162I, K162L, K162M, K162F, K162P, K162T, K162W, K162Y, K162V, and K162S; preferably K162N, K162Q, K162S, K162T, K162C and K162M; most preferably K162N.
Clause 21B-36: The nuclease; preferably the endonuclease, according to any of the preceding Clauses, wherein the amino acid sequence is substituted compared to SEQ ID NO:1 in position P195; preferably wherein the substitution is selected from the group consisting of P195A, P195R, P195N, P195D, P195C, P195Q, P195E, P195G, P195H, P195I, P195L, P195K, P195M, P195F, P195T, P195W, P195Y, P195V, and P195S; preferably P195K, P195H and P195R; most preferably P195R.
Clause 21B-37: The nuclease; preferably the endonuclease, according to any of the preceding Clauses, wherein the amino acid sequence is substituted compared to SEQ ID NO:1 in position K196; preferably wherein the substitution is selected from the group consisting of K196A, K196R, K196N, K196D, K196C, K196Q, K196E, K196G, K196H, K196I, K196L, K196M, K196F, K196P, K196T, K196W, K196Y, K196V, and K196S; preferably K196G, K196A, K196V, K196L, K196I, K196P, K196R, K196H, K196N, K196Q, K196S, K196T, K196C, and K196M; more preferably K196R, and K196H; most preferably K196R.
Clause 21B-38: The nuclease; preferably the endonuclease, according to any of the preceding Clauses, wherein the amino acid sequence is substituted compared to SEQ ID NO:1 in position G197; preferably wherein the substitution is selected from the group consisting of G197A, G197R, G197N, G197D, G197C, G197Q, G197E, G197H, G197I, G197L, G197K, G197M, G197F, G197P, G197T, G197W, G197Y, G197V, and G197S; preferably G197D, G197E, G197R, G197K, G197H, G197A, G197V, G197L, G197I, and G197P; preferably G197K, G197H, and G197R; more preferably G197K.
Clause 21B-39: The nuclease; preferably the endonuclease, according to any of the preceding Clauses, wherein the amino acid sequence is substituted compared to SEQ ID NO:1 in position D199; preferably wherein the substitution is selected from the group consisting of D199G, D199A, D199V, D199L, D199I, D199P, D199W, D199F, D199Y, D199C, D199M, D199S, D199Q, D199T, D199N, D199E, D199H, D199R and D199K; preferably D199G, D199A, D199V, D199L, D199I, D199P, D199W, D199F, D199Y, D199C, D199M, D199N, D199S, D199Q, D199T, and D199K; preferably D199N, D199S, D199Q, D199T, D199C, D199M, D199H, and D199K; more preferably D199N, D199S, and D199K.
Clause 21B-40: The nuclease; preferably the endonuclease, according to any of the preceding Clauses, wherein the amino acid sequence is substituted compared to SEQ ID NO:1 in position R204; preferably wherein the substitution is selected from the group consisting of R204A, R204N, R204D, R204C, R204Q, R204E, R204G, R204H, R204I, R204L, R204K, R204M, R204F, R204P, R204T, R204W, R204Y, R204V, and R204S; preferably R204G, R204A, R204V, R204L, R204I, R204P, R204K, R204H, R204N, R204Q, R204S, R204T, R204C, and R204M; more preferably R204Q, R204N, R204S, R204T, R204C and R204M; most preferably R204Q.
Clause 21B-41: The nuclease; preferably the endonuclease, according to any of the preceding Clauses, wherein the amino acid sequence is substituted compared to SEQ ID NO:1 in position I218; preferably wherein the substitution is selected from the group consisting of I218R, I218N, I218D, I218C, I218Q, I218E, I218G, I218H, I218A, I218L, I218K, I218M, I218F, I218P, I218T, I218W, I218Y, I218V, and I218S; preferably I218F, I218W and I218Y; more preferably I218F.
Clause 21B-42: The nuclease; preferably the endonuclease, according to any of the preceding Clauses, wherein the amino acid sequence is substituted compared to SEQ ID NO:1 in position A228; preferably wherein the substitution is selected from the group consisting of A228R, A228N, A228D, A228C, A228Q, A228E, A228G, A228H, A228I, A228L, A228K, A228M, A228F, A228P, A228T, A228W, A228Y, A228V, and A228S; preferably A228K, A228H and A228R; more preferably A228R.
Clause 21B-43: The nuclease; preferably the endonuclease, according to any of the preceding Clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five positions selected from the group consisting of the positions P51, K55, T56, N58, A74, T77, N80, A81, K84, D86, A94, S116, D117, A124, D128, K132, D135, E151, K162, D191, G197, R204, I218, A228, and E239, wherein, (i) when the amino acid substitution comprises a substitution at amino acid position P51, then the amino acid substitution at position P51 is selected from the group consisting of the substitutions D, E, R, K, H, G, A, V, L, and I; preferably D, E, G, A, V, L, and I; more preferably G, L, and D; (ii) when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; (iii) when the amino acid substitution comprises a substitution at amino acid position A74, then the amino acid substitution at position A74 is selected from the group consisting of the substitutions G, I, V, L, P, W, F, Y, C, M, N, Q, S, T, D, E, H, R, and K; preferably R, Q, and H; more preferably R; (iv) when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H; (v) when the amino acid substitution comprises a substitution at amino acid position A81, then the amino acid substitution at position A81 is selected from the group consisting of the substitutions G, V, L, I, P, W, F, Y, C, M, S, N, T, Q, H, R, K, D and E; preferably R, K, and H; more preferably K; (vi) when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; (vii) when the amino acid substitution comprises a substitution at amino acid position A94, then the amino acid substitution at position A94 is selected from the group consisting of the substitutions G, I, V, L, P, W, F, Y, C, M, N, Q, S, T, D, E, H, R, and K; preferably G, I, V, L, and P; more preferably G; (viii) when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K; (ix) when the amino acid substitution comprises a substitution at amino acid position D117, then the amino acid substitution at position D117 is selected from the group consisting of the substitutions R, K, H, G, A, V, L, I, P, W, F, Y, C, M, S, Q, T, and N; preferably C, M, S, Q, T, and N; more preferably C, M, S, Q, and N; still more preferably N; (x) when the amino acid substitution comprises a substitution at amino acid position A124, then the amino acid substitution at position A124 is selected from the group consisting of the substitutions D, E, R, K, H, G, V, L, I, and P; preferably K, H and R; more preferably K; (xi) when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably W, F, Y, C, M, N, Q, S, T, and R; more preferably R, M, F, and Y; (xii) when the amino acid substitution comprises a substitution at amino acid position K132, then the amino acid substitution at position K132 is selected from the group consisting of the substitutions R, H, G, A, V, L, I, P, W, F, Y, C, M, S, Q, T, and N; preferably R, and H; more preferably R; (xiii) when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably G, A, M, R, and N; (xiv) when the amino acid substitution comprises a substitution at amino acid position E151, then the amino acid substitution at position E151 is selected from the group consisting of the substitutions D, R, K, H, G, A, V, L, I, P, W, F, Y, C, M, S, Q, T, and N; preferably G, A, V, L, I, and P; more preferably L; (xv) when the amino acid substitution comprises a substitution at amino acid position K162, then the amino acid substitution at position K162 is selected from the group consisting of the substitutions R, H, G, A, V, L, I, P, W, F, Y, C, M, S, Q, T, and N; preferably C. M, S, Q, T, and N; more preferably N; (xi) when the amino acid substitution comprises a substitution at amino acid position G197, then the amino acid substitution at position G197 is selected from the group consisting of the substitutions D, E, R, K, H, G, V, L, I, N, T, S, Q, C, M, Y, F, W and P; preferably R, K, and H; more preferably K; (xii) when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q; (xiii) when the amino acid substitution comprises a substitution at amino acid position I218, then the amino acid substitution at position I218 is selected from the group consisting of the substitutions G, A, V, L, P, W, F, Y, C, M, N, Q, S, T, D, E, H, R, and K; preferably W, Y, and F; more preferably F; or (xiv) when the amino acid substitution comprises a substitution at amino acid position A228, then the amino acid substitution at position A228 is selected from the group consisting of the substitutions G, I, V, L, P, W, F, Y, C, M, N, Q, S, T, D, E, H, R, and K; preferably H, R, and K; more preferably R.
Clause 21B-44: The nuclease; preferably the endonuclease, according to any of the preceding Clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, at least twenty-five substitution selected from the group consisting of the substitutions P51L, P51G, P51D, K55R, T56A, T56V, T56L, T56I, T56P, T56D, T56S, T56G, T56R, N58A, N58G, N58V, N58L, N58I, N58P, N58R, A74R, T77K, T77F, T77A, T77G, T77V, T77I, T77P, T77L, T77R, N80H, A81K, K84G, K84A, K84V; K84L, K84I, K84P, D86S, A94G, S116K, D117N, A124K, D128R, D128M, D128Y, D128F, K132R, D135A, D135G, D135N, D135M, D135R, E151L, K162N, D191A, D191G, D191V, D191L, D191I, D191P, G197K, R204Q, I218F, A228R, E239G, E239V, E239L, E239I, E239P, and E239A; preferably P51L, P51G, P51D, K55R, T56P, T56D, T56S, T56G, T56R, N58V, N58R, A74R, T77K, T77F, T77L, T77R, N80H, A81K, K84G, D86S, A94G, S116K, D117N, A124K, D128R, D128M, D128Y, D128F, K132R, D135A, D135G, D135N, D135M, D135R, E151L, K162N, D191P, G197K, R204Q, I218F, A228R, and E239A; more preferably P51L, P51G, P51D, K55R, T56P, T56G, N58V, A74R, T77L, N80H, A81K, K84G, D86S, A94G, S116K, D117N, A124K, D128R, D128M, D128Y, D128F, K132R, D135A, D135G, D135N, D135M, D135R, E151L, K162N, D191P, G197K, R204Q, I218F, A228R, and E239A.
Clause 21B-45a: The nuclease; preferably the endonuclease, according to any of the preceding Clauses comprising an amino acid sequence with at least 65%; identity to SEQ ID NO:1; and comprising (i) a substitution in position T56, wherein the position is substituted by an amino acid selected from the group consisting of A, G, V, L, I, and P; preferably P; and (ii) a substitution in position K84, wherein the position is substituted by an amino acid selected from the group consisting of A, G, V, L, I, and P; preferably G,
Clause 21B-45b: The nuclease; preferably the endonuclease, according to any of the preceding Clauses comprising an amino acid sequence with at least 65%; identity to SEQ ID NO:1; and comprising (i) a substitution in position T56, wherein the position is substituted by an amino acid selected from the group consisting of A, G, V, L, I, and P; preferably P; (ii) a substitution in position K84, wherein the position is substituted by an amino acid selected from the group consisting of A, G, V, L, I, and P; preferably G; and (iii) a substitution in position P51, wherein the position is substituted by an amino acid selected from the group consisting of A, G, V, L, and I; preferably L.
21B-45c: The nuclease; preferably the endonuclease, according to any of the preceding Clauses comprising an amino acid sequence with at least 65%; identity to SEQ ID NO: 1; and comprising (i) a substitution in position P51, wherein the position is substituted by an amino acid selected from the group consisting of A, G, V, L, and I; preferably L; and (ii) a substitution in position T56, wherein the position is substituted by an amino acid selected from the group consisting of A, G, V, L, I, and P; preferably P.
Clause 21B-45d: The nuclease; preferably the endonuclease, according to any of the preceding Clauses comprising an amino acid sequence with at least 65%; identity to SEQ ID NO:1; and comprising (i) a substitution in position P51, wherein the position is substituted by an amino acid selected from the group consisting of A, G, V, L, and I; preferably L; (ii) a substitution in position T56, wherein the position is substituted by an amino acid selected from the group consisting of A, G, V, L, I, and P; preferably P; and (iii) a substitution in position D135, wherein the position is substituted by an amino acid selected from the group consisting of N, T, Q, S, C, M, A, G, V, L, I, and P; preferably N, and A.
Clause 21B-45e: The nuclease; preferably the endonuclease, according to any of the preceding Clauses comprising an amino acid sequence with at least 65%; identity to SEQ ID NO:1; and comprising (i) a substitution in position E239, wherein the position is substituted by an amino acid selected from the group consisting of A, G, V, L, I, and P; preferably A; (ii) a substitution in position P51, wherein the position is substituted by an amino acid selected from the group consisting of A, G, V, L, and I; prefer-ably L; (iii) a substitution in position T56, wherein the position is substituted by an amino acid selected from the group consisting of D, E, C, M, N, Q, S, A, G, V, L, I, and P; preferably P, G, D, and S; more preferably D;
Clause 21B-46: The nuclease; preferably the endonuclease, according to any of the preceding Clauses, wherein the amino acid sequence has an identity to SEQ ID NO: 1 of at least 76%; yet more preferably at least 78%; even more preferably at least 80%; most preferably at least 84%, and in particular at least 87%.
Clause 21B-47: The nuclease; preferably the endonuclease, according to any of the preceding Clauses, wherein the amino acid sequence has an identity to SEQ ID NO:1 of at least 88%; preferably at least 89%; more preferably at least 90%; still more preferably at least 91%; yet more preferably at least 92%; even more preferably at least 93%; most preferably at least 94%, and in particular at least 95%.
Clause 21B-48: The nuclease; preferably the endonuclease, according to any of the preceding Clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 4, 5, 8, 9, 10, 13, 19, 21, 22, 23, 24, 25, 26, 27, 28, 32, 34, 36, 37, 41, 44, 46, 48, 53, 54, 68, 72, 73, 74, 75, 76, 77, 81, 99, 100, 101, 102, 106, 107, 108, 109, 110, 111, 122, 123, 124, 128, 129, 130, 131, or 137.
Clause 21B-49: The nuclease; preferably the endonuclease, according to any of the preceding Clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 4, 5, 8, 9, 10, 13, 19, 21, 22, 23, 24, 25, 26, 27, 28, 32, 34, 36, 37, 41, 44, 46, 48, 53, 54, 68, 72, 73, 74, 75, 76, 77, 106, 107, 108, 109, 110, or 111.
Clause 21B-50: The nuclease; preferably the endonuclease, according to any of the preceding Clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to (i) SEQ ID NO: 9, 13, 21, 26, 48, 54, 73, 74, 75, 75, 76, 76, 81, 102, 122, 123, 124, 128, 128, 129, 130, 131, 131, and 137; or (ii) SEQ ID NO: 9, 13, 21, 26, 48, 54, 73, 74, 75, 75, 76, or 76.
Clause 21B-51: The nuclease; preferably the endonuclease, according to any of the preceding Clauses, which is characterized by (A) an increased thermal stability in comparison to the wildtype nuclease of SEQ ID NO:1.
Clause 21B-52: The nuclease; preferably the endonuclease, according to any of the preceding Clauses, which has an increased thermal stability in comparison to the thermal stability of the wildtype nuclease of SEQ ID NO:1, wherein said thermal stability (i) is at least 16%; preferably at least 23%; more preferably at least 29%, and still more preferably at least 59% increased in comparison to SEQ ID NO:1, when the residual activity is determined at a temperature T_{stability} of 51°C; or (ii) is at least 16%; prefer-ably at least 23%; more preferably at least 29%, and still more preferably at least 59% increased in comparison to SEQ ID NO:1.
Clause 21B-53: The nuclease; preferably the endonuclease, according to any of the preceding Clauses, which has a thermal stability that compared to SEQ ID NO:1 is increased (i) at least 1.051-fold, at least 1.5-fold, or at least 2-fold; more preferably at least 3-fold, at least 4-fold, or at least 5-fold, and still more preferably at least 6-fold, at least 6.5-fold, or at least 7-fold; or (ii) is increased from 1.051-fold to 100-fold; preferably from 1.5-fold to 50-fold; more preferably from 2-fold to 40-fold; still more prefer-ably from 3-fold, to 30-fold; yet more preferably from 4-fold to 23-fold; even more preferably from 5-fold to 21-fold, and most preferably from 6-fold to 15-fold.
Clause 21B-54: The nuclease; preferably the endonuclease, according to any of the preceding Clauses, wherein the nuclease; preferably the endonuclease, is capable of hydrolyzing of phosphodiesters of polynucleotide substrate into cleaved polynucleotides or oligonucleotides; preferably wherein the polynucleotide substrates are selected from the group consisting of single strand RNA, single strand DNA, double strand DNA, double strand RNA and hybrid strand DNA/RNA; preferably double strand DNA.
Clause 21B-55: A method of hydrolyzing polynucleotide substrates according to any embodiment of any aspect of the invention, comprising the steps of (a) providing a nuclease according to any of the Clauses 121B-1 to 121B-54.
Clause 21B-56: Use of a nuclease according to any of 121B-1 to 121B-54 for hydrolyzing polynucleotide substrates; preferably being contained in a composition, according to any embodiment of any aspect of the invention.
Clause 21C-1: A nuclease; preferably an endonuclease, with at least 70% identity to SEQ ID NO:1, wherein the amino acid sequence is substituted compared to SEQ ID NO:1 in at least two amino acid position selected from the group consisting of P51, T56, K84, and N58, wherein in each case the native amino acid of the selected position is substituted by an amino acid selected from the group consisting of L, P, A, G, V, and I; preferably selected from the group consisting of L, P, I, G, and V; more preferably selected from the group consisting of L, P, I, and G; still more preferably selected from the group consisting of L, P, and G; yet more preferably selected from the group consisting of L, and P.
Clause 21C-2: The nuclease; preferably the endonuclease, according to any of the preceding Clauses; preferably Clause 21C-1, wherein the amino acid sequence is substituted compared to SEQ ID NO: 1 in position P51; preferably wherein the substitution is selected from the group consisting of P51G, P51A, P51V, P51I, and P51L; preferably P51G, and P51L; more preferably P51L.
Clause 21C-3: The nuclease; preferably the endonuclease, according to any of the preceding Clauses; preferably Clause 31C-1 or Clause 21C-2, wherein the amino acid sequence is substituted compared to SEQ ID NO: 1 in position T56; preferably wherein the substitution is selected from the group consisting of T56G, T56A, T56V, T56L, T56I, and T56P; preferably T56G, and T56P.
Clause 21C-4: The nuclease; preferably the endonuclease, according to any of the preceding Clauses; preferably Clause 31C-1, Clause 21C-2, or Clause 21C-3, wherein the amino acid sequence is substituted compared to SEQ ID NO:1 in position K84; preferably wherein the substitution is selected from the group consisting of K84G, K84A, K84V, K84L, K84I, and K84P; preferably G.
Clause 21C-5: The nuclease; preferably the endonuclease, according to any of the preceding Clauses; preferably Clause 31C-1, Clause 21C-2, Clause 21C-3, or Clause 21C-4, wherein the amino acid sequence is substituted compared to SEQ ID NO:1 in position N58; preferably wherein the substitution is selected from the group consisting of N58G, N58A, N58V, N58L, N58I, and N58P; preferably V.
Clause 21C-6: The nuclease; preferably the endonuclease, according to any of the preceding Clauses, wherein the amino acid sequence is substituted compared to SEQ ID NO:1 in one, two, three, four, five, six, seven, eight, nine, ten, eleven, twelve, thirteen, fourteen, fifteen, six-teen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, or thirty-seven additional positions selected from the group consisting ofP51, A52, S53, G54, K55, T56, N58, A72, P73, A74, T77, G78, N80, A81, A82, K84, D86, A94, S116, D117, N119, Q120, A124, D128, Q129, K132, D135, E151, K162, D191, P195, K196, G197, D199, R204, I218, A228, and E239; preferably P51, K55, T56, N58, P73, A74, T77, G78, A81, K84, D86, S116, D117, Q120, A124, D128, Q129, D135, K196, G197, D199, and E239; more preferably P51, T56, N58, T77, A81, K84, D86, S116, D117, Q120, A124, D128, Q129, D135, K196, and E239; still more preferably P51, T56, N58, T77, K84, D86, S116, and D135.
Clause 21C-7: The nuclease; preferably the endonuclease, according to any of the preceding Clauses, which comprises
   (i) two substitutions in a position selected from the group consisting of P51, T56, K84, and N58; wherein in each case the native amino acid of SEQ ID NO:1 has been substituted by an amino acid selected from the group consisting of L, P, A, G, V, and I; and additionally
   (ii) a third substitution in another position selected from the group consisting of P51, T56, K84, and N58; wherein in each case the native amino acid of SEQ ID NO:1 has been substituted by an amino acid selected from the group consisting of G, P, A, V, L, I, M, C, F, Y, W, H, K, R, Q, N, E, D, S, and T.
Clause 21C-8: The nuclease; preferably endonuclease, according to Clause 21C-7, wherein the (ii) third substitution is selected from the group consisting of
   - P51L, P51D, P51E, P51R, P51K, P51H, P51G, P51A, P51V, P51I, P51F, P51W, P51Y, P51N, P51T, P51S, P51Q, P51C, and P51M; preferably P51G, P51A, P51V, P51I, and P51L; more preferably P51G, and P51L; still more preferably P51L;
   - T56P, T56G, T56D, T56E, T56R, T56K, T56H, T56A, T56V, T56L, T56I, T56F, T56W, T56Y, T56N, T56S, T56Q, T56C, and T56M; preferably T56R, T56K, T56H, T56G, T56A, T56V, T56L, T56I, T56P, and T56D; more preferably T56G, T56A, T56V, T56L, T56I, T56P, and T56D; still more preferably T56G, T56P, and T56D;
   - K84G, K84D, K84E, K84R, K84H, K84A, K84V, K84L, K84I, K84P, K84F, K84W, K84Y, K84N, K84T, K84S, K84Q, K84C, and K84M; preferably K84G, K84A, K84V, K84L, K84I, and K84P; more preferably K84G; and
   - N58V, N58D, N58E, N58R, N58K, N58H, N58G, N58A, N58L, N58I, N58P, N58F, N58W, N58Y, N58T, N58S, N58Q, N58C, and N58M; N58R, N58K, N58H, N58G, N58A, N58V, N58L, N58I, and N58P; more preferably N58R, N58K, and N58V; still more preferably N58R, and N58V.
Clause 21C-9: The nuclease; preferably the endonuclease, according to any of the preceding Clauses, wherein the amino acid sequence is substituted compared to SEQ ID NO:1 in position A52; preferably wherein the substitution is selected from the group consisting of A52R, A52N, A52D, A52C, A52Q, A52E, A52G, A52H, A52I, A52L, A52K, A52M, A52F, A52P, A52T, A52W, A52Y, A52V, and A52S; preferably A52R, A52K, A52H; more preferably A52R.
Clause 21C-10: The nuclease; preferably the endonuclease, according to any of the preceding Clauses, wherein the amino acid sequence is substituted compared to SEQ ID NO:1 in position S53; preferably wherein the substitution is selected from the group consisting of S53A, S53R, S53N, S53D, S53C, S53Q, S53E, S53G, S53H, S53I, S53L, S53K, S53M, S53F, S53P, S53T, S53W, S53Y, and S53V; preferably S53R, S53K, S53H, S53G, S53A, S53V, S53L, S53I, S53P, S53W, S53F, S53Y, and S53D; more preferably S53R, S53K, S53H, S53G, S53A, S53V, S53L, S53I and S53P; even more preferably S53K, and S53A; most preferably S53K.
Clause 21C-11: The nuclease; preferably the endonuclease, according to any of the preceding Clauses, wherein the amino acid sequence is substituted compared to SEQ ID NO:1 in position G54; preferably wherein the substitution is selected from the group consisting of G54A, G54R, G54N, G54D, G54C, G54Q, G54E, G54H, G54I, G54L, G54K, G54M, G54F, G54P, G54T, G54W, G54Y, G54V, and G54S; preferably G54D, G54E, G54R, G54K, G54H, G54A, G54V, G54L, G54I, and G54P; preferably G54K, G54H, and G54R; more preferably G54R.
Clause 21C-12: The nuclease; preferably the endonuclease, according to any of the preceding Clauses, wherein the amino acid sequence is substituted compared to SEQ ID NO:1 in position K55; preferably wherein the substitution is selected from the group consisting of K55A, K55R, K55N, K55D, K55C, K55Q, K55E, K55G, K55H, K55I, K55L, K55M, K55F, K55P, K55T, K55W, K55Y, K55V, and K55S; preferably K55G, K55A, K55V, K55L, K55I, K55P, K55R, K55H, K55N, K55Q, K55S, K55T, K55C, and K55M; more preferably K55H and K55R; most preferably K55R.
Clause 21C-13: The nuclease; preferably the endonuclease, according to any of the preceding Clauses, wherein the amino acid sequence is substituted compared to SEQ ID NO:1 in position A72; preferably wherein the substitution is selected from the group consisting of A72R, A72N, A72D, A72C, A72Q, A72E, A72G, A72H, A72I, A72L, A72K, A72M, A72F, A72P, A72T, A72W, A72Y, A72V, and A72S; preferably A72D, A72E, A72R, A72K, A72H, A72G, A72V, A72L, A72I, and A72P; more preferably A72K, A72H and A72R; most preferably A72K.
Clause 21C-14: The nuclease; preferably the endonuclease, according to any of the preceding Clauses, wherein the amino acid sequence is substituted compared to SEQ ID NO:1 in position P73; preferably wherein the substitution is selected from the group consisting of P73A, P73R, P73N, P73D, P73C, P73Q, P73E, P73G, P73H, P73I, P73L, P73K, P73M, P73F, P73T, P73W, P73Y, P73V, and P73S; preferably P73D, P73E, P73R, P73K, P73H, P73G, P73V, P73L, P73I, and P73A; more preferably P73K, P73H and P73R; most preferably P73K, and P73R.
Clause 21C-15: The nuclease; preferably the endonuclease, according to any of the preceding Clauses, wherein the amino acid sequence is substituted compared to SEQ ID NO: 1 in position A74; preferably wherein the substitution is selected from the group consisting of A74R, A74N, A74D, A74C, A74Q, A74E, A74G, A74H, A74I, A74L, A74K, A74M, A74F, A74P, A74T, A74W, A74Y, A74V, and A74S; preferably A74D, A74E, A74R, A74K, A74H, A74G, A74V, A74L, A74I, and A74P; more preferably A74K, A74H, and A74R; most preferably A74K, and A74R.
Clause 21C-16: The nuclease; preferably the endonuclease, according to any of the preceding Clauses, wherein the amino acid sequence is substituted compared to SEQ ID NO: 1 in position T77; preferably wherein the substitution is selected from the group consisting of T77R, T77N, T77D, T77C, T77Q, T77E, T77G, T77H, T77I, T77L, T77K, T77M, T77F, T77P, T77A T77W, T77Y, T77V, and T77S; preferably T77R, T77K, T77H, T77G, T77A, T77V, T77L, T77I, T77P, T77W, T77F, T77Y, and T77D; preferably T77L, T77F, T77K, T77R, and T77H; more preferably T77K, T77R, and T77H; still more preferably T77R.
Clause 21C-17: The nuclease; preferably the endonuclease, according to any of the preceding Clauses, wherein the amino acid sequence is substituted compared to SEQ ID NO: 1 in position G78; preferably wherein the substitution is selected from the group consisting of G78A, G78R, G78N, G78D, G78C, G78Q, G78E, G78H, G78I, G78L, G78K, G78M, G78F, G78P, G78T, G78W, G78Y, G78V, and G78S; preferably G78R, G78K, and G78H; more preferably G78R.
Clause 21C-18: The nuclease; preferably the endonuclease, according to any of the preceding Clauses, wherein the amino acid sequence is substituted compared to SEQ ID NO: 1 in position N80; preferably wherein the substitution is selected from the group consisting of N80A, N80R, N80D, N80C, N80Q, N80E, N80G, N80H, N80I, N80L, N80K, N80M, N80F, N80P, N80T, N80W, N80Y, N80V, and N80S; preferably N80R, N80K, N80H, N80G, N80A, N80V, N80L, N80I, N80P, N80W, N80F, N80Y, and N80D; preferably N80K, N80R, and N80H; more preferably N80H and N80R; most preferably N80H.
Clause 21C-19: The nuclease; preferably the endonuclease, according to any of the preceding Clauses, wherein the amino acid sequence is substituted compared to SEQ ID NO: 1 in position A81; preferably wherein the substitution is selected from the group consisting of A81R, A81N, A81D, A81C, A81Q, A81E, A81G, A81H, A81I, A81L, A81K, A81M, A81F, A81P, A81T, A81W, A81Y, A81V, and A81S; preferably A81K, A81H, and A81R; more preferably A81K.
Clause 21C-20: The nuclease; preferably the endonuclease, according to any of the preceding Clauses, wherein the amino acid sequence is substituted compared to SEQ ID NO: 1 in position A82; preferably wherein the substitution is selected from the group consisting of A82R, A82N, A82D, A82C, A82Q, A82E, A82G, A82H, A82I, A82L, A82K, A82M, A82F, A82P, A82T, A82W, A82Y, A82V, and A82S; preferably A82K, A82H, and A82R; more preferably A82R.
Clause 21C-21: The nuclease; preferably the endonuclease, according to any of the preceding Clauses, wherein the amino acid sequence is substituted compared to SEQ ID NO:1 in position D86; preferably wherein the substitution is selected from the group consisting of D86A, D86R, D86N, D86C, D86Q, D86E, D86G, D86H, D86I, D86L, D86K, D86M, D86F, D86P, D86T, D86W, D86Y, D86V, and D86S; D86G, D86A, D86V, D86L, D86I, D86P, D86C, D86M, D86S, D86T, and D86Q; preferably D86C, D86M, D86S, D86T, and D86Q; more preferably D86S.
Clause 21C-22: The nuclease; preferably the endonuclease, according to any of the preceding Clauses, wherein the amino acid sequence is substituted compared to SEQ ID NO:1 in position A94; preferably wherein the substitution is selected from the group consisting of A94R, A94N, A94D, A94C, A94Q, A94E, A94G, A94H, A94I, A94L, A94K, A94M, A94F, A94P, A94T, A94W, A94Y, A94V, and A94S; preferably A94G, A94V, A94L, A94I, and A94P; more preferably A94G.
Clause 21C-23: The nuclease; preferably the endonuclease, according to any of the preceding Clauses, wherein the amino acid sequence is substituted compared to SEQ ID NO:1 in position S116; preferably wherein the substitution is selected from the group consisting of S116A, S116R, S116N, S116D, S116C, S116Q, S116E, S116G, S116H, S116I, S116L, S116K, S116M, S116F, S116P, S116T, S116W, S116Y, and S116V; preferably S116R, S116K, S116H, S116G, S116A, S116V, S116L, S116I, S116P, S116W, S116F, S116Y, and S116D; preferably S116K, S116H, and S116R; more preferably S116K.
Clause 21C-24: The nuclease; preferably the endonuclease, according to any of the preceding Clauses, wherein the amino acid sequence is substituted compared to SEQ ID NO:1 in position D117; preferably wherein the substitution is selected from the group consisting of D117G, D117A, D117V, D117L, D117I, D117P, D117W, D117F, D117Y, D117C, D117M, D117S, D117Q, D117T, D117N, D117E, D117H, D117R and D117K; preferably D117G, D117A, D117V, D117L, D117I, D117P, D117W, D117F, D117Y, D117C, D117M, D117S, D117Q, D117T, D117N, D117H, D117R and D117K; more preferably D117K, D117R, D117H, D117W, D117F and D117Y; still more preferably D117R, D117K, D117H, and D117Y; yet more preferably D117R, D117K, and D117H; even more preferably D117K.
Clause 21C-25: The nuclease; preferably the endonuclease, according to any of the preceding Clauses, wherein the amino acid sequence is substituted compared to SEQ ID NO:1 in position N119; preferably wherein the substitution is selected from the group consisting of N119A, N119R, N119D, N119C, N119Q, N119E, N119G, N119H, N119I, N119L, N119K, N119M, N119F, N119P, N119T, N119W, N119Y, N119V, and N119S; preferably N119R, N119K, and N119H; more preferably N119R.
Clause 21C-26: The nuclease; preferably the endonuclease, according to any of the preceding Clauses, wherein the amino acid sequence is substituted compared to SEQ ID NO:1 in position Q120; preferably wherein the substitution is selected from the group consisting of Q120A, Q120R, Q120N, Q120D, Q120C, Q120E, Q120G, Q120H, Q120I, Q120L, Q120K, Q120M, Q120F, Q120P, Q120T, Q120W, Q120Y, Q120V, and Q120S; preferably Q120R, Q120K, Q120H, Q120G, Q120A, Q120V, Q120L, Q120I, Q120P, Q120W, Q120F, Q120Y, and Q120D; more preferably Q120K, Q120H, and Q120R; most preferably Q120R.
Clause 21C-27: The nuclease; preferably the endonuclease, according to any of the preceding Clauses, wherein the amino acid sequence is substituted compared to SEQ ID NO:1 in position A124; preferably wherein the substitution is selected from the group consisting of A124R, A124N, A124D, A124C, A124Q, A124E, A124G, A124H, A1241, A124L, A124K, A124M, A124F, A124P, A124T, A124W, A124Y, A124V, and A124S; preferably A124D, A124E, A124R, A124K, A124H, A124G, A124V, A124L, A 124I, and A124P; preferably A124K, A124H and A124R; more preferably A124K.
Clause 21C-28: The nuclease; preferably the endonuclease, according to any of the preceding Clauses, wherein the amino acid sequence is substituted compared to SEQ ID NO:1 in position D128; preferably wherein the substitution is selected from the group consisting of D128A, D128R, D128N, D128C, D128Q, D128E, D128G, D128H, D128I, D128L, D128K, D128M, D128F, D128P, D128T, D128W, D128Y, D128V, and D128S; preferably D128G, D128A, D128V, D128L, D128I, D128P, D128W, D128F, D128Y, D128C, D128M, D128N, D128Q, D128S, D128T, D128H, D128R and D128K; most preferably D128G, D128Y, D128F, D128M, D128N, D128S, D128R and D128K.
Clause 21C-29: The nuclease; preferably the endonuclease, according to any of the preceding Clauses, wherein the amino acid sequence is substituted compared to SEQ ID NO: 1 in position Q129; preferably wherein the substitution is selected from the group consisting of Q129A, Q129R, Q129N, Q129D, Q129C, Q129E, Q129G, Q129H, Q129I, Q129L, Q129K, Q129M, Q129F, Q129P, Q129T, Q129W, Q129Y, Q129V, and Q129S; preferably Q129R, Q129K, Q129H, Q129G, Q129A, Q129V, Q129L, Q129I, Q129P, Q129W, Q129F, Q129Y, and Q129D; preferably Q129K, Q129H, and Q129R; more preferably Q129K, Q129R; still more preferably Q129R.
Clause 21C-30: The nuclease; preferably the endonuclease, according to any of the preceding Clauses, wherein the amino acid sequence is substituted compared to SEQ ID NO:1 in position K132; preferably wherein the substitution is selected from the group consisting of K132A, K132R, K132N, K132D, K132C, K132Q, K132E, K132G, K132H, K132I, K132L, K132M, K132F, K132P, K132T, K132W, K132Y, K132V, and K132S; preferably K132R, and K132H; most preferably K132R.
Clause 21C-31: The nuclease; preferably the endonuclease, according to any of the preceding Clauses, wherein the amino acid sequence is substituted compared to SEQ ID NO:1 in position D135; preferably wherein the substitution is selected from the group consisting of D135A, D135R, D135N, D135C, D135Q, D135E, D135G, D135H, D135I, D135L, D135K, D135M, D135F, D135P, D135T, D135W, D135Y, D135V, and D135S; preferably D135G, D135A, D135V, D135L, D135I, D135P, D135C, D135M, D135N, D135Q, D135S, D135T, D135R, D135H, and D135K; more preferably D135G, D135A, D135V, D135L, D135I, D135P, D135C, D135M, D135N, D135Q, D135S, and D135T; still more preferably D135A, and D135N.
Clause 21C-32: The nuclease; preferably the endonuclease, according to any of the preceding Clauses, wherein the amino acid sequence is substituted compared to SEQ ID NO: 1 in position E151; preferably wherein the substitution is selected from the group consisting of E151A, E151R, E151N, E151D, E151C, E151Q, E151G, E151H, E151I, E151L, E151K, E151M, E151F, E151P, E151T, E151W, E151Y, E151V, and E151S; E151G, E151A, E151V, E151L, E151I, E151P, E151W, E151F, E151Y, E151C, E151M, E151N, E151Q, E151S, E151T, E151H and E151K; preferably E151A, E151G, E151V, E151L, E151I, and E151P; more preferably E151L.
Clause 21C-33: The nuclease; preferably the endonuclease, according to any of the preceding Clauses, wherein the amino acid sequence is substituted compared to SEQ ID NO:1 in position K162; preferably wherein the substitution is selected from the group consisting of K162A, K162R, K162N, K162D, K162C, K162Q, K162E, K162G, K162H, K162I, K162L, K162M, K162F, K162P, K162T, K162W, K162Y, K162V, and K162S; preferably K162N, K162Q, K162S, K162T, K162C and K162M; most preferably K162N.
Clause 21C-34: The nuclease; preferably the endonuclease, according to any of the preceding Clauses, wherein the amino acid sequence is substituted compared to SEQ ID NO:1 in position D191, wherein the substitution is selected from the group consisting of D191G, D191A, D191V, D191L, D191I, D191P, D191W, D191F, D191Y, D191C, D191M, D191S, D191Q, D191T, D191N, D191E, D191H, D191R and D191K; preferably D191G, D191A, D191V, D191L, D191I, D191P, D191C, D191M, D191S, D191Q, and D191T; more preferably D191G, D191A, D191V, D191L, D191I, and D191P; most preferably D191P.
Clause 21C-35: The nuclease; preferably the endonuclease, according to any of the preceding Clauses, wherein the amino acid sequence is substituted compared to SEQ ID NO:1 in position P195; preferably wherein the substitution is selected from the group consisting of P195A, P195R, P195N, P195D, P195C, P195Q, P195E, P195G, P195H, P195I, P195L, P195K, P195M, P195F, P195T, P195W, P195Y, P195V, and P195S; preferably P195K, P195H and P195R; most preferably P195R.
Clause 21C-36: The nuclease; preferably the endonuclease, according to any of the preceding Clauses, wherein the amino acid sequence is substituted compared to SEQ ID NO: 1 in position K196; preferably wherein the substitution is selected from the group consisting of K196A, K196R, K196N, K196D, K196C, K196Q, K196E, K196G, K196H, K196I, K196L, K196M, K196F, K196P, K196T, K196W, K196Y, K196V, and K196S; preferably K196G, K196A, K196V, K196L, K196I, K196P, K196R, K196H, K196N, K196Q, K196S, K196T, K196C, and K196M; more preferably K196R, and K196H; most preferably K196R.
Clause 21C-37: The nuclease; preferably the endonuclease, according to any of the preceding Clauses, wherein the amino acid sequence is substituted compared to SEQ ID NO:1 in position G197; preferably wherein the substitution is selected from the group consisting of G197A, G197R, G197N, G197D, G197C, G197Q, G197E, G197H, G197I, G197L, G197K, G197M, G197F, G197P, G197T, G197W, G197Y, G197V, and G197S; preferably G197D, G197E, G197R, G197K, G197H, G197A, G197V, G197L, G197I, and G197P; preferably G197K, G197H, and G197R; more preferably G197K.
Clause 21C-38: The nuclease; preferably the endonuclease, according to any of the preceding Clauses, wherein the amino acid sequence is substituted compared to SEQ ID NO:1 in position D199; preferably wherein the substitution is selected from the group consisting of D199G, D199A, D199V, D199L, D199I, D199P, D199W, D199F, D199Y, D199C, D199M, D199S, D199Q, D199T, D199N, D199E, D199H, D199R and D199K; preferably D199G, D199A, D199V, D199L, D199I, D199P, D199W, D199F, D199Y, D199C, D199M, D199N, D199S, D199Q, D199T, and D199K; preferably D199N, D199S, D199Q, D199T, D199C, D199M, D199H, and D199K; more preferably D199N, D199S, and D199K.
Clause 21C-39: The nuclease; preferably the endonuclease, according to any of the preceding Clauses, wherein the amino acid sequence is substituted compared to SEQ ID NO:1 in position R204; preferably wherein the substitution is selected from the group consisting of R204A, R204N, R204D, R204C, R204Q, R204E, R204G, R204H, R204I, R204L, R204K, R204M, R204F, R204P, R204T, R204W, R204Y, R204V, and R204S; preferably R204G, R204A, R204V, R204L, R204I, R204P, R204K, R204H, R204N, R204Q, R204S, R204T, R204C, and R204M; more preferably R204Q, R204N, R204S, R204T, R204C and R204M; most preferably R204Q.
Clause 21C-40: The nuclease; preferably the endonuclease, according to any of the preceding Clauses, wherein the amino acid sequence is substituted compared to SEQ ID NO:1 in position I218; preferably wherein the substitution is selected from the group consisting of I218R, I218N, I218D, I218C, I218Q, I218E, I218G, I218H, I218A, I218L, I218K, I218M, I218F, I218P, I218T, I218W, I218Y, I218V, and I218S; preferably I218F, I218W and I218Y; more preferably I218F.
Clause 21C-41: The nuclease; preferably the endonuclease, according to any of the preceding Clauses, wherein the amino acid sequence is substituted compared to SEQ ID NO:1 in position A228; preferably wherein the substitution is selected from the group consisting of A228R, A228N, A228D, A228C, A228Q, A228E, A228G, A228H, A228I, A228L, A228K, A228M, A228F, A228P, A228T, A228W, A228Y, A228V, and A228S; preferably A228K, A228H and A228R; more preferably A228R.
Clause 21C-42: The nuclease; preferably the endonuclease, according to any of the preceding Clauses, wherein the amino acid sequence is substituted compared to SEQ ID NO:1 in positionE239, wherein the substitution is selected from the group consisting of E239A, E239R, E239N, E239D, E239C, E239Q, E239G, E239H, E239I, E239L, E239K, E239M, E239F, E239P, E239T, E239W, E239Y, E239V, and E239S; preferably E239G, E239A, E239V, E239L, E239I, E239P, E239W, E239F, E239Y, E239C, E239M, E239N, E239Q, E239S, E239T, E239H and E239K; preferably E239A, E239G, E239V, E239L, E239I, and E239P; more preferably E239A.
Clause 21C-43: The nuclease; preferably the endonuclease, according to any of the preceding Clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions selected from the group consisting of the positions P51, K55, T56, N58, P73, A74, T77, G78, A81, K84, D86, S116, D117, Q120, A124, D128, Q129, D135, K196, G197, D199, and E239; preferably P51, T56, N58, T77, A81, K84, D86, S116, D117, Q120, A124, D128, Q129, D135, K196, and E239; more preferably P51, T56, N58, T77, K84, D86, S116, and D135, and exhibits an increased Tlow activity in comparison to the Tlow activity of SEQ ID NO: 1, wherein,
   when the amino acid substitution comprises a substitution at amino acid position P51, then the amino acid substitution at position P51 is selected from the group consisting of the substitutions D, E, R, K, H, G, A, V, L, and I; preferably G, A, V, I, and L; more preferably G, and L; still more preferably L;
   when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R;
   when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably R, K, H, G, A, V, L, I, P, and D; more preferably G, A, V, L, I, P, and D; still more preferably G, P, and D;
   when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably R, K, and V; still more preferably R, and V;
   when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R;
   when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R;
   when the amino acid substitution comprises a substitution at amino acid position T77, then the amino acid substitution at position T77 is selected from the group consisting of the substitutions R, K, H, G, A, V, L, I, P, W, F, Y, and D; preferably L, F, K, R, and H; more preferably K, R, and H; still more preferably R;
   when the amino acid substitution comprises a substitution at amino acid position G78, then the amino acid substitution at position G78 is selected from the group consisting of the substitutions R, K, H, A, V, L, I, P, W, F, Y, N, Q, S, T, C, M and D; preferably K, R, and H; more preferably R;
   when the amino acid substitution comprises a substitution at amino acid position A81, then the amino acid substitution at position A81 is selected from the group consisting of the substitutions R, K, and H; preferably K;
   when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G;
   when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S;
   when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K;
   when the amino acid substitution comprises a substitution at amino acid position D 117, then the amino acid substitution at position D 1 17 is selected from the group consisting of the substitutions G, A, V, L, I, P, W, F, Y, C, M, S, N, T, Q, H, R, and K; preferably G, V, L, I, P, W, F, Y, C, M, S, N, Q, H, R, and K; more preferably Y, W, F, K, R, and H; still more preferably Y, K, R, and H; yet preferably K, R, and H; even more preferably K;
   when the amino acid substitution comprises a substitution at amino acid position Q120, then the amino acid substitution at position Q 120 is selected from the group consisting of the substitutions R, K, H, G, A, V, L, I, P, W, F, Y, and D; preferably K, H, and R; more preferably R;
   when the amino acid substitution comprises a substitution at amino acid position A124, then the amino acid substitution at position A124 is selected from the group consisting of the substitutions D, E, R, K, H, G, V, L, I, and P; preferably K, H and R; more preferably K;
   when the amino acid substitution comprises a substitution at amino acid position D 128, then the amino acid substitution at position D 128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, R, and K; more preferably G, Y, F, M, N, S, R, and K;
   when the amino acid substitution comprises a substitution at amino acid position Q129, then the amino acid substitution at position Q 129 is selected from the group consisting of the substitutions R, K, H, G, A, V, L, I, P, W, F, Y, and D; preferably K, H, and R; more preferably R;
   when the amino acid substitution comprises a substitution at amino acid position D135, then the amino acid substitution at position D 135 is selected from the group consisting of the substitutions G, A, V, L, I, P, W, F, Y, C, M, N, Q, S, T, H, R, and K; preferably R, K, G, A, V, L, I, P, C, M, N, Q, S, and T; more preferably G, A, V, L, I, P, C, M, N, Q, S, and T; still more preferably A, and N;
   when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R;
   when the amino acid substitution comprises a substitution at amino acid position G197, then the amino acid substitution at position G 197 is selected from the group consisting of the substitutions D, E, R, K, H, A, V, L, I, and P; preferably K, H, and R; more preferably K;
   when the amino acid substitution comprises a substitution at amino acid position D199, then the amino acid substitution at position D 199 is selected from the group consisting of the substitutions G, A, V, L, I, P, W, F, Y, C, M, Q, T, N, S, H, R, and K; preferably G, A, V, L, I, P, W, F, Y, C, M, Q, T, N, S, H, and K; more preferably S, Q, T, N, C, M, H, and K; still more preferably S, N, and K; or
   when the amino acid substitution comprises a substitution at amino acid position E239, then the amino acid substitution at position E239 is selected from the group consisting of the substitutions G, A, V, L, I, P, W, F, Y, C, M, Q, T, N, S, H, R, K, and D; preferably G, A, V, L, I, and P; more preferably A.
Clause 21C-44: The nuclease; preferably the endonuclease, according to any of the preceding Clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two positions such that it comprises at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, or at least twenty-two substitutions selected from the group consisting of the substitutions P51L, P51D, P51G, P51R, K55R, T56M, T56L, T56S, T56A, T56V, T56I, T56G, T56D, T56P, T56R, T56K, T56F, N58V, N58K, N58R, P73K, P73R, A74K, A74R, T77K, T77L, T77R, T77F, G78R, K84G, D86S, S116K, D117K, D117H, D117Y, D117R, Q120R, A124K, D128Y, D128M, D128N, D128K, D128F, D128R, D128S, D128G, Q129R, D135A, D135K, D135R, D135N, D135M, D135G, K196R, G197K, D199K, D199S, D199N, and E239A; preferably P51L, T56A, T56V, T56I, T56M, T56L, T56F, T56D, T56P, T56G, T56K, T56S, N58V, N58R, T77K, T77R, K84G, D86S, S116K, D117H, D117R, D117Y, Q120R, A124K, D128G, D128N, D128S, Q129R, D135A, D135M, D135N, K196R, and E239A; more preferably P51L, T56D, T56P, T56G, N58V, N58R, T77R, K84G, D86S, S116K, D135A, and D135N.
Clause 21C-45: The nuclease; preferably the endonuclease, comprising an amino acid sequence with at least 70%; identity to SEQ ID NO:1; and comprising
   (i) a substitution in position P51, wherein the position is substituted by an amino acid selected from the group consisting of A, G, V, L, and I; preferably L; and
   (ii) a substitution in position T56, wherein the position is substituted by an amino acid selected from the group consisting of A, G, V, L, I, and P; preferably G, A, L, I, and P; more preferably G, and P; still more preferably P.
Clause 21C-46: The nuclease; preferably the endonuclease, comprising an amino acid sequence with at least 70%; identity to SEQ ID NO:1; and comprising
   (i) a substitution in position T56, wherein the position is substituted by an amino acid selected from the group consisting of A, G, V, L, I, and P; preferably G, A, L, I, and P; more preferably G, and P; still more preferably P; and
   (ii) a substitution in position K84, wherein the position is substituted by an amino acid selected from the group consisting of A, G, V, L, I, and P; preferably G.
Clause 21C-47: The nuclease; preferably the endonuclease, comprising an amino acid sequence with at least 70%; identity to SEQ ID NO: 1; and comprising
   (i) a substitution in position T56, wherein the position is substituted by an amino acid selected from the group consisting of A, G, V, L, I, and P; preferably G, A, L, I, and P; more preferably G, and P; still more preferably P;
   (ii) a substitution in position K84, wherein the position is substituted by an amino acid selected from the group consisting of A, G, V, L, I, and P; preferably G; and
   (iii) a substitution in position P51, wherein the position is substituted by an amino acid selected from the group consisting of A, G, V, L, and I; preferably L.
Clause 21C-48: The nuclease; preferably the endonuclease, comprising an amino acid sequence with at least 70%; identity to SEQ ID NO: 1; and comprising
   (i) a substitution in position P51, wherein the position is substituted by an amino acid selected from the group consisting of A, G, V, L, and I; preferably L;
   (ii) a substitution in position T56, wherein the position is substituted by an amino acid selected from the group consisting of A, G, V, L, I, and P; preferably G, A, L, I, and P; more preferably G, and P; still more preferably P; and
   (iii) a substitution in position D135, wherein the position is substituted by an amino acid selected from the group consisting of N, T, Q, S, C, M, A, G, V, L, I, and P; preferably N, and A.
Clause 21C-49: The nuclease; preferably the endonuclease, comprising an amino acid sequence with at least 70%; identity to SEQ ID NO: 1; and comprising
   (i) a substitution in position P51, wherein the position is substituted by an amino acid selected from the group consisting of A, G, V, L, and I; preferably L;
   (ii) a substitution in position T56, wherein the position is substituted by an amino acid selected from the group consisting of A, G, V, L, I, and P; preferably G, A, L, I, and P; more preferably G, and P; still more preferably G; and
   (iii) a substitution in position A81, wherein the position is substituted by an amino acid selected from the group consisting of R, K, and H; preferably R, and K; more preferably K.
Clause 21C-50: The nuclease; preferably the endonuclease, comprising an amino acid sequence with at least 70%; identity to SEQ ID NO: 1; and comprising
   (i) a substitution in position P51, wherein the position is substituted by an amino acid selected from the group consisting of A, G, V, L, and I; preferably L;
   (ii) a substitution in position T56, wherein the position is substituted by an amino acid selected from the group consisting of D, E, C, M, N, Q, S, A, G, V, L, I, and P; preferably P, G, D, and S; more preferably D;
Clause 21C-51: The nuclease; preferably the endonuclease, comprising an amino acid sequence with at least 70%; identity to SEQ ID NO:1; and comprising
   (i) a substitution in position P51, wherein the position is substituted by an amino acid selected from the group consisting of A, G, V, L, and I; preferably L;
   (ii) a substitution in position T56, wherein the position is substituted by an amino acid selected from the group consisting of D, E, C, M, N, Q, S, A, G, V, L, I, and P; preferably P, G, D, and S; more preferably D;
   (iii) a substitution in position E239, wherein the position is substituted by an amino acid selected from the group consisting of A, G, V, L, I, and P; preferably A;
Clause 21C-52: The nuclease; preferably the endonuclease, according to any of the preceding Clauses, wherein the amino acid sequence has an identity to SEQ ID NO:1 of at least 76%; yet more preferably at least 78%; even more preferably at least 80%; most preferably at least 84%, and in particular at least 87%.
Clause 21C-53: The nuclease; preferably the endonuclease, according to any of the preceding Clauses, wherein the amino acid sequence has an identity to SEQ ID NO:1 of at least 88%; preferably at least 89%; more preferably at least 90%; still more preferably at least 91%; yet more preferably at least 92%; even more preferably at least 93%; most preferably at least 94%, and in particular at least 95%.
Clause 21C-54: The nuclease; preferably the endonuclease, according to any of the preceding Clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 8, 9, 10, 11, 12, 13, 14, 15, 17, 18, 19, 20, 21, 22, 23, 24, 26, 27, 28, 29, 36, 37, 38, 39, 40, 41, 42, 43, 44, 45, 46, 47, 49, 50, 51, 52, 57, 60, 61, 62, 63, 64, 65, 66, 73, 74, 75, 76, 77, 78, 79, 80, 81, 82, 83, 84, 85, 86, 87, 88, 89, 90, 91, 92, 93, 94, 95, 96, 97, 98, 99, 102, 112, 116, 119, 120, 121, 122, 123, 124, 125, 126, 127, 128, 129, 130, 131, 132, 133, 134, 135, 136, 137, 138, 139, 140, 141, 142, 143, or 144.
Clause 21C-55: The nuclease; preferably the endonuclease, according to any of the preceding Clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 8, 9, 10, 11, 12, 13, 14, 15, 17, 18, 19, 20, 21, 22, 23, 24, 26, 27, 28, 29, 36, 37, 38, 39, 40, 41, 42, 43, 44, 45, 46, 47, 49, 50, 51, 52, 57, 60, 61, 62, 63, 64, 65, 66, 73, 74, 75, 76, 77, 112, 116, or 119.
Clause 21C-56: The nuclease; preferably the endonuclease, according to any of the preceding Clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to
   (i) SEQ ID NO: 64, 65, 66, 73, 74, 75, 76, 81, 83, 88, 89, 90, 94, 120, 122, 123, 128, 131, and 135; or
   (ii) SEQ ID NO: 64, 65, 66, 73, 74, 75, 76.
Clause 21C-57: The nuclease; preferably the endonuclease, according to any of the preceding Clauses, which is characterized by,
   (A) an increased Tlow activity in comparison to the Tlow activity of SEQ ID NO: 1; and/or
   (B) an increased relative cold activity compared to the relative cold activity of SEQ ID NO:1.
Clause 21C-58: The nuclease; preferably the endonuclease, according to any of the preceding Clauses, which has a relative cold activity that compared to SEQ ID NO: 1 is increased,
   (i) at least 1.05-fold; preferably at least 1.5-fold; more preferably at least 2.0-fold, and still more preferably at least 3.0-fold.; or
   (ii) is increased from 1.05-fold to 5-fold; preferably from 1.5-fold to 5-fold; more preferably from 2-fold to 5-fold, and still more preferably about 3-fold.
Clause 21C-59: The nuclease; preferably the endonuclease, according to any of the preceding Clauses, which has a Tlow activity that compared to SEQ ID NO: 1 is increased,
   (i) at least 1.01-fold ; preferably at least 1.1-fold; more preferably at least 1.3-fold; still more preferably at least 1.5-fold, and yet more preferably at least 1.55-fold; or
   (ii) is increased from 1.01-fold to 2-fold; preferably from 1.1-fold to 2-fold; more preferably from 1.3-fold to 1.6-fold; still more preferably from 1.5-fold to 1.6-fold, and yet more preferably about 1.55-fold. Clause 21C-60: The nuclease; preferably the endonuclease, according to any of the preceding Clauses, wherein the cold activity and the relative cold activity are determined at a Tlow of 5°C and a Thigh of 30°C.
Clause 21C-61: The nuclease; preferably the endonuclease, according to any of the preceding Clauses, wherein the nuclease; preferably the endonuclease, is capable of hydrolyzing of phosphodiesters of polynucleotide substrate into cleaved polynucleotides or oligonucleotides; preferably wherein the polynucleotide substrates are selected from the group consisting of single strand RNA, single strand DNA, double strand DNA, double strand RNA and hybrid strand DNA/RNA; preferably double strand DNA.
Clause 21C-62: A method of hydrolyzing polynucleotide substrates according to any embodiment of any aspect of the invention, comprising the steps of (a) providing a nuclease according to any of the preceding Clauses.
Clause 21C-63: Use of a nuclease according to any of the preceding Clauses for hydrolyzing polynucleotide substrates; preferably being contained in a composition, according to any embodiment of any aspect of the invention.
Clause 22: The nuclease according to any of the preceding Clauses, wherein the amino acid sequence is substituted compared to SEQ ID NO:1 in position P51, wherein the substitution is selected from the group consisting of P5 1D, P51E, P51R, P51K, P51H, P51G, P51A, P51V, P51L, and P51I; preferably P51D, P51R, P51G, and P51L.
Clause 23: The nuclease according to any of the preceding Clauses, wherein the amino acid sequence is substituted compared to SEQ ID NO:1 in position S53, wherein the substitution is selected from the group consisting of S53R, S53K, S53H, S53G, S53A, S53V, S53L, S53I, S53P, S53W, S53F, S53Y, and S53D; preferably S53R, S53K, S53H, S53G, S53A, S53V, S53L, S53I and S53P; more preferably S53K, and S53A.
Clause 24: The nuclease according to any of the preceding Clauses, wherein the amino acid sequence is substituted compared to SEQ ID NO:1 in position K55, wherein the substitution is selected from the group consisting of K55G, K55A, K55V, K55L, K55I, K55P, K55R, K55H, K55N, K55Q, K55S, K55T, K55C, and K55M; preferably K55H and K55R; more preferably K55R.
Clause 25: The nuclease according to any of the preceding Clauses, wherein the amino acid sequence is substituted compared to SEQ ID NO:1 in position T56, wherein the substitution is selected from the group consisting of T56R, T56K, T56H, T56G, T56A, T56V, T56L, T56I, T56P, T56W, T56F, T56Y, T56S, T56M, and T56D; preferably T56R, T56K, T56H, T56G, T56A, T56V, T56L, T56I, T56P, T56S, T56M, and T56D; more preferably T56K, T56R, T56P, and T56D, and even more preferably T56K, T56R, and T56D.
Clause 26: The nuclease according to any of the preceding Clauses, wherein the amino acid sequence is substituted compared to SEQ ID NO:1 in position N58, wherein the substitution is selected from the group consisting of N58R, N58K, N58H, N58G, N58A, N58V, N58L, N58I, N58P, N58W, N58F, N58Y, and N58D; preferably N58R, N58K, N58H, N58G, N58A, N58V, N58L, N58I, and N58P; more preferably N58K, N58R, and N58V.
Clause 27: The nuclease according to any of the preceding Clauses, wherein the amino acid sequence is substituted compared to SEQ ID NO:1 in position A72, wherein the substitution is selected from the group consisting of A72D, A72E, A72R, A72K, A72H, A72G, A72V, A72L, A72I, and A72P; preferably A72K, A72H and A72R; more preferably A72K.
Clause 28: The nuclease according to any of the preceding Clauses, wherein the amino acid sequence is substituted compared to SEQ ID NO:1 in position P73, wherein the substitution is selected from the group consisting of P73D, P73E, P73R, P73K, P73H, P73G, P73V, P73L, P73I, and P73A; preferably P73K, P73H and P73R; more preferably P73K, and P73R.
Clause 29: The nuclease according to any of the preceding Clauses, wherein the amino acid sequence is substituted compared to SEQ ID NO:1 in position A74, wherein the substitution is selected from the group consisting of A74D, A74E, A74R, A74K, A74H, A74G, A74V, A74L, A74I, and A74P; preferably A74K, A74H, and A74R; more preferably A74K, and A74R.
Clause 30: The nuclease according to any of the preceding Clauses, wherein the amino acid sequence is substituted compared to SEQ ID NO:1 in position T77, wherein the substitution is selected from the group consisting of T77R, T77K, T77H, T77G, T77A, T77V, T77L, T77I, T77P, T77W, T77F, T77Y, and T77D; preferably T77K, T77R, T77F, and T77L.
Clause 31: The nuclease according to any of the preceding Clauses, wherein the amino acid sequence is substituted compared to SEQ ID NO:1 in position N80, wherein the substitution is selected from the group consisting of N80R, N80K, N80H, N80G, N80A, N80V, N80L, N80I, N80P, N80W, N80F, N80Y, and N80D; preferably N80K, N80R, and N80H; more preferably N80H.
Clause 32: The nuclease according to any of the preceding Clauses, wherein the amino acid sequence is substituted compared to SEQ ID NO:1 in position K84, wherein the substitution is selected from the group consisting of K84G, K84A, K84V, K84L, K84I, K84P, K84R, K84H, K84N, K84Q, K84S, K84T, K84C, and K84M; preferably K84G, K84A, K84V, K84L, K84I, and K84P; more preferably K84G.
Clause 33: The nuclease according to any of the preceding Clauses, wherein the amino acid sequence is substituted compared to SEQ ID NO:1 in position D86, wherein the substitution is selected from the group consisting of D86G, D86A, D86V, D86L, D86I, D86P, D86C, D86M, D86S, D86T, and D86Q; preferably D86C, D86M, D86S, D86T, and D86Q; more preferably D86S.
Clause 34: The nuclease according to any of the preceding Clauses, wherein the amino acid sequence is substituted compared to SEQ ID NO:1 in position S116, wherein the substitution is selected from the group consisting of S116R, S116K, S116H, S116G, S116A, S116V, S116L, S116I, S116P, S116W, S116F, S116Y, and S116D; preferably S116K, S116H, and S116R; more preferably S116K.
Clause 35: The nuclease according to any of the preceding Clauses, wherein the amino acid sequence is substituted compared to SEQ ID NO:1 in position D117, wherein the substitution is selected from the group consisting of D117G, D 117A, D117V, D117L, D117I, D 117P, D 117W, D 117F, D117Y, D117C, D117M, D117S, D117Q, D117T, D117N, D117H, D117R and D117K; preferably D117G, D117A, D117V, D117L, D117I, D117P, D117W, D117F, D117Y, D117C, D117M, D117S, D117Q, D117T, D117N, D117H, and D117K; more preferably D117G, D117A, D117V, D117L, D1171, D117P, D117C, D117M, D117S, D117Q, D117T, D117N, and D117K; most preferably D117A, D117N, D117K, and D117S.
Clause 36: The nuclease according to any of the preceding Clauses, wherein the amino acid sequence is substituted compared to SEQ ID NO:1 in position Q120, wherein the substitution is selected from the group consisting of Q120R, Q120K, Q120H, Q120G, Q120A, Q120V, Q120L, Q120I, Q120P, Q120W, Q120F, Q120Y, and Q120D; preferably Q120K, Q120H, and Q120R; more preferably Q120R;
Clause 37: The nuclease according to any of the preceding Clauses, wherein the amino acid sequence is substituted compared to SEQ ID NO:1 in position A124, wherein the substitution is selected from the group consisting of A124D, A124E, A124R, A124K, A124H, A124G, A124V, A124L, A1241, and A124P; preferably A124K, A124H and A124R; more prefer-ably A124K, and A124R.
Clause 38: The nuclease according to any of the preceding Clauses, wherein the amino acid sequence is substituted compared to SEQ ID NO:1 in position D128, wherein the substitution is selected from the group consisting of D128G, D128A, D128V, D128L, D128I, D128P, D128W, D128F, D128Y, D128C, D128M, D128N, D128Q, D128S, D128T, D128H, and D128K; preferably D128G, D128Y, D128F, D128M, D128N, D128S, and D128K.
Clause 39: The nuclease according to any of the preceding Clauses, wherein the amino acid sequence is substituted compared to SEQ ID NO:1 in position Q129, wherein the substitution is selected from the group consisting of Q129R, Q129K, Q129H, Q129G, Q129A, Q129V, Q129L, Q129I, Q129P, Q129W, Q129F, Q129Y, and Q129D; preferably Q129K, Q129H, and Q129R; more preferably Q129K, Q129R; still more preferably Q129R.
Clause 40: The nuclease according to any of the preceding Clauses, wherein the amino acid sequence is substituted compared to SEQ ID NO:1 in position D135, wherein the substitution is selected from the group consisting of D135G, D135A, D135V, D135L, D135I, D135P, D135W, D135F, D135Y, D135C, D135M, D135N, D135Q, D135S, D135T, D135K, D135R; preferably D135G, D135A, D135V, D135L, D135I, D135P, D135W, D135F, D135Y, D135C, D135M, D135N, D135Q, D135S, D135T, D135K; more preferably D135G, D135A, D135V, D135L, D135I, D135P, D135W, D135F, D135Y, D135C, D135M, D135N, D135Q, D135S, D135T, D135K; even more preferably D135G, D135A, D135V, D135L, D135I, D135P, D135C, D135M, D135N, D135Q, D135S, D135T, D135H, and D135K; most preferably D135A, D135M, D135G, D135N, and D135K.
Clause 41: The nuclease according to any of the preceding Clauses, wherein the amino acid sequence is substituted compared to SEQ ID NO:1 in position D191, wherein the substitution is selected from the group consisting of D191G, D191A, D191V, D191L, D191I, D191P, D191C, D191M, D191S, D191Q, and D191T; preferably D191G, D191A, D191V, D191L, D191I, and D191P; more preferably D191P.
Clause 42: The nuclease according to any of the preceding Clauses, wherein the amino acid sequence is substituted compared to SEQ ID NO:1 in position K196, wherein the substitution is selected from the group consisting ofK196G, K196A, K196V, K196L, K196I, K196P, K196R, K196H, K196N, K196Q, K196S, K196T, K196C, and K196M; preferably K196R, and K196H; more preferably K196R.
Clause 43: The nuclease according to any of the preceding Clauses, wherein the amino acid sequence is substituted compared to SEQ ID NO:1 in position G197, wherein the substitution is selected from the group consisting of G197D, G197E, G197R, G197K, G197H, G197A, G197V, G197L, G197I, and G197P; preferably G197K, G197H, and G197R; more preferably G197K.
Clause 44: The nuclease according to any of the preceding Clauses, wherein the amino acid sequence is substituted compared to SEQ ID NO:1 in position D199, wherein the substitution is selected from the group consisting ofD199G, D199A, D199V, D199L, D199I, D199P, D199W, D199F, D199Y, D199C, D199M, D199N, D199S, D199Q, D199T, and D199K; preferably D199N, D199S, D199Q, D199T, D199C, D199M, D199H, and D199K; more preferably D199N, D199S, and D199K.
Clause 45: The nuclease according to any of the preceding Clauses, wherein the amino acid sequence is substituted compared to SEQ ID NO:1 in position R204, wherein the substitution is selected from the group consisting of R204G, R204A, R204V, R204L, R204I, R204P, R204K, R204H, R204N, R204Q, R204S, R204T, R204C, and R204M; preferably R204Q, R204N, R204S, R204T, R204C and R204M; more preferably R204Q.
Clause 46: The nuclease according to any of the preceding Clauses, wherein the amino acid sequence is substituted compared to SEQ ID NO:1 in position E239, wherein the substitution is selected from the group consisting of E239G, E239A, E239V, E239L, E239I, E239P, E239W, E239F, E239Y, E239C, E239M, E239N, E239Q, E239S, E239T, E239H and E239K; preferably E239A, E239G, E239V, E239L, E239I, and E239P; more preferably E239A.
Clause 47: The nuclease according to any of the preceding Clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51, S53, K55, T56, N58, A72, P73, A74, T77, N80, K84, D86, S116, D117, Q120, A124, D128, Q129, K132, D135, D191, K196, G197, D199, R204, and E239; preferably P51, T56, N58, T77, K84, Q120, A124, D128, Q129, D135, K196, and G197.
Clause 48: The nuclease according to any of the preceding Clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51, S53, K55, T56, N58, A72, P73, A74, T77, N80, K84, D86, S116, D117, Q120, A124, D128, Q129, K132, D135, D191, K196, G197, D199, R204, and E239; preferably P51, T56, N58, T77, K84, Q120, A124, D128, Q129, D135, K196, and G197; and wherein, when the amino acid substitution comprises a substitution at amino acid position P51, then the amino acid substitution at position P51 is selected from the group consisting of the substitutions D, E, R, K, H, G, A, V, L, and I; preferably D, R, G, and L; when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, and A; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions S, M, R, K, H, G, A, V, L, I, P, W, F, Y, and D; preferably S, M, R, K, G, A, V, L, I, F, P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and V; when the amino acid substitution comprises a substitution at amino acid position A72, then the amino acid substitution at position A72 is selected from the group consisting of the substitutions D, E, R, K, H, G, V, L, I, and P; preferably K, H and R; more preferably K; when the amino acid substitution comprises a substitution at amino acid position P73, then the amino acid substitution at position P73 is selected from the group consisting of the substitutions D, E, R, K, H, G, V, L, I, and A; preferably K, H and R; more preferably K, and R; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L; when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K; when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably A, G, V, L, I, P, C, M, N, T, Q, K, H, R, and S; more preferably A, N, Y, K, H, R, and S; when the amino acid substitution comprises a substitution at amino acid position Q120, then the amino acid substitution at position Q120 is selected from the group consisting of the substitutions R, K, H, G, A, V, L, I, P, W, F, Y, and D; preferably K, H, and R; more preferably R; when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, Y, F, M, N, S, R, and K; when the amino acid substitution comprises a substitution at amino acid position Q129, then the amino acid substitution at position Q129 is selected from the group consisting of the substitutions R, K, H, G, A, V, L, I, P, W, F, Y, and D; preferably K, H, and R; more preferably K, and R; when the amino acid substitution comprises a substitution at amino acid position K132, then the amino acid substitution at position K132 is selected from the group consisting of the substitutions G, A, V, L, I, P, R, H, N, Q, S, T, C, and M; preferably H, and R; more preferably R; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably A, M, G, N, R, and K; when the amino acid substitution comprises a substitution at amino acid position D191, then the amino acid substitution at position D191 is selected from the group consisting of the substitutions G, A, V, L, I, P, C, M, S, Q, and T; preferably G, A, V, L, I, and P; more preferably P; when the amino acid substitution comprises a substitution at amino acid position K196, then the amino acid substitution at position K196 is selected from the group consisting of the substitutions G, A, V, L, I, P, R, H, N, Q, S, T, C, and M; preferably H, and R; more preferably R; when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably S, Q, T, N, C, M, H, R, and K; more preferably N, S, and K; when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q; or when the amino acid substitution comprises a substitution at amino acid position E239, then the amino acid substitution at position E239 is selected from the group consisting of the substitutions G, A, V, L, I, P, W, F, Y, C, M, N, Q, S, T, H, R, and K; preferably A, G, V, L, I, and P; more preferably A.
Clause 49: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five amino acid positions selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A; preferably P51R, P51G, P51L, T56S, T56M, T56R, T56K, T56G, T56A, T56V, T56L, T56I, T56F, T56P, T56D, N58K, N58R, N58V, T77K, T77R, T77F, T77L, K84G, Q120R, A124K, A124R, D128G, D128Y, D128F, D128M, D128N, D128S, D128R, D128K, Q129R, D135A, D135M, D135G, D135N, D135R, D135K, K196R, and G197K.
Clause 50: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five, or at least twenty-six, or at least twenty-seven, or at least twenty-eighth amino acid positions selected from the group consisting of P51, S53, K55, T56, N58, A72, P73, A74, T77, G78, N80, A81, A82, K84, D86, S116, D117, Q120, A124, D128, Q129, K132, D135, D191, K196, G197, D199, R204, and E239, and wherein the amino acid sequence in addition comprises at least one further substitution, wherein the at least one further substitution position is selected from the group consisting ofP51, S53, K55, T56, N58, A72, P73, A74, T77, G78, N80, A81, A82, K84, D86, S116, D117, Q120, A124, D128, Q129, K132, D135, D191, K196, G197, D199, R204, and E239.
Clause 51: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty-one, at least twenty-two, at least twenty-three, at least twenty-four, or at least twenty-five, or at least twenty-six, or at least twenty-seven, or at least twenty-eighth amino acid positions selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A, and wherein the amino acid sequence in addition comprises at least one further substitution, wherein the at least one further substitution position is selected from the group consisting of P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A.
Clause 52: The nuclease according to any of the preceding clauses, wherein the identity of the amino acid sequence of the nuclease with the sequence of SEQ ID NO:1 is at least 65%, or at least 66%, or at least 67%, or at least 68%, or at least 69%, or at least 70%, or at least 71%, or at least 72%, or at least 73%, or at least 74%, or at least 75%, or at least 76%, or at least 77%, or at least 78%, or at least 79%, or at least 80%, or at least 81%, or at least 82%, or at least 83%, or at least 84%, or at least 85%; preferably at least 86%, or at least 87%, or at least 88%, or at least 89%, or at least 90%; more preferably at least 91%, or at least 92%, or at least 93%, or at least 94%, or at least 95%; still more preferably at least 96%, or at least 97%, or at least 98%, or at least 99%; yet more preferably at least 99.1%, or at least 99.2%, or at least 99.3%, or at least 99.4, or at least 99.5%; even more preferably at least 99.6%. Clause 53: The nuclease according to any of the preceding clauses, wherein the identity of the amino acid sequence of the nuclease with the sequence of SEQ ID NO:1 is at least 89%, or at least 90%, or at least 91%, or at least 92%, or at least 93%, or at least 94%, or at least 95%; preferably at least 96%, or at least 97%, or at least 98%, or at least 99%; more preferably at least 99.1%, or at least 99.2%, or at least 99.3%, or at least 99.4, or at least 99.5%; still more preferably at least 99.6%.
Clause 54: The nuclease according to any of the preceding clauses, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, or 102.
Clause 55: The nuclease according to any of the preceding clauses, which is characterized by either (A) an increased thermal stability in comparison to the wildtype nuclease of SEQ ID NO:1; and/or (B) an increased relative salt activity in comparison to the relative salt activity of the wildtype nuclease of SEQ ID NO:1; and/or (C) an increased S_{high} activity in comparison to the S_{high} activity of the wildtype nuclease of SEQ ID NO:1; and/or (D) an increased relative cold activity in comparison to the relative cold activity of the wildtype nuclease of SEQ ID NO:1; and/or (E) an increased T_{low} activity in comparison to the T_{low} activity of the wildtype nuclease of SEQ ID NO:1.
Clause 56: The nuclease according to any of the preceding clauses, which has an increased thermal stability than SEQ ID NO:1, wherein said thermal stability is characterized by a higher residual activity after incubation for 15 minutes at a temperature T_{stability}.
Clause 57: The nuclease according to clause 56, wherein the residual activity of an individual nuclease is detected by (i) separately incubating two vessels with a specific amount of nuclease in a buffer containing 50 mM Tris-HCl, pH 8.0, 5 mM MgCl2, 0.1 mg/ml BSA for 15 min at either 23°C or T_{stability}, respectively, and (ii) subsequently cooling down the two vessels, (iii) measuring the residual activity of either nuclease from step (ii) by transferring amounts of either nuclease in a specific ratio into new separate vessels and mixing with DNA sodium salt from salmon testes to a final concentration of 0.8 mg/ml, and incubating each new vessel for 1 h at 30°C, then adding 100 µl of 4 % perchloric acid to terminate the reaction, incubating the two obtained composition on ice for 45 min, and subsequently centrifuging the obtained composition at 3,800xg at 4°C for 30 minutes, and measuring the absorbance of each obtained supernatant at 260 nm which absorbance correlates to the enzymatic activity of the nuclease of the corresponding vessel, and (iv) calculating the residual activity as the ratio of absorbances measured for the sample at T_{stability} and at 23°C expressed as percentage (A260 [T_{stability}]: A260 [T_{23°C}]) wherein for any nuclease variant the specific ratio of the amounts of nucleases being transferred into the two vessels in step (ii) is defined by the amounts of nuclease of SEQ ID NO:1 after incubation at either 23°C or T_{stability} that are required to obtain a detectable and linear signal in measuring the residual activity in step (iii).
Clause 58: The nuclease according to clause 56 or 57, wherein the residual activity is determined at a temperature T_{stability} from 40°C to 60°C; preferably from 45°C to 55°C; more preferably from 49°C to 53°C, and still more preferably 51°C.
Clause 59: The nuclease according to any of clauses 56 to 58, wherein the thermal stability of the nuclease is at least 16%; preferably at least 23%; more preferably at least 29%, and still more preferably at least 59% increased in comparison to SEQ ID NO:1, when the residual activity is determined at a temperature T_{stability} of 51°C.
Clause 60: The nuclease according to any of clauses 56 to 59, wherein the thermal stability of the nuclease is at least 16%; preferably at least 23%; more preferably at least 29%, and still more preferably at least 59% increased in comparison to SEQ ID NO: 1.
Clause 61: The nuclease according to any of clauses 56 to 60, which has a thermal stability that compared to SEQ ID NO:1 is increased at least 1.051- fold, at least 1.5-fold, or at least 2-fold; more preferably at least 3-fold, at least 4-fold, or at least 5-fold, and still more preferably at least 6-fold, at least 6.5-fold, or at least 7-fold.
Clause 62: The nuclease according to any of clauses 56 to 61, which has a thermal stability that compared to SEQ ID NO:1 is increased from 1.051- fold to 100-fold; preferably from 1.5-fold to 50-fold; more preferably from 2-fold to 40-fold; still more preferably from 3-fold, to 30-fold; yet more preferably from 4-fold to 23-fold; even more preferably from 5-fold to 21-fold, and most preferably from 6-fold to 15-fold.
Clause 63: The nuclease according to any of the preceding clauses, which has (i) a higher enzymatic activity at a salt concentration S_{high} (S_{high} activity) than SEQ ID NO: 1; and/or (ii) a higher relative salt activity in comparison to SEQ ID NO:1, wherein said relative salt activity is the ratio of enzymatic activity at the salt concentration S_{high} in comparison to the salt concentration S_{low}.
Clause 64: The nuclease according to clause 63, wherein the S_{high} activity, the S_{low} activity, and the relative salt activity are determined by (i) separately detecting the enzymatic activity of the nuclease at salt concentrations S_{low} and S_{high} by transferring amounts of such nuclease in a specific ratio into two separate vessels, mixing with buffer to a final concentration of 50 mM Tris-HCl, pH 8.0, 5.0 mM MgCl2, 0.1 mg/ml BSA, and 0.8 mg/ml DNA sodium salt from salmon testes, and a salt concentration of S_{low} or S_{high}, respectively, incubating at 30°C for 1 h, then adding 100 µl of 4 % perchloric acid to terminate the reaction, incubating the two obtained compositions on ice for 45 min, and subsequently centrifuging the obtained compositions at 3,800xg at 4°C for 30 minutes, and measuring the absorbance of each obtained supernatant at 260 nm, wherein the detected absorbances correlate to the enzymatic activity of the nuclease at salt concentrations S_{low} (S_{low} activity) and S_{high} (S_{high} activity), respectively, and (ii) calculating the relative salt activity as the ratio of absorbances measured for the sample incubated at S_{high} (S_{high} activity) and the sample at S_{low} (S_{low} activity) ex-pressed as percentage (A260 [S_{high}]: A260 [S_{low}]), wherein for any nuclease variant the specific ratio of the amounts of nucleases being transferred into the two vessels in step (i) is defined by the amounts of nuclease of SEQ ID NO:1 being required to obtain a detectable and linear signal in measuring the S_{low} activity or the S_{high} activity in step (ii), respectively. Clause 65: The nuclease according to clause 63 or 64, wherein the salt is NaCl or KCl; preferably NaCl. Clause 66: The nuclease according to any of clauses 63 to 65, wherein in determining of the S_{high} activity, the S_{low} activity, and the relative salt activity the concentration of - S_{low} is within the range of from 0.00M to 0.15M; preferably at 0.00M; and - S_{high} is from 0.01M to LOOM; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.
Clause 67: The nuclease according to any of clauses 63 to 66, which has a relative salt activity that compared to SEQ ID NO:1 is increased at least 1.05-fold; preferably at least 7-fold; more preferably at least 9-fold; still more preferably at least 13-fold; yet more preferably at least 60-fold, and even more preferably at least 110-fold.
Clause 68: The nuclease according to any of clauses 63 to 67, which has a relative salt activity that compared to SEQ ID NO:1 is increased from 1.05-fold to 300-fold preferably from 7-fold to 250-fold; more preferably from 9-fold to 200-fold; still more preferably from 13-fold to 150-fold, and yet more preferably about 115-fold.
Clause 69: The nuclease according to any of clauses 63 to 68, wherein the relative salt activity is at least 3%; preferably at least 25%; more preferably at least 50%; still more preferably at least 100%; yet more preferably at least 130%; even more preferably at least 200%, and most preferably at least 800% in-creased in comparison to SEQ ID NO:1.
Clause 70: The nuclease according to any of clauses 63 to 69, which has a S_{high} activity that compared to the S_{high} activity of SEQ ID NO:1 is increased at least 1.01-fold; preferably at least 1.5-fold; more preferably at least 2-fold; still more preferably at least 5-fold, yet more prefer-ably at least 12-fold, and even more preferably at least 19-fold.
Clause 71: The nuclease according to any of clauses 63 to 70, which has a S_{high} activity that compared to the SEQ ID NO: 1 is increased from 1.01-fold to 200-fold; preferably from 1.5-fold to 150-fold; more preferably from 2-fold to 100-fold; still more preferably from 5-fold to 75-fold; yet more preferably from 12-fold to 50-fold, and even more preferably about 20-fold.
Clause 72: The nuclease according to any of clauses 63 to 71 wherein the salt activity and the relative salt activity are determined at a S_{low} of 0.00M and S_{high} of 500 mM.
Clause 73: The nuclease according to any of the preceding clauses, which has (i) a higher enzymatic activity at a temperature T_{low} below 30°C (T_{low} activity) than SEQ ID NO: 1; and/or (ii) a higher relative cold activity than SEQ ID NO: 1, wherein said relative cold activity is the ratio of the enzymatic activity at a temperature T_{low} below 30°C to the enzymatic activity at a temperature T_{high} of 30°C or above (T_{low} activity: T_{high} activity).
Clause 74: The nuclease according to clause 73, wherein the T_{low} activity, the T_{high} activity, and the relative cold activity are determined by (i) separately detecting the enzymatic activity of the nuclease at temperatures T_{low} and T_{high} by transferring amounts of such nuclease in a specific ratio into two separate vessels, mixing with buffer to a final concentration of 50 mM Tris-HCl, pH 8.0, 5.0 mM MgCl2, 0.1 mg/ml BSA, and 0.8 mg/ml DNA sodium salt from salmon testes, and incubating at temperatures T_{low} and at T_{high}, respectively for 1.0 h, then adding 100 µl of 4 % perchloric acid to terminate the reaction, incubating the two obtained composition on ice for 45 min, and subsequently centrifuging the obtained composition at 3,800xg at 4°C for 30 minutes, and measuring the absorbance of each obtained supernatant at 260 nm, wherein the detected absorbances correlate to the enzymatic activity of the nuclease at T_{low} (T_{low} activity) and at T_{high} (T_{high} activity), respectively, and (ii) calculating the relative cold activity as the ratio of absorbances measured for the sample incubated at T_{low} (T_{low} activity) and the sample at T_{high} (T_{high} activity) ex-pressed as percentage (A260 [T_{low}]: A260 [T_{high}]) wherein for any nuclease variant the specific ratio of the amounts of nucleases being transferred into the two vessels in step (i) is defined by the amounts of nuclease of SEQ ID NO:1 being required to obtain a detectable and linear signal in measuring the T_{low} activity or a T_{high} activity in step (ii), respectively.
Clause 75: The nuclease according to clause 73 or 74, wherein - T_{low} is within the range of from 4°C to 25°C; preferably from 4.0°C to 15°C; more preferably from 4.0°C to 10°C; still more preferably from 4.0°C to 6.0°C; yet more preferably 5.0°C; and - T_{high} is within the range of from 30°C to 60°C; preferably from 30°C to 50°C; more preferably from 30°C to 40°C; still more preferably 30°C.
Clause 76: The nuclease according to any of clauses 73 to 75, wherein T_{low} is from 4.0°C to 15°C and T_{high} is from 25°C to 60°C; preferably T_{low} is from 4.0°C to 10°C and T_{high} is from 25°C to 50°C; more preferably T_{low} is from 4.0°C to 6.0°C and T_{high} is from 25°C to 40°C; still more preferably T_{low} is 5.0°C and T_{high} is 30°C.
Clause 77: The nuclease according to any of clauses 73 to 76, which has a relative cold activity that compared to SEQ ID NO:1 is increased at least 1.05-fold; preferably at least 1.5-fold; more preferably at least 2.0-fold, and still more preferably at least 3.0-fold.
Clause 78: The nuclease according to any of clauses 73 to 77, which has a relative cold activity that compared to SEQ ID NO:1 is increased from 1.05-fold to 5-fold; preferably from 1.5-fold to 5-fold; more preferably from 2-fold to 5-fold, and still more preferably about 3-fold.
Clause 79: The nuclease according to any of clauses 73 to 78, which has a T_{low} activity that compared to SEQ ID NO:1 is increased at least 1.01-fold ; preferably at least 1.1-fold; more preferably at least 1.3-fold; still more preferably at least 1.5-fold, and yet more preferably at least 1.55-fold.
Clause 80: The nuclease according to any of clauses 73 to 79, which has a T_{low} activity that compared to SEQ ID NO:1 is increased from 1.01-fold to 2-fold; preferably from 1.1-fold to 2-fold; more preferably from 1.3-fold to 1.6-fold; still more preferably from 1.5-fold to 1.6-fold, and yet more preferably about 1.55-fold.
Clause 81: The nuclease according to any of clauses 73 to 80 wherein the cold activity and the relative cold activity are determined at a T_{low} of 5°C and a T_{high} of 30°C.
Clause 82: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen amino acid positions selected from the group consisting of the positions P51, K55, T56, N58, T77, N80, K84, D86, S116, A124, D128, D135, D191, R204, and E239, and exhibits an increased thermal stability in comparison to SEQ ID NO: 1.
Clause 83: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen positions select-ed from the group consisting of the positions P51, K55, T56, N58, T77, N80, K84, D86, S116, A124, D128, D135, D191, R204, and E239, and exhibits an increased thermal stability of at least 1,051- fold, at least 1,5-fold, or at least 2-fold; more preferably at least 3-fold, at least 4-fold, or at least 5-fold, and still more preferably at least 6-fold, at least 6,5-fold, or at least 7-fold in comparison to the thermal stability of SEQ ID NO: 1.
Clause 84: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen positions selected from the group consisting of the positions P51, K55, T56, N58, T77, N80, K84, D86, S116, A124, D128, D135, D191, R204, and E239, and exhibits an increased thermal stability in comparison to SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably D, E, G, A, V, L, and I; more preferably L, and D; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably G, A, V, L, I, P, and D; still more preferably P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably G, A, V, L, I, and P; still more preferably V; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L; when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K; when the amino acid substitution comprises a substitution at amino acid position A124, then the amino acid substitution at position A124 is selected from the group consisting of the substitutions D, E, R, K, H, G, V, L, I, and P; preferably K, Hand R; more preferably K; when the amino acid substitution comprises a substitution at amino acid position D 128, then the amino acid substitution at position D 128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably W, F, Y, C, M, N, Q, S, T; most preferably M, and Y; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R and K; preferably G, A, V, L, I, P, W, F, Y, C, M, N, Q, S, T, H, and K; more preferably G, A, V, L, I, P, C, M, N, Q, S, T, H, and K; still more preferably C, M, Q, S, T and N; most preferably N; when the amino acid substitution comprises a substitution at amino acid position D191, then the amino acid substitution at position D191 is selected from the group consisting of the substitutions G, A, V, L, I, P, C, M, S, Q, and T; preferably G, A, V, L, I, and P; more preferably P; when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q; or when the amino acid substitution comprises a substitution at amino acid position E239, then the amino acid substitution at position E239 is selected from the group consisting of the substitutions G, A, V, L, I, P, W, F, Y, C, M, N, Q, S, T, H, R, and K; preferably A, G, V, L, I, and P; more preferably A.
Clause 85: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen positions such that it comprises at least one, at least two, at least three, at least four, at least five, at least six, at least seven, at least eight, at least nine, at least ten, at least eleven, at least twelve, at least thirteen, at least fourteen, or at least fifteen substitution selected from the group consisting of the substitutions P51L, P51D, K55R, T56P, T56D, N58V, T77K, T77F, T77L, T77R, N80H, K84G, D86S, S116K, A124K, D128M, D128Y, D135N, D191P, R204Q, and E239A, exhibits an increased thermal stability in comparison to SEQ ID NO:1.
Clause 86: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, or at least seventeen positions, wherein the substitution is selected from the group consisting of the positions P51, S53, K55, T56, N58, A72, P73, A74, T77, S116, D117, A124, D128, D135, K196, G197, and D199, and wherein the nuclease exhibits an increased relative salt activity compared to the relative salt activity of SEQ ID NO:1.
Clause 87: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, or at least seventeen positions, wherein the substitution is selected from the group consisting of the positions P51, S53, K55, T56, N58, A72, P73, A74, T77, S116, D117, A124, D128, D135, K196, G197, and D199, and wherein the nuclease exhibits an increased relative salt activity of at least 1.05-fold; preferably at least 7-fold; more preferably at least 9-fold; still more preferably at least 13-fold; yet more preferably at least 60-fold, and even more preferably at least 110-fold compared to the relative salt activity of SEQ ID NO:1.
Clause 88: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, or at least seventeen positions selected from the group consisting of the positions P51, S53, K55, T56, N58, A72, P73, A74, T77, S116, D117, A124, D128, D135, K196, G197, and D199, and exhibits an increased relative salt activity compared to the relative salt activity of SEQ ID NO: 1, wherein, when the amino acid substitution comprises a substitution at amino acid position P51, then the amino acid substitution at position P51 is selected from the group consisting of the substitutions D, E, R, K, H, G, A, V, L, and I; preferably R, G, and L; when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, and A; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably K, R, and H; still more preferably K, and R; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R; when the amino acid substitution comprises a substitution at amino acid position A72, then the amino acid substitution at position A72 is selected from the group consisting of the substitutions D, E, R, K, H, G, V, L, I, and P; preferably K, H and R; more preferably K; when the amino acid substitution comprises a substitution at amino acid position P73, then the amino acid substitution at position P73 is selected from the group consisting of the substitutions D, E, R, K, H, G, V, L, I, and A; preferably K, H and R; more preferably K, and R; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, H, W, F, and Y; more preferably K, R, and F; when the amino acid substitution comprises a substitution at amino acid position S 116, then the amino acid substitution at position S116 is selected from the group consisting of the substitutions R, K, H, G, A, V, L, I, P, W, F, Y, and D; preferably K, H, and R; more preferably K; when the amino acid substitution comprises a substitution at amino acid position D1 17, then the amino acid substitution at position D117 is selected from the group consisting of the substitutions G, A, V, L, I, P, W, F, Y, C, M, S, N, T, Q, H, and K; preferably A, G, V, L, I, P, C, M, N, T, Q, K, H, and S; more preferably A, K, N, and S; when the amino acid substitution comprises a substitution at amino acid position A124, then the amino acid substitution at position A124 is selected from the group consisting of the substitutions D, E, R, K, H, G, V, L, I, and P; preferably K, Hand R; more preferably K, and R; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably G, Y, F, M, N, S, and K; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably G, A, V, L, I, P, C, M, N, Q, S, T, H, and K; most preferably G, N, and K; when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R; when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably G, A, V, L, I, P, W, F, Y, C, M, Q, T, N, S, H, and K; more preferably S, Q, T, N, C, M, H, and K; most preferably S, N, and K.
Clause 89: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, at least fifteen, at least sixteen, or at least seventeen positions such that it comprises at least one, at least two, at least three, at least four, at least five, at least six, at least seven, at least eight, at least nine, at least ten, at least eleven, at least twelve, at least thirteen, at least fourteen, at least fifteen, at least six-teen, or at least seventeen substitutions selected from the group consisting of the substitutions P51R, P51G, P51L, S53K, S53A, K55R, T56R, T56K, N58R, N58K, A72K, P73K, P73R, A74R, A74K, T77F, T77K, T77R, S116K, D117A, D117S, D117K, D117N, A124K, A124R, D128K, D128Y, D128S, D128M, D128N, D128F, D128G, D135G, D135N, D135K, K196R, G197K, D199K, D199N, and D199S, and exhibits an increased relative salt activity compared to the relative salt activity of SEQ ID NO:1.
Clause 90: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, or at least thirteen positions selected from the group consisting of the positions P51, S53, T56, N58, A72, A74, T77, D117, A124, D128, D135, K196, and G197, and exhibits an increased S_{high} activity compared to the S_{high} activity of SEQ ID NO:1.
Clause 91: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, or at least thirteen positions selected from the group consisting of the positions P51, S53, T56, N58, A72, A74, T77, D117, A124, D128, D135, K196, and G197, and exhibits an increased S_{high} activity of at least 1.01-fold; preferably at least 1.5-fold; more preferably at least 2-fold; still more preferably at least 5-fold, yet more prefer-ably at least 12-fold, and even more preferably at least 19-fold compared to the S_{high} activity of SEQ ID NO: 1.
Clause 92: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, or at least thirteen positions selected from the group consisting of the positions P51, S53, T56, N58, A72, A74, T77, D117, A124, D128, D135, K196, and G197, and exhibits an increased S_{high} activity compared to the S_{high} activity of SEQ ID NO:1 wherein, when the amino acid substitution comprises a substitution at amino acid position P51, then the amino acid substitution at position P51 is selected from the group consisting of the substitutions D, E, R, K, H, G, A, V, L, and I; preferably R, G, and L; when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, R, and H; still more preferably K; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably K, R, and H; still more preferably K, and R; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R; when the amino acid substitution comprises a substitution at amino acid position A72, then the amino acid substitution at position A72 is selected from the group consisting of the substitutions D, E, R, K, H, G, V, L, I, and P; preferably K, Hand R; more preferably K; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, and H; more preferably K, and R; when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably G, A, V, L, I, P, W, F, Y, C, M, S, N, T, Q, H, and K; more preferably A, G, V, L, I, P, C, M, N, T, Q, K, H, and S; still more preferably A, G, V, L, I, P, C, M, N, T, Q, and S; most preferably A, and S; when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably G, Y, F, M, N, S, and K; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, W, F, Y, C, M, N, Q, S, T, H, and K; more preferably G, A, V, L, I, P, C, M, N, Q, S, T, H, and K; most preferably G, N, and K; when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R; or when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K.
Clause 93: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, or at least thirteen positions such that it comprises at least one, at least two, at least three, at least four, at least five, at least six, at least seven, at least eight, at least nine, at least ten, at least eleven, at least twelve, or at least thirteen substitutions selected from the group consisting of the substitutions P51L, P51R, P51G, S53K, T56R, T56K, N58R, N58K, A72K, A74K, A74R, T77R, T77K, D117A, D117S, A124K, A124R, D128S, D128N, D128K, D128G, D128M, D128Y, D128F, D135K, D135G, D135N, K196R, and G197K, and exhibits an increased S_{high} activity compared to the S_{high} activity of SEQ ID NO:1.
Clause 94: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen positions select-ed from the group consisting of the positions P51, K55, T56, N58, P73, A74, T77, K84, D86, S116, D117, D128, D135, G197, and D199, and exhibits an increased relative cold activity compared to the relative cold activity of SEQ ID NO:1. Clause 95: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen position select-ed from the group consisting of the positions P51, K55, T56, N58, P73, A74, T77, K84, D86, S116, D117, D128, D135, G197, and D199, and exhibits an increased relative cold activity of at least 1.05-fold; preferably at least 1.5-fold; more preferably at least 2.0-fold, and still more preferably at least 3.0-fold compared to the relative cold activity of SEQ ID NO:1.
Clause 96: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen positions select-ed from the group consisting of the positions P51, K55, T56, N58, P73, A74, T77, K84, D86, S116, D117, D128, D135, G197, and D199, and exhibits an increased relative cold activity compared to the relative cold activity of SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably D, R, G, and L; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably K, R, P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and V; when the amino acid substitution comprises a substitution at amino acid position P73, then the amino acid substitution at position P73 is selected from the group consisting of the substitutions D, E, R, K, H, G, V, L, I, and A; preferably K, H and R; more preferably K, and R; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K; when the amino acid substitution comprises a substitution at amino acid position D117, then the amino acid substitution at position D117 is selected from the group consisting of the substitutions G, A, V, L, I, P, W, F, Y, C, M, S, N, T, Q, H, R and K; preferably G, A, V, L, I, P, W, F, Y, C, M, S, N, T, Q, H, and K; more preferably A, G, V, L, I, P, C, M, N, T, Q, K, H, and S; even more preferably H, and K; most preferably K; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R and K; preferably G, A, V, L, I, P, W, F, Y, C, M, N, Q, S, T, H, and K; more preferably G, Y, F, M, N, S, and K; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R and K; preferably G, A, V, L, I, P, W, F, Y, C, M, N, Q, S, T, H, and K; more preferably G, A, V, L, I, P, C, M, N, Q, S, T, H, and K; most preferably G, N, and K; when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or when the amino acid substitution comprises a substitution at amino acid position D199, then the amino acid substitution at position D199 is selected from the group consisting of the substitutions G, A, V, L, I, P, W, F, Y, C, M, Q, T, N, S, H, and K; preferably S, Q, T, N, C, M, H, and K; more preferably N, S, and K.
Clause 97: The nuclease according to any of the preceding clauses, wherein the amino acid sequence is substituted compared to SEQ ID NO:1 in at least one, at least two, at least three, at least four, at least five, at least six, at least seven, at least eight, at least nine, at least ten, at least eleven, at least twelve, at least thirteen, at least fourteen, or at least fifteen positions such that it comprises at least one, at least two, at least three, at least four, at least five, at least six, at least seven, at least eight, at least nine, at least ten, at least eleven, at least twelve, at least thirteen, at least fourteen, or at least fifteen substitutions selected from the group consisting of the substitutions P51L, P51D, P51G, P51R, K55R, T56D, T56R, T56P, T56K, N58V, N58K, N58R, P73R, P73K, A74K, A74R, T77K, T77L, T77R, T77F, K84G, D86S, S116K, D117K, D128Y, D128M, D128N, D128K, D128F, D128S, D128G, D135K, D135N, D135G, G197K, D199K, D199N, and D199S, and exhibits an increased relative cold activity compared to the relative cold activity of SEQ ID NO:1.
Clause 98: The nuclease according to any of the preceding clauses, wherein the amino acid sequence is substituted compared to SEQ ID NO:1 in at least one, at least two, at least three, at least four, at least five, or at least six positions selected from the group consisting of the posi-tions P51, T56, N58, K84, D86, and S116, and exhibits an increased T_{low} activity in com-parison to the T_{low} activity of SEQ ID NO:1.
Clause 99: The nuclease according to any of the preceding clauses, wherein the amino acid sequence is substituted compared to SEQ ID NO:1 in at least one, at least two, at least three, at least four, at least five, or at least six positions selected from the group consisting of the positions P51, T56, N58, K84, D86, and S116, and exhibits an increased T_{low} activity of at least 1.01-fold ; preferably at least 1.1-fold; more preferably at least 1.3-fold; still more preferably at least 1.5-fold, and yet more preferably at least 1.55-fold in comparison to the T_{low} activity of SEQ ID NO: 1.
Clause 100: The nuclease according to any of the preceding clauses, wherein the amino acid sequence is substituted compared to SEQ ID NO: 1 in at least one, at least two, at least three, at least four, at least five, or at least six positions selected from the group consisting of the positions P51, T56, N58, K84, D86, and S116, and exhibits an increased T_{low} activity in com-parison to the T_{low} activity of SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably G, A, V, I, and L; more preferably L; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably G, A, V, L, I, P, and D; still more preferably P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably G, A, L, I, P, and V; still more preferably V; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; or when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K.
Clause 101: The nuclease according to any of the preceding clauses, wherein the amino acid sequence is substituted compared to SEQ ID NO: 1 in at least one, at least two, at least three, at least four, at least five, or at least six positions such that it comprises at least one, at least two, at least three, at least four, at least five, or at least six substitutions selected from the group consisting of the substitutions P51L, T56D, T56P, N58V, K84G, D86S, and S116K, and exhibits an increased T_{low} activity in comparison to the T_{low} activity of SEQ ID NO: 1.
Clause 102: The nuclease according to any of the preceding clauses, wherein the amino acid sequence is substituted compared to SEQ ID NO: 1 in at least two, at least three, at least four, at least five, at least six, at least seven, at least eight, at least nine, at least ten, at least eleven, at least twelve, at least thirteen, at least fourteen, or at least fifteen amino acid positions selected from the group consisting of the positions P51, K55, T56, N58, T77, N80, K84, D86, S116, A124, D128, D135, D191, R204, and E239, and exhibits an increased thermal stability in comparison to SEQ ID NO: 1.
Clause 103: The nuclease according to any of the preceding clauses, wherein the amino acid sequence is substituted compared to SEQ ID NO: 1 in at least two, at least three, at least four, at least five, at least six, at least seven, at least eight, at least nine, at least ten, at least eleven, at least twelve, at least thirteen, at least fourteen, or at least fifteen positions selected from the group consisting of the positions P51, K55, T56, N58, T77, N80, K84, D86, S116, A124, D128, D135, D191, R204, and E239, and exhibits an increased thermal stability of at least 1.051- fold, at least 1.5-fold, or at least 2-fold; more preferably at least 3-fold, at least 4-fold, or at least 5-fold, and still more preferably at least 6-fold, at least 6.5-fold, or at least 7-fold in comparison to the thermal stability of SEQ ID NO:1.
Clause 104: The nuclease according to any of the preceding clauses, wherein the amino acid sequence is substituted compared to SEQ ID NO: 1 in at least two, at least three, at least four, at least five, at least six, at least seven, at least eight, at least nine, at least ten, at least eleven, at least twelve, at least thirteen, at least fourteen, or at least fifteen positions selected from the group consisting of the positions P51, K55, T56, N58, T77, N80, K84, D86, S116, A124, D128, D135, D191, R204, and E239, and exhibits an increased thermal stability in comparison to SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably D, E, G, A, V, L, and I; more preferably L, and D; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably G, A, V, L, I, P, and D; still more preferably P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably R, and V; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L; when the amino acid substitution comprises a substitution at amino acid position N80, then the amino acid substitution at position N80 is selected from the group consisting of the substitutions R, K, H, G, A, V, L, I, P, W, F, Y, and D; preferably K, R, and H; more preferably H; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K; when the amino acid substitution comprises a substitution at amino acid position A124, then the amino acid substitution at position A124 is selected from the group consisting of the substitutions D, E, R, K, H, G, V, L, I, and P; preferably K, H and R; more preferably K; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably W, F, Y, C, M, N, Q, S, T, and R; more preferably R, M, and Y; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably M, R, and N; when the amino acid substitution comprises a substitution at amino acid position D191, then the amino acid substitution at position D191 is selected from the group consisting of the substitutions G, A, V, L, I, P, C, M, S, Q, and T; preferably G, A, V, L, I, and P; more preferably P; when the amino acid substitution comprises a substitution at amino acid position R204, then the amino acid substitution at position R204 is selected from the group consisting of the substitutions G, A, V, L, I, P, K, H, N, Q, S, T, C, and M; preferably Q, N, S, T, C and M; more preferably Q; or when the amino acid substitution comprises a substitution at amino acid position E239, then the amino acid substitution at position E239 is selected from the group consisting of the substitutions G, A, V, L, I, P, W, F, Y, C, M, N, Q, S, T, H, R, and K; preferably A, G, V, L, I, and P; more preferably A.
Clause 105: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, or at least fifteen positions such that it comprises at least two, at least three, at least four, at least five, at least six, at least seven, at least eight, at least nine, at least ten, at least eleven, at least twelve, at least thirteen, at least fourteen, or at least fifteen substitution selected from the group consisting of the substitutions P51L, P51D, K55R, T56P, T56D, N58V, N58R, T77K, T77F, T77L, T77R, N80H, K84G, D86S, S116K, A124K, D128R, D128M, D128Y, D135N, D135M, D135R, D191P, R204Q, and E239A, exhibits an increased thermal stability in comparison to SEQ ID NO:1.
Clause 106: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, or at least seventeen positions, wherein the substitution is selected from the group consisting of the positions P51, S53, K55, T56, N58, A72, P73, A74, T77, S116, D117, Q120, A124, D128, Q129, D135, K196, G197, and D199, and wherein the nuclease exhibits an in-creased relative salt activity compared to the relative salt activity of SEQ ID NO:1.
Clause 107: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, or at least seventeen positions, wherein the substitution is selected from the group consisting of the positions P51, S53, K55, T56, N58, A72, P73, A74, T77, S116, D117, Q120, A124, D128, Q129, D135, K196, G197, and D199, and wherein the nuclease exhibits an in-creased relative salt activity of at least 1.05-fold; preferably at least 7-fold; more preferably at least 9-fold; still more preferably at least 13-fold; yet more preferably at least 60-fold, and even more preferably at least 110-fold compared to the relative salt activity of SEQ ID NO:1.
Clause 108: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, at least sixteen, or at least seventeen positions selected from the group consisting of the positions P51, S53, K55, T56, N58, A72, P73, A74, T77, S116, D117, Q120, A124, D128, Q129, D135, K196, G197, and D199, and exhibits an increased relative salt activity compared to the relative salt activity of SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably R, G, and L; when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, and A; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably R, K, G, A, V, L, I, P, F, S, M, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R; when the amino acid substitution comprises a substitution at amino acid position A72, then the amino acid substitution at position A72 is selected from the group consisting of the substitutions D, E, R, K, H, G, V, L, I, and P; preferably K, H and R; more preferably K; when the amino acid substitution comprises a substitution at amino acid position P73, then the amino acid substitution at position P73 is selected from the group consisting of the substitutions D, E, R, K, H, G, V, L, I, and A; preferably K, Hand R; more preferably K, and R; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, H, W, F, and Y; more preferably K, R, and F; when the amino acid substitution comprises a substitution at amino acid position S116, then the amino acid substitution at position S 116 is selected from the group consisting of the substitutions R, K, H, G, A, V, L, I, P, W, F, Y, and D; preferably K, H, and R; more preferably K; when the amino acid substitution comprises a substitution at amino acid position D1 17, then the amino acid substitution at position D117 is selected from the group consisting of the substitutions G, A, V, L, I, P, W, F, Y, C, M, S, N, T, Q, H, R, and K; preferably N, Y, A, K, H, R, and S; when the amino acid substitution comprises a substitution at amino acid position Q120, then the amino acid substitution at position Q120 is selected from the group consisting of the substitutions R, K, H, G, A, V, L, I, P, W, F, Y, and D; preferably K, H, and R; more preferably R; when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, Y, F, M, N, S, R, and K; when the amino acid substitution comprises a substitution at amino acid position Q129, then the amino acid substitution at position Q129 is selected from the group consisting of the substitutions R, K, H, G, A, V, L, I, P, W, F, Y, and D; preferably K, H, and R; more preferably R; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably G, N, R, and K; when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R; when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably S, Q, T, N, C, M, H, R, and K; more preferably N, S, and K.
Clause 109: The nuclease according to any of the preceding clauses, wherein the amino acid sequence is substituted compared to SEQ ID NO: 1 in at least two, at least three, at least four, at least five, at least six, at least seven, at least eight, at least nine, at least ten, at least eleven, at least twelve, at least thirteen, at least fourteen, at least fifteen, at least sixteen, or at least seventeen positions such that it comprises at least two, at least three, at least four, at least five, at least six, at least seven, at least eight, at least nine, at least ten, at least eleven, at least twelve, at least thirteen, at least fourteen, at least fifteen, at least sixteen, or at least seventeen substitutions selected from the group consisting of the substitutions P51R, P51G, P51L, S53K, S53A, K55R, T56R, T56K, T56M, T56L, T56F, T56I, T56V, T56A, T56G, T56P, T56S, T56D, N58R, N58K, A72K, P73K, P73R, A74R, A74K, T77F, T77K, T77R, S116K, D117A, D117S, D117K, D117N, D117Y, D117H, D117R, Q120R, A124K, A124R, D128R, D128K, D128Y, D128S, D128M, D128N, D128F, D128G, Q129R, D135R, D135G, D135N, D135K, K196R, G197K, D199N, D199K, and D199S, and exhibits an increased relative salt activity compared to the relative salt activity of SEQ ID NO:1.
Clause 110: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, or at least thirteen positions selected from the group consisting of the positions P51, S53, T56, N58, A72, A74, T77, D117, Q120, A124, D128, Q129, D135, K196, and G197, and exhibits an increased S_{high} activity compared to the S_{high} activity of SEQ ID NO:1.
Clause 111: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, or at least thirteen positions selected from the group consisting of the positions P51, S53, T56, N58, A72, A74, T77, D117, Q120, A124, D128, Q129, D135, K196, and G197, and exhibits an increased S_{high} activity of at least 1.01-fold; preferably at least 1.5-fold; more preferably at least 2-fold; still more preferably at least 5-fold; yet more preferably at least 12-fold, and even more preferably at least 19-fold compared to the S_{high} activity of SEQ ID NO:1.
Clause 112: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, or at least thirteen positions selected from the group consisting of the positions P51, S53, T56, N58, A72, A74, T77, D117, Q120, A124, D128, Q129, D135, K196, and G197, and exhibits an increased S_{high} activity compared to the S_{high} activity of SEQ ID NO:1 wherein, when the amino acid substitution comprises a substitution at amino acid position P51, then the amino acid substitution at position P51 is selected from the group consisting of the substitutions D, E, R, K, H, G, A, V, L, and I; preferably R, G, and L; when the amino acid substitution comprises a substitution at amino acid position S53, then the amino acid substitution at position S53 is selected from the group consisting of the substitutions R, K, H, G, A, V, L, I, P, W, F, Y, and D; preferably R, K, H, G, A, V, L, I and P; more preferably K, R, and H; still more preferably K; when the amino acid substitution comprises a substitution at amino acid position T56, then the amino acid substitution at position T56 is selected from the group consisting of the substitutions R, K, H, G, A, V, L, I, P, W, F, Y, N, S, Q, C, M, and D; preferably G, A, V, L, I, P, F, S, M, D, K, and R; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and H; still more preferably K, and R; when the amino acid substitution comprises a substitution at amino acid position A72, then the amino acid substitution at position A72 is selected from the group consisting of the substitutions D, E, R, K, H, G, V, L, I, and P; preferably K, H and R; more preferably K; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, and H; more preferably K, and R; when the amino acid substitution comprises a substitution at amino acid position D117, then the amino acid substitution at position D117 is selected from the group consisting of the substitutions G, A, V, L, I, P, W, F, Y, C, M, S, N, T, Q, H, R, and K; preferably Y, H, R, A, and S; when the amino acid substitution comprises a substitution at amino acid position Q120, then the amino acid substitution at position Q120 is selected from the group consisting of the substitutions R, K, H, G, A, V, L, I, P, W, F, Y, and D; preferably K, H, and R; more preferably R; when the amino acid substitution comprises a substitution at amino acid position A124, then the amino acid substitution at position A124 is selected from the group consisting of the substitutions D, E, R, K, H, G, V, L, I, and P; preferably K, H and R; more preferably K, and R; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R, and K; preferably G, Y, F, M, N, S, R, and K; when the amino acid substitution comprises a substitution at amino acid position Q129, then the amino acid substitution at position Q129 is selected from the group consisting of the substitutions R, K, H, G, A, V, L, I, P, W, F, Y, and D; preferably K, H, and R; more preferably R; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably G, A, V, L, I, P, C, M, N, Q, S, T, H, R, and K; more preferably A, G, N, R, and K; when the amino acid substitution comprises a substitution at amino acid position K196, then the amino acid substitution at position K196 is selected from the group consisting of the substitutions G, A, V, L, I, P, R, H, N, Q, S, T, C, and M; preferably H and R; more preferably R; or when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K. Clause 113: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, or at least thirteen positions such that it comprises at least two, at least three, at least four, at least five, at least six, at least seven, at least eight, at least nine, at least ten, at least eleven, at least twelve, or at least thirteen substitutions selected from the group consisting of the substitutions P51L, P51R, P51G, S53K, T56R, T56K, T56M, T56L, T56F, T56I, T56V, T56A, T56G, T56P, T56S, T56D, N58R, N58K, A72K, A74K, A74R, T77R, T77K, D117A, D117S, D117Y, D117H, D117R, Q120R, A124K, A124R, D128R, D128S, D128N, D128K, D128G, D128M, D128Y, D128F, Q129R, D135A, D135K, D135G, D135N, D135R, K196R, and G197K, and exhibits an increased S_{high} activity compared to the S_{high} activity of SEQ ID NO:1.
Clause 114: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, or at least sixteen positions selected from the group consisting of the positions P51, K55, T56, N58, P73, A74, T77, K84, D86, S116, D117, A124, D128, D135, G197, and D199, and exhibits an increased relative cold activity compared to the relative cold activity of SEQ ID NO:1.
Clause 115: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, or at least sixteen position selected from the group consisting of the positions P51, K55, T56, N58, P73, A74, T77, K84, D86, S116, D117, A124, D128, D135, G197, and D199, and exhibits an increased relative cold activity of at least 1.05-fold; preferably at least 1.5-fold; more preferably at least 2.0-fold, and still more preferably at least 3.0-fold compared to the relative cold activity of SEQ ID NO:1.
Clause 116: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, at least fifteen, or at least sixteen positions selected from the group consisting of the positions P51, K55, T56, N58, P73, A74, T77, K84, D86, S116, D117, A124, D128, D135, G197, and D199, and exhibits an increased relative cold activity compared to the relative cold activity of SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably D, R, G, and L; when the amino acid substitution comprises a substitution at amino acid position K55, then the amino acid substitution at position K55 is selected from the group consisting of the substitutions G, A, V, L, I, P, R, H, N, Q, S, T, C, and M; preferably H and R; more preferably R; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably K, R, P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably K, R, and V; when the amino acid substitution comprises a substitution at amino acid position P73, then the amino acid substitution at position P73 is selected from the group consisting of the substitutions D, E, R, K, H, G, V, L, I, and A; preferably K, H and R; more preferably K, and R; when the amino acid substitution comprises a substitution at amino acid position A74, then the amino acid substitution at position A74 is selected from the group consisting of the substitutions D, E, R, K, H, G, V, L, I, and P; preferably K, H, and R; more preferably K, and R; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, F, and L; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; when the amino acid substitution comprises a substitution at amino acid position S 116, then the amino acid substitution at position S 116 is selected from the group consisting of the substitutions R, K, H, G, A, V, L, I, P, W, F, Y, and D; preferably K, H, and R; more preferably K; when the amino acid substitution comprises a substitution at amino acid position D1 17, then the amino acid substitution at position D117 is selected from the group consisting of the substitutions G, A, V, L, I, P, W, F, Y, C, M, S, N, T, Q, H, R, and K; preferably A, G, V, L, I, P, C, M, N, T, Q, R, K, H, and S; more preferably H, R, and K; still more preferably K; when the amino acid substitution comprises a substitution at amino acid position A124, then the amino acid substitution at position A124 is selected from the group consisting of the substitutions D, E, R, K, H, G, V, L, I, and P; preferably K, H and R; more preferably K; when the amino acid substitution comprises a substitution at amino acid position D128, then the amino acid substitution at position D128 is selected from the group consisting of the substitutions G, A, V, L, I, P, W, F, Y, C, M, N, Q, S, T, H, R and K; preferably G, Y, F, M, N, S, R and K; when the amino acid substitution comprises a substitution at amino acid position D135, then the amino acid substitution at position D135 is selected from the group consisting of the substitutions G, A, V, L, I, P, W, F, Y, C, M, N, Q, S, T, H, R and K; preferably G, A, V, L, I, P, C, M, N, Q, S, T, H, R and K; more preferably G, N, R and K; when the amino acid substitution comprises a substitution at amino acid position G197, then the amino acid substitution at position G197 is selected from the group consisting of the substitutions D, E, R, K, H, A, V, L, I, and P; preferably K, H, and R; more preferably K; or when the amino acid substitution comprises a substitution at amino acid position D199, then the amino acid substitution at position D199 is selected from the group consisting of the substitutions G, A, V, L, I, P, W, F, Y, C, M, Q, T, N, S, H, R, and K; preferably S, Q, T, N, C, M, H, R, and K; more preferably N, S, and K.
Clause 117: The nuclease according to any of the preceding clauses, wherein the amino acid sequence is substituted compared to SEQ ID NO:1 in at least two, at least three, at least four, at least five, at least six, at least seven, at least eight, at least nine, at least ten, at least eleven, at least twelve, at least thirteen, at least fourteen, or at least fifteen positions such that it comprises at least two, at least three, at least four, at least five, at least six, at least seven, at least eight, at least nine, at least ten, at least eleven, at least twelve, at least thirteen, at least fourteen, at least fifteen, or at least sixteen substitutions selected from the group consisting of the substitutions P51L, P51D, P51G, P51R, K55R, T56D, T56R, T56P, T56K, N58V, N58K, N58R, P73R, P73K, A74K, A74R, T77K, T77L, T77R, T77F, K84G, D86S, S116K, D117K, A124K, D128Y, D128M, D128N, D128K, D128F, D128S, D128G, D128R, D135K, D135R, D135N, D135G, G197K, D199N, D199K, and D199S, and exhibits an increased relative cold activity compared to the relative cold activity of SEQ ID NO:1.
Clause 118: The nuclease according to any of the preceding clauses, wherein the amino acid sequence is substituted compared to SEQ ID NO:1 in at least two, at least three, at least four, at least five, or at least six positions selected from the group consisting of the positions P51, T56, N58, T77, K84, D86, S116, and D135, and exhibits an increased T_{low} activity in comparison to the T_{low} activity of SEQ ID NO:1.
Clause 119: The nuclease according to any of the preceding clauses, wherein the amino acid sequence is substituted compared to SEQ ID NO:1 in at least two, at least three, at least four, at least five, or at least six positions selected from the group consisting of the positions P51, T56, N58, T77, K84, D86, S116, and D135, and exhibits an increased T_{low} activity of at least 1.01-fold; preferably at least 1.1-fold; more preferably at least 1.3-fold, still more prefer-ably at least 1.5-fold, and yet more preferably at least 1.55-fold in comparison to the T_{low} activity of SEQ ID NO:1.
Clause 120: The nuclease according to any of the preceding clauses, wherein the amino acid sequence is substituted compared to SEQ ID NO:1 in at least two, at least three, at least four, at least five, or at least six positions selected from the group consisting of the positions P51, T56, N58, T77, K84, D86, S116, and D135, and exhibits an increased T_{low} activity in comparison to the T_{low} activity of SEQ ID NO:1, wherein, when the amino acid substitution comprises a substitution at amino acid position P51, then the amino acid substitution at position P51 is selected from the group consisting of the substitutions D, E, R, K, H, G, A, V, L, and I; preferably G, A, V, I, and L; more preferably L; when the amino acid substitution comprises a substitution at amino acid position T56, then the amino acid substitution at position T56 is selected from the group consisting of the substitutions R, K, H, G, A, V, L, I, P, W, F, Y, and D; preferably R, K, H, G, A, V, L, I, P, and D; more preferably G, A, V, L, I, P, and D; still more preferably P, and D; when the amino acid substitution comprises a substitution at amino acid position N58, then the amino acid substitution at position N58 is selected from the group consisting of the substitutions R, K, H, G, A, V, L, I, P, W, F, Y, and D; preferably R, K, H, G, A, V, L, I, and P; more preferably R, and V; when the amino acid substitution comprises a substitution at amino acid position T77, then the amino acid substitution at position T77 is selected from the group consisting of the substitutions R, K, H, G, A, V, L, I, P, W, F, Y, and D; preferably K, R, and H; more preferably R; when the amino acid substitution comprises a substitution at amino acid position K84, then the amino acid substitution at position K84 is selected from the group consisting of the substitutions G, A, V, L, I, P, R, H, N, Q, S, T, C, and M; preferably G, A, V, L, I, and P; more preferably G; when the amino acid substitution comprises a substitution at amino acid position D86, then the amino acid substitution at position D86 is selected from the group consisting of the substitutions G, A, V, L, I, P, C, M, S, Q, and T; preferably Q, T, and S; more preferably S; when the amino acid substitution comprises a substitution at amino acid position S116, then the amino acid substitution at position S116 is selected from the group consisting of the substitutions R, K, H, G, A, V, L, I, P, W, F, Y, and D; preferably K, H, and R; more preferably K; or when the amino acid substitution comprises a substitution at amino acid position D135, then the amino acid substitution at position D135 is selected from the group consisting of the substitutions G, A, V, L, I, P, W, F, Y, C, M, N, Q, S, T, H, R, and K; preferably C, M, N, Q, S, and T; more preferably N.
Clause 121: The nuclease according to any of the preceding clauses, wherein the amino acid sequence is substituted compared to SEQ ID NO:1 in at least two, at least three, at least four, at least five, or at least six positions such that it comprises at least two, at least three, at least four, at least five, or at least six substitutions selected from the group consisting of the substitutions P51L, T56D, T56P, N58V, N58R, T77R, K84G, D86S, S116K, and D135N, and exhibits an increased T_{low} activity in comparison to the T_{low} activity of SEQ ID NO:1.
Clause 122: The nuclease according to any of the preceding clauses, wherein the nuclease is capable of hydrolyzing of phosphodiesters of polynucleotide substrate into cleaved polynucleotides or oligonucleotides.
Clause 123: The nuclease according to any of the preceding clauses, wherein the nuclease catalyzes the hydrolysis of phosphodiesters of polynucleotide substrate into cleaved polynucleotides or oligonucleotides.
Clause 124: The nuclease according to any of the preceding clauses, wherein the polynucleotide substrates are selected from the group consisting of single strand RNA, single strand DNA, double strand DNA, double strand RNA and hybrid strand DNA/RNA; preferably double strand DNA.
Clause 125: The nuclease according to any of the preceding clauses, wherein the cleaved polynucleotide substrate is an oligonucleotide; preferably, wherein the oligonucleotide is at least 200 nucleotides long; more preferably at least 150 nucleotides long; still more preferably at least 100 nucleotides long; yet more preferably at least 50 nucleotides long; even more preferably at least 25 nucleotides long; most preferably at least 13 nucleotides long, utmost preferably at least 5 nucleotides long, and in particular 2 nucleotides long, optionally, wherein the cleaved polynucleotide is a mononucleotide.
Clause 126: The nuclease according to any of the preceding clauses, wherein the nuclease is contained in a liquid formulation.
Clause 127: The nuclease according to any of the preceding clauses, wherein the nuclease is contained in a liquid formulation containing buffer and glycerol, sucrose, or other stabilizing agents and/or benzoate or other preservative agents, each in a suitable range of percentages.
Clause 128: The nuclease according to any of the preceding clauses, wherein the nuclease is present in solid form or contained in a solid formulation; preferably a powder.
Clause 129: The nuclease according to any of the preceding clauses, wherein the nuclease is a powder or is present in such a powder obtained by lyophilization, spray drying or other techniques for drying known in the state of the art.
Clause 130: The nuclease according to any of the preceding clauses, wherein the nuclease is a crude cell lysate, or a crude cell supernatant, or a partially purified enzyme from any of the foregoing, or a purified enzyme from any of the foregoing.
Clause 131: The nuclease according to any of the preceding clauses, wherein the nuclease is immobilized on a carrier.
Clause 132: A method of hydrolyzing polynucleotide substrates; preferably being contained in a sample, comprising the steps of (a) providing a nuclease according to any of the preceding clauses; preferably according to any of clauses 18 to 126; (b) providing a composition containing a polynucleotide substrate; (c) contacting the polynucleotide substrate in the composition provided in step (b) with the nuclease provided in step (a) and allowing the nuclease to hydrolyze the polynucleotide substrate thereby obtaining a composition containing cleaved polynucleotide.
Clause 133: The method according to clause 132, wherein the nuclease is hydrolyzing the polynucleotide substrate into cleaved polynucleotides or oligonucleotides.
Clause 134: The method according to clause 132 or 133, wherein step (c) is carried out at a temperature, between 4°C and 90°C; preferably between 10°C and 80°C; more preferably between 20°C and 65°C; still more preferably between 25°C and 65°; yet more preferably between 30°C and 50°C; even more preferably between 34°C and 42°C; most preferably at 37°C; or between 20°C and 90°C; preferably between 23°C and 80°C; more preferably between 25°C and 65°C; still more preferably between 30°C and 65°C; yet more preferably be-tween 37°C and 65°C; even more preferably 50°C and 65°C; most preferably at 51°C; or between 4°C and 65°C; preferably between 4°C and 38°C; more preferably be-tween 4°C and 25°C; still more preferably between 4°C and 20°C; yet more preferably between 4°C and 15°C; even more preferably between 4°C and 10°C; most preferably between 4°C and 6°C, and utmost preferably at 5°C.
Clause 135: The method according to any of clauses 132 to 134, wherein step (c) is carried out at a temperature between 20°C and 42°C; preferably between 25°C and 42°C; more preferably between 28°C and 40°C, and still more preferably 37°C.
Clause 136: The method according to any of clauses 132 to 135, wherein step (c) is carried out at a temperature between 30°C and 65°C; preferably between 30°C and 55°C; more preferably between 40°C and 52°C, and still more preferably 51°C.
Clause 137: The method according to any of clauses 132 to 136, wherein step (c) is carried out at a temperature between 4°C and 25°C; preferably between 4°C and 15°C; more preferably between 4°C and 10°C; still more preferably between 4°C and 6°C, and yet more preferably at 5°C.
Clause 138: The method according to any of clauses 132 to 137, wherein step (c) is carried out in the presence of salts, wherein the salt is NaCl or KCl; preferably NaCl.
Clause 139: The method according to any of clauses 132 to 138, wherein step (c) is carried out at a concentration of salt from 0.00M to 1.00M, or from 0.01M to 1.00M; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.
Clause 140: The method according to any of clauses 132 to 137, wherein step (c) is carried out in the absence of salts.
Clause 141: The method according to any of clauses 132 to 140 comprising the additional step of (d) inactivating the nuclease after step (c) thereby obtaining a composition containing cleaved polynucleotides and an inactivated nuclease.
Clause 142: The method according to clause 141, wherein the inactivation of the nuclease in step (d) is carried out (a) by heat inactivation; and/or (b) by decreasing the pH value of the composition to lower than pH 5; and/or (c) by increasing the pH value of the composition to higher than pH 11; and/or (d) by adding chelating agents to the composition; and/or (e) by adding oxidizing agents to the composition; and/or (f) by adding protein denaturant agents; preferably detergents; and/or (g) by adding enzymes hydrolyzing proteins; preferably a protease.
Clause 143: The method according to any of clauses 132 to 142, comprising the additional step of (e) separating the nuclease from the composition containing cleaved polynucleotide obtained in step (c) and optionally inactivated nuclease obtained in step (d).
Clause 144: The method according to clause 143, wherein step (e) is carried out by use of a chromatographic method, and/or ultrafiltration, and/or tangential flow filtration.
Clause 145: Use of a nuclease according to any of clauses 1 to 131; preferably according to any of clauses 18 to 126; for hydrolyzing polynucleotide substrates; preferably being contained in a composition.
Clause 146: The use according to clause 145, wherein the polynucleotide substrates in the composition are derived from a cell; preferably a cell selected from - a cell from the domain Eukaryota; preferably an immortalized cell line, or a stem cell, or a tissue cell, or a fungal cell; more preferably from the specie Pichia pastoris, or from a specie from the genus Aspergillus, or from a specie from the genus Saccharomyces; and/or - a cell from the domain Bacteria; preferably a bacterial cell; more preferably from the specie Escherichia coli, or from a specie from the genus Bacillus; and/or - a cell from the domain Archea.
Clause 147: The use according to clause 145 or 146, wherein the polynucleotide substrate is released into the composition from a cell by cell disruption, secretion, lysis, or cell burst.
Clause 148: The use according to any of clauses 145 to 147, wherein the composition is derived from lysed cells from a cell fermentation process.
Clause 149: The use according to any of clauses 145 to 148, wherein the composition is an intermediate product or product from a production process for biopharmaceuticals, pharmaceutical compositions, medicaments, vaccines, or viral vectors.
Clause 150: The use according to clause 149, wherein the viral vector is selected from the group consisting of adeno associated virus, retrovirus, lentivirus, adenovirus, retrovirus, plant virus, bacteriophage, and hybrid vector virus.
Clause 151: The use according to clause 149, wherein the composition is an intermediate product or product during preparation of patient tissue samples for use in vitro diagnostics.
Clause 152: The use according to any of clauses 149 to 151, wherein the biopharmaceuticals, pharmaceutical compositions, medicaments, vaccines, or viral vectors are used in gene therapy, and/or tissue therapy, and/or in vitro diagnostic.
Clause 153: The use according to any of clauses 145 to 152, for the reduction of viscosity in cell lysates. Clause 154: The use according to any of clauses 145 to 153, for sample preparation in electrophoresis and/or chromatographic methods.
Clause 155: The use according to any of clauses 145 to 154, which is carried out at a temperature (a) between 4°C and 90°C; preferably between 10°C and 80°C; more preferably be-tween 20°C and 65°C; still more preferably between 25°C and 65°; yet more preferably between 30°C and 50°C; even more preferably between 34°C and 42°C; most preferably at 37°C; or (b) between 20°C and 90°C; preferably between 23°C and 80°C; more preferably between 25°C and 65°C; still more preferably between 30°C and 65°C; yet more preferably between 37°C and 65°C; even more preferably 50°C and 65°C; most preferably at 51°C; or (c) between 4°C and 65°C; preferably between 4°C and 38°C; more preferably be-tween 4°C and 25°C; still more preferably between 4°C and 20°C; yet more preferably between 4°C and 15°C; even more preferably between 4°C and 10°C; most preferably between 4°C and 6°C, and utmost preferably at 5°C.
Clause 156: The use according to any of clauses 145 to 155, which is carried out at a concentration of salt from 0.00M to 1.00M, or from 0.01M to LOOM; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.
Clause 157: The use according to any of clauses 145 to 155, which is carried out in the absence of salts. Clause 158: A kit for cleaving a polynucleotide, the kit comprising - the nuclease according to any of clauses 1 to 131; preferably according to any of clauses 18 to 126, - a buffer for hydrolyzing polynucleotide substrates, and - optionally, an agent and/or device for inactivation and/or removal of the nuclease; preferably wherein the kit is for use in the method according to any of clauses 132 to 144 or in the use according to any of clauses 145 to 156.

### Examples

The following examples further illustrate the invention but are not to be construed as limiting its scope.

### Example 1: General methods

Design and expression of variants: The gene coding for the *Serratia marcescens* nuclease with protein sequence SEQ ID NO:1 without its natural signal peptide sequence was codon-optimized for expression in *Bacillus subtilis* and was cloned into the *Bacillus* expression plasmid vector pLE2E02. For expression and secretion of the nuclease, the gene was fused onto the sequence encoding the secretion sequence of AmyE from *Bacillus subtilis* for proper secretion of the endocuclease into the cultivation medium. Plasmid vector pLE2E02 contains a maltose-inducible promoter from *Bacillus subtilis* for induction of recombinant nuclease expression, a chloramphenicol resistance gene for selection as well as functional elements for replication in the *Bacillus* host cells. The resulting plasmid was used for transformation of *Bacillus subtilis* strain LE2A106 (a derivative of *Bacillus subtilis* DSM No. 402).

Variants of the *Serratia marcescens* nuclease were created by standard site directed mutagenesis technologies as known to the person skilled in the art and recombinantly expressed. Exemplary variants of SEQ ID NO: 1 are listed in Table 1.

Recombinant *Serratia marcescens* nuclease enzymes were routinely expressed in 96-deep-well plates in LB medium (10 g/l trypton, 5 g/l yeast extract, 10 g/l NaCl, pH 7.0) with 10 µg/ml chloramphenicol and 1 mM MgSO4. For the cultivation of precultures the medium was supplemented with 2 % glucose, and 0.4 ml medium per well were inoculated with a single clone and incubated overnight at 37 °C and 1000 rpm for agitation. For expression cultures the medium was supplemented with 0.05 % glucose and 2 % maltose and 1 ml of medium per well was inoculated with 10 µl of a fresh overnight preculture. The cultures were incubated for 19 hours with shaking at 30 °C for expression and secretion of nuclease into the medium. The supernatants containing secreted nuclease were separated from cells by centrifugation (3300xg; 20 min; 4 °C) and the resulting enzyme samples were used for further analysis.

### Example 2: Nuclease Activity Assay

The Nuclease Activity Assay bases upon the release of short, acid soluble oligonucleotides (cleaved polynucleotides or oligonucleotides) from DNA substrate (polynucleotide substrate) which leads to an increase in absorbance at 260 nm.

Enzyme samples to be tested were usually separated from cells, cell debris or any denatured protein or particles that may result from any earlier step (like expression from host cells described in Example 1 or the incubation at T_{stability} described in Example 3) by centrifugation (3300xg; ~20 min; 4 °C) prior to testing.

In a first step the enzyme samples were diluted in enzyme dilution buffer (50 mM Tris-HCl pH 8.0, 5 mM MgCl₂, 0.1 mg/ml BSA) to appropriate dilutions by the dilution factor (D), which factor (D) is chosen as described in this Example 2 after step 5 below. In parallel, background control samples were set up containing only enzyme dilution buffer without any nuclease.

Optionally, a certain salt concentration of the substrate buffer may be adjusted, for example when testing enzymatic activity at a certain salt concentration like a S_{high} activity, or a S_{low} activity as described in Example 4.

The diluted nuclease samples, control samples, and substrate buffer (50 mM Tris-HCl pH 8.0, 5 mM MgCl₂, 0.1 mg/ml BSA, 1 mg/ml DNA sodium salt from salmon testes (Sigma-Aldrich, Cat. No. D1626)) were separately preheated to 30 °C in 96-well microtiter plates (MTP) in a second step.

In a third step, the reaction was setup by mixing 20 µl of nuclease samples, or control sample respectively, with 80 µl of substrate buffer in MTPs. Reactions were incubated for 60 minutes at 30 °C. The final concentration of the polynucleotide substrate in the reaction is 0.8 mg/ml DNA sodium salt from salmon testes. Then, the reactions were stopped by addition of 1 volume (100 µl) 4 % perchloric acid to each well.

Optionally, the reactions may be incubated at a different reaction temperature in preheating or the reaction, for example when testing enzymatic activity at low or at high temperatures, like the T_{low} activity, or the T_{high} activity as described in Example 5.

In a fourth step, the stopped reaction assay samples from step 3 were incubated on ice for 45 to 60 minutes to assure a complete precipitation of the unconverted DNA, and the precipitated DNA was then removed by centrifugation (3800xg; 30 min; 4 °C).

In a fifth step, 100 µl of the supernatant were transferred to flat bottom UV transparent MTPs and absorbance was measured at 260 nm. The absorbance signal of control wells (reactions without nuclease) was subtracted from nuclease containing wells to calculate the normalized absorbance ("A260").

The assay was linear to nuclease activity in the range of A260-values from 0-1.9. For comparative detection of samples, the amount of nuclease to be provided in an enzyme sample in the nuclease activity assay requires to be adjusted in such a way that the A260-values were within the linear range of the assay. This is achieved by the dilution of the enzyme sample with a suitable dilution factor (D) in step 1. The detected A260 values measured in step 5 were multiplied by the dilution factor (D) to calculate enzyme activities corrected to the enzyme amount tested.

### Example 3: Detection of thermal stability

Thermal stability is defined as the property of the nuclease enzyme to retain enzymatic activity upon incubation at elevated temperatures for a given time. Thermal stability is expressed as the residual activity of the nuclease after incubation for 15 minutes at 51°C compared to its activity after incubation at 23 °C. SEQ ID NO:1 shows a residual activity of around 10-20% after incubation at 51 °C.

To determine the thermal stability of the *S. marcescens* wild-type (SEQ ID NO:1) and the variants, the corresponding enzyme samples were diluted in enzyme dilution buffer (50 mM Tris-HCl, pH 8.0, 5 mM MgCl2, 0.1 mg/ml BSA) at a suitable dilution factor (D). For each enzyme sample, 70 µl aliquot of the enzyme dilution was incubated for 15 minutes at 51 °C (T_{stabiliy}) in a first vessel, and a second 70 µl aliquot was separately incubated at 23 °C (T_{23°C}) in a second vessel for reference activity. After incubation at the corresponding temperatures, the samples were incubated for 15 minutes on ice for regeneration and denatured protein was separated by centrifugation (3300xg; 15 min; 4 °C). Each supernatant was transferred into a new vessel and the nuclease activity in each supernatant was determined using a Nuclease Activity Assay as described in Example 2. The enzymatic activity in the Nuclease Activity Assay was measured without NaCl, or at a salt concentration of 150 mM NaCl, respectively, and in each case at a temperature of 30 °C.

For calculating the residual activity of each individual enzyme sample, the ratio of the absorbance value A260 of the sample incubated at T_{stabiliy} and the absorbance value A260 of the sample incubated at T_{23°C} is calculated (A260 [T_{stability}]: A260 [T_{23°C}]).

For calculating the thermal stability of an enzyme sample in comparison to SEQ ID NO: 1, the ratio of the residual activity of such enzyme variant and the residual activity of SEQ ID NO: 1 is calculated. Variants showing an increased residual activity than SEQ ID NO: 1 possess a higher thermal stability compared to the thermal stability of SEQ ID NO: 1 The increased thermal stability of a variant is expressed as x-fold increase in comparison to SEQ ID NO:1. The increased thermal stability of variants in comparison to SEQ ID NO: 1 are listed in Table 2.

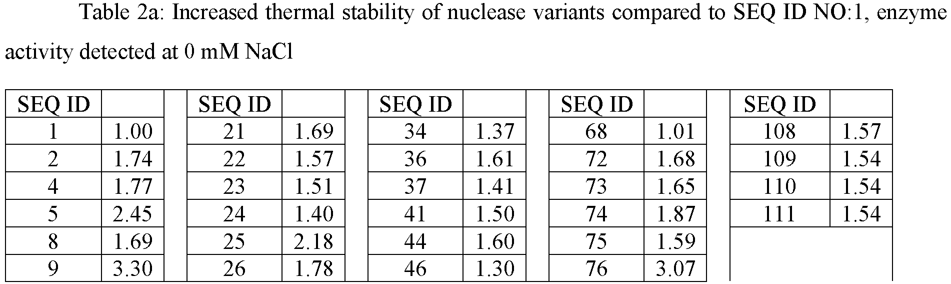

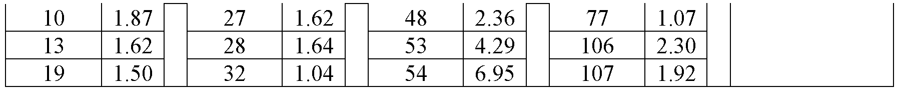

**Table 2b: Increased thermal stability of nuclease variants compared to SEQ ID NO: 1, enzyme activity detected at 150 mM NaCl**

| SEQ ID | | | SEQ ID | | | SEQ ID | |
|---|---|---|---|---|---|---|---|
| 1 | 1.00 | | 102 | 2.93 | | 129 | 2.65 |
| 81 | 1.66 | | 122 | 1.32 | | 130 | 3.15 |
| 99 | 1.08 | | 123 | 2.09 | | 131 | 2.43 |
| 100 | 1.53 | | 124 | 3.00 | | 137 | 1.38 |
| 101 | 2.93 | | 128 | 1.56 | | | |

The data of Tables 2a and 2b show that the nucleases according to the invention have improved thermal stability compared to SEQ ID NO:1. Improved thermal stability especially relies upon substitutions in positions P51, T56, T77, and A124, especially upon substitution of a negatively charged or aliphatic nonpolar amino acid in position P51 or T56, of a positively charged, an aromatic, or an aliphatic nonpolar amino acid in position T77, or of a positively charged amino acid in position A124, and in particular upon a substitution selected from the group consisting ofP51L, P51D, P51G, T56P, T56D, T77L, T77K, T77F, T77R, and A124K, either alone or in combination with each other.

Improved thermal stability also relies upon substitutions in position K55, N58, A74, N80, K84, D86, S116, D117, D128, D128, D128, D135, D135, D191, R204, E239, especially upon substitution of a positively charged amino acid in positions K55, A74, or N80, an aliphatic nonpolar amino acid in positions N58, K84, D135, D191, or E239, an aromatic amino acid in positions D128, or D128, or an aliphatic polar non-charged amino acid in positions D86, D117, D128, D135, or R204, and in particular upon a substitution selected from the group consisting of K55R, N58V, A74R, N80H, K84G, D86S, S116K, D117N, D128M, D128Y, D128F, D135N, D135G, D191P, R204Q, E239A, either alone or in combination with each other.

### Example 4: Detection of salt activity and relative salt activity

To determine the salt activity of the *S. marcescens* wild-type (SEQ ID NO:1) and the variants, the corresponding enzyme samples were analyzed as described in the Nuclease Activity Assay (Example 2). For adjusting a certain salt concentration S_{low} (for S_{low} activity) or S_{high} (for S_{high} activity) during the reaction of step 3, the salt concentration of the substrate buffer in step 2 is changed in such way, that a final concentration S_{low} or S_{high}, respectively, is obtained upon mixture with the enzyme sample or control sample, respectively.

Specifically, each enzyme sample was incubated at a salt concentration S_{low} of 0 mM NaCl (without NaCl) and a salt concentration S_{high} at 500 mM NaCl (for a final concentration of 500 mM NaCl after mixing with samples the substrate buffer was adjusted to 625 mM NaCl). Enzymatic activity at each salt concentration was measured as described in the Nuclease Activity Assay. The A260 value at S_{low} corresponds to the S_{low} activity, the A260 value at S_{high} corresponds to the S_{high} activity. The relative salt activity corresponds to the ratio of the S_{high} activity and the S_{low} activity (A260 [S_{high}]: A260 [S_{low}], or S_{high} activity : S_{low} activity, respectively).

Table 3 shows the relative salt activity (S_{high} activity : S_{low} activity) of variants of SEQ ID NO: 1 in comparison to the relative salt activity of SEQ ID NO: 1 at a salt concentration S_{high} of 500 mM NaCl and a salt concentration S_{low} without salt. Table 4 shows the S_{high} activity of variants of SEQ ID NO: 1 in comparison to the S_{high} activity of SEQ ID NO: 1 at 500 mM NaCl.

**Table 3: Relative salt activity of variants compared to relative salt activity of SEQ ID NO: 1.**

| SEQ ID | | | SEQ ID | | | SEQ ID | | | SEQ ID | | | SEQ ID | |
|---|---|---|---|---|---|---|---|---|---|---|---|---|---|
| 1 | 1.00 | | 31 | 1.83 | | 57 | 24.34 | | 83 | 2.07 | | 120 | 2.55 |
| 2 | 2.37 | | 32 | 1.71 | | 58 | 34.53 | | 84 | 2.88 | | 121 | 8.60 |
| 3 | 2.47 | | 33 | 4.47 | | 59 | 20.63 | | 85 | 2.43 | | 122 | 3.79 |
| 4 | 2.28 | | 34 | 2.37 | | 60 | 22.97 | | 86 | 1.70 | | 123 | 3.93 |
| 6 | 1.56 | | 35 | 6.94 | | 61 | 26.27 | | 87 | 2.56 | | 124 | 2.95 |
| 7 | 1.54 | | 36 | 3.46 | | 62 | 25.36 | | 88 | 1.59 | | 125 | 7.92 |
| 8 | 1.31 | | 37 | 4.35 | | 63 | 26.22 | | 89 | 1.46 | | 126 | 1.81 |
| 11 | 4.57 | | 38 | 9.08 | | 64 | 12.48 | | 90 | 1.31 | | 127 | 11.12 |
| 12 | 2.62 | | 39 | 3.66 | | 65 | 13.64 | | 91 | 4.97 | | 128 | 6.71 |
| 14 | 2.61 | | 40 | 3.37 | | 66 | 11.05 | | 92 | 1.19 | | 129 | 1.48 |
| 15 | 1.38 | | 41 | 3.26 | | 67 | 9.37 | | 93 | 1.06 | | 131 | 9.23 |
| 16 | 1.84 | | 42 | 2.87 | | 68 | 36.35 | | 94 | 1.12 | | 132 | 14.73 |
| 17 | 5.08 | | 43 | 8.73 | | 69 | 34.38 | | 95 | 1.04 | | 133 | 18.64 |
| 18 | 3.95 | | 44 | 2.22 | | 70 | 116.31 | | 96 | 1.11 | | 134 | 9.91 |
| 19 | 2.61 | | 45 | 5.20 | | 71 | 62.50 | | 97 | 1.09 | | 135 | 21.09 |
| 20 | 2.55 | | 46 | 2.23 | | 73 | 4.90 | | 107 | 1.12 | | 136 | 2.91 |
| 22 | 2.67 | | 47 | 4.53 | | 74 | 2.42 | | 113 | 1.15 | | 137 | 5.19 |
| 23 | 1.15 | | 49 | 2.51 | | 75 | 1.48 | | 114 | 8.08 | | 138 | 6.48 |
| 24 | 2.47 | | 50 | 1.36 | | 78 | 4.64 | | 115 | 1.17 | | 139 | 28.84 |
| 27 | 1.04 | | 51 | 1.82 | | 79 | 4.79 | | 116 | 2.50 | | 140 | 16.29 |
| 28 | 1.11 | | 52 | 1.44 | | 80 | 2.26 | | 117 | 1.94 | | 141 | 14.31 |
| 29 | 3.68 | | 55 | 31.92 | | 81 | 2.16 | | 118 | 1.24 | | 142 | 15.68 |
| 30 | 1.99 | | 56 | 35.93 | | 82 | 3.29 | | 119 | 1.17 | | 143 | 21.70 |

The data of Table 3 show that the nucleases according to the invention have improved relative salt activity compared to the relative salt activity of SEQ ID NO: 1. Improved relative salt activity especially relies upon substitutions in positions P51, T77, and A124, especially upon substitution of an aliphatic nonpolar amino acid in position P51 (SEQ ID NO: 2 or 4), or of a positively charged amino acid in position T77 (SEQ ID NO: 22 or 24), or of a positively charged amino acid in position A124 (SEQ ID NO: 34, 35). When all substitutions according to the invention are realized, even synergistic effects may be observed, for example in SEQ ID NO: 55, 57, 58, 59, 61, 64, 66, 69, 132, 133, 139, 140, or 143.

The data of Table 3 show that the nucleases according to the invention have improved relative salt activity compared to the relative salt activity of SEQ ID NO:1. Improved relative salt activity especially relies upon substitutions in positions P51, T56, T77, and A124, especially upon substitution of a positively charged or aliphatic nonpolar amino acid in position P51, or of a positively charged amino acid in position T56, or of a an aromatic, or a positively charged amino acid in position T77, or of a positively charged amino acid in position A124, and in particular upon a substitution selected from the group consisting ofP51L, P51R, P51G, T56R, T56K, T77K, T77F, T77R, A124R and A124K, either alone or in combination with each other.

Improved relative salt activity compared to the relative salt activity of SEQ ID NO:1 also relies upon substitutions in position K55, N58, A74, N80, K84, D86, S116, D117, D128, D128, D128, D135, D135, D191, R204, E239, especially upon substitution of a positively charged amino acid in positions S53, K55, N58, N58, A72, P73, P73, A74, A74, S116, D117, D117, D128, D128, D135, D135, G197, or D199, an aliphatic nonpolar amino acid in positions S53, D117, D128, or D135, an aromatic amino acid in positions D128, or D128, or an aliphatic polar non-charged amino acid in positions D86, D117, D117, D128, D128, D128, D135, D199, or D199, and in particular upon a substitution selected from the group consisting of S53, S53, K55, N58, N58, A72, P73, P73, A74, A74, D86, S116, D117, D117, D117, D117, D117, D128, D128, D128, D128, D128, D128, D128, D128, D135, D135, D135, D135, G197, D199, D199, D199, either alone or in combination with each other.

Furthermore, substitutions in positions Q120 and Q129, especially upon substitution with a negatively charged amino acid, and in particular the substitution Q120R or Q129R exhibit further beneficial effects in respect to relative salt activity of nuclease variants compared to the relative salt activity of SEQ ID NO:1.

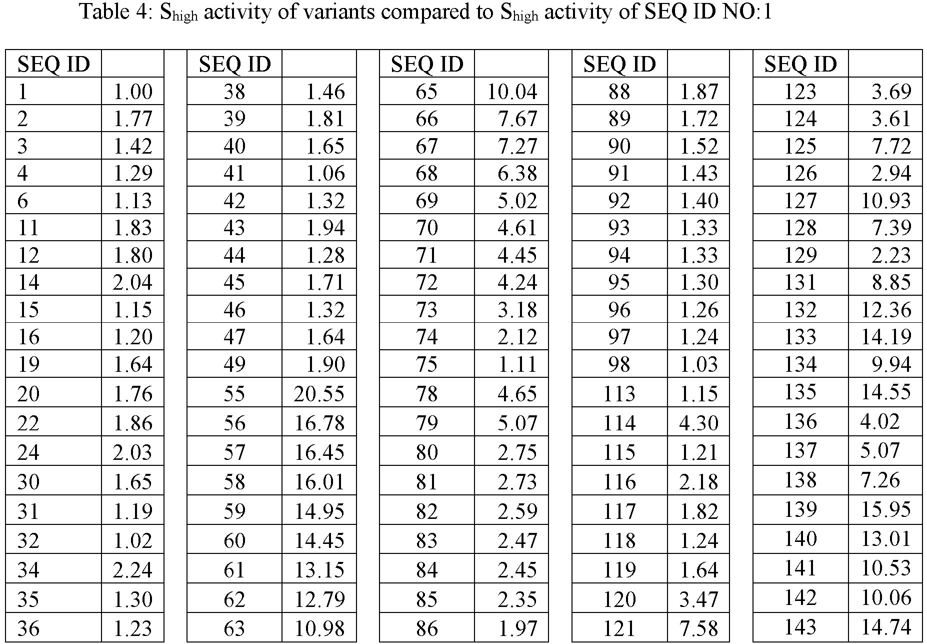

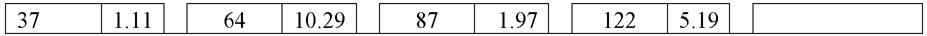

The data of Table 4 show that the nucleases according to the invention have improved relative salt activity compared to the relative salt activity of SEQ ID NO: 1. Improved relative salt activity especially relies upon substitutions in positions P51, T77, and A124, especially upon substitution of an aliphatic nonpolar amino acid in position P51 (SEQ ID NO: 2 or 4), or of a positively charged amino acid in position T77 (SEQ ID NO: 22 or 24), or of a positively charged amino acid in position A124 (SEQ ID NO: 34, 35). When all substitutions according to the invention are realized, even synergistic effects may be observed, for example in SEQ ID NO: 55, 57, 58, 59, 61, 64, 132, 133, 139, 140, or 143.

The data of Table 4 show that the nucleases according to the invention have improved S_{high} activity compared to the S_{high} activity of SEQ ID NO:1. Improved S_{high} activity especially relies upon substitutions in positions P51, T56, T77, and A124, especially upon substitution of a positively charged or aliphatic nonpolar amino acid in position P51, of a positively charged amino acid in position T56, of a positively charged amino acid in position T77, or of a positively charged amino acid in position A124, and in particular upon a substitution selected from the group consisting of P51L, P51R, P51G, T56R, T56K, T77K, T77R and A124K, either alone or in combination with each other.

Improved S_{high} activity also relies upon substitutions in position S53, N58, N58, A72, A74, A74, D128, D128, D128, D135, and D135, especially upon substitution of a positively charged amino acid in positions S53, N58, N58, A72, A74, A74, D128, or D135, an aromatic amino acid in position D128, or an aliphatic polar non-charged amino acid in position D128, and in particular upon a substitution selected from the group consisting of S53K, N58R, N58K, A72K, A74R, A74K, D128M, D128Y, D128R, D135N, and D135R, either alone or in combination with each other.

Furthermore, substitutions in positions Q120 and Q129, especially upon substitution with a negatively charged amino acid, and in particular the substitution Q120R or Q129R exhibit further beneficial effects in respect to improved S_{high} activity of nuclease variants.

### Example 5: Detection of cold activity and relative cold activity assay

To determine the cold activity of the *S. marcescens* wild-type (SEQ ID NO: 1) and the variants, the corresponding enzyme samples were analyzed as described in the Nuclease Activity Assay (Example 2), with the exception that the temperature is adjusted to either T_{low} (for T_{low} activity) or T_{high} (for T_{high} activity) during the preincubation (step 2) and the reaction of step 3, respectively.

Specifically, each enzyme sample was incubated at T_{low} of 5 °C or T_{high} of 30 °C. Enzymatic activity at each temperature was measured as described in the Nuclease Activity Assay. The A260 value at T_{low} corresponds to the T_{low} activity, the A260 value at T_{high} corresponds to the T_{high} activity. The relative cold activity corresponds to the ratio of the T_{low} activity and the T_{high} activity (A260 [T_{low}]: A260 [T_{high}], or T_{low} activity : T_{high} activity, respectively).

Table 5 shows the relative cold activity of variants of SEQ ID NO: 1 in comparison to the relative cold activity of SEQ ID NO: 1, each at a temperature T_{low} of 5 °C and T_{high} of 30 °C. Table 6 shows the ratio of T_{low} activity of variants of SEQ ID NO: 1 in comparison to the T_{low} activity of SEQ ID NO: 1 at 5 °C each.

**Table 5a: Ratio of relative cold activity of variants compared to relative cold activity of SEQ ID NO: 1; enzyme activity is detected at 0 mM NaCl**

| SEQ ID | | | SEQ ID | | | SEQ ID | | | SEQ ID | | | SEQ ID | |
|---|---|---|---|---|---|---|---|---|---|---|---|---|---|
| 1 | 1.00 | | 15 | 1.15 | | 29 | 1.12 | | 47 | 1.85 | | 66 | 1.46 |
| 2 | 1.70 | | 17 | 3.30 | | 36 | 1.51 | | 49 | 1.19 | | 73 | 2.18 |
| 3 | 1.46 | | 18 | 1.48 | | 37 | 2.00 | | 50 | 1.06 | | 74 | 1.76 |
| 4 | 1.53 | | 19 | 1.14 | | 38 | 1.38 | | 51 | 1.20 | | 75 | 2.06 |
| 5 | 1.76 | | 20 | 1.28 | | 39 | 1.12 | | 52 | 1.08 | | 76 | 1.49 |
| 8 | 1.28 | | 21 | 1.17 | | 40 | 1.44 | | 57 | 1.13 | | 77 | 1.64 |
| 9 | 1.42 | | 22 | 1.19 | | 41 | 1.18 | | 60 | 1.06 | | 112 | 1.04 |
| 10 | 1.69 | | 23 | 1.08 | | 42 | 1.10 | | 61 | 1.25 | | 116 | 1.18 |
| 11 | 1.60 | | 24 | 1.10 | | 43 | 1.16 | | 62 | 1.02 | | | |
| 12 | 1.38 | | 26 | 1.47 | | 44 | 1.66 | | 63 | 1.27 | | | |
| 13 | 1.24 | | 27 | 1.13 | | 45 | 2.42 | | 64 | 1.15 | | | |
| 14 | 1.11 | | 28 | 1.20 | | 46 | 1.27 | | 65 | 1.30 | | | |

**Table 5b: Ratio of relative cold activity of variants compared to relative cold activity of SEQ ID NO: 1, enzyme activity detected at 150 mM NaCl**

| SEQ ID | | | SEQ ID | | | SEQ ID | | | SEQ ID | | | SEQ ID | |
|---|---|---|---|---|---|---|---|---|---|---|---|---|---|
| 1 | 1.00 | | 89 | 1.11 | | 99 | 1.17 | | 128 | 1.12 | | 137 | 1.50 |
| 79 | 1.37 | | 90 | 1.07 | | 102 | 1.05 | | 129 | 1.17 | | 138 | 1.39 |
| 80 | 1.14 | | 91 | 1.24 | | 120 | 1.18 | | 130 | 1.30 | | 139 | 1.96 |
| 81 | 1.25 | | 92 | 1.12 | | 121 | 1.89 | | 131 | 1.63 | | 140 | 1.18 |
| 82 | 1.41 | | 93 | 1.06 | | 122 | 1.59 | | 132 | 1.30 | | 141 | 1.15 |
| 83 | 1.13 | | 94 | 1.10 | | 123 | 1.71 | | 133 | 1.79 | | 142 | 1.03 |
| 84 | 1.42 | | 96 | 1.13 | | 124 | 1.08 | | 134 | 1.66 | | 143 | 1.26 |
| 86 | 1.25 | | 97 | 1.04 | | 126 | 1.26 | | 135 | 1.54 | | 144 | 1.03 |
| 88 | 1.18 | | 98 | 1.23 | | 127 | 1.67 | | 136 | 1.19 | | | |

The data of Tables 5a/b show that the nucleases according to the invention have relative cold activity compared to the relative cold activity of SEQ ID NO:1. Improved relative cold activity especially relies upon substitutions in positions P51, T56, and T77, especially upon substitution of a positively charged amino acid, of a negatively charged amino acid, or of aliphatic nonpolar amino acid in position P51 or T56, or upon substitution of a an aromatic amino acid, an aliphatic nonpolar amino acid, or a positively charged amino acid in position T77, and in particular upon a substitution selected from the group consisting ofP51L, P51R, P51G, P51D, T56P, T56D, T56R, T56K, T77L, T77K, T77F, and T77R, either alone or in combination with each other.

Improved relative cold activity also relies upon substitutions in position K55, N58, N58, N58, P73, P73, A74, A74, K84, D86, S116, D117, D128, D128, D128, D128, D128, D128, D128, D128, D135, D135, D135, D135, G197, D199, D199, and D199, especially upon substitution of a positively charged amino acid in positions K55, N58, N58, P73, P73, A74, A74, S116, D117, D128, D128, D135, D135, G197, or D199K, an aliphatic nonpolar amino acid in positions N58, K84, D128, or D135, an aromatic amino acid in positions D128, or D128, or an aliphatic polar non-charged amino acid in positions D86, D128, D128, D128, D135, D199, or D199, and in particular upon a substitution selected from the group consisting of K55R, N58V, N58R, N58K, P73K, P73R, A74R, A74K, K84G, D86S, S116K, D117K, D128M, D128Y, D128K, D128S, D128N, D128F, D128G, D128R, D135N, D135K, D135G, D135R, G197K, D199N, D199K, and D199S, either alone or in combination with each other.

**Table 6a: Ratio of T_{low} activity of variants compared to T_{low} activity of SEQ ID NO:1; enzyme activity is detected at 0 mM NaCl**

| SEQ ID | | | SEQ ID | | | SEQ ID | | | SEQ ID | |
|---|---|---|---|---|---|---|---|---|---|---|
| 1 | 1.00 | | 13 | 1.10 | | 66 | 1.01 | | 76 | 1.43 |
| 2 | 1.28 | | 26 | 1.11 | | 73 | 1.42 | | 77 | 1.59 |
| 9 | 1.01 | | 27 | 1.01 | | 74 | 1.55 | | 116 | 1.03 |
| 10 | 1.08 | | 28 | 1.01 | | 75 | 1.55 | | 119 | 1.39 |

**Table 6b: Ratio of T_{low} activity of variants compared to T_{low} activity of SEQ ID NO: 1, enzyme activity detected at 150 mM NaCl**

| SEQ ID | | | SEQ ID | | | SEQ ID | | | SEQ ID | | | SEQ ID | |
|---|---|---|---|---|---|---|---|---|---|---|---|---|---|
| 1 | 1.00 | | 87 | 1.58 | | 97 | 1.34 | | 127 | 1.82 | | 137 | 1.86 |
| 78 | 1.48 | | 88 | 1.59 | | 98 | 1.30 | | 128 | 1.93 | | 138 | 2.39 |
| 79 | 2.18 | | 89 | 1.56 | | 99 | 1.21 | | 129 | 1.81 | | 139 | 1.54 |
| 80 | 1.77 | | 90 | 1.46 | | 120 | 2.11 | | 130 | 1.87 | | 140 | 2.05 |
| 81 | 1.93 | | 91 | 1.26 | | 121 | 2.02 | | 131 | 1.72 | | 141 | 1.95 |
| 82 | 1.35 | | 92 | 1.43 | | 122 | 1.82 | | 132 | 2.07 | | 142 | 1.45 |
| 83 | 1.68 | | 93 | 1.49 | | 123 | 1.86 | | 133 | 1.95 | | 143 | 1.74 |
| 84 | 1.48 | | 94 | 1.44 | | 124 | 1.85 | | 134 | 2.16 | | 144 | 1.41 |
| 85 | 1.29 | | 95 | 1.12 | | 125 | 1.84 | | 135 | 1.47 | | | |
| 86 | 1.21 | | 96 | 1.44 | | 126 | 2.12 | | 136 | 1.94 | | | |

The data of Tables 6a/b show that the nucleases according to the invention have increased T_{low} activity compared to the T_{low} activity of SEQ ID NO:1. Improved T_{low} activity especially relies upon substitutions in positions P51, T56, and T77, especially upon substitution of an aliphatic nonpolar amino acid in position P51, or of an aliphatic nonpolar amino acid in position or a negatively charged amino acid in position T56, and in particular upon a substitution selected from the group consisting of P51L, T56P, and T56D, either alone or in combination with each other.

Improved T_{low} activity also relies upon substitutions in position N58 and D86, especially upon substitution of an aliphatic nonpolar amino acid in position N58, or an aliphatic polar non-charged amino acid in position D86, and in particular upon a substitution selected from the group consisting of N58V and D86S, either alone or in combination with each other.

### Example 6: Salt activity profiles of different nucleases

The salt activity profiles of SEQ ID NO: 1, the variants SEQ ID NO:55 and SEQ ID NO:59 and the commercially available Salt Active Nuclease (SAN) (Sigma-Aldrich Cat. No. SRE0015) were compared. SAN is a general, unspecific endonuclease which exhibits its optimum enzymatic activity at high concentrations of salt. The amino acid sequence of SAN is assumed to correspond to SEQ ID NO: 103 of this application, which is the endonuclease I from the psychro-halophilic marine bacterium *Vibrio salmonicida* (synonym: *Aliivibrio salmonicida*) and being disclosed as SEQ ID NO:4 in WO2013121228A1/US9650618B2, or a variant thereof.

The salt activity of the nuclease enzymes was analyzed as described in the Nuclease Activity Assay (Example 2). For each of the enzymes, in particular for SAN, a suitable dilution factor (D) was determined in preparatory tests. For adjusting a certain salt concentration S_{low} (for S_{low} activity) or S_{high} (for S_{high} activity) during the reaction of step 3, the salt concentration of the substrate buffer in step 2 was changed in such way that the following final concentrations S_{low} or S_{high}, respectively, were obtained upon mixture with the enzyme sample or control sample:

Final salt concentrations in reaction: S_{low}: 0 mM NaCl; S_{high-1}: 100 mM NaCl; S_{high-2}: 300 mM NaCl; S_{high-3}: 500 mM NaCl; S_{high-4}: 650 mM NaCl; S_{high-5}: 800 mM NaCl. Enzymatic activity at each salt concentration was measured as described in the Nuclease Activity Assay. The A260 values at S_{low} and the respective corresponds S_{high} correspond to the respective S_{low} or S_{high} activity.

For each enzyme, the concentration at which the maximum S_{high} activity values were detected is defined as 100%, and the enzyme activity values detected at other concentrations are expressed as relative enzymatic activity in comparison to that maximum value as a percentage, resulting in a salt activity profile for each enzyme. The salt activity profiles for each enzyme are listed in Table 5.

**Table 5: Salt activity profile for different nucleases**

| Nuclease SEQ ID NO | NaCl concentration | | | | | |
|---|---|---|---|---|---|---|
| | 0 mM | 100 mM | 300 mM | 500 mM | 650 mM | 800 mM |
| | S_{low} | S_{high-1} | S_{high-2} | S_{high-3} | S_{high-4} | S_{high-5} |
| SEQ ID NO:1 | 95% | 100% | 41% | 7% | 2% | 0% |
| SEQ ID NO: 59 | 54% | 52% | 100% | 66% | 30% | 10% |
| SEQ ID NO: 55 | 48% | 45% | 96% | 100% | 58% | 23% |
| Putative: SEQ ID NO:103 | 13% | 18% | 35% | 69% | 98% | 100% |

The data of Table 5 indicate that the nucleases variants of SEQ ID NO: 1 according to the invention exhibit a salt activity maximum at higher concentrations than the wild-type enzyme of SEQ ID NO: 1 and cover optimum salt concentration ranges between physiological conditions (~100mM) and extreme high salt conditions (800mM and higher).

### Example 7: Expression and analysis of endonucleases from Serratia ficaria (SEQ ID NO: 104) and Serratia odorifera (SEQ ID NO:105) and variants thereof

The gene of the nucleases of *Serratia ficaria* and *Serratia odorifera* with SEQ ID NO:104 and SEQ ID NO:105 are codon-optimized and cloned and expressed in *Bacillus subtilis* as described in Example 1 for SEQ ID NO:1.

Variants of SEQ ID NO:104 and SEQ ID NO:105 are created with substitutions at one or more positions corresponding to the positions P51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A from SEQ ID NO:1 in a sequence alignment established in accordance with the description and are expressed as described in Example 1.

Thermal stability, salt (S_{high}) activity, relative salt activity, cold (T_{low}) activity and relative cold activity of SEQ ID NO:104 and SEQ ID NO:105 and variants thereof are analyzed as described in Examples 2, 3, 4 and 5.

Variants of SEQ ID NO:104 and SEQ ID NO:105 show a higher residual activity than their corresponding wild-type enzymes SEQ ID NO:104 and SEQ ID NO:105, respectively, which means they possess an improved thermal stability compared to the wild-type nucleases.

Furthermore, Variants of SEQ ID NO:104 and SEQ ID NO:105 exhibit a higher relative salt activity and/or S_{high} activity than their corresponding wild-type enzymes SEQ ID NO:104 and SEQ ID NO:105, respectively.

Certain variants of SEQ ID NO:104 and SEQ ID NO:105 exhibit a higher relative cold activity and/or T_{low} activity than their corresponding wild-type enzymes SEQ ID NO:104 and SEQ ID NO:105, respectively.

### Example 8: Calculation of physicochemical characteristics of SEQ ID NO: 1 and variants thereof

Modeling the interaction between SEQ ID NO:1 and DNA: The crystal structure of the Serratia marcescens nuclease (protein data bank accession code: 1g8t) and the crystal structure of the B-DNA Dodecamer (protein data bank accession code: 1D65) were used to model the interaction of the nuclease with DNA. The initial complex nuclease-DNA comprised the DNA located next to the DNA-binding site of the nuclease. In order to obtain a stable DNA-nuclease complex two sequential steps of Molecular Dynamics simulations and an energy minimization were performed. First, the complex was subjected to 6 ns in water with the YASARA2 force field (YASARA structure, version 22.8.22). In the second simulation the complex resulting from the firs simulation was subjected to a 125 ns simulation using the AMBER14 force field in water. For that, the default settings included in the md_run.mcr macro of YASARA were used. The final model was subjected to energy minimization. The binding site of DNA on the surface of the nuclease was determined from this final model as it is seen in Figures 1 to 4.

Solvent Accessibility: The solvent accessible surface area (SASA) per amino acid was calculated from the model by the software Pymol 2.5.1 (Schrödinger, LLC.). The SASA shown in table # correspond to the area of the native amino acid from SEQ ID NO:1 in square Angstrom (Å²). The calculated SASA demonstrates that the amino acid residues A52, S53, G54, T56, N58, P73, A74, G78, N80, A81, N119, Q120, A124, and P195 have solvent accessibility at the surface of the protein, this was also demonstrated for P51 and T77 which SASA is 40,09 Å2 and 111,41 Å2, respectively.

From the final model it was determined that the alpha carbon of the positions P51, A52, S53, G54, T56, N58, P73, A74, T77, G78, N80, A81, N119, Q120, A124, and P195 was located within a distance shorter than 10Å of the doble strand DNA, and as previously mentioned their side chains have solvent accessibility to the surface of the protein.

Nuclease-DNA binding energy calculation: The binding energy of the previously calculated final model in vacuum was obtained by use of the software YASARA structure (version 22.9.24) and the NOVA force field. The YASARA routine to calculate the binding energy of the double strand DNA to the nuclease was executed with the command "BindEnergyObj". As shown in Table 6, the calculated binding energy between Serratia marcescens nuclease and the double strand DNA was 518,1 kJ/mol.

In order to calculate the binding energies between the mutated variants and DNA, the residues in the positions of interest were substituted in the model by using the "SwapRes" command in YASARA. Subsequently, the side chain of the substituted residue was subjected to energy minimization by use of the "Energy minimization" routine of YASARA.

As shown in Table 6, the variants having a single substitutions A52R, S53K, G54R, T56K, T56R, N58R, N58K, P73R, A74R, G78R, N80R, A81K, N119R, Q120R, A124K, and P195R demonstrate a superior binding energy in comparison to SEQ ID NO:1. The double mutation of P51L and T77R; P51L and T77K; P51G and T77K; P51G and T77R; P51L and T77H; P51G and T77H; P51A and T77R; P51V and T77R; P51I and T77R, also demonstrated a superior binding energy in comparison to SEQ ID NO:1. It was also demonstrated that the addition of a third substitution selected from the group of A52R, S53K, G54R, T56K, T56R, N58R, N58K, P73R, A74R, G78R, N80R, A81K, N119R, Q120R, A124H, A124K, A124R, and P195R produces a variant which binding energy is superior to the binding energy of both the same double mutant variant and SEQ ID NO:1. For example the triple mutant having the substitutions P51L, T77R, and A124K (668.6 kJ/mol) has a higher binding energy than the double mutant variant P51L and T77R (578.8 kJ/mol), and SEQ ID NO:1 (518.1 kJ/mol).

**Table 6**

| SE Q ID NO | No of mutations | 1st mutation | 2nd mutation | 3rd /4th mutation | Binding Energy [kJ/mol] | Delta binding energy (Wt-variant) | Delta binding energy (double vs triple mutant) | Shigh | SASA |
|---|---|---|---|---|---|---|---|---|---|
| 1 | 0 | WT | WT | WT | 518.1 | 0 | | | |
| 153 | 2 | P51L | T77R | | 578.8 | 60.7 | | | |
| 154 | 2 | P51L | T77K | | 576.4 | 58.3 | | | |
| 34 | 1 | A124K | | | 605.6 | 87.5 | | 2.24 | 26.28 |
| 57 | 4 | P51L | T77K | A124K; N58R | 741.7 | 223.56 | | 16.45 | |
| 61 | 4 | P51L | T77R | A124K; T56R | 715.6 | 197.5 | | 13.15 | |
| 162 | 3 | P51L | T77R | A124H | 666.9 | 148.8 | 88.1 | | |
| 64 | 3 | P51L | T77R | A124K | 668.6 | 150.5 | 89.8 | 10.29 | |
| 163 | 3 | P51L | T77R | A124R | 675 | 156.9 | 96.2 | | |
| 114 | 1 | Q 120R | | | 628.1 | 110 | | 4.3 | 103.44 |
| 164 | 3 | P51L | T77R | Q 120R | 675.7 | 157.6 | 96.9 | | |
| 113 | 1 | A81K | | | 586.5 | 68.4 | | 1.15 | 130.76 |
| 165 | 3 | P51L | T77R | A81K | 645.2 | 127.1 | 66.4 | | |
| 6 | 1 | S53K | | | 604.5 | 86.4 | | 1.13 | 123.40 |
| 166 | 3 | P51L | T77R | S53K | 662.3 | 144.2 | 83.5 | | |
| 145 | 1 | A52R | | | 577.0 | 58.9 | | | 15.38 |
| 167 | 3 | P51L | T77R | A52R | 634.4 | 116.3 | 55.6 | | |
| 146 | 1 | G54R | | | 611.5 | 93.4 | | | 99.30 |
| 168 | 3 | P51L | T77R | G54R | 668.4 | 150.3 | 89.6 | | |
| 147 | 1 | P73R | | | 580.4 | 62.3 | | | 61.05 |
| 169 | 3 | P51L | T77R | P73R | 638.2 | 120.1 | 59.4 | | |
| 148 | 1 | A74R | | | 568.0 | 49.9 | | 1.64 | 77.27 |
| 170 | 3 | P51L | T77R | A74R | 624.0 | 105.9 | 45.2 | | |
| 149 | 1 | G78R | | | 573.1 | 55 | | | 28.22 |
| 171 | 3 | P51L | T77R | G78R | 630.9 | 112.8 | 52.1 | | |
| 150 | 1 | N80R | | | 609.5 | 91.4 | | | 74.98 |
| 172 | 3 | P51L | T77R | N80R | 667.5 | 149.4 | 88.7 | | |
| 151 | 1 | N119R | | | 611.1 | 93 | | | 13.35 |
| 173 | 3 | P51L | T77R | N119R | 668.9 | 150.8 | 90.1 | | |
| 152 | 1 | P195R | | | 589.2 | 71.1 | | | 93.77 |
| 174 | 3 | P51L | T77R | P195R | 647.2 | 129.1 | 68.4 | | |
| 12 | 1 | T56K | | | 581.2 | 63.1 | | 1.80 | 65.0 |
| 175 | 3 | P51L | T77R | T56K | 638.9 | 120.8 | 60.1 | | |
| 11 | 1 | T56R | | | 580.6 | 62.5 | | 1.83 | 65.01 |
| 176 | 3 | P51L | T77R | T56R | 638.9 | 120.8 | 60.1 | | |
| 14 | 1 | N58R | | | 599.0 | 80.9 | | 2.04 | 102.90 |
| 66 | 3 | P51L | T77R | N58R | 649.2 | 131.1 | 70.4 | 7.67 | |
| 15 | 1 | N58K | | | 586.3 | 68.2 | | 1.15 | 102.9 |
| 177 | 3 | P51L | T77R | N58K | 644.4 | 126.3 | 65.6 | | |
| 178 | 3 | P51L | T77K | N58R | 647.9 | 129.8 | 71.5 | | |
| 179 | 3 | P51L | T77K | A124H | 664.6 | 146.5 | 88.2 | | |
| 180 | 3 | P51L | T77K | A124K | 667.8 | 149.7 | 91.4 | | |
| 155 | 2 | P51G | T77K | | 574.7 | 56.6 | | | |
| 181 | 3 | P51G | T77K | A124K | 663.7 | 145.6 | 89 | | |
| 182 | 3 | P51G | T77K | A124R | 658.9 | 140.8 | 84.2 | | |
| 183 | 3 | P51G | T77K | A124H | 664.3 | 146.2 | 89.6 | | |
| 156 | 2 | P51G | T77R | | 576.8 | 58.7 | | | |
| 184 | 3 | P51G | T77R | A124R | 661.2 | 143.1 | 84.4 | | |
| 185 | 3 | P51G | T77R | A124K | 666 | 147.9 | 89.2 | | |
| 186 | 3 | P51G | T77R | A124H | 664.9 | 146.8 | 88.1 | | |
| 157 | 2 | P51L | T77H | | 578.6 | 60.5 | | | |
| 187 | 3 | P51L | T77H | A124R | 670.6 | 152.5 | 92 | | |
| 188 | 3 | P51L | T77H | A124K | 673.5 | 155.4 | 94.9 | | |
| 189 | 3 | P51L | T77H | A124H | 668.5 | 150.4 | 89.9 | | |
| 158 | 2 | P51G | T77H | | 578.5 | 60.4 | | | |
| 190 | 3 | P51G | T77H | A124R | 670.4 | 152.3 | 670.4 | | |
| 191 | 3 | P51G | T77H | A124K | 673.2 | 155.1 | 673.2 | | |
| 192 | 3 | P51G | T77H | A124H | 668.2 | 150.1 | 668.2 | | |
| 159 | 2 | P51A | T77R | | 576.6 | 58.5 | | | |
| 193 | 3 | P51A | T77R | A124K | 665.6 | 147.5 | 89 | | |
| 160 | 2 | P51V | T77R | | 577.8 | 59.7 | | | |
| 194 | 3 | P51V | T77R | A124K | 666.8 | 148.7 | 89 | | |
| 161 | 2 | P51I | T77R | | 577.1 | 59 | | | |
| 195 | 3 | P51I | T77R | A124K | 666.1 | 148 | 89 | | |

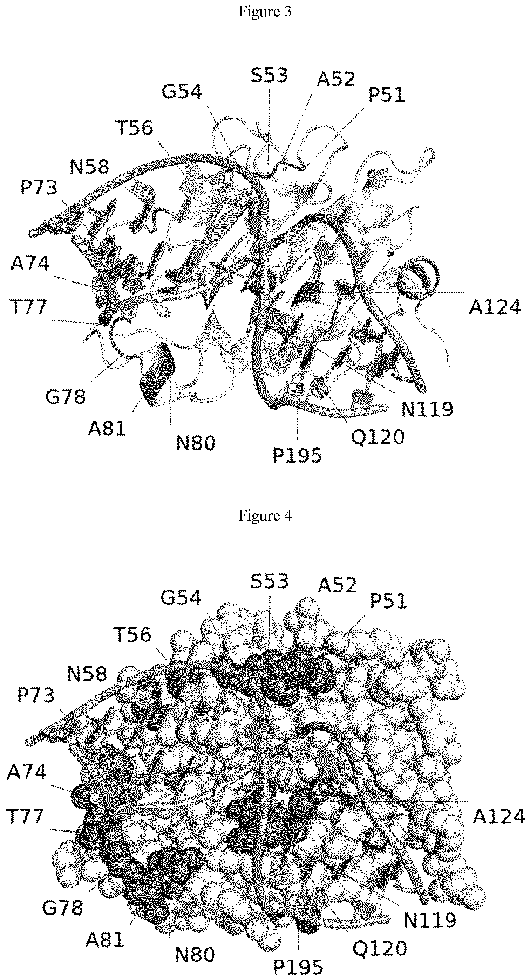

Figures 1 to 4 show a three-dimensional representation of the final model calculated by methods shown in example 8. Figure 1 shows the structure of the model (nuclease only) represented as cartoon, the secondary structures of the nuclease are visible. Figure 2 shows the structure of the model (nuclease only) represented as balls, the highlighted amino acids have solvent accessibility. Figures 3 and 4 show the structure of the final model complexed with the DNA. The amino acids in the binding site of the DNA are identified.

## Claims

1. A nuclease comprising an amino acid sequence with at least 89% identity to SEQ ID NO:1; wherein the amino acid sequence is substituted compared to SEQ ID NO:1 in at least one amino acid position selected from the group consisting of T77R, T77K, A124R and A124K.

2. The nuclease according to claim 1, wherein the substitution is selected from the group consisting of T77K and T77R.

3. The nuclease according to claim 1, wherein the substitution is selected from the group consisting of A 124K, and A124R.

4. The nuclease according to any of the preceding claims, wherein the amino acid sequence is substituted compared to SEQ ID NO:1 in at least two amino acid positions selected from the group consisting of T77K, T77R A 124K, and A124R.

5. The nuclease according to any of the preceding claims, wherein the amino acid sequence is substituted compared to SEQ ID NO:1 in at least two amino acid positions selected from the group consisting of T77K, T77R, A124K, and A124R, and
wherein the amino acid sequence in addition comprises at least one further substitution, wherein the at least one further substitution position is selected from the group consisting ofP51D, P51R, P51G, P51L, S53K, S53A, K55H, K55R, T56S, T56M, T56R, T56K, T56G, T56A, T56V, T56L, T56I, T56F, T56P, T56D, N58K, N58R, N58V, A72K, A72H, A72R, P73K, P73R, A74K, A74H, A74R, T77K, T77R, T77F, T77L, N80K, N80R, N80H, K84G, D86S, S116K, D117A, D117N, D117Y, D117K, D117H, D117R, D117S, Q120R, A124K, A124R, D128G, D128Y, D128F, D128M, D128N, D128S, D128R, D128K, Q129R, K132R, D135A, D135M, D135G, D135N, D135R, D135K, D191P, K196R, G197K, D199N, D199S, D199K, R204Q, and E239A.

6. The nuclease according to any of the preceding claims, wherein the identity of the amino acid sequence of the nuclease with the sequence of SEQ ID NO:1 is at least 90%, or at least 91%, or at least 92%, or at least 93%, or at least 94%, or at least 95%; preferably at least 96%, or at least 97%, or at least 98%, or at least 99%; more preferably at least 99.1%, or at least 99.2%, or at least 99.3%, or at least 99.4, or at least 99.5%; still more preferably at least 99.6%.

7. The nuclease according to any of the preceding claims, wherein the amino acid sequence has at least 90% identity; preferably at least 91%; more preferably at least 92%; still more preferably at least 93%; yet more preferably at least 94%; even more preferably at least 95%; even more preferably at least 96%; most preferably at least 97%, utmost preferably at least 98%, and in particular at least 99%, or 100% to SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, or 195.

8. The nuclease according to any of the preceding claims, which is **characterized by** either
(A) an increased thermal stability in comparison to the wildtype nuclease of SEQ ID NO:1; and/or
(B) an increased relative salt activity in comparison to the relative salt activity of the wildtype nuclease of SEQ ID NO:1; and/or
(C) an increased S_{high} activity in comparison to the S_{high} activity of the wildtype nuclease of SEQ ID NO:1; and/or
(D) an increased relative cold activity in comparison to the relative cold activity of the wildtype nuclease of SEQ ID NO:1; and/or
(E) an increased T_{low} activity in comparison to the T_{low} activity of the wildtype nuclease of SEQ ID NO:1.

9. The nuclease according to any of the preceding claims, which has an increased thermal stability compared to SEQ ID NO:1, wherein said thermal stability is **characterized by** a higher residual activity after incubation for 15 minutes at a temperature T_{stability}.

10. The nuclease according to any of the preceding claims, which has
(i) a higher enzymatic activity at a salt concentration S_{high} (S_{high} activity) than SEQ ID NO:1; and/or
(ii) a higher relative salt activity in comparison to SEQ ID NO:1, wherein said relative salt activity is the ratio of enzymatic activity at the salt concentration S_{high} in comparison to the salt concentration S_{low}.

11. A method of hydrolyzing polynucleotide substrates; preferably being contained in a sample, comprising the steps of
(a) providing a nuclease according to any of the preceding claims;
(b) providing a composition containing a polynucleotide substrate;
(c) contacting the polynucleotide substrate in the composition provided in step (b) with the nuclease provided in step (a) and allowing the nuclease to hydrolyze the polynucleotide substrate thereby obtaining a composition containing cleaved polynucleotide.

12. The method according to claim 11, wherein step (c) is carried out in the presence of salts;
wherein the salt is NaCl or KCl; preferably NaCl; and/or
wherein step (c) is carried out at a concentration of salt from 0.00M to 1.00M, or from 0.01M to 1.00M; preferably from 0.15M to 0.85M; more preferably from 0.20M to 0.70M; still more preferably from 0.25M to 0.60M; yet more preferably from 0.30M to 0.60M; even more preferably from 0.35M to 0.60M; most preferably from 0.40M to 0.60M, and in particular 0.50M.

13. The method according to any of claims 12 to 13 comprising the additional step of (d) inactivating the nuclease after step (c) thereby obtaining a composition containing cleaved polynucleotides and an inactivated nuclease.

14. Use of a nuclease according to any of claims 1 to 10 for hydrolyzing polynucleotide substrates; preferably being contained in a composition.

15. A kit for cleaving a polynucleotide, the kit comprising
- the nuclease according to any of claims 1 to 10;
- a buffer for hydrolyzing polynucleotide substrates, and
- optionally, an agent and/or device for inactivation and/or removal of the nuclease;
preferably wherein the kit is for use in the method according to any of claims 11 to 13 or in the use according to claim 14.
